(19) Europäisches Patentamt
European Patent Office
Office européen des brevets

(11) EP 4 692 079 A1

(12) EUROPEAN PATENT APPLICATION
published in accordance with Art. 153(4) EPC

(43) Date of publication:
11.02.2026 Bulletin 2026/07

(21) Application number: 24778048.9

(22) Date of filing: 27.03.2024

(51) International Patent Classification (IPC):
C07D 405/14 (2006.01) C07D 209/44 (2006.01)
C07D 405/06 (2006.01) C07D 417/14 (2006.01)
A61P 35/00 (2006.01) A61K 31/404 (2006.01)

(52) Cooperative Patent Classification (CPC):
A61K 31/404; A61P 35/00; C07D 209/44;
C07D 405/06; C07D 405/14; C07D 417/14

(86) International application number:
PCT/CN2024/083998

(87) International publication number:
WO 2024/199262 (03.10.2024 Gazette 2024/40)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.03.2023 CN 202310320916
20.04.2023 CN 202310428094
07.06.2023 CN 202310667272
06.07.2023 CN 202310821299
04.08.2023 CN 202310979027
22.08.2023 CN 202311059482
20.09.2023 CN 202311216619
07.12.2023 CN 202311669033

(71) Applicant: Xizang Haisco Pharmaceutical Co., Ltd.
Lhoka, Tibet 856099 (CN)

(72) Inventors:
- LI, Yao
Lhoka, Tibet 856099 (CN)
- SHI, Zongjun
Lhoka, Tibet 856099 (CN)
- ZHANG, Guobiao
Lhoka, Tibet 856099 (CN)
- PEI, Yunpeng
Lhoka, Tibet 856099 (CN)
- SHU, Tianbo
Lhoka, Tibet 856099 (CN)
- WANG, Yaoling
Lhoka, Tibet 856099 (CN)
- WANG, Pengcheng
Lhoka, Tibet 856099 (CN)
- ZHONG, Qijie
Lhoka, Tibet 856099 (CN)
- LIU, Xin
Lhoka, Tibet 856099 (CN)
- SHI, Shaohui
Lhoka, Tibet 856099 (CN)
- TANG, Pingming
Lhoka, Tibet 856099 (CN)
- ZHANG, Chen
Lhoka, Tibet 856099 (CN)
- YAN, Pangke
Lhoka, Tibet 856099 (CN)

(74) Representative: AWA Denmark A/S
Strandgade 56
1401 Copenhagen K (DK)

# (54) CYP11A1 INHIBITOR AND USE THEREOF

(57) A compound as represented by formula (I), or a stereoisomer, tautomer, deuterated compound, solvate, co-crystal or pharmaceutically acceptable salt of the compound and a pharmaceutical composition thereof, and the use thereof in the preparation of a drug for treating/preventing CYP11A1-mediated diseases, wherein each group in formula (I) is as defined in the description.

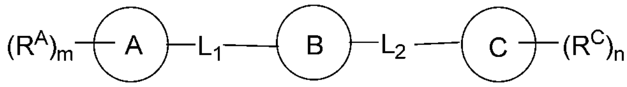

(I)

## Description

The present application claims to be entitled to the right of priority for

the earlier application with the patent application number of 202310320916.4, entitled "CYP11A1 Inhibitor and Use Thereof" and submitted to the China National Intellectual Property Administration on 29 March 2023;
the earlier application with the patent application number of 202310428094.1, entitled "CYP11A1 Inhibitor and Use Thereof" and submitted to the China National Intellectual Property Administration on 20 April 2023;
the earlier application with the patent application number of 202310667272.6, entitled "CYP11A1 Inhibitor and Use Thereof" and submitted to the China National Intellectual Property Administration on 7 June 2023;
the earlier application with the patent application number of 202310821299.6, entitled "CYP11A1 Inhibitor and Use Thereof" and submitted to the China National Intellectual Property Administration on 6 July 2023;
the earlier application with the patent application number of 202310979027.9, entitled "CYP11A1 Inhibitor and Use Thereof" and submitted to the China National Intellectual Property Administration on 4 August 2023;
the earlier application with the patent application number of 202311059482.3, entitled "CYP11A1 Inhibitor and Use Thereof" and submitted to the China National Intellectual Property Administration on 22 August 2023;
the earlier application with the patent application number of 202311216619.1, entitled "CYP11A1 Inhibitor and Use Thereof" and submitted to the China National Intellectual Property Administration on 20 September 2023;
the earlier application with the patent application number of 202311669033.0, entitled "CYP11A1 Inhibitor and Use Thereof" and submitted to the China National Intellectual Property Administration on 7 December 2023;
the contents of which are incorporated herein by reference in their entireties.

### Technical Field

The present invention belongs to the field of medicine and in particular relates to a small molecule compound and a stereoisomer, tautomer, deuterated compound, solvate, co-crystal or pharmaceutically acceptable salt thereof, which have a selective inhibitory activity against CYP11A1, and the use thereof in the preparation of a drug for treating a related disease.

### Background Art

Cytochrome p450 monooxygenase 11a1 (CYP11A1), also termed cholesterol side-chain cleavage enzyme, primarily participates in drug metabolism-related catalytic reactions, as well as the synthesis of cholesterol, steroids, and other lipids. CYP11A1 protein is localized within the inner mitochondrial membrane and catalyses the conversion of cholesterol to pregnenolone, representing the first and rate-limiting step in the synthesis of steroid hormones. This reaction occurs in the mitochondria of the adrenal cortex, and is catalysed by cytochrome CYP11A1 (also referred to as P450scc), which functions in conjunction with adrenodoxin (Adx) and adrenodoxin reductase (AdR). CYP11A1, Adx, and AdR belong to the cholesterol hydroxylase/lyase system (CH/L system), which catalyses the initial step of steroid synthesis in mammals - the production of pregnenolone from cholesterol. Pregnenolone serves as a critical precursor for steroid hormones. The reaction process involves three consecutive monooxygenation reactions: production of 22R-hydroxycholesterol (22HC), production of 20R,22R-dihydroxycholesterol, and cleavage of the C20-C22 bond. Each monooxygenation reaction requires two electrons and one molecular oxygen. The electrons are supplied by NADPH and transferred to P450scc via NADPH-AdR and Adx. Adx forms a complex with P450scc and can function as a mobile electron shuttle.

CYP11A1 is primarily expressed in the placenta in response to the synthesis of placenta-derived hormones, such as progesterone and testosterone. It is also highly expressed in the adrenal glands and testes, but is almost absent in other tissues. By inhibiting CYP11A1 (a key enzyme upstream of CYP17a1 in steroid biosynthesis), complete blockade of the entire steroid biosynthesis pathway can be achieved. Therefore, CYP11A1 inhibitors hold great potential for treating steroid hormone-dependent cancers such as prostate cancer, even in advanced disease stages, especially in patients exhibiting hormone-refractory status. It has been recently confirmed that compounds having inhibitory effects on CYP11A1 significantly suppress tumour growth *in vivo* in mouse castration-resistant prostate cancer (CRPC) xenograft models.

There is an urgent need to discover CYP11A1 inhibitors with good activity, high safety and minor side effects, which have a good clinical development prospect and can be used for treating cancers or other proliferative diseases or conditions.

Summary of the Invention

The present invention provides a small molecule compound with CYP11A1 inhibitory activity, and a stereoisomer, tautomer, deuterated compound, solvate, co-crystal or pharmaceutically acceptable salt thereof, wherein the compound is as represented by formula (I), (II), (I-a), (I-b), (I-c), (I-d), or (I-d1),

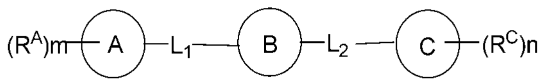 (I) 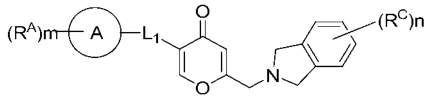 (II)

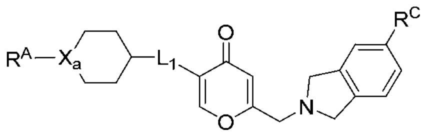 (I-a) 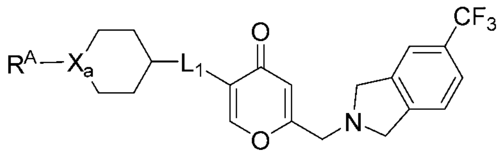 (I-b)

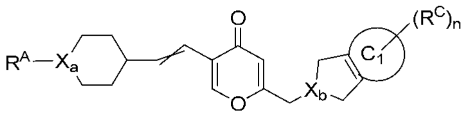 (I-c) 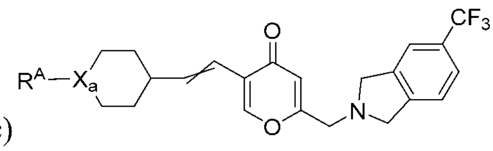 (I-d)

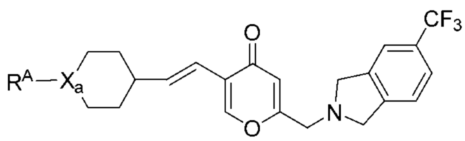 (I-d1)

wherein Xa is selected from CH or N; in some embodiments, Xa is CH; in some embodiments, Xa is N;

$X_b$ is selected from CH or N; in some embodiments, $X_b$ is CH; in some embodiments, $X_b$ is N;

ring $C_1$ is selected from 5- to 6-membered heteroaryl, phenyl or benzo 5- to 6-membered cycloalkyl; in some embodiments, ring $C_1$ is selected from 5- to 6-membered heteroaryl, or phenyl; in some embodiments, ring $C_1$ is selected from 5-membered heteroaryl, or phenyl; in some embodiments, ring $C_1$ is phenyl;

ring A is selected from 4- to 8-membered cycloalkyl, 4- to 7-membered monocyclic heterocycloalkyl, 6- to 12-membered bicyclic heterocycloalkyl, or 8- to 14-membered tricyclic heterocycloalkyl;

in some embodiments, ring A is selected from 5- to 8-membered cycloalkyl, 4-to 7-membered monocyclic heterocycloalkyl, 6- to 12-membered bicyclic heterocycloalkyl, or 8- to 14-membered tricyclic heterocycloalkyl;

in some embodiments, ring A is selected from 4- to 8-membered cycloalkyl, 4-to 7-membered monocyclic heterocycloalkyl, 5- to 6-membered heterocycloalkyl fused 5- to 6-membered heterocycloalkyl, 4- to 6-membered heterocycloalkyl spiro 5- to 6-membered heterocycloalkyl, 5- to 6-membered heterocycloalkyl spiro 4- to 6-memberedcycloalkyl, 5- to 6-membered heterocycloalkyl spiro 5- to 6-membered cycloalkyl, 5- to 6-membered heterocycloalkyl spiro 3- to 6-membered cycloalkyl, 10- to 14-membered partially unsaturated tricyclic heterocycloalkyl, or 7- to 8-membered bicyclic bridged heterocyclyl;

in some embodiments, ring A is selected from 5- to 8-membered cycloalkyl, 4-to 7-membered monocyclic heterocycloalkyl, 5- to 6-membered heterocycloalkyl fused 5- to 6-membered heterocycloalkyl, 4- to 6-membered heterocycloalkyl spiro 5- to 6-membered heterocycloalkyl, 5- to 6-membered heterocycloalkyl spiro 4- to 6-memberedcycloalkyl, 5- to 6-membered heterocycloalkyl spiro 5- to 6-membered cycloalkyl, 5- to 6-membered heterocycloalkyl fused 3- to 6-membered cycloalkyl, 10- to 14-membered partially unsaturated tricyclic heterocycloalkyl, or 7- to 8-membered bicyclic bridged heterocyclyl;

in some embodiments, ring A is selected from 5- to 8-membered cycloalkyl, 4-to 7-membered monocyclic heterocycloalkyl, 5- to 6-membered heterocycloalkyl fused 5- to 6-membered heterocycloalkyl, 4- to 6-membered heterocycloalkyl spiro 5- to 6-membered heterocycloalkyl, 5- to 6-membered heterocycloalkyl spiro 5- to 6-membered cycloalkyl, 5- to 6-membered heterocycloalkyl fused 5- to 6-membered cycloalkyl, or 10- to 14-membered partially unsaturated tricyclic heterocycloalkyl;

in some embodiments, ring A is selected from 5- to 8-membered cycloalkyl, 4-to 7-membered monocyclic heterocycloalkyl, 5- to 6-membered heterocycloalkyl fused 5- to 6-membered heterocycloalkyl, 4- to 6-membered heterocycloalkyl spiro 5- to 6-membered heterocycloalkyl, 5- to 6-membered heterocycloalkyl spiro 4- to 6-memberedcycloalkyl, 5- to 6-membered heterocycloalkyl spiro 5- to 6-membered cycloalkyl, 5- to 6-membered heterocycloalkyl fused 5- to 6-membered cycloalkyl, or 10- to 14-membered partially unsaturated tricyclic heterocycloalkyl;

in some embodiments, ring A is selected from 4- to 6-membered cycloalkyl, 4-to 7-membered monocyclic heterocycloalkyl, 5-membered heterocycloalkyl fused 5-membered heterocycloalkyl, 5-membered heterocycloalkyl fused 6-membered heterocycloalkyl, 5-membered heterocycloalkyl spiro 5-membered heterocycloalkyl, 5-membered heterocycloalkyl spiro 6-membered heterocycloalkyl, 4-membered heterocycloalkyl spiro 6-membered heterocycloalkyl, 4-membered cycloalkyl spiro 6-membered heterocycloalkyl, 5-membered cycloalkyl spiro 6-membered heterocycloalkyl, 5-membered heterocycloalkyl fused 5-membered cycloalkyl, 6-membered heterocycloalkyl fused 5-membered cycloalkyl, or 10- to 14-membered partially unsaturated tricyclic heterocycloalkyl;

in some embodiments, ring A is selected from 5- to 6-membered cycloalkyl, 4-to 7-membered monocyclic heterocycloalkyl, 5-membered heterocycloalkyl fused 5-membered heterocycloalkyl, 5-membered heterocycloalkyl fused 6-membered heterocycloalkyl, 5-membered heterocycloalkyl spiro 5-membered heterocycloalkyl, 5-membered heterocycloalkyl spiro 6-membered heterocycloalkyl, 4-membered heterocycloalkyl spiro 6-membered heterocycloalkyl, 4-membered cycloalkyl spiro 6-membered heterocycloalkyl, 5-membered cycloalkyl spiro 6-membered heterocycloalkyl, 5-membered heterocycloalkyl fused 5-membered cycloalkyl, 6-membered heterocycloalkyl fused 5-membered cycloalkyl, or 10- to 14-membered partially unsaturated tricyclic heterocycloalkyl;

in some embodiments, ring A is selected from 5- to 6-membered cycloalkyl, 4-to 7-membered monocyclic heterocycloalkyl, 5-membered heterocycloalkyl fused 5-membered heterocycloalkyl, 5-membered heterocycloalkyl fused 6-membered heterocycloalkyl, 5-membered heterocycloalkyl spiro 5-membered heterocycloalkyl, 5-membered heterocycloalkyl spiro 6-membered heterocycloalkyl, 4-membered heterocycloalkyl spiro 6-membered heterocycloalkyl, 5-membered heterocycloalkyl fused 5-membered cycloalkyl, or 6-membered heterocycloalkyl fused 5-membered cycloalkyl;

in some embodiments, ring A has one of the following structures:

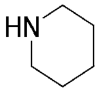,

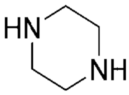, 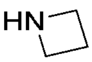, 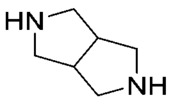, 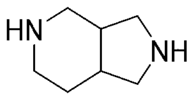, 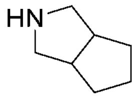, 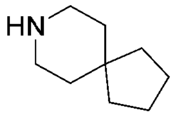,

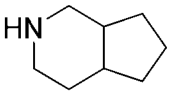, 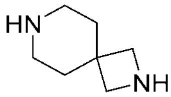, 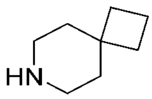, 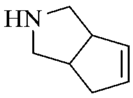, 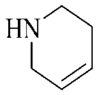, 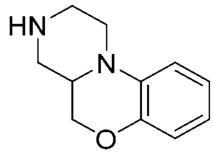,

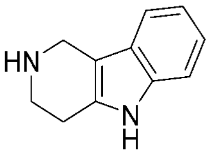, 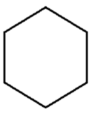, 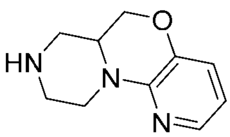, 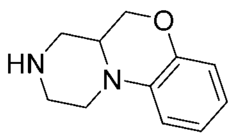, 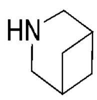, 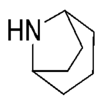,

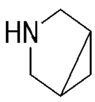, 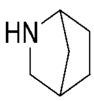, or 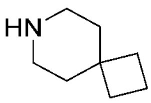,

or is selected from

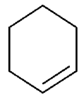;

in some embodiments, ring A has one of the following structures:

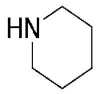,

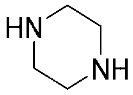, 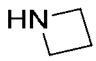, 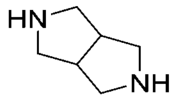, 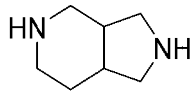, 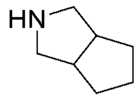, 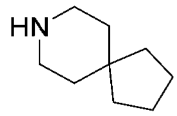,

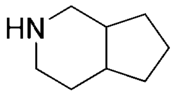, 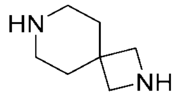, 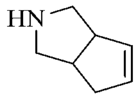, 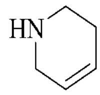, 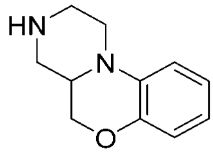,

or

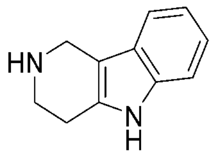,

or is selected from

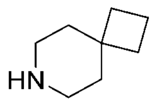,

or is selected from

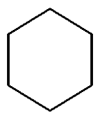,

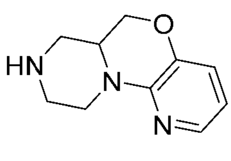, 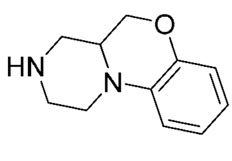, 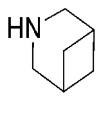, 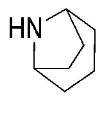, 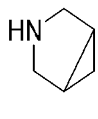, or 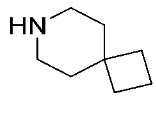,

or is selected from

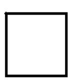, 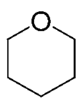, 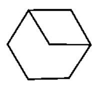, 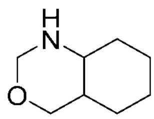, 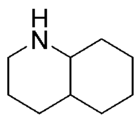, 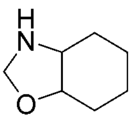,

or

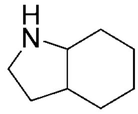;

ring B is

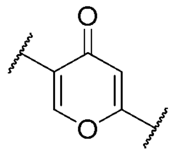,

and ring B is connected to $L_1$ on the left side;

ring C is selected from phenyl, 5- to 6-membered heteroaryl, 6- to 12-membered bicyclic carbocyclyl, 6- to 12-membered bicyclic heterocycloalkyl, or 8-to 14-membered tricyclic heterocycloalkyl, and ring C is not

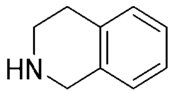,

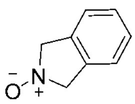, 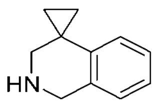, 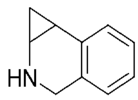 and 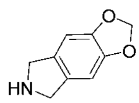;

in some embodiments, ring C is selected from phenyl, 5-membered heteroaryl, 6-membered heteroaryl, 5- to 7-membered heterocycloalkyl fused phenyl, 5- to 6-membered carbocyclyl fused phenyl, 5- to 6-membered heterocycloalkyl fused 5- to 6-membered heteroaryl, 5- to 6-membered heterocycloalkyl fused 3- to 6-membered cycloalkyl, or 9- to 12-membered tricyclic heterocycloalkyl, and ring C is not

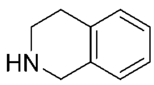, 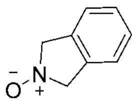, 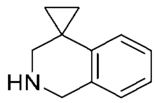, 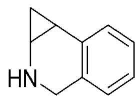 and 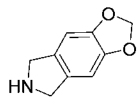;

in some embodiments, ring C is selected from 5- to 7-membered heterocycloalkyl fused phenyl, 5- to 6-membered heterocycloalkyl fused 5- to 6-membered heteroaryl, 5- to 6-membered heterocycloalkyl fused 3- to 6-membered cycloalkyl, or 9- to 12-membered tricyclic heterocycloalkyl, and ring C is not

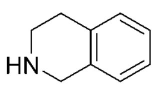, 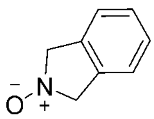, 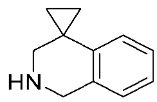, 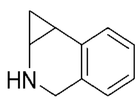 and 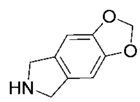;

in some embodiments, ring C is selected from 5- to 7-membered heterocycloalkyl fused phenyl, 5- to 6-membered heterocycloalkyl fused 5- to 6-membered heteroaryl, or 5- to 6-membered heterocycloalkyl fused 3- to 6-membered cycloalkyl, and ring C is not

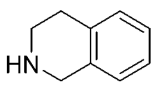, 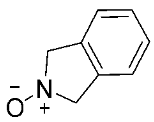, 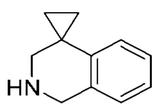, 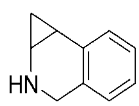

and

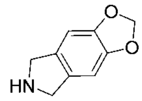 ;

in some embodiments, ring C has one of the following structures:

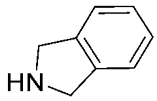 ,

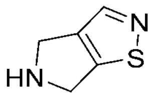 , 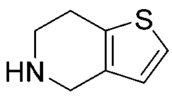 , 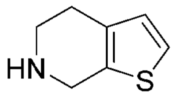 , 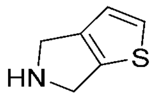 , 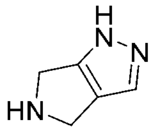 , 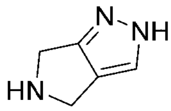 ,

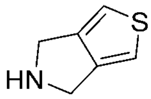 , 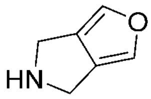 , 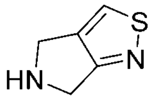 , 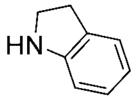 , 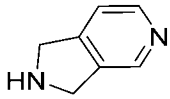 , 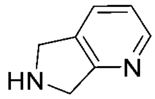 ,

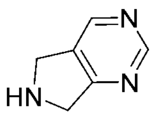 , 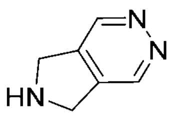 , 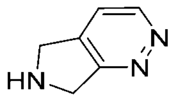 , 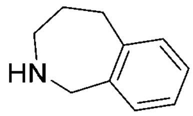 , 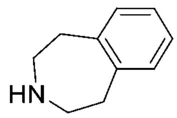 ,

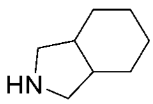 , 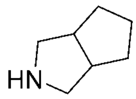 , 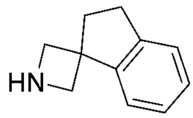 , 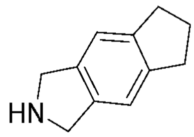 , or 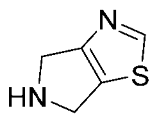 ,

or is selected from

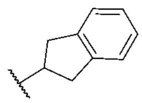 ;

in some embodiments, ring C has one of the following structures:

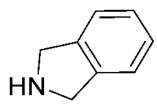 ,

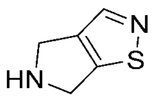 , 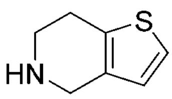 , 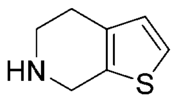 , 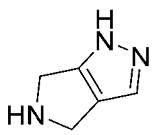 , 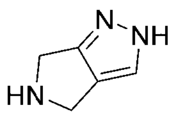 , 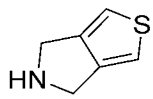 ,

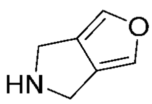 , 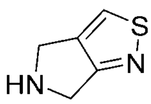 , 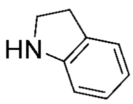 , 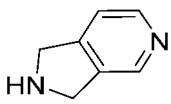 , 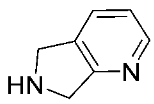 , 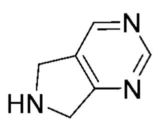 ,

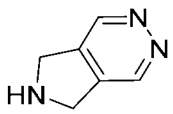 , 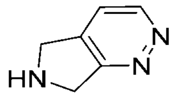 , 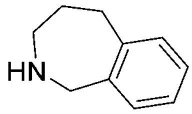 , 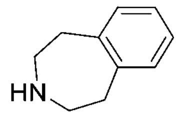 , 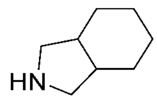 ,

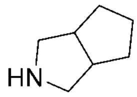 , or 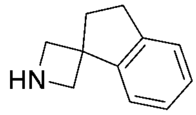 ,

or is selected from

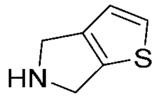 ;

$L_1$ is $W_1$-$R^{La}$-$W_2$, and $L_1$ is connected to A on the left side;

$L_2$ is $W_3$-$R^{Lb}$-$W_4$, and $L_2$ is connected to B on the left side, and $L_1$ and $L_2$ are not both a bond;

$R^{La}$ and $R^{Lb}$ are each independently selected from a bond, $C_{1-4}$ alkylene, $C_{2-4}$ alkenylene, or $C_{2-4}$ alkynylene, wherein the alkylene and alkenylene are optionally further substituted with 1-4 $R^{L1}$;

in some embodiments, $R^{La}$ and $R^{Lb}$ are each independently selected from a bond, $C_{1-4}$ alkylene, or $C_{2-4}$ alkenylene, wherein the alkylene and alkenylene are optionally further substituted with 1-4 $R^{L1}$;

in some embodiments, $R^{La}$ and $R^{Lb}$ are each independently selected from a bond, $C_{1-2}$ alkylene, or $C_{2-4}$ alkenylene, wherein the alkylene and alkenylene are optionally further substituted with 1-4 $R^{L1}$;

in some embodiments, $R^{La}$ and $R^{Lb}$ are each independently selected from a bond, $C_{1-2}$ alkylene, or $C_{2-4}$ alkenylene, wherein the alkylene and alkenylene are optionally further substituted with 1-4 $R^{L1}$;

in some embodiments, $R^{La}$ and $R^{Lb}$ are each independently selected from a bond, $-CH_2-$, $-CH_2CH_2-$, -CH=CH-, $-CH_2-CH=CH-$, $-CH_2CH_2-CH=CH-$, $-CH(CH_3)-CH=CH-$, or $-CH_2-CH=CH-CH_2-$, or is selected from $-C(CH_3)=CH-$, or-$CH=C(CH_3)-$, wherein the $CH_3$, $CH_2$, and CH are optionally further substituted with 1-4 $R^{L1}$;

each $R^{L1}$ is independently selected from halogen, =O, $C_{1-4}$ alkyl, $C_{2-4}$ alkenyl, $C_{1-4}$ alkoxy, or 3- to 6-membered cycloalkyl, wherein the alkyl, alkenyl, alkoxy, and cycloalkyl are optionally further substituted with 1-4 substituents selected from halogen, CN, OH and $NH_2$, or two $R^{L1}$ on the same carbon atom or two adjacent carbon atoms, together with the atom to which they are connected, form 3- to 8-membered cycloalkyl, 5- to 10-membered heterocycloalkyl, or 5- to 6-membered heteroaryl;

in some embodiments, each $R^{L1}$ is independently selected from halogen, $C_{1-4}$ alkyl, $C_{2-4}$ alkenyl, $C_{1-4}$ alkoxy, or 3- to 6-membered cycloalkyl, wherein the alkyl, alkenyl, alkoxy, and cycloalkyl are optionally further substituted with 1-4 substituents selected from halogen, CN, OH and $NH_2$, or two $R^{L1}$ on the same carbon atom or two adjacent carbon atoms, together with the atom to which they are connected, form 3- to 8-membered cycloalkyl, 5- to 10-membered heterocycloalkyl, or 5- to 6-membered heteroaryl;

in some embodiments, each $R^{L1}$ is independently selected from halogen, =O, $C_{1-4}$ alkyl, $C_{2-4}$ alkenyl, $C_{1-4}$ alkoxy, or 3- to 6-membered cycloalkyl, wherein the alkyl, alkoxy, and cycloalkyl are optionally further substituted with 1-4 substituents selected from halogen, CN, OH and $NH_2$, or two $R^{L1}$ on the same carbon atom or two adjacent carbon atoms, together with the atom to which they are connected, form 3- to 8-membered cycloalkyl, 5- to 10-membered heterocycloalkyl, or 5- to 6-membered heteroaryl;

in some embodiments, each $R^{L1}$ is independently selected from halogen, =O, $C_{1-4}$ alkyl, $C_{2-4}$ alkenyl, $C_{1-4}$ alkoxy, or 3- to 6-membered cycloalkyl, wherein the alkyl, alkoxy, and cycloalkyl are optionally further substituted with 1-4 substituents selected from halogen, CN, OH and $NH_2$, or two adjacent $R^{L1}$, together with the atom to which they are connected, form 3- to 8-membered cycloalkyl, 5- to 10-membered heterocycloalkyl, or 5- to 6-membered heteroaryl;

in some embodiments, each $R^{L1}$ is independently selected from halogen, =O, $C_{1-2}$ alkyl, $C_{2-4}$ alkenyl, $C_{1-2}$ alkoxy, or 3- to 6-membered cycloalkyl, wherein the alkyl, alkoxy, and cycloalkyl are optionally further substituted with 1-4 substituents selected from halogen, CN, OH and $NH_2$, or two $R^{L1}$ on the same carbon atom or two adjacent carbon atoms, together with the atom to which they are connected, form 3- to 6-membered cycloalkyl;

in some embodiments, each $R^{L1}$ is independently selected from halogen, $C_{1-2}$ alkyl, $C_{2-4}$ alkenyl, $C_{1-2}$ alkoxy, or 3- to 6-membered cycloalkyl, wherein the alkyl, alkoxy, and cycloalkyl are optionally further substituted with 1-4 substituents selected from halogen, CN, OH and $NH_2$, or two $R^{L1}$ on the same carbon atom or two adjacent carbon atoms, together with the atom to which they are connected, form 3- to 6-membered cycloalkyl;

in some embodiments, each $R^{L1}$ is independently selected from halogen, =O, $C_{1-2}$ alkyl, $C_{2-4}$ alkenyl, $C_{1-2}$ alkoxy, or 3- to 6-membered cycloalkyl, wherein the alkyl, alkoxy, and cycloalkyl are optionally further substituted with 1-4 substituents selected from halogen, CN, OH and $NH_2$, or two adjacent $R^{L1}$, together with the atom to which they are connected, form 3- to 6-membered cycloalkyl;

in some embodiments, each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl, or two $R^{L1}$ on the same carbon atom or two adjacent carbon atoms, together with the atom to which they are connected, form 3- to 6-membered cycloalkyl;

in some embodiments, each $R^{L1}$ is independently selected from halogen, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl, or two $R^{L1}$ on the same carbon atom or two adjacent carbon atoms, together with the atom to which they are connected, form 3- to 6-membered cycloalkyl;

in some embodiments, each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl, or two $R^{L1}$ on the same carbon atom or two adjacent carbon atoms, together with the atom to which they are connected, form cyclopropyl, cyclobutyl, cyclopentyl, or cyclohexyl;

in some embodiments, each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl, or two adjacent $R^{L1}$, together with the atom to which they are connected, form cyclopropyl, cyclobutyl, cyclopentyl, or cyclohexyl;

in some embodiments, each $R^{L1}$ is independently selected from F, Cl, Br, =O, methyl, ethyl, $-CF_3$, $-CHF_2$, $-CH_2F$, vinyl, propenyl, methoxy, ethoxy, $-OCF_3$,$-OCHF_2$, $-OCH_2F$, cyclopropyl, or cyclobutyl, or two adjacent $R^{L1}$, together with the atom to which they are connected, form cyclopropyl, cyclobutyl, cyclopentyl, or cyclohexyl;

$W_1$, $W_2$, $W_3$, and $W_4$ are each independently selected from a bond, -O-, -S-,$-NR^{W1}$-, -Se-, or -C(O)-;

in some embodiments, $W_1$, $W_2$, $W_3$, and $W_4$ are each independently selected from a bond, -O-, -S-, $-NR^{W1}$-, or -Se-;

in some embodiments, $W_1$, $W_2$, $W_3$, and $W_4$ are each independently selected from a bond, -O-, -S-, or $-NR^{W1}$-;

in some embodiments, $W_1$, $W_2$, $W_3$, and $W_4$ are each independently selected from a bond, -O-, or $-NR^{W1}$-;

in some embodiments, $W_1$, $W_2$, $W_3$, and $W_4$ are each independently selected from a bond or -O-;

$R^{W1}$ is selected from H, halogen, or $C_{1-4}$ alkyl;

in some embodiments, $R^{W1}$ is selected from H, halogen, or $C_{1-2}$ alkyl;

in some embodiments, $R^{W1}$ is selected from H, F, Cl, Br, methyl, or ethyl;

in some embodiments, $L_1$ is selected from a bond, $-(C_{1-2}$ alkylene)-O-, $C_{1-2}$ alkylene, $-O-(C_{1-2}$ alkylene)-, $C_{2-4}$ alkenylene, $-C(=O)-N(C_{1-2}$ alkylene)-, -O-,-C(=O)-O-, or $C_{2-4}$ alkynylene, wherein the alkylene and alkenylene are optionally further substituted with 1-3 $R^{L1}$;

in some embodiments, $L_1$ is selected from a bond, $-(C_{1-2}$ alkylene)-O-, $C_{1-2}$ alkylene, $-O-(C_{1-2}$ alkylene)-, $C_{2-4}$ alkenylene, $-C(=O)-N(C_{1-2}$ alkylene)-, -O-, or-C(=O)-O-, wherein the alkylene and alkenylene are optionally substituted with 1-3 $R^{L1}$;

in some embodiments, $L_1$ is selected from a bond, $-(C_{1-2}$ alkylene)-O-, $-(C_{1-2}$ alkylene)-Se-, $C_{1-2}$ alkylene, $-O-(C_{1-2}$ alkylene)-, $C_{2-4}$ alkenylene, -O-, or $C_{2-4}$ alkynylene, wherein the alkylene and alkenylene are optionally further substituted with 1-3 $R^{L1}$;

in some embodiments, $L_1$ is selected from a bond, $-(C_{1-2}$ alkylene)-O-, $-(C_{1-2}$ alkylene)-Se-, $C_{1-2}$ alkylene, $-O-(C_{1-2}$ alkylene)-, $C_{2-4}$ alkenylene, or -O-, wherein the alkylene and alkenylene are optionally further substituted with 1-3 $R^{L1}$;

in some embodiments, $L_1$ is selected from a bond, $-CH_2O-$, $-CH_2CH_2O-$, $-CH_2-$, $-CH_2CH_2-$, $-OCH_2-$, $-OCH_2CH_2-$, -CH=CH-, $-CH_2-CH=CH-$, $-CH_2CH_2-CH=CH-$,$-CH(CH_3)-CH=CH-$, $-CH_2-CH=CH-CH_2-$, $-C(=O)-N(CH_3)-$, $-C(=O)-N(CH_2CH_3)-$,-O-, or -C(=O)-O-, or is selected from $-C(CH_3)=CH-$, or $-CH=C(CH_3)-$, wherein the $CH_3$, $CH_2$, and CH are optionally further substituted with 1-3 $R^{L1}$;

in some embodiments, $L_1$ is selected from a bond, $-CH_2-O-$, $-CH_2-Se-$,-CH=CH-, -CF=CH-, $-CH_2-$, $-CH_2CH_2-$,

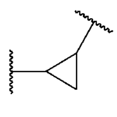
,

-O-, $-C(CH_3)=CH-$,$-CH=C(CH_3)-$, or

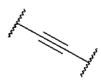
;

in some embodiments, $L_1$ is selected from a bond, $-CH_2-O-$, $-CH_2-Se-$, - CH=CH-, -CF=CH-, $-CH_2-$, $-CH_2CH_2-$,

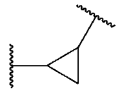,

or -O-;

in some embodiments, $L_1$ is selected from a bond, $-CH_2-O-$, -CH=CH-, $-CH_2-$, $-CH_2CH_2-$,

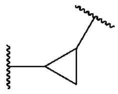,

or -O-;

in some embodiments, $L_1$ is selected from a bond, $-CH_2-O-$, -CH=CH-, $-CH_2-$, or

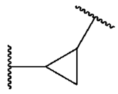;

in some embodiments, $L_1$ is $-CH_2O-$, -CH=CH-;

in some embodiments, $L_1$ is -CH=CH-;

$L_2$ is selected from a bond, $C_{1-2}$ alkylene, -C(=O)-, -NH-, or -O-, wherein the alkylene is optionally further substituted with 1-3 $R^{L1}$;

in some embodiments, $L_2$ is selected from a bond, $C_{1-2}$ alkylene, -NH-, or -O-, wherein the alkylene is optionally further substituted with 1-3 $R^{L1}$;

in some embodiments, $L_2$ is selected from a bond, $-CH_2-$, $-CH_2CH_2-$, -C(=O)-, -NH-, or -O-, wherein the $CH_3$ and $CH_2$ are optionally further substituted with 1-3 $R^{L1}$.

in some embodiments, $L_2$ is $-CH_2-$;

each $R^A$ is independently selected from halogen, =O, CN, COOH, $C_{1-6}$ alkyl, $C_{2-6}$ alkenyl, $C_{2-6}$ alkynyl, -O-$C_{1-4}$ alkyl, $-S(O)_2-C_{1-4}$ alkyl, $-S(O)_2-(CH_2)_{1-3}-R^a$,-P(O)-(C1-4 alkyl)2, -C(O)-O-(CH2)$_p$-$R^a$, $-(CH_2)_{1-2}-C(O)-R^a$, $-C(O)-(CH_2)_{1-2}-R^a$,-C(O)-O-halo $C_{1-4}$ alkyl, -C(O)-(6- to 9-membered bicyclic heterocycloalkyl), -NH-C(O)-$(CH_2)_p$-$R^a$, -NH-$R^a$, $-N(CH_3)-C(O)-(CH_2)_p-R^a$, $-NH-SO_2-(CH_2)_{1-2}-R^a$, -NH-C(O)-O-$R^a$, -C(O)-$(CH_2)_p$-(4- to 6-membered monocyclic heterocycloalkyl), -C(O)-$(CH_2)_p$-(4- to 6-membered monocyclic cycloalkyl), -NH-C(O)-NH-$R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), $-C(O)-NH-S(O)_2-C_{1-4}$ alkyl, -NH-C(O)deuterated $C_{1-2}$ alkyl, -NH-C(O)-NH-$C_{1-2}$ alkyl or -NH-C(O)-$C_{1-2}$ alkyl, wherein the alkyl, alkenyl, alkynyl, cycloalkyl, and heterocycloalkyl are optionally further substituted with 1-4 groups selected from halogen, OH, $NH_2$, CN, $-S(O)_2-CH_3$, -O-halo $C_{1-4}$ alkyl, or $-NH-S(O)_2-CH_3$;

in some embodiments, each $R^A$ is independently selected from halogen, =O, CN, COOH, $C_{1-4}$ alkyl, $C_{2-4}$ alkenyl, $C_{2-4}$ alkynyl, $-OC_{1-4}$ alkyl, $-S(O)_2-C_{1-4}$ alkyl,$-S(O)_2-(CH_2)_{1-3}-R^a$, -P(O)-($C_{1-4}$ alkyl)2, $-C(O)-O-(CH_2)_p-R^a$, -(CH2)$_{1-2}$-C(O)-$R^a$,$-C(O)-(CH_2)_{1-2}-R^a$, -NH-$R^a$, -C(O)-O-halo $C_{1-4}$ alkyl, -C(O)-(6- to 9-membered bicyclic heterocycloalkyl), -NH-C(O)-$(CH_2)_p$-$R^a$, $-N(CH_3)-C(O)-(CH_2)_p-R^a$, $-NH-SO_2-(CH_2)_{1-2}-R^a$, -NH-C(O)-O-$R^a$, -C(O)-$(CH_2)_p$-(4- to 6-membered monocyclic heterocycloalkyl), -C(O)-$(CH_2)_p$-(4- to 6-membered monocyclic cycloalkyl), -NH-C(O)-NH-$R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), $-C(O)-NH-S(O)_2-C_{1-4}$ alkyl, -NH-C(O)deuterated $C_{1-2}$ alkyl,-NH-C(O)-NH-$C_{1-2}$ alkyl or -NH-C(O)-$C_{1-2}$ alkyl, wherein the alkyl, alkenyl, alkynyl, cycloalkyl, and heterocycloalkyl are optionally further substituted with 1-4 groups selected from halogen, OH, $NH_2$, CN, $-S(O)_2-CH_3$, or $-NH-S(O)_2-CH_3$;

in some embodiments, each $R^A$ is independently selected from halogen, =O, CN, COOH, $C_{1-4}$ alkyl, $C_{2-4}$ alkenyl, $C_{2-4}$ alkynyl, $-OC_{1-4}$ alkyl, $-S(O)_2-C_{1-4}$ alkyl,$-S(O)_2-(CH_2)_{1-3}-R^a$, $-C(O)-O-(CH_2)_p-R^a$, $-(CH_2)_{1-2}-C(O)-R^a$, $-C(O)-(CH_2)_{1-2}-R^a$,-C(O)-O-halo $C_{1-4}$ alkyl, -NH-$R^a$, -C(O)-(6- to 9-membered bicyclic heterocycloalkyl), $-N(CH_3)-C(O)-(CH_2)_p-R^a$, -NH-C(O)-O-$R^a$, -C(O)-$(CH_2)_p$-(4- to 6-membered monocyclic heterocycloalkyl), -C(O)-$(CH_2)_p$-(4- to 6-membered monocyclic cycloalkyl), -NH-C(O)-NH-$R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), $-C(O)-NH-S(O)_2-C_{1-4}$ alkyl, -NH-C(O)deuterated $C_{1-2}$ alkyl, -NH-C(O)-NH-$C_{1-2}$ alkyl or -NH-C(O)-$C_{1-2}$ alkyl, wherein the alkyl, alkenyl, alkynyl, cycloalkyl, and heterocycloalkyl are optionally further substituted with 1-4 groups selected from halogen, OH, $NH_2$, CN, $-S(O)_2-CH_3$, or $-NH-S(O)_2-CH_3$;

in some embodiments, each $R^A$ is independently selected from =O, $C_{1-4}$ alkyl, $-S(O)_2-C_{1-4}$ alkyl, $-S(O)_2-(CH_2)_{1-3}-R^a$, $-C(O)-O-(CH_2)_p-R^a$, $-(CH_2)_{1-2}-C(O)-R^a$,$-C(O)-(CH_2)_{1-2}-R^a$, -C(O)-O-halo $C_{1-3}$ alkyl, -C(O)-(6- to 9-membered bicyclic

heterocycloalkyl), -NH-C(O)-$(CH_2)_p$-$R^a$, -NH-$R^a$, -N($CH_3$)-C(O)-$(CH_2)_p$-$R^a$, -NH-$SO_2$-$(CH_2)_{1-2}$-$R^a$, -NH-C(O)-O-$R^a$, -C(O)-$(CH_2)_p$-(4- to 6-membered monocyclic heterocycloalkyl), -C(O)-$(CH_2)_p$-(4- to 6-membered monocyclic cycloalkyl), -NH-C(O)-NH-$R^a$, -C(O)-deuterated $C_{1-2}$ alkyl, -C(O)-NH-(4- to 6-membered monocyclic cycloalkyl), -C(O)-NH-(4- to 6-membered monocyclic heterocycloalkyl), -C(O)-NH-$S(O)_2$-$C_{1-4}$ alkyl, -NH-C(O)deuterated $C_{1-2}$ alkyl,-NH-C(O)-NH-$C_{1-2}$ alkyl or -NH-C(O)-$C_{1-2}$ alkyl, wherein the alkyl, cycloalkyl, and heterocycloalkyl are optionally further substituted with 1-4 groups selected from halogen, OH, $NH_2$, CN, -$S(O)_2$-$CH_3$, or -NH-$S(O)_2$-$CH_3$;

in some embodiments, $R^A$ is selected from -$S(O)_2$-$(CH_2)_{1-3}$-$R^a$, -C(O)-O-$(CH_2)_p$-$R^a$, -(CH2)$_{1-2}$-C(O)-$R^a$, -C(O)-$(CH_2)_{1-2}$-$R^a$, -C(O)-O-halo $C_{1-4}$ alkyl, -C(O)-(6- to 9-membered bicyclic heterocycloalkyl), -NH-C(O)-$(CH_2)_p$-$R^a$, -N($CH_3$)-C(O)-$(CH_2)_p$-$R^a$, -NH-$SO_2$-$(CH_2)_{1-2}$-$R^a$, -NH-C(O)-O-$R^a$, -NH-$R^a$, -C(O)-$(CH_2)_p$-(4-to 6-membered monocyclic heterocycloalkyl), -C(O)-$(CH_2)_p$-(4- to 6-membered monocyclic cycloalkyl), -NH-C(O)-NH-$R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), -C(O)-NH-$S(O)_2$-$C_{1-4}$ alkyl, -NH-C(O)deuterated $C_{1-2}$ alkyl, -NH-C(O)-NH-$C_{1-2}$ alkyl or -NH-C(O)-$C_{1-2}$ alkyl, wherein the alkyl, cycloalkyl, and heterocycloalkyl are optionally further substituted with 1-3 groups selected from halogen, OH, $NH_2$, CN, -$S(O)_2$-$CH_3$, -O-halo $C_{1-4}$ alkyl, or -NH-$S(O)_2$-$CH_3$;

in some embodiments, each $R^A$ is independently selected from halogen, =O, CN, COOH, $C_{1-6}$ alkyl, $C_{2-6}$ alkenyl, $C_{2-6}$ alkynyl, -O-$C_{1-4}$ alkyl, -$S(O)_2$-$C_{1-4}$ alkyl,-$S(O)_2$-(CH2)$_{1-3}$-$R^a$, -P(O)-(C1-4 alkyl)2, -C(O)-O-(CH2)$_p$-$R^a$, -$(CH_2)_{1-2}$-C(O)-$R^a$,-C(O)-$(CH_2)_{1-2}$-$R^a$, -C(O)-O-halo $C_{1-4}$ alkyl, -C(O)-(6- to 9-membered bicyclic heterocycloalkyl), -NH-C(O)-$(CH_2)_p$-$R^a$, -N($CH_3$)-C(O)-$(CH_2)_p$-$R^a$, -NH-$SO_2$-$(CH_2)_{1-2}$-$R^a$, -NH-C(O)-O-$R^a$, -C(O)-$(CH_2)_p$-(4- to 6-membered monocyclic heterocycloalkyl), -C(O)-$(CH_2)_p$-(4- to 6-membered monocyclic cycloalkyl), -NH-C(O)-NH-$R^a$, -C(O)-deuterated $C_{1-4}$ alkyl or -C(O)-NH-(4- to 6-membered monocyclic cycloalkyl), -C(O)-NH-(4- to 6-membered monocyclic heterocycloalkyl), -C(O)-NH-$S(O)_2$-$C_{1-4}$ alkyl, -NH-C(O)deuterated $C_{1-2}$ alkyl,-NH-C(O)-NH-$C_{1-2}$ alkyl or -NH-C(O)-$C_{1-2}$ alkyl, wherein the alkyl, alkenyl, alkynyl, cycloalkyl, and heterocycloalkyl are optionally further substituted with 1-4 groups selected from halogen, OH, $NH_2$, CN, -$S(O)_2$-$CH_3$, -O-halo $C_{1-4}$ alkyl, or -NH-$S(O)_2$-$CH_3$;

in some embodiments, each $R^A$ is independently selected from -$S(O)_2$-$C_{1-4}$ alkyl, -$S(O)_2$-$(CH_2)_{1-3}$-$R^a$, -C(O)-O-$(CH_2)_p$-$R^a$, -$(CH_2)_{1-2}$-C(O)-$R^a$, -C(O)-$(CH_2)_{1-2}$-$R^a$,-C(O)-O-halo $C_{1-3}$ alkyl, -C(O)-6- to 8-membered bicyclic heterocycloalkyl, -NH-C(O)-$(CH_2)_p$-$R^a$, -N($CH_3$)-C(O)-$(CH_2)_p$-$R^a$, or -NH-$SO_2$-$(CH_2)_{12}$-$R^a$, wherein the alkyl and heterocycloalkyl are optionally further substituted with 1-4 groups selected from halogen, OH, $NH_2$, CN, or -$S(O)_2$-$CH_3$;

in some embodiments, each $R^A$ is independently selected from halogen, CN, COOH, $C_{2-6}$ alkenyl, $C_{2-6}$ alkynyl, -O-$C_{1-4}$ alkyl, -$S(O)_2$-$C_{1-4}$ alkyl, -$S(O)_2$-$(CH_2)_{1-3}$-$R^a$, -P(O)-($C_{1-4}$ alkyl)$_2$, -C(O)-O-$(CH_2)_p$-$R^a$, -(CH2)$_{1-2}$-C(O)-$R^a$, -C(O)-$(CH_2)_{1-2}$-$R^a$,-C(O)-O-halo $C_{1-4}$ alkyl, -NH-C(O)-$(CH_2)_p$-$R^a$, or -NH-$SO_2$-$(CH_2)_{1-2}$-$R^a$, wherein the alkyl, alkenyl, and alkynyl are optionally further substituted with 1-4 groups selected from halogen, OH, $NH_2$, CN, or -O-halo $C_{1-4}$ alkyl;

in some embodiments, each $R^A$ is independently selected from halogen, CN, COOH, $C_{2-6}$ alkenyl, $C_{2-6}$ alkynyl, -O-$C_{1-4}$ alkyl, -$S(O)_2$-$C_{1-4}$ alkyl, -$S(O)_2$-$(CH_2)_{1-3}$-$R^a$, -P(O)-($C_{1-4}$ alkyl)$_2$, -C(O)-O-$(CH_2)_p$-$R^a$, -(CH2)$_{1-2}$-C(O)-$R^a$, -C(O)-$(CH_2)_{1-2}$-$R^a$, or -C(O)-O-halo $C_{1-4}$ alkyl, wherein the alkyl, alkenyl, and alkynyl are optionally further substituted with 1-4 groups selected from halogen, OH, $NH_2$, CN, or -O-halo $C_{1-4}$ alkyl;

in some embodiments, each $R^A$ is independently selected from halogen, =O, CN, COOH, $C_{1-4}$ alkyl, $C_{2-4}$ alkenyl, $C_{2-4}$ alkynyl, -O$C_{1-4}$ alkyl, -$S(O)_2$-$C_{1-4}$ alkyl,-$S(O)_2$-$(CH_2)_{1-3}$-$R^a$, -P(O)-($C_{1-4}$ alkyl)$_2$, -C(O)-O-$(CH_2)_p$-$R^a$, -$(CH_2)_{1-2}$-C(O)-$R^a$,-C(O)-$(CH_2)_{1-2}$-$R^a$, -C(O)-O-halo $C_{1-4}$ alkyl, -C(O)-(6- to 9-membered bicyclic heterocycloalkyl), -NH-C(O)-$(CH_2)_p$-$R^a$, -N($CH_3$)-C(O)-$(CH_2)_p$-$R^a$, -NH-$SO_2$-$(CH_2)_{1-2}$-$R^a$, -NH-C(O)-O-$R^a$, -C(O)-$(CH_2)_p$-(4- to 6-membered monocyclic heterocycloalkyl), -C(O)-$(CH_2)_p$-(4- to 6-membered monocyclic cycloalkyl), -NH-C(O)-NH-$R^a$, or -C(O)-deuterated $C_{1-4}$ alkyl, wherein the alkyl, alkenyl, alkynyl, cycloalkyl, and heterocycloalkyl are optionally further substituted with 1-4 groups selected from halogen, OH, $NH_2$, CN, -$S(O)_2$-$CH_3$, or -NH-$S(O)_2$-$CH_3$;

in some embodiments, each $R^A$ is independently selected from halogen, =O, CN, COOH, $C_{1-4}$ alkyl, $C_{2-4}$ alkenyl, $C_{2-4}$ alkynyl, -O$C_{1-4}$ alkyl, -$S(O)_2$-$C_{1-4}$ alkyl,-$S(O)_2$-$(CH_2)_{1-3}$-$R^a$, -C(O)-O-$(CH_2)_p$-$R^a$, -(CH2)$_{1-2}$-C(O)-$R^a$, -C(O)-$(CH_2)_{1-2}$-$R^a$,-C(O)-O-halo $C_{1-4}$ alkyl, -C(O)-(6- to 9-membered bicyclic heterocycloalkyl),-N($CH_3$)-C(O)-$(CH_2)_p$-$R^a$, -NH-C(O)-O-$R^a$, -C(O)-$(CH_2)_p$-(4- to 6-membered monocyclic heterocycloalkyl), -C(O)-$(CH_2)_p$-(4- to 6-membered monocyclic cycloalkyl), -NH-C(O)-NH-$R^a$, or -C(O)-deuterated $C_{1-4}$ alkyl, wherein the alkyl, alkenyl, alkynyl, cycloalkyl, and heterocycloalkyl are optionally further substituted with 1-4 groups selected from halogen, OH, $NH_2$, CN, -$S(O)_2$-$CH_3$, or -NH-$S(O)_2$-$CH_3$;

in some embodiments, each $R^A$ is independently selected from halogen, CN, COOH, $C_{2-4}$ alkenyl, $C_{2-4}$ alkynyl, -O-$C_{1-4}$ alkyl, -$S(O)_2$-$C_{1-4}$ alkyl, -$S(O)_2$-$(CH_2)_{1-3}$-$R^a$, -P(O)-($C_{1-4}$ alkyl)2, -C(O)-O-(CH2)$_p$-$R^a$, -$(CH_2)_{1-2}$-C(O)-$R^a$, -C(O)-$(CH_2)_{1-2}$-$R^a$,-C(O)-O-halo $C_{1-4}$ alkyl, -NH-C(O)-$(CH_2)_p$-$R^a$, or -NH-$SO_2$-$(CH_2)_{1-2}$-$R^a$, wherein the alkyl, alkenyl, and alkynyl are optionally further substituted with 1-4 groups selected from halogen, OH, $NH_2$, or CN;

in some embodiments, each $R^A$ is independently selected from halogen, CN, COOH, $C_{2-4}$ alkenyl, $C_{2-4}$ alkynyl, -O-$C_{1-4}$ alkyl, -$S(O)_2$-$C_{1-4}$ alkyl, -$S(O)_2$-$(CH_2)_{1-3}$-$R^a$, -P(O)-($C_{1-4}$ alkyl)$_2$, -C(O)-O-(CH2)$_p$-$R^a$, -$(CH_2)_{1-2}$-C(O)-$R^a$, -C(O)-$(CH_2)_{1-2}$-$R^a$, or -C(O)-O-halo $C_{1-4}$ alkyl, wherein the alkyl, alkenyl, and alkynyl are optionally further substituted

with 1-4 groups selected from halogen, OH, $NH_2$, or CN;

in some embodiments, each $R^A$ is independently selected from halogen, CN, COOH, $C_{2-4}$ alkenyl, $C_{2-4}$ alkynyl, -O-$C_{1-4}$ alkyl, $-S(O)_2-C_{1-4}$ alkyl, $-S(O)_2-(CH_2)_{1-3}-R^a$, $-C(O)-O-(CH2)_p-R^a$, $-(CH_2)_{1-2}-C(O)-R^a$, $-C(O)-(CH_2)_{1-2}-R^a$, or -C(O)-O-halo $C_{1-4}$ alkyl, wherein the alkyl, alkenyl, and alkynyl are optionally further substituted with 1-4 groups selected from halogen, OH, $NH_2$, or CN;

in some embodiments, each $R^A$ is independently selected from halogen, CN, COOH, $C_{2-4}$ alkenyl, $C_{2-4}$ alkynyl, -O-$C_{1-4}$ alkyl, $-S(O)_2-C_{1-4}$ alkyl, $-S(O)_2-(CH_2)_{1-3}-R^a$, $-C(O)-O-(CH_2)_p-R^a$, $-(CH_2)_{1-2}-C(O)-R^a$, or $-C(O)-(CH_2)_{1-2}-R^a$, wherein the alkyl, alkenyl, and alkynyl are optionally further substituted with 1-4 groups selected from halogen, OH, $NH_2$, or CN;

in some embodiments, each $R^A$ is independently selected from $-S(O)_2-C_{1-4}$ alkyl, $-S(O)_2-(CH_2)_{1-3}-R^a$, $-C(O)-O-(CH_2)_p-R^a$, $-(CH2)_{1-2}-C(O)-R^a$, $-C(O)-(CH_2)_{1-2}-R^a$, or-C(O)-O-halo $C_{1-3}$ alkyl, wherein the alkyl is optionally further substituted with 1-4 groups selected from halogen, OH, $NH_2$, or CN; in some embodiments, each $R^A$ is independently selected from $-S(O)_2-C_{1-4}$ alkyl, $-S(O)_2-(CH_2)_{1-3}-R^a$, $-C(O)-O-(CH_2)_p-R^a$, $-(CH_2)_{1-2}-C(O)-R^a$, or $-C(O)-(CH_2)_{1-2}-R^a$, wherein the alkyl is optionally further substituted with 1-4 groups selected from halogen, OH, $NH_2$, or CN;

in some embodiments, each $R^A$ is independently selected from $-S(O)_2-CH_3$,$-S(O)_2-CH_2CH_3$, $-S(O)_2-CH_2(CH_3)_2$, $-S(O)_2-CH_2CH_2CH_3$, $-S(O)_2-CH_2-R^a$, $-S(O)_2-(CH_2)_2-R^a$, $-C(O)-O-R^a$, $-C(O)-O-CH_2-R^a$, $-CH_2-C(0)-R^a$, $-(CH_2)_2-C(O)-R^a$, $-C(O)-CH_2-R^a$, $-C(O)-(CH_2)_2-R^a$, or -C(O)-O-halo $C_{1-3}$alkyl, wherein the $CH_3$ and $CH_2$ are optionally further substituted with 1-4 groups selected from halogen, OH, $NH_2$, or CN;

in some embodiments, each $R^A$ is independently selected from $-S(O)_2-CH_3$,$-S(O)_2-CH_2CH_3$, $-S(O)_2-CH_2(CH_3)_2$, $-S(O)_2-CH_2CH_2CH_3$, $-S(O)_2-CH_2-R^a$, $-S(O)_2-(CH_2)_2-R^a$, $-C(O)-O-R^a$, $-C(O)-O-CH_2-R^a$, $-CH_2-C(O)-R^a$, $-(CH_2)_2-C(O)-R^a$, $-C(O)-CH_2-R^a$, or $-C(O)-(CH_2)_2-R^a$, wherein the $CH_3$ and $CH_2$ are optionally further substituted with 1-4 groups selected from halogen, OH, $NH_2$, or CN;

in some embodiments, each $R^A$ is independently selected from $-S(O)_2-CH_3$,$-S(O)_2-CH_2CH_3$, $-S(O)_2-CH_2(CH_3)_2$, $-S(O)_2-CH_2CH_2CH_3$, $-S(O)_2-CH_2-R''$, $-S(O)_2-(CH_2)_2-R^a$, $-C(O)-O-R^a$, $-C(O)-O-CH_2-R^a$, $-CH_2-C(O)-R^a$, $-(CH_2)_2-C(O)-R^a$, $-C(O)-CH_2-R^a$, $-C(O)-(CH_2)_2-R^a$, or -C(O)-O-halo $C_{1-3}$ alkyl;

in some embodiments, each $R^A$ is independently selected from $-S(O)_2-CH_3$,$-S(O)_2-CH_2CH_3$, $-S(O)_2-CH_2(CH_3)_2$, $-S(O)_2-CH_2CH_2CH_3$, $-S(O)_2-CH_2-R''$, $-S(O)_2-(CH_2)_2-R^a$, $-C(O)-O-R^a$, $-C(O)-O-CH_2-R^a$, $-CH_2-C(O)-R^a$, $-(CH_2)_2-C(O)-R^a$, $-C(O)-CH_2-R^a$, or $-C(O)-(CH_2)_2-R^a$;

in some embodiments, each $R^A$ is independently selected from $-S(O)_2-CH_3$,$-S(O)_2-CH_2CH_3$, $-S(O)_2-CH_2(CH_3)_2$, $-S(O)_2-CH_2CH_2CH_3$, $-S(O)_2-CH_2$-3- to 5-membered cycloalkyl, $-S(O)_2-CH_2$-4- to 6-membered heterocycloalkyl, $-S(O)_2-CH_2$-5- to 6-membered heteroaryl, $-S(O)_2-(CH_2)_2$-3- to 5-membered cycloalkyl, $-S(O)_2-(CH_2)_2$-4- to 6-membered heterocycloalkyl, $-S(O)_2-(CH_2)_2$-5- to 6-membered heteroaryl, -C(O)-O-3- to 5-membered cycloalkyl, -C(O)-O-4- to 6-membered heterocycloalkyl, -C(O)-O-5- to 6-membered heteroaryl, $-C(O)-O-CH_2$-3- to 5-membered cycloalkyl, $-C(O)-O-CH_2$-4- to 6-membered heterocycloalkyl, $-C(O)-O-CH_2$-5- to 6-membered heteroaryl, $-CH_2-C(O)$-3- to 5-membered cycloalkyl, $-CH_2-C(O)$-4- to 6-membered heterocycloalkyl, $-CH_2-C(O)$-5- to 6-membered heteroaryl, $-(CH_2)_2-C(O)$-3- to 5-membered cycloalkyl, $-(CH_2)_2-C(O)$-4- to 6-membered heterocycloalkyl, $-(CH_2)_2-C(O)$-5- to 6-membered heteroaryl, $-C(O)-CH_2$-3- to 5-membered cycloalkyl, $-C(O)-CH_2$-4- to 6-membered heterocycloalkyl, $-C(O)-CH_2$-5- to 6-membered heteroaryl, $-C(O)-(CH_2)_2$-3- to 5-membered cycloalkyl, $-C(O)-(CH_2)_2$-4- to 6-membered heterocycloalkyl, $-C(O)-(CH_2)_2$-5- to 6-membered heteroaryl, or -C(O)-O-halo $C_{1-3}$ alkyl, wherein the cycloalkyl, heterocycloalkyl, and heteroaryl are optionally further substituted with groups selected from F, Cl, Br, OH, methyl, or ethyl;

in some embodiments, each $R^A$ is independently selected from $-S(O)_2-CH_3$,$-S(O)_2-CH_2CH_3$, $-S(O)_2-CH_2(CH_3)_2$, $-S(O)_2-CH_2CH_2CH_3$, $-S(O)_2-CH_2$-3- to 5-membered cycloalkyl, $-S(O)_2-CH_2$-4- to 6-membered heterocycloalkyl, $-S(O)_2-CH_2$-5- to 6-membered heteroaryl, $-S(O)_2-(CH_2)_2$-3- to 5-membered cycloalkyl, $-S(O)_2-(CH_2)_2$-4- to 6-membered heterocycloalkyl, $-S(O)_2-(CH_2)_2$-5- to 6-membered heteroaryl, -C(O)-O-3- to 5-membered cycloalkyl, -C(O)-O-4- to 6-membered heterocycloalkyl, -C(O)-O-5- to 6-membered heteroaryl, $-C(O)-O-CH_2$-3- to 5-membered cycloalkyl, $-C(O)-O-CH_2$-4- to 6-membered heterocycloalkyl, $-C(O)-O-CH_2$-5- to 6-membered heteroaryl, $-CH_2-C(O)$-3- to 5-membered cycloalkyl, $-CH_2-C(O)$-4- to 6-membered heterocycloalkyl, $-CH_2-C(O)$-5- to 6-membered heteroaryl, $-(CH_2)_2-C(O)$-3- to 5-membered cycloalkyl, $-(CH_2)_2-C(O)$-4- to 6-membered heterocycloalkyl, $-(CH_2)_2-C(O)$-5- to 6-membered heteroaryl, $-C(O)-CH_2$-3- to 5-membered cycloalkyl, $-C(O)-CH_2$-4- to 6-membered heterocycloalkyl, $-C(O)-CH_2$-5- to 6-membered heteroaryl, $-C(O)-(CH_2)_2$-3- to 5-membered cycloalkyl, $-C(O)-(CH_2)_2$-4- to 6-membered heterocycloalkyl, or $-C(O)-(CH_2)_2$-5- to 6-membered heteroaryl, wherein the cycloalkyl, heterocycloalkyl, and heteroaryl are optionally further substituted with groups selected from F, Cl, Br, OH, methyl, or ethyl;

in some embodiments, each $R^A$ is independently selected from $-S(O)_2-CH_3$,$-S(O)_2-CH_2CH_3$, -C(O)-O-4- to 6-membered heterocycloalkyl, -C(O)-O-3- to 5-membered cycloalkyl, $-C(O)-CH_2$-3- to 5-membered cycloalkyl, $-S(O)_2-CH_2$-3- to 5-membered cycloalkyl, or -C(O)-O-halo $C_{1-3}$ alkyl, wherein the cycloalkyl and heterocycloalkyl

are optionally further substituted with groups selected from F, Cl, Br, OH, methyl, or ethyl;

in some embodiments, each $R^A$ is independently selected from $-S(O)_2-CH_3$,$-S(O)_2-CH_2CH_3$, -C(O)-O-4- to 6-membered heterocycloalkyl, -C(O)-O-3- to 5-membered cycloalkyl, $-C(O)-CH_2-$3- to 5-membered cycloalkyl, or $-S(O)_2-CH_2-$3-to 5-membered cycloalkyl, wherein the cycloalkyl and heterocycloalkyl are optionally further substituted with groups selected from F, Cl, Br, OH, methyl, or ethyl;

in some embodiments, each $R^A$ is independently selected from $-S(O)_2-CH_3$,$-S(O)_2-CH_2CH_3$, -C(O)-O-oxetanyl, -C(O)-O-azetidinyl, -C(O)-O-cyclobutyl,-C(O)-O-piperidyl, $-C(O)-CH_2-$cyclopropyl, $-S(O)_2-CH_2-$cyclopropyl, $-S(O)_2-CH_2-$cyclobutyl, $-S(O)_2-CH_2-$cyclopentyl, or -C(O)-O-halo $C_{1-3}$alkyl, wherein the oxetanyl, azetidinyl, cyclobutyl, piperidyl, cyclopropyl, and cyclopentyl are optionally further substituted with groups selected from F, Cl, Br, OH, methyl, or ethyl;

in some embodiments, each $R^A$ is independently selected from $-S(O)_2-CH_3$,$-S(O)_2-CH_2CH_3$, -C(O)-O-oxetanyl, -C(O)-O-azetidinyl, -C(O)-O-cyclobutyl,-C(O)-O-piperidyl, $-C(O)-CH_2-$cyclopropyl, $-S(O)_2-CH_2-$cyclopropyl, $-S(O)_2-CH_2-$cyclobutyl, or $-S(O)_2-CH_2-$cyclopentyl, wherein the oxetanyl, azetidinyl, cyclobutyl, piperidyl, cyclopropyl, and cyclopentyl are optionally further substituted with groups selected from F, Cl, Br, OH, methyl, or ethyl;

in some embodiments, $R^A$ is selected from $-C(O)-O-(CH_2)_p-R^a$, $-C(O)-(CH_2)_{1-2}-R^a$, $-NH-C(O)-(CH_2)_p-R^a$, $-N(CH_3)-C(O)-(CH_2)_p-R^a$, or $-NH-C(O)-O-R^a$;

in some embodiments,

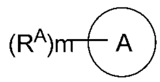

has one of the following structures:

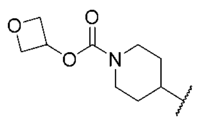 , 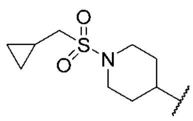 , 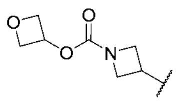 , 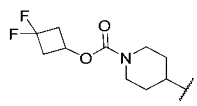 , 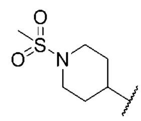 ,

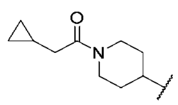 , 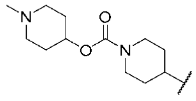 , 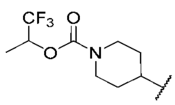 , 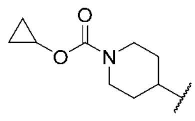 , 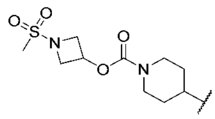 ,

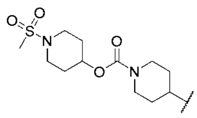 , 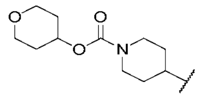 , 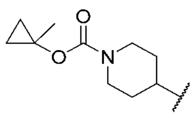 , 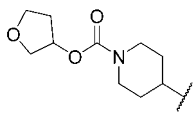 , 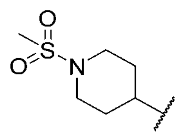 ,

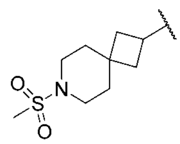 , 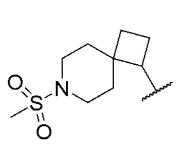 , 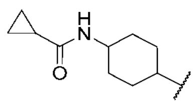 , 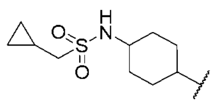 ,

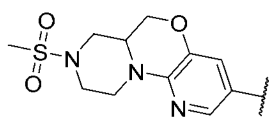 , 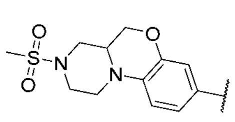 , 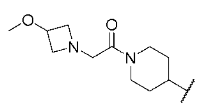 , 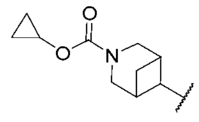 ,

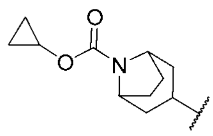 , 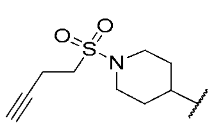 , 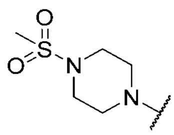 , 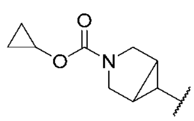 , 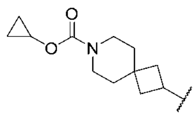 ,

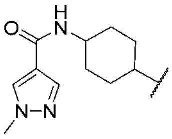, 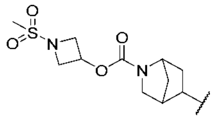, 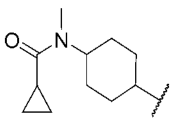, 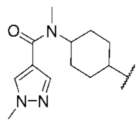, 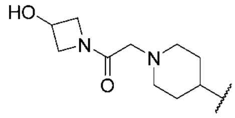,

or

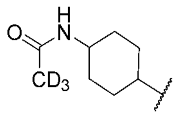,

or is selected from the following structures:

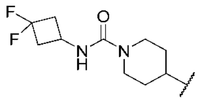,

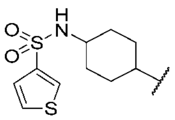, 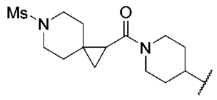, 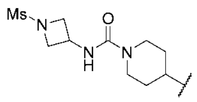, 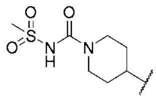, 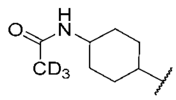,

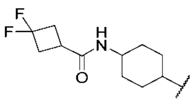, 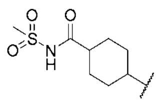, 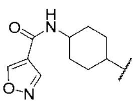, or 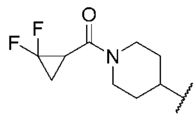,

or is selected from the following structures:

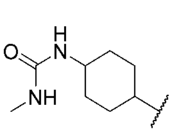, 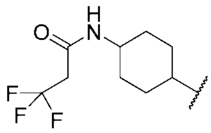, 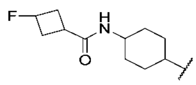, 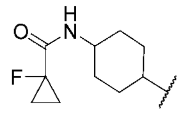,

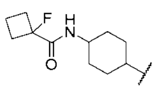, 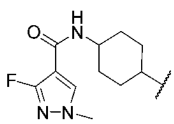, or 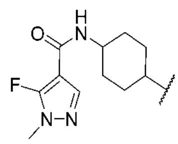,

or is selected from the following structures:

, 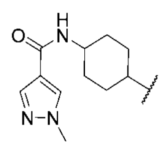, 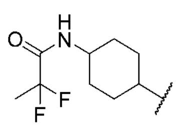, 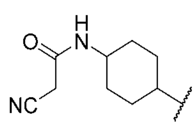, 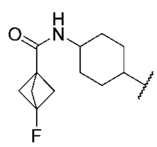, 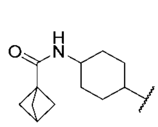,

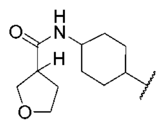, 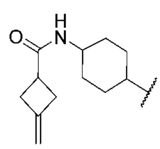, or 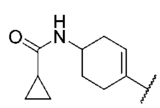,

or is selected from

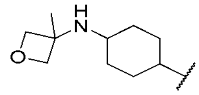

or

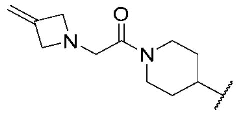;

in some embodiments,

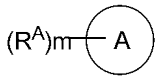

has one of the following structures:

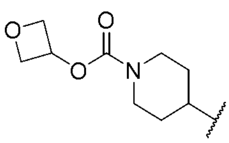, 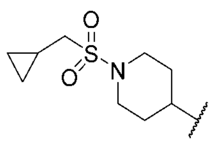, 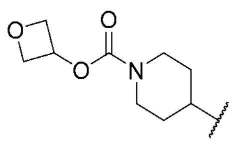, 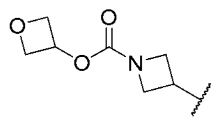,

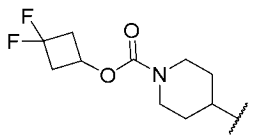, 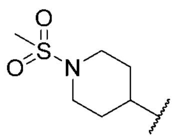, 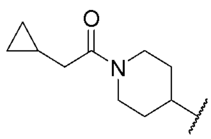, or 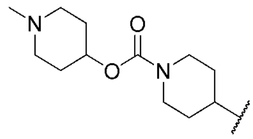,

or is selected from

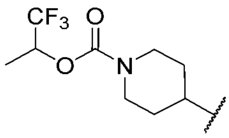, 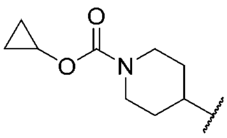, 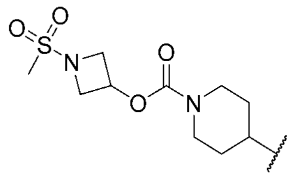,

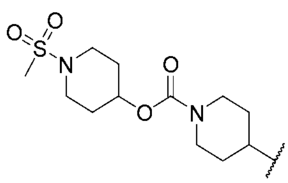, 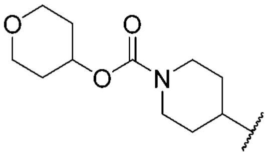, 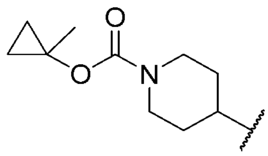,

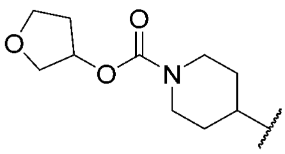, 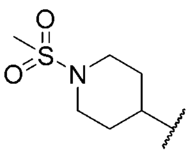, 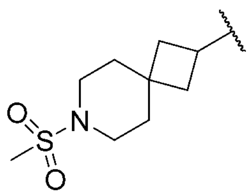, or 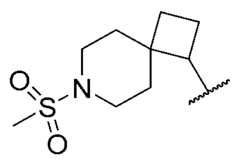,

or is selected from

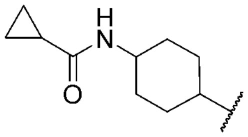 , 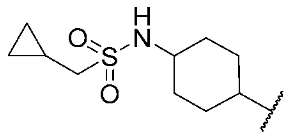 ,

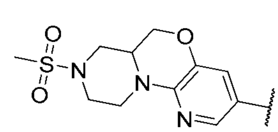 , 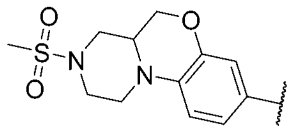 , 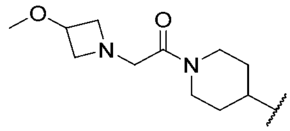 ,

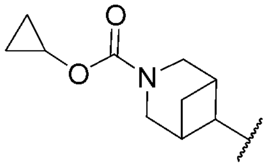 , 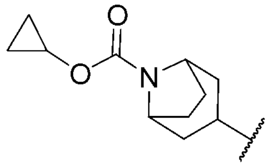 , 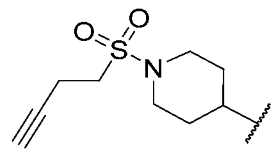 , 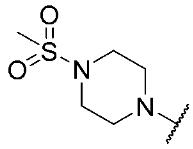 ,

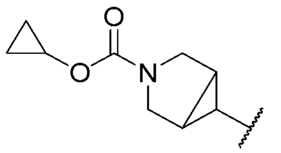 , or 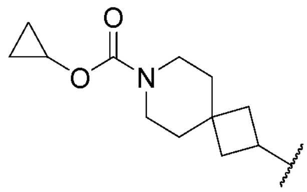 ,

or is selected from

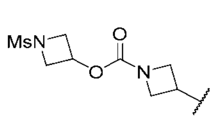 , 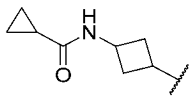 , 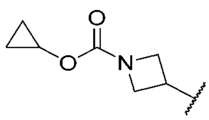 , 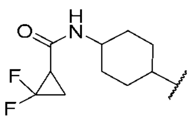 , 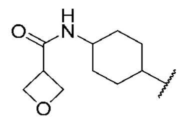 ,

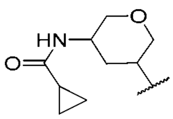 , 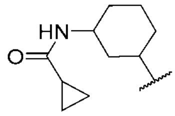 , 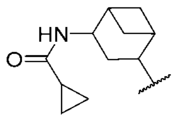 , 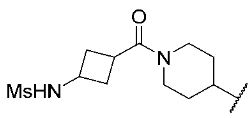 , 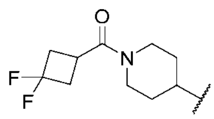 ,

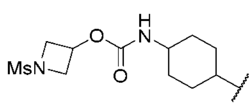 , 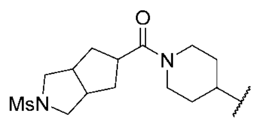 , 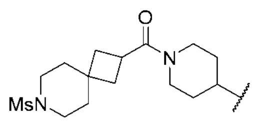 , 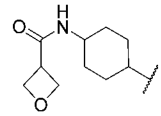 ,

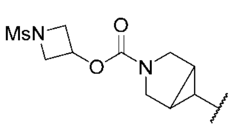 , 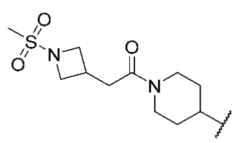 , 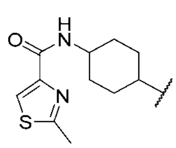 , 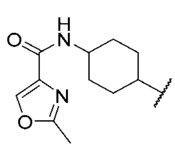 , 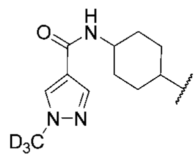 ,

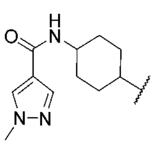 , 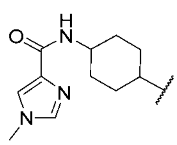 , 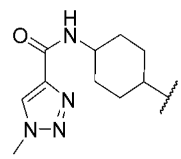 , 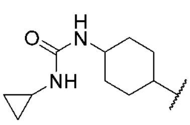 , 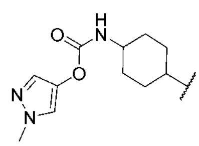 ,

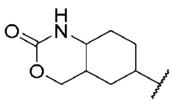, 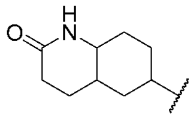, 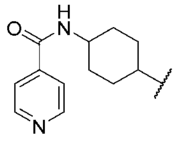, 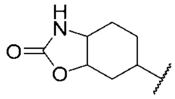, 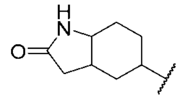,

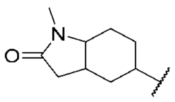, 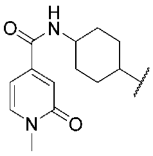, 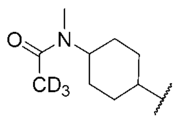, or 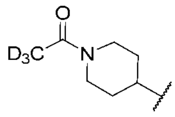;

in some embodiments,

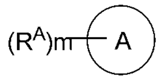

has one of the following structures:

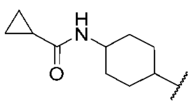, 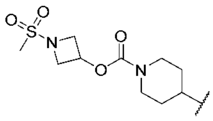, 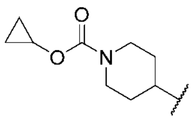, 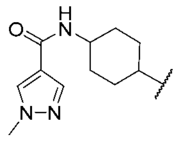, 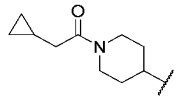,

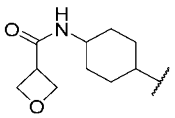, 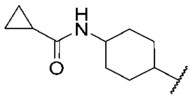, 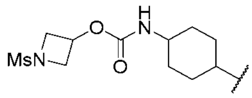, 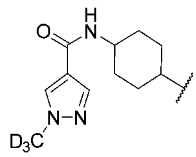,

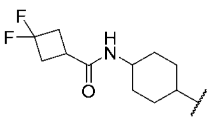, 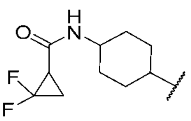, or 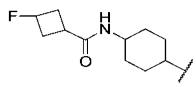;

in some embodiments,

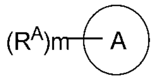

has one of the following structures:

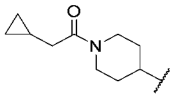, 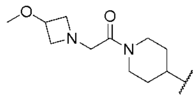, 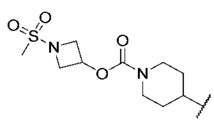, 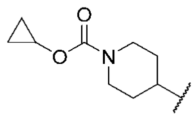, 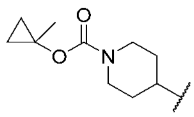,

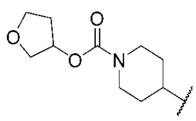, 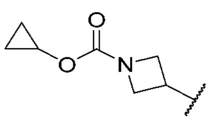, 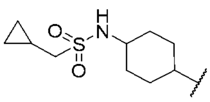, 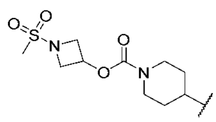, 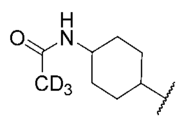,

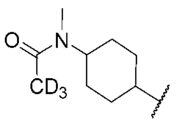, 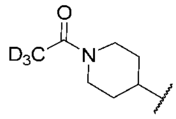, 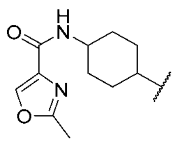, 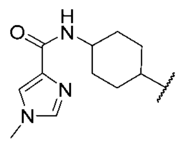, 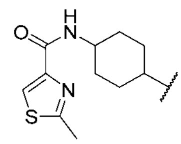,

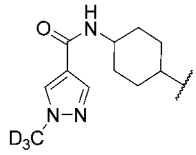, 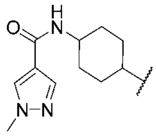, 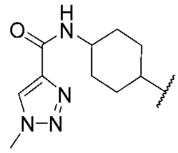, 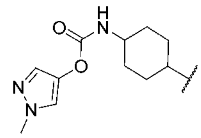, 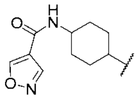,

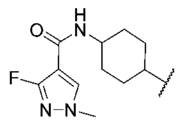, 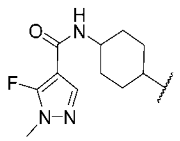, 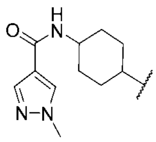, 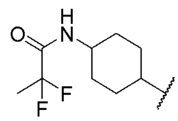, 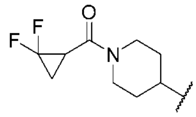,

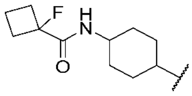, 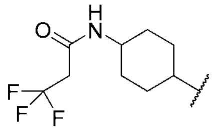, 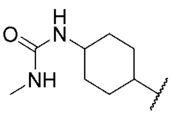, 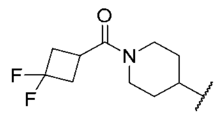, 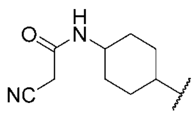,

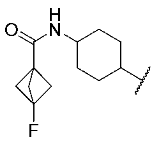, 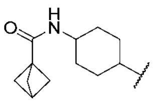, 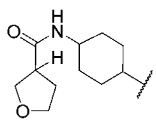, 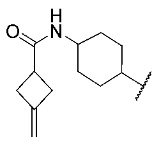, 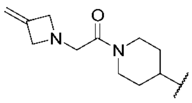, 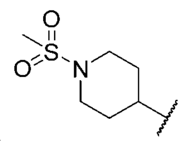,

or

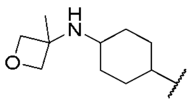;

$R^a$ is selected from deuterated $C_{1-4}$ alkyl, $C_{2-4}$ alkynyl, 3- to 5-membered cycloalkyl, 3- to 6-membered heterocycloalkyl, or 5- to 6-membered heteroaryl, wherein the alkynyl, cycloalkyl, heterocycloalkyl, and heteroaryl are optionally further substituted with 1-4 groups selected from halogen, OH, =O, $C_{1-4}$ alkyl, deuterated $C_{1-4}$ alkyl, $C_{1-4}$ alkoxy, $C_{2-4}$ alkynyl, -O-halo $C_{1-4}$ alkyl, $-S(O)_2-CH_3$ or $=CH_2$;

in some embodiments, $R^a$ is selected from deuterated $C_{1-4}$ alkyl, $C_{2-4}$ alkynyl, 3- to 5-membered cycloalkyl, 3- to 6-membered heterocycloalkyl, or 5- to 6-membered heteroaryl, wherein the alkynyl, cycloalkyl, heterocycloalkyl, and heteroaryl are optionally further substituted with 1-4 groups selected from halogen, OH, $C_{1-4}$ alkyl, $C_{1-4}$ alkoxy, $C_{2-4}$ alkynyl, -O-halo $C_{1-4}$ alkyl, or $-S(O)_2-CH_3$;

in some embodiments, $R^a$ is selected from $C_{2-4}$ alkynyl, 3- to 5-membered cycloalkyl, 3- to 6-membered heterocycloalkyl, or 5- to 6-membered heteroaryl, wherein the alkynyl, cycloalkyl, heterocycloalkyl, and heteroaryl are optionally further substituted with 1-4 groups selected from halogen, OH, $C_{1-4}$ alkyl, $C_{1-4}$ alkoxy, $C_{2-4}$ alkynyl, -O-halo $C_{1-4}$ alkyl, or $-S(O)_2-CH_3$;

in some embodiments, $R^a$ is selected from 3- to 5-membered cycloalkyl, 3- to 6-membered heterocycloalkyl, or 5- to 6-membered heteroaryl, wherein the cycloalkyl, heterocycloalkyl, and heteroaryl are further substituted with 1, 2, 3 or 4 groups selected from halogen, $C_{1-4}$ alkyl, deuterated $C_{1-4}$ alkyl, or $-S(O)_2-CH_3$;

in some embodiments, $R^a$ is selected from $C_{2-4}$ alkynyl, 3- to 5-membered cycloalkyl, 3- to 6-membered heterocycloalkyl, or 5- to 6-membered heteroaryl, wherein the alkynyl, cycloalkyl, heterocycloalkyl, and heteroaryl are optionally further substituted with 1-4 groups selected from halogen, OH, $C_{1-4}$ alkyl, $C_{2-4}$ alkynyl, -O-halo $C_{1-4}$ alkyl, or $-S(O)_2-CH_3$;

in some embodiments, $R^a$ is selected from $C_{2-4}$ alkynyl, 3- to 5-membered cycloalkyl, 4- to 6-membered heterocycloalkyl, 5-membered heteroaryl, or 6-membered heteroaryl, wherein the cycloalkyl, heterocycloalkyl, and heteroaryl are optionally further substituted with groups selected from halogen, $C_{1-2}$ alkyl, OH, or $-S(O)_2-CH_3$;

in some embodiments, $R^a$ is selected from deuterated $C_{1-2}$ alkyl, $C_{2-4}$ alkynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, $C_{1-2}$ alkyl, OH, =O, $-S(O)_2-CH_3$, $C_{1-2}$ alkoxy, or deuterated $C_{1-2}$ alkyl;

in some embodiments, $R^a$ is selected from $C_{2-4}$ alkynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, $C_{1-2}$ alkyl, OH, $-S(O)_2-CH_3$, or $C_{1-2}$ alkoxy;

in some embodiments, $R^a$ is selected from $C_{2-4}$ alkynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, $C_{1-2}$ alkyl, OH, or $-S(O)_2-CH_3$;

in some embodiments, $R^a$ is selected from deuterated $C_{1-2}$ alkyl, ethynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, OH, =O, $C_{1-2}$ alkyl, deuterated $C_{1-2}$ alkyl, $C_{1-2}$ alkoxy, or $-S(O)_2-CH_3$;

in some embodiments, $R^a$ is selected from deuterated $C_{1-2}$ alkyl, ethynyl, 3- to 5-membered cycloalkyl, 4- to 6-membered heterocycloalkyl, or 5- to 6-membered heteroaryl, wherein the cycloalkyl, heterocycloalkyl, and heteroaryl are optionally further substituted with groups selected from halogen, methyl, deuterated methyl, methoxy, ethoxy, OH, =O, or $-S(O)_2-CH_3$;

in some embodiments, $R^a$ is selected from ethynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, OH, $C_{1-2}$ alkyl, $C_{1-2}$ alkoxy, or $-S(O)_2-CH_3$;

in some embodiments, $R^a$ is selected from ethynyl, 3- to 5-membered cycloalkyl, or 4- to 6-membered heterocycloalkyl, wherein the cycloalkyl and heterocycloalkyl are optionally further substituted with groups selected from halogen, methyl, methoxy, ethoxy, OH, or $-S(O)_2-CH_3$;

in some embodiments, $R^a$ is selected from 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, OH, $C_{1-2}$ alkyl, or $-S(O)_2-CH_3$;

in some embodiments, $R^a$ is selected from 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, OH, or $C_{1-2}$ alkyl; in some embodiments, $R^a$ is selected from 3- to 5-membered cycloalkyl, or 4- to 6-membered heterocycloalkyl, wherein the cycloalkyl and heterocycloalkyl are optionally further substituted with groups selected from F, Cl, Br, OH, methyl, ethyl, or $-S(O)_2-CH_3$;

in some embodiments, $R^a$ is selected from 3- to 5-membered cycloalkyl, or 4-to 6-membered heterocycloalkyl, wherein the cycloalkyl and heterocycloalkyl are optionally further substituted with groups selected from F, Cl, Br, OH, methyl, or ethyl;

in some embodiments, $R^a$ is selected from cyclopropyl, cyclobutyl, cyclopentyl, oxetanyl, azetidinyl, oxolanyl, or oxolanyl, wherein the cyclopropyl, cyclobutyl, cyclopentyl, oxetanyl, azetidinyl, oxolanyl, and oxolanyl are optionally further substituted with groups selected from F, Cl, Br, OH, methyl, ethyl, or $-S(O)_2-CH_3$;

in some embodiments, $R^a$ is selected from cyclopropyl, cyclobutyl, cyclopentyl, oxetanyl, azetidinyl, oxolanyl, or oxolanyl, wherein the cyclopropyl, cyclobutyl, cyclopentyl, oxetanyl, azetidinyl, oxolanyl, and oxolanyl are optionally further substituted with groups selected from F, Cl, Br, OH, methyl, or ethyl;

each $R^C$ is independently selected from H, CN, halogen, OH, $C_{1-4}$ alkyl, halo $C_{1-4}$ alkyl, -O-halo $C_{1-4}$ alkyl, -Se-halo $C_{1-4}$ alkyl, $C_{2-4}$ alkenyl, $C_{2-4}$ alkynyl, =O, $C_{3-6}$ cycloalkyl, $-SCF_3$, $-SF_5$, $-(NH)_q-P(O)(C_{1-4}\ alkyl)_2$, $-(CH_2)_p-O-C_{1-4}$ alkyl, $-O-(CH_2)_p-C_{3-6}$ cycloalkyl or $-(CH_2)_p-O-C_{3-6}$ cycloalkyl, or any two $R^C$, together with the atom to which they are connected, form 3- to 8-membered cycloalkyl;

in some embodiments, each $R^C$ is independently selected from H, halogen, OH, $C_{1-4}$ alkyl, halo $C_{1-4}$ alkyl, -O-halo $C_{1-4}$ alkyl, $C_{2-4}$ alkenyl, $C_{2-4}$ alkynyl, =O, $C_{3-6}$ cycloalkyl, $-SCF_3$, $-SF_5$, $-(NH)_q-P(O)(C_{1-4}\ alkyl)_2$, $-(CH_2)_p-O-C_{1-4}$ alkyl, $-O-(CH_2)_p-C_{3-6}$ cycloalkyl or $-(CH_2)_p-O-C_{3-6}$ cycloalkyl, or any two $R^C$ together with the atom to which they are connected, form 3- to 8-membered cycloalkyl;

in some embodiments, each $R^C$ is independently selected from H, halogen, OH, $C_{1-4}$ alkyl, halo $C_{1-4}$ alkyl, -O-halo $C_{1-4}$ alkyl, $C_{2-4}$ alkenyl, $C_{2-4}$ alkynyl, =O, $C_{3-6}$ cycloalkyl, $-SCF_3$, $-SF_5$, $-(NH)_q-P(O)(C_{1-4}\ alkyl)_2$, $-(CH_2)_p-O-C_{1-4}$ alkyl, or $-(CH_2)_p-O-C_{3-6}$ cycloalkyl, or any two $R^C$, together with the atom to which they are connected, form 3- to 8-membered cycloalkyl;

in some embodiments, each $R^C$ is independently selected from H, CN, halogen, OH, $C_{1-4}$ alkyl, halo $C_{1-4}$ alkyl, -O-halo $C_{1-4}$ alkyl, -Se-halo $C_{1-2}$ alkyl, $C_{2-4}$ alkenyl, $C_{2-4}$ alkynyl, =O, $C_{3-6}$ cycloalkyl, $-SCF_3$, $-SF_5$, $-(NH)_q-P(O)(C_{1-4}\ alkyl)_2$, $-(CH_2)_p-O-C_{1-4}$ alkyl, $-O-(CH_2)_p-C_{3-6}$ cycloalkyl or $-(CH_2)_p-O-C_{3-6}$ cycloalkyl, or any two $R^C$ together with the atom to which they are connected, form 3- to 6-membered cycloalkyl;

in some embodiments, each $R^C$ is independently selected from H, halogen, OH, $C_{1-4}$ alkyl, halo $C_{1-4}$ alkyl, -O-halo $C_{1-4}$ alkyl, $C_{2-4}$ alkenyl, $C_{2-4}$ alkynyl, =O, $C_{3-6}$ cycloalkyl, $-SCF_3$, $-SF_5$, $-(NH)_q-P(O)(C_{1-4}\ alkyl)_2$, $-(CH_2)_p-O-C_{1-4}$ alkyl, or $-(CH_2)_p-O-C_{3-6}$ cycloalkyl, or any two $R^C$, together with the atom to which they are connected, form 3- to 6-membered cycloalkyl;

in some embodiments, $R^C$ is selected from F, Cl, halo $C_{1-2}$ alkyl, $C_{2-4}$ alkynyl, 3-4-membered cycloalkyl, or $-O-C_{3-4}$ cycloalkyl;

in some embodiments, $R^C$ is selected from F, Cl, $CHF_2$, $CF_3$, ethynyl, cyclopropyl, cyclobutyl, -O-cyclopropyl, or -O-cyclobutyl;

in some embodiments, $R^C$ is $CF_3$;

in some embodiments, each $R^C$ is independently selected from H, CN, F, Cl, Br, OH, $-SF_5$, methyl, difluoromethyl, trifluoromethyl, ethyl, ethynyl, cyclopropyl, -O-cyclopropyl, $-O-CH_2$-cyclopropyl, cyclobutyl, -O-cyclobutyl, $-O-CH_2$-cyclobutyl, or -Se-trifluoromethyl, or any two $R^C$, together with the atom to which they are connected, form 3- to 6-membered cycloalkyl;

in some embodiments, each $R^C$ is independently selected from F, Cl, Br, OH,$-CH_3$, $-CH_2CH_3$, $-CH_2F$, $-CHF_2$, $-CF_3$, $-CH_2Cl$, $-CHCl_2$, $-CCl_3$, $-CHFCH_3$, $-CF_2CH_3$, $-CH_2CH_2F$, $-CH_2CHF_2$, $-CH_2CF_3$, $-CHClCH_3$, $-CCl_2CH_3$, $-CH_2CH_2Cl$, $-CH_2CHCl_2$, $-CH_2CCl_3$, $-OCH_2F$, $-OCHF_2$, $-OCF_3$, $-OCH_2Cl$, $-OCHCl_2$, $-OCCl_3$, $-OCHFCH_3$,$-OCF_2CH_3$, $-OCH_2CH_2F$, $-OCH_2CHF_2$, $-OCH_2CF_3$, $-OCHClCH_3$, $-OCCl_2CH_3$,$-OCH_2CH_2Cl$, $-OCH_2CHCl_2$, $-OCH_2CCl_3$, ethynyl, =O, cyclopropyl, cyclobutyl, cyclopentyl, cyclohexyl, $-SCF_3$, $-SF_5$, $-P(O)(CH_3)_2$, $-P(O)(CH_2CH_3)_2$, $-NH-P(O)(CH_3)_2$, $-NH-P(O)(CH_2CH_3)_2$, $-OCH_3$, $-OCH_2CH_3$, $-CH_2OCH_3$, $-CH_2OCH_2CH_3$, $-CH_2CH_2OCH_3$, $-CH_2CH_2OCH_2CH_3$,

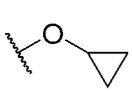, 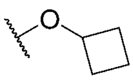, 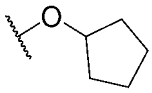,

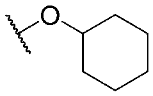, 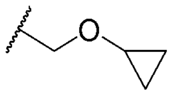, 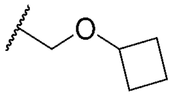, 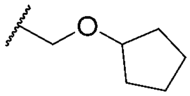, 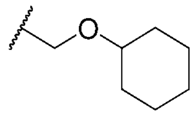,

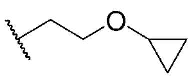, 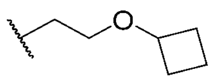, 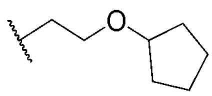, or 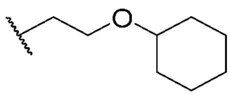,

or any two $R^C$, together with the atom to which they are connected, form cyclopropyl, cyclobutyl, cyclopentyl, or cyclohexyl;

in some embodiments, each $R^C$ is independently selected from H, F, Cl, Br, OH, methyl, ethyl, cyclopropyl, cyclobutyl, trifluoromethyl, or ethynyl, or any two $R^C$ together with the atom to which they are connected, form cyclopropyl, cyclobutyl, cyclopentyl, or cyclohexy;

in some embodiments, each $R^C$ is independently selected from H, F, Cl, Br, OH, methyl, ethyl, cyclopropyl, or cyclobutyl, or any two $R^C$, together with the atom to which they are connected, form cyclopropyl, cyclobutyl, cyclopentyl, or cyclohexyl;

in some embodiments,

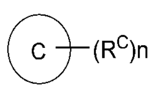

has one of the following structures:

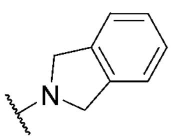, 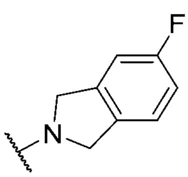, 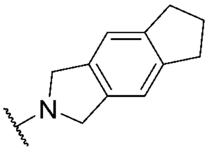, 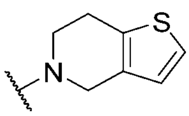, 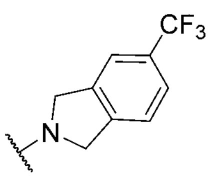,

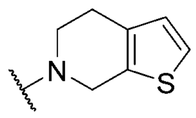, or 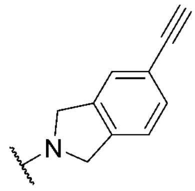,

or is selected from

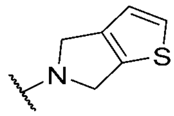,

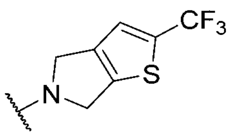, 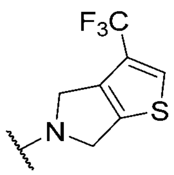, or 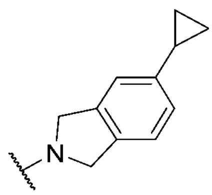,

or is selected from

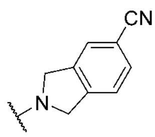 or 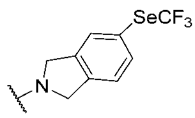,

or is selected from

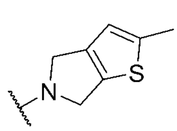 or 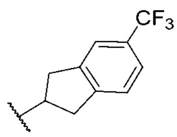;

q is selected from 0 or 1;
in some embodiments, q is 0;
in some embodiments, q is 1;
each p is independently selected from 0, 1, 2, 3, or 4;
in some embodiments, each p is independently selected from 0, 1, 2, or 3;
in some embodiments, each p is independently selected from 0, 1, or 2;
m is selected from 1, 2, 3, or 4;
in some embodiments, m is selected from 1, 2, or 3;
in some embodiments, m is selected from 1 or 2;
in some embodiments, m is 1;
n is selected from 1, 2, 3, or 4;
in some embodiments, n is selected from 1, 2, or 3;
in some embodiments, n is selected from 1 or 2;
in some embodiments, n is 1;
provided that

(1) when ring A is

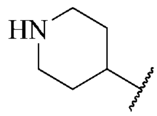,

$R^A$ is not $-S(O)_2-(CH_2)_{0-1}CH_3$, and

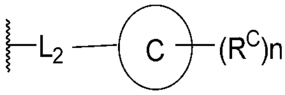

is not

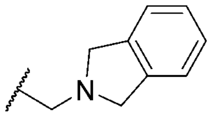 and 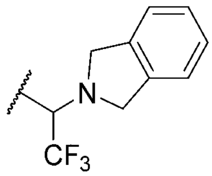 ;

(2) when $R^A$ is $-S(O)_2-(CH_2)_{0-1}CH_3$, ring A is not

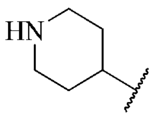 and 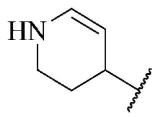,

and

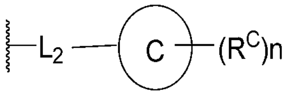

is not

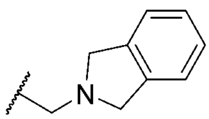 and 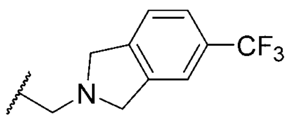 ;

(3) when ring C is

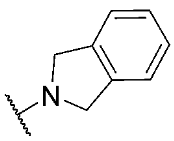,

$R^C$ is not H, and

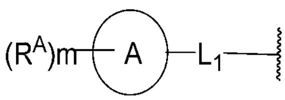

is not

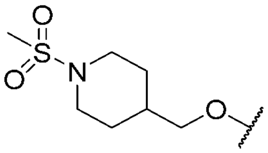,
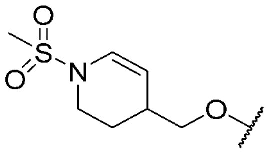,
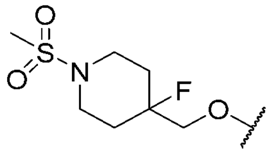

and

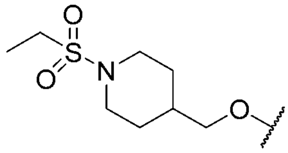;

(4) when $R^C$ is H, ring C is not

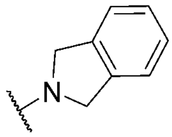,

and

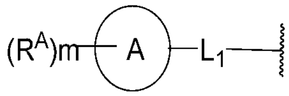

is not

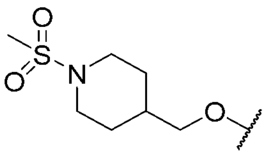 and 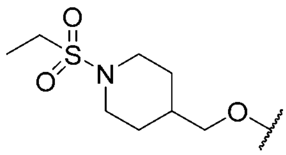.

.

More specifically, as a first technical solution of the present invention, provided is a compound as represented by formula (I), or a stereoisomer, tautomer, deuterated compound, solvate, co-crystal or pharmaceutically acceptable salt, wherein

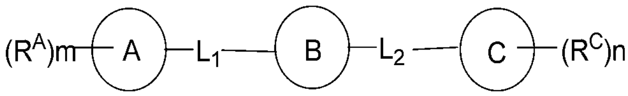

(I)

ring A is selected from 4- to 8-membered cycloalkyl, 4- to 7-membered monocyclic heterocycloalkyl, 6- to 12-membered bicyclic heterocycloalkyl, or 8- to 14-membered tricyclic heterocycloalkyl; in some embodiments, ring A is selected from 5- to 8-membered cycloalkyl, 4- to 7-membered monocyclic heterocycloalkyl, 6- to 12-membered bicyclic heterocycloalkyl, or 8- to 14-membered tricyclic heterocycloalkyl;

ring B is

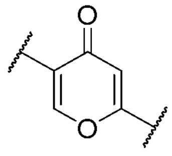,

and ring B is connected to $L_1$ on the left side;

ring C is selected from phenyl, 5- to 6-membered heteroaryl, 6- to 12-membered bicyclic carbocyclyl, 6- to 12-membered bicyclic heterocycloalkyl, or 8-to 14-membered tricyclic heterocycloalkyl, and ring C is not

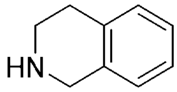,

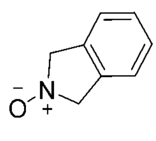, 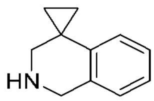, 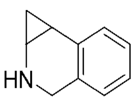 and 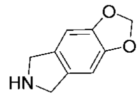;

$L_1$ is $W_1$-$R^{La}$-$W_2$, and $L_1$ is connected to A on the left side;

$L_2$ is $W_3$-$R^{Lb}$-$W_4$, and $L_2$ is connected to B on the left side, and $L_1$ and $L_2$ are not both a bond;

$R^{La}$ and $R^{Lb}$ are each independently selected from a bond, $C_{1-4}$ alkylene, $C_{2-4}$ alkenylene, or $C_{2-4}$ alkynylene, wherein the alkylene and alkenylene are optionally further substituted with 1-4 $R^{L1}$;

each $R^{L1}$ is independently selected from halogen, =O, $C_{1-4}$ alkyl, $C_{2-4}$ alkenyl, $C_{1-4}$ alkoxy, or 3- to 6-membered cycloalkyl, wherein the alkyl, alkenyl, alkoxy, and cycloalkyl are optionally further substituted with 1-4 substituents selected from halogen, CN, OH and $NH_2$, or two $R^{L1}$ on the same carbon atom or two adjacent carbon atoms, together with the atom to which they are connected, form 3- to 8-membered cycloalkyl, 5- to 10-membered heterocycloalkyl, or 5- to 6-membered heteroaryl; in some embodiments, each $R^{L1}$ is independently selected from halogen, $C_{1-4}$ alkyl, $C_{2-4}$ alkenyl, $C_{1-4}$ alkoxy, or 3- to 6-membered cycloalkyl, wherein the alkyl, alkenyl, alkoxy, and cycloalkyl are optionally further substituted with 1-4 substituents selected from halogen, CN, OH and $NH_2$, or two $R^{L1}$ on the same carbon atom or two adjacent carbon atoms, together with the atom to which they are connected, form 3- to 8-membered cycloalkyl, 5- to 10-membered heterocycloalkyl, or 5- to 6-membered heteroaryl; in some embodiments, each $R^{L1}$ is independently selected from halogen, =O, $C_{1-4}$ alkyl, $C_{2-4}$ alkenyl, $C_{1-4}$ alkoxy, or 3- to 6-membered cycloalkyl, wherein the alkyl, alkoxy, and cycloalkyl are optionally further substituted with 1-4 substituents selected from halogen, CN, OH and $NH_2$, or two $R^{L1}$ on the same carbon atom or two adjacent carbon atoms, together with the atom to which they are connected, form 3- to 8-membered cycloalkyl, 5- to 10-membered heterocycloalkyl, or 5- to 6-membered heteroaryl; in some embodiments, each $R^{L1}$ is independently selected from halogen, =O, $C_{1-4}$ alkyl, $C_{2-4}$ alkenyl, $C_{1-4}$ alkoxy, or 3- to 6-membered cycloalkyl, wherein the alkyl, alkoxy, and cycloalkyl are optionally further substituted with 1-4 substituents selected from halogen, CN, OH and $NH_2$, or two adjacent $R^{L1}$, together with the atom to which they are connected, form 3- to 8-membered cycloalkyl, 5- to 10-membered heterocycloalkyl, or 5- to 6-membered heteroaryl;

$W_1$, $W_2$, $W_3$, and $W_4$ are each independently selected from a bond, -O-, -S-, - $NR^{W1}$-, -Se-, or -C(O)-; in some embodiments, $W_1$, $W_2$, $W_3$, and $W_4$ are each independently selected from a bond, -O-, -S-, or -$NR^{W1}$-;

$R^{W1}$ is selected from H, halogen, or $C_{1-4}$ alkyl;

each $R^A$ is independently selected from halogen, =O, CN, COOH, $C_{1-6}$ alkyl, $C_{2-6}$ alkenyl, $C_{2-6}$ alkynyl, -O-$C_{1-4}$ alkyl, -$S(O)_2$-$C_{1-4}$ alkyl, -$S(O)_2$-$(CH_2)_{1-3}$-$R^a$, - P(O)-$(C_{1-4}$ alkyl$)_2$, -C(O)-O-$(CH_2)_p$-$R^a$, -$(CH_2)_{1-2}$-C(O)-$R^a$, -C(O)-$(CH_2)_{1-2}$-$R^a$, - C(O)-O-halo $C_{1-4}$ alkyl, -C(O)-(6- to 9-membered bicyclic heterocycloalkyl), -NH-C(O)-$(CH_2)_p$-$R^a$, -NH-$R^a$, -N($CH_3$)-C(O)-$(CH_2)_p$-$R^a$, -NH-$SO_2$-$(CH_2)_{1-2}$-$R^a$, -NH-C(O)-O-$R^a$, -C(O)-$(CH_2)_p$-(4- to 6-membered monocyclic heterocycloalkyl), -C(O)-$(CH_2)_p$-(4- to 6-membered monocyclic cycloalkyl), -NH-C(O)-NH-$R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), -C(O)-NH-$S(O)_2$-$C_{1-4}$ alkyl, -NH-C(O)deuterated $C_{1-2}$ alkyl, -NH-C(O)-NH-$C_{1-2}$ alkyl or -NH-C(O)-$C_{1-2}$ alkyl, wherein the alkyl, alkenyl, alkynyl, cycloalkyl, and heterocycloalkyl are optionally further substituted with 1-4 groups selected from halogen, OH, $NH_2$, CN, -$S(O)_2$-$CH_3$, -O-halo $C_{1-4}$ alkyl, or -NH-$S(O)_2$-$CH_3$; in some embodiments, each $R^A$ is independently selected from halogen, =O, CN, COOH, $C_{1-6}$ alkyl, $C_{2-6}$ alkenyl, $C_{2-6}$ alkynyl, -O-$C_{1-4}$ alkyl, -$S(O)_2$-$C_{1-4}$ alkyl, -$S(O)_2$-$(CH_2)_{1-3}$-$R^a$, -P(O)-$(C_{1-4}$ alkyl$)_2$, - C(O)-O-$(CH_2)_p$-$R^a$, -$(CH_2)_{1-2}$-C(O)-$R^a$, -C(O)-$(CH_2)_{1-2}$-$R^a$, -C(O)-O-halo $C_{1-4}$ alkyl, -C(O)-(6- to 9-membered bicyclic heterocycloalkyl), -NH-C(O)-$(CH_2)_p$-$R^a$, - N($CH_3$)-C(O)-$(CH_2)_p$-$R^a$, -NH-$SO_2$-$(CH_2)_{1-2}$-$R^a$, -NH-C(O)-O-$R^a$,

$-C(O)-(CH_2)_p-$(4-to 6-membered monocyclic heterocycloalkyl), $-C(O)-(CH_2)_p-$(4- to 6-membered monocyclic cycloalkyl), $-NH-C(O)-NH-R^a$, -C(O)-deuterated $C_{1-4}$ alkyl or -C(O)-NH-(4- to 6-membered monocyclic cycloalkyl), -C(O)-NH-(4- to 6-membered monocyclic heterocycloalkyl), $-C(O)-NH-S(O)_2-C_{1-4}$ alkyl, -NH-C(O)deuterated $C_{1-2}$ alkyl, $-NH-C(O)-NH-C_{1-2}$ alkyl or $-NH-C(O)-C_{1-2}$ alkyl, wherein the alkyl, alkenyl, alkynyl, cycloalkyl, and heterocycloalkyl are optionally further substituted with 1-4 groups selected from halogen, OH, $NH_2$, CN, $-S(O)_2-CH_3$, -O-halo $C_{1-4}$ alkyl, or - $NH-S(O)_2-CH_3$; in some embodiments, each $R^A$ is independently selected from halogen, CN, COOH, $C_{2-6}$ alkenyl, $C_{2-6}$ alkynyl, $-OC_{1-4}$ alkyl, $-S(O)_2-C_{1-4}$ alkyl, - $S(O)_2-(CH_2)_{1-3}-R^a$, $-P(O)-(C_{1-4}$ alkyl)2, $-C(O)-O-(CH_2)_p-R^a$, $-(CH_2)_{1-2}-C(O)-R^a$, - $C(O)-(CH_2)_{1-2}-R^a$, -C(O)-O-halo $C_{1-4}$ alkyl, -C(O)-6- to 8-membered bicyclic heterocycloalkyl, $-NH-C(O)-(CH_2)_p-R^a$, $-N(CH_3)-C(O)-(CH_2)_p-R^a$, or $-NH-SO_2-(CH_2)_{1-2}-R^a$, wherein the alkyl, alkenyl, alkynyl, and heterocycloalkyl are optionally further substituted with 1-4 groups selected from halogen, OH, $NH_2$, CN, $-S(O)_2-CH_3$, or -O-halo $C_{1-4}$ alkyl; in some embodiments, each $R^A$ is independently selected from halogen, CN, COOH, $C_{2-6}$ alkenyl, $C_{2-6}$ alkynyl, $-OC_{1-4}$ alkyl, $-S(O)_2-C_{1-4}$ alkyl, $-S(O)_2-(CH_2)_{1-3}-R^a$, $-P(O)-(C_{1-4}$ alkyl$)_2$, $-C(O)-O-(CH_2)_p-R^a$, $-(CH_2)_{1-2}-C(O)-R^a$, - $C(O)-(CH_2)_{1-2}-R^a$, -C(O)-O-halo $C_{1-4}$ alkyl, $-NH-C(O)-(CH_2)_p-R^a$, or $-NH-SO_2-(CH_2)_{1-2}-R^a$, wherein the alkyl, alkenyl, and alkynyl are optionally further substituted with 1-4 groups selected from halogen, OH, $NH_2$, CN, or -O-halo $C_{1-4}$ alkyl; in some embodiments, each $R^A$ is independently selected from halogen, CN, COOH, $C_{2-6}$ alkenyl, $C_{2-6}$ alkynyl, $-OC_{1-4}$ alkyl, $-S(O)_2-C_{1-4}$ alkyl, $-S(O)_2-(CH_2)_{1-3}-R^a$, $-P(O)-(C_{1-4}$ alkyl$)_2$, $-C(O)-O-(CH_2)_p-R^a$, $-(CH_2)_{1-2}-C(O)-R^a$, $-C(O)-(CH_2)_{1-2}-R^a$, or -C(O)-O-halo $C_{1-4}$ alkyl, wherein the alkyl, alkenyl, and alkynyl are optionally further substituted with 1-4 groups selected from halogen, OH, $NH_2$, CN, or -O-halo $C_{1-4}$ alkyl;

$R^a$ is selected from deuterated $C_{1-4}$ alkyl, $C_{2-4}$ alkynyl, 3- to 5-membered cycloalkyl, 3- to 6-membered heterocycloalkyl, or 5- to 6-membered heteroaryl, wherein the alkynyl, cycloalkyl, heterocycloalkyl, and heteroaryl are optionally further substituted with 1-4 groups selected from halogen, OH, =O, $C_{1-4}$ alkyl, deuterated $C_{1-4}$ alkyl, $C_{1-4}$ alkoxy, $C_{2-4}$ alkynyl, -O-halo $C_{1-4}$ alkyl, $-S(O)_2-CH_3$ or $=CH_2$; in some embodiments, $R^a$ is selected from deuterated $C_{1-4}$ alkyl, $C_{2-4}$ alkynyl, 3- to 5-membered cycloalkyl, 3- to 6-membered heterocycloalkyl, or 5- to 6-membered heteroaryl, wherein the alkynyl, cycloalkyl, heterocycloalkyl, and heteroaryl are optionally further substituted with 1-4 groups selected from halogen, OH, $C_{1-4}$ alkyl, $C_{1-4}$ alkoxy, $C_{2-4}$ alkynyl, -O-halo $C_{1-4}$ alkyl, or $-S(O)_2-CH_3$; in some embodiments, $R^a$ is selected from $C_{2-4}$ alkynyl, 3- to 5-membered cycloalkyl, 3- to 6-membered heterocycloalkyl, or 5- to 6-membered heteroaryl, wherein the alkynyl, cycloalkyl, heterocycloalkyl, and heteroaryl are optionally further substituted with 1-4 groups selected from halogen, OH, $C_{1-4}$ alkyl, $C_{1-4}$ alkoxy, $C_{2-4}$ alkynyl, -O-halo $C_{1-4}$ alkyl, or $-S(O)_2-CH_3$; in some embodiments, $R^a$ is selected from $C_{2-4}$ alkynyl, 3- to 5-membered cycloalkyl, 3- to 6-membered heterocycloalkyl, or 5- to 6-membered heteroaryl, wherein the alkynyl, cycloalkyl, heterocycloalkyl, and heteroaryl are optionally further substituted with 1-4 groups selected from halogen, OH, $C_{1-4}$ alkyl, $C_{2-4}$ alkynyl, -O-halo $C_{1-4}$ alkyl, or $-S(O)_2-CH_3$;

each $R^C$ is independently selected from H, CN, halogen, OH, $C_{1-4}$ alkyl, halo $C_{1-4}$ alkyl, -O-halo $C_{1-4}$ alkyl, -Se-halo $C_{1-4}$ alkyl, $C_{2-4}$ alkenyl, $C_{2-4}$ alkynyl, =O, $C_{3-6}$ cycloalkyl, $-SCF_3$, $-SF_5$, $-(NH)_q-P(O)(C_{1-4}$ alkyl$)_2$, $-(CH_2)_p-O-C_{1-4}$ alkyl, $-O-(CH_2)_p-C_{3-6}$ cycloalkyl or $-(CH_2)_p-O-C_{3-6}$ cycloalkyl, or any two $R^C$, together with the atom to which they are connected, form 3- to 8-membered cycloalkyl; in some embodiments, each $R^C$ is independently selected from H, halogen, OH, $C_{1-4}$ alkyl, halo $C_{1-4}$ alkyl, -O-halo $C_{1-4}$ alkyl, $C_{2-4}$ alkenyl, $C_{2-4}$ alkynyl, =O, $C_{3-6}$ cycloalkyl, - $SCF_3$, $-SF_5$, $-(NH)_q-P(O)(C_{1-4}$ alkyl$)_2$, $-(CH_2)_p-O-C_{1-4}$ alkyl, $-O-(CH_2)_p-C_{3-6}$ cycloalkyl or $-(CH_2)_p-O-C_{3-6}$ cycloalkyl, or any two $R^C$ together with the atom to which they are connected, form 3- to 8-membered cycloalkyl; in some embodiments, each $R^C$ is independently selected from H, halogen, OH, $C_{1-4}$ alkyl, halo $C_{1-4}$ alkyl, -O-halo $C_{1-4}$ alkyl, $C_{2-4}$ alkenyl, $C_{2-4}$ alkynyl, =O, $C_{3-6}$ cycloalkyl, $-SCF_3$, $-SF_5$, - $(NH)_q-P(O)(C_{1-4}$ alkyl$)_2$, $-(CH_2)_p-O-C_{1-4}$ alkyl, or $-(CH_2)_p-O-C_{3-6}$ cycloalkyl, or any two $R^C$, together with the atom to which they are connected, form 3- to 8-membered cycloalkyl;

q is selected from 0 or 1;

each p is independently selected from 0, 1, 2, 3, or 4;

m is selected from 1, 2, 3, or 4;

n is selected from 1, 2, 3, or 4;

provided that

(1) when ring A is

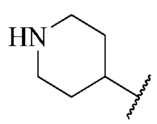

,

$R^A$ is not $-S(O)_2-(CH_2)_{0-1}CH_3$, and

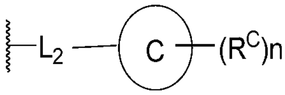

is not

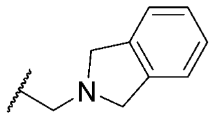 and 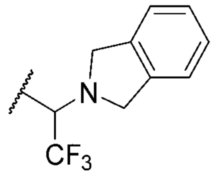 ;

(2) when $R^A$ is $-S(O)_2-(CH_2)_{0-1}CH_3$, ring A is not

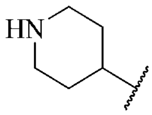 and 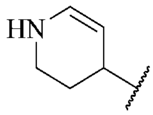 ,

and

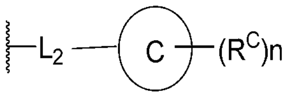

is not

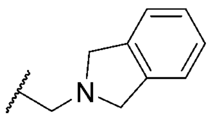 and 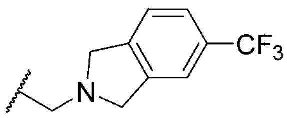 ;

(3) when ring C is

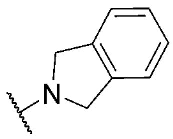 ,

$R^C$ is not H, and

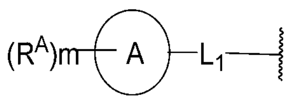

is not

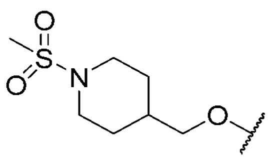 , 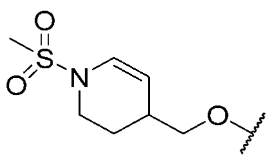 , 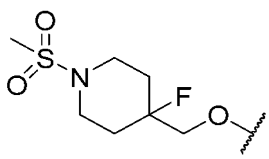

and

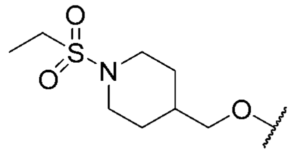;

(4) when $R^C$ is H, ring C is not

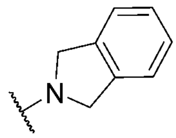, and 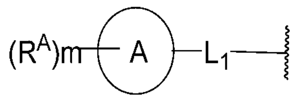

is not

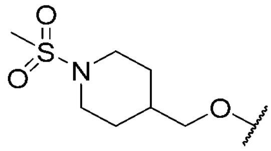 and 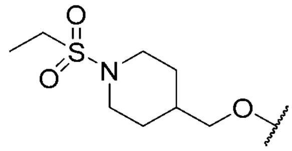.

More specifically, as a second technical solution of the present invention, provided is a compound as represented by formula (I), or a stereoisomer, tautomer, deuterated compound, solvate, co-crystal or pharmaceutically acceptable salt, wherein

ring A is selected from 4- to 8-membered cycloalkyl, 4- to 7-membered monocyclic heterocycloalkyl, 5- to 6-membered heterocycloalkyl fused 5- to 6-membered heterocycloalkyl, 4- to 6-membered heterocycloalkyl spiro 5- to 6-membered heterocycloalkyl, 5- to 6-membered heterocycloalkyl spiro 4- to 6-memberedcycloalkyl, 5- to 6-membered heterocycloalkyl spiro 5- to 6-membered cycloalkyl, 5- to 6-membered heterocycloalkyl spiro 3- to 6-membered cycloalkyl, 10- to 14-membered partially unsaturated tricyclic heterocycloalkyl, or 7- to 8-membered bicyclic bridged heterocyclyl; in some embodiments, ring A is selected from 5- to 8-membered cycloalkyl, 4- to 7-membered monocyclic heterocycloalkyl, 5- to 6-membered heterocycloalkyl fused 5- to 6-membered heterocycloalkyl, 4- to 6-membered heterocycloalkyl spiro 5- to 6-membered heterocycloalkyl, 5- to 6-membered heterocycloalkyl spiro 4- to 6-memberedcycloalkyl, 5- to 6-membered heterocycloalkyl spiro 5- to 6-membered cycloalkyl, 5- to 6-membered heterocycloalkyl fused 3- to 6-membered cycloalkyl, 10- to 14-membered partially unsaturated tricyclic heterocycloalkyl, or 7- to 8-membered bicyclic bridged heterocyclyl; in some embodiments, ring A is selected from 5- to 8-membered cycloalkyl, 4- to 7-membered monocyclic heterocycloalkyl, 5- to 6-membered heterocycloalkyl fused 5- to 6-membered heterocycloalkyl, 4- to 6-membered heterocycloalkyl spiro 5- to 6-membered heterocycloalkyl, 5- to 6-membered heterocycloalkyl spiro 5- to 6-membered cycloalkyl, 5- to 6-membered heterocycloalkyl fused 5- to 6-membered cycloalkyl, 10- to 14-membered partially unsaturated tricyclic heterocycloalkyl, or 5- to 6-membered heterocycloalkyl spiro 4- to 6-membered cycloalkyl; in some embodiments, ring A is selected from 5- to 8-membered cycloalkyl, 4- to 7-membered monocyclic heterocycloalkyl, 5- to 6-membered heterocycloalkyl fused 5- to 6-membered heterocycloalkyl, 4- to 6-membered heterocycloalkyl spiro 5- to 6-membered heterocycloalkyl, 5- to 6-membered heterocycloalkyl spiro 5- to 6-membered cycloalkyl, 5- to 6-membered heterocycloalkyl fused 5- to 6-membered cycloalkyl, or 10- to 14-membered partially unsaturated tricyclic heterocycloalkyl;

ring C is selected from phenyl, 5-membered heteroaryl, 6-membered heteroaryl, 5- to 7-membered heterocycloalkyl fused phenyl, 5- to 6-membered carbocyclyl fused phenyl, 5- to 6-membered heterocycloalkyl fused 5- to 6-membered heteroaryl, 5- to 6-membered heterocycloalkyl fused 3- to 6-membered cycloalkyl, or 9- to 12-membered tricyclic heterocycloalkyl;

$R^{La}$ and $R^{Lb}$ are each independently selected from a bond, $C_{1-2}$ alkylene, $C_{2-4}$ alkenylene, or $C_{2-4}$ alkynylene, wherein the alkylene and alkenylene are optionally further substituted with 1-4 $R^{L1}$;

each $R^{L1}$ is independently selected from halogen, =O, $C_{1-2}$ alkyl, $C_{2-4}$ alkenyl, $C_{1-2}$ alkoxy, or 3- to 6-membered cycloalkyl, wherein the alkyl, alkoxy, and cycloalkyl are optionally further substituted with 1-4 substituents selected from halogen, CN, OH and $NH_2$, or two $R^{L1}$ on the same carbon atom or two adjacent carbon atoms, together with the atom to which they are connected, form 3- to 6-membered cycloalkyl; in some embodiments, each $R^{L1}$ is independently selected from halogen, $C_{1-2}$ alkyl, $C_{2-4}$ alkenyl, $C_{1-2}$ alkoxy, or 3- to 6-membered cycloalkyl, wherein the alkyl, alkoxy, and cycloalkyl are optionally further substituted with 1-4 substituents selected from halogen, CN, OH and $NH_2$,

or two $R^{L1}$ on the same carbon atom or two adjacent carbon atoms, together with the atom to which they are connected, form 3- to 6-membered cycloalkyl; in some embodiments, each $R^{L1}$ is independently selected from halogen, =O, $C_{1-2}$ alkyl, $C_{2-4}$ alkenyl, $C_{1-2}$ alkoxy, or 3-to 6-membered cycloalkyl, wherein the alkyl, alkoxy, and cycloalkyl are optionally further substituted with 1-4 substituents selected from halogen, CN, OH and $NH_2$, or two adjacent $R^{L1}$, together with the atom to which they are connected, form 3- to 6-membered cycloalkyl;

$R^{W1}$ is selected from H, halogen, or $C_{1-2}$ alkyl;

each $R^A$ is independently selected from halogen, =O, CN, COOH, $C_{1-4}$ alkyl, $C_{2-4}$ alkenyl, $C_{2-4}$ alkynyl, -$OC_{1-4}$ alkyl, -$S(O)_2$-$C_{1-4}$ alkyl, -$S(O)_2$-$(CH_2)_{1-3}$-$R^a$, -P(O)-($C_{1-4}$ alkyl)$_2$, -C(O)-O-$(CH_2)_p$-$R^a$, -$(CH_2)_{1-2}$-C(O)-$R^a$, -C(O)-$(CH_2)_{1-2}$-$R^a$, -NH-$R^a$, - C(O)-O-halo $C_{1-4}$ alkyl, -C(O)-(6- to 9-membered bicyclic heterocycloalkyl), -NH-C(O)-$(CH_2)_p$-$R^a$, -N($CH_3$)-C(O)-$(CH_2)_p$-$R^a$, -NH-$SO_2$-$(CH_2)_{1-2}$-$R^a$, -NH-C(O)-O-$R^a$, -C(O)-$(CH_2)_p$-(4- to 6-membered monocyclic heterocycloalkyl), -C(O)-$(CH_2)_p$-(4-to 6-membered monocyclic cycloalkyl), -NH-C(O)-NH-$R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), -C(O)-NH-$S(O)_2$-$C_{1-4}$ alkyl, -NH-C(O)deuterated $C_{1-2}$ alkyl, -NH-C(O)-NH-$C_{1-2}$ alkyl or -NH-C(O)-$C_{1-2}$ alkyl, wherein the alkyl, alkenyl, alkynyl, cycloalkyl, and heterocycloalkyl are optionally further substituted with 1-4 groups selected from halogen, OH, $NH_2$, CN, -$S(O)_2$-$CH_3$, or -NH-$S(O)_2$-$CH_3$; in some embodiments, each $R^A$ is independently selected from halogen, =O, CN, COOH, $C_{1-4}$ alkyl, $C_{2-4}$ alkenyl, $C_{2-4}$ alkynyl, -$OC_{1-4}$ alkyl, - $S(O)_2$-$C_{1-4}$ alkyl, -$S(O)_2$-$(CH_2)_{1-3}$-$R^a$, -P(O)-($C_{1-4}$ alkyl)$_2$, -C(O)-O-$(CH_2)_p$-$R^a$, - $(CH_2)_{1-2}$-C(O)-$R^a$, -C(O)-$(CH_2)_{1-2}$-$R^a$, -C(O)-O-halo $C_{1-4}$ alkyl, -C(O)-(6- to 9-membered bicyclic heterocycloalkyl), -NH-C(O)-$(CH_2)_p$-$R^a$, -N($CH_3$)-C(O)-$(CH_2)_p$-$R^a$, -NH-$SO_2$-$(CH_2)_{1-2}$-$R^a$, -NH-C(O)-O-$R^a$, -C(O)-$(CH_2)_p$-(4- to 6-membered monocyclic heterocycloalkyl), -C(O)-$(CH_2)_p$-(4- to 6-membered monocyclic cycloalkyl), -NH-C(O)-NH-$R^a$, -C(O)-deuterated $C_{1-4}$ alkyl, or -C(O)-NH-(4- to 6-membered monocyclic cycloalkyl), -C(O)-NH-(4- to 6-membered monocyclic heterocycloalkyl), -C(O)-NH-$S(O)_2$-$C_{1-4}$ alkyl, -NH-C(O)deuterated $C_{1-2}$ alkyl, - NH-C(O)-NH-$C_{1-2}$ alkyl or -NH-C(O)-$C_{1-2}$ alkyl, wherein the alkyl, alkenyl, alkynyl, cycloalkyl, and heterocycloalkyl are optionally further substituted with 1-4 groups selected from halogen, OH, $NH_2$, CN, -$S(O)_2$-$CH_3$, or -NH-$S(O)_2$-$CH_3$; in some embodiments, each $R^A$ is independently selected from halogen, CN, COOH, $C_{2-4}$ alkenyl, $C_{2-4}$ alkynyl, -$OC_{1-4}$ alkyl, -$S(O)_2$-$C_{1-4}$ alkyl, -$S(O)_2$-$(CH_2)_{1-3}$-$R^a$, -P(O)-($C_{1-4}$ alkyl)$_2$, -C(O)-O-$(CH_2)_p$-$R^a$, -$(CH_2)_{1-2}$-C(O)-$R^a$, -C(O)-$(CH_2)_{1-2}$-$R^a$, -C(O)-O-halo $C_{1-4}$ alkyl, -C(O)-6- to 8-membered bicyclic heterocycloalkyl, -NH-C(O)-$(CH_2)_p$-$R^a$, -N($CH_3$)-C(O)-$(CH_2)_p$-$R^a$, or -NH-$SO_2$-$(CH_2)_{1-2}$-$R^a$, wherein the alkyl, alkenyl, alkynyl, and heterocycloalkyl are optionally further substituted with 1-4 groups selected from halogen, OH, $NH_2$, CN, or -$S(O)_2$-$CH_3$; in some embodiments, each $R^A$ is independently selected from halogen, CN, COOH, $C_{2-4}$ alkenyl, $C_{2-4}$ alkynyl, -$OC_{1-4}$ alkyl, -$S(O)_2$-$C_{1-4}$ alkyl, -$S(O)_2$-$(CH_2)_{1-3}$-$R^a$, -P(O)-($C_{1-4}$ alkyl)$_2$, -C(O)-O-$(CH_2)_p$-$R^a$, -$(CH_2)_{1-2}$-C(O)-$R^a$, -C(O)-$(CH_2)_{1-2}$-$R^a$, -C(O)-O-halo $C_{1-4}$ alkyl, -NH-C(O)-$(CH_2)_p$-$R^a$, or -NH-$SO_2$-$(CH_2)_{1-2}$-$R^a$, wherein the alkyl, alkenyl, and alkynyl are optionally further substituted with 1-4 groups selected from halogen, OH, $NH_2$, or CN; in some embodiments, each $R^A$ is independently selected from halogen, CN, COOH, $C_{2-4}$ alkenyl, $C_{2-4}$ alkynyl, -$OC_{1-4}$ alkyl, -$S(O)_2$-$C_{1-4}$ alkyl, -$S(O)_2$-$(CH_2)_{1-3}$-$R^a$, -P(O)-($C_{1-4}$ alkyl)$_2$, -C(O)-O-$(CH_2)_p$-$R^a$, -$(CH_2)_{1-2}$-C(O)-$R^a$, -C(O)-$(CH_2)_{1-2}$-$R^a$, or -C(O)-O-halo $C_{1-4}$ alkyl, wherein the alkyl, alkenyl, and alkynyl are optionally further substituted with 1-4 groups selected from halogen, OH, $NH_2$, or CN;

$R^a$ is selected from deuterated $C_{1-2}$ alkyl, $C_{2-4}$ alkynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, $C_{1-2}$ alkyl, OH, =O, -$S(O)_2$-$CH_3$, $C_{1-2}$ alkoxy, deuterated $C_{1-2}$ alkyl or =$CH_2$; in some embodiments, $R^a$ is selected from deuterated $C_{1-2}$ alkyl, $C_{2-4}$ alkynyl, 3- to 5-membered cycloalkyl, 4-to 6-membered heterocycloalkyl, or 5-membered heteroaryl, wherein the cycloalkyl, heterocycloalkyl, and heteroaryl are optionally further substituted with groups selected from halogen, $C_{1-2}$ alkyl, OH, -$S(O)_2$-$CH_3$, or $C_{1-2}$ alkoxy; in some embodiments, $R^a$ is selected from $C_{2-4}$ alkynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, $C_{1-2}$ alkyl, OH, -$S(O)_2$-$CH_3$, or $C_{1-2}$ alkoxy; in some embodiments, $R^a$ is selected from $C_{2-4}$ alkynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, $C_{1-2}$ alkyl, OH, or -$S(O)_2$-$CH_3$;

each $R^C$ is independently selected from H, CN, halogen, OH, $C_{1-4}$ alkyl, halo $C_{1-4}$ alkyl, -O-halo $C_{1-4}$ alkyl, -Se-halo $C_{1-2}$ alkyl, $C_{2-4}$ alkenyl, $C_{2-4}$ alkynyl, =O, $C_{3-6}$ cycloalkyl, -$SCF_3$, - $SF_5$, -$(NH)_q$-P(O)($C_{1-4}$ alkyl)$_2$, -$(CH_2)_p$-O-$C_{1-4}$ alkyl, -O-$(CH_2)_p$-$C_{3-6}$ cycloalkyl or -$(CH_2)_p$-O-$C_{3-6}$ cycloalkyl, or any two $R^C$, together with the atom to which they are connected, form 3- to 6-membered cycloalkyl; in some embodiments, each $R^C$ is independently selected from H, halogen, OH, $C_{1-4}$ alkyl, halo $C_{1-4}$ alkyl, -O-halo $C_{1-4}$ alkyl, $C_{2-4}$ alkenyl, $C_{2-4}$ alkynyl, =O, $C_{3-6}$ cycloalkyl, - $SCF_3$, - $SF_5$, -$(NH)_q$-P(O)($C_{1-4}$ alkyl)$_2$, -$(CH_2)_p$-O-$C_{1-4}$ alkyl, -O-$(CH_2)_p$-$C_{3-6}$ cycloalkyl or -$(CH_2)_p$-O-$C_{3-6}$ cycloalkyl, or any two $R^C$ together with the atom to which they are connected, form 3- to 6-membered cycloalkyl; in some embodiments, each $R^C$ is independently selected from H, halogen, OH, $C_{1-4}$ alkyl, halo $C_{1-4}$ alkyl, -O-halo $C_{1-4}$ alkyl, $C_{2-4}$ alkenyl, $C_{2-4}$ alkynyl, =O, $C_{3-6}$ cycloalkyl, -$SCF_3$, - $SF_5$, - $(NH)_q$-P(O)($C_{1-4}$ alkyl)$_2$, -$(CH_2)_p$-O-$C_{1-4}$ alkyl, or -$(CH_2)_p$-O-$C_{3-6}$

cycloalkyl, or any two $R^C$, together with the atom to which they are connected, form 3- to 6-membered cycloalkyl;
each p is independently selected from 0, 1, 2, or 3;
m is selected from 1, 2, or 3;
n is selected from 1, 2, or 3;
the remaining groups are as described in the preceding first technical solution.

More specifically, as a third technical solution of the present invention, provided is a compound as represented by formula (I), or a stereoisomer, tautomer, deuterated compound, solvate, co-crystal or pharmaceutically acceptable salt, wherein

ring A is selected from 4- to 6-membered cycloalkyl, 4- to 7-membered monocyclic heterocycloalkyl, 5-membered heterocycloalkyl fused 5-membered heterocycloalkyl, 5-membered heterocycloalkyl fused 6-membered heterocycloalkyl, 5-membered heterocycloalkyl spiro 5-membered heterocycloalkyl, 5-membered heterocycloalkyl spiro 6-membered heterocycloalkyl, 4-membered heterocycloalkyl spiro 6-membered heterocycloalkyl, 4-membered cycloalkyl spiro 6-membered heterocycloalkyl, 5-membered cycloalkyl spiro 6-membered heterocycloalkyl, 5-membered heterocycloalkyl fused 5-membered cycloalkyl, 6-membered heterocycloalkyl fused 5-membered cycloalkyl, or 10- to 14-membered partially unsaturated tricyclic heterocycloalkyl; in some embodiments, ring A is selected from 5- to 6-membered cycloalkyl, 4- to 7-membered monocyclic heterocycloalkyl, 5-membered heterocycloalkyl fused 5-membered heterocycloalkyl, 5-membered heterocycloalkyl fused 6-membered heterocycloalkyl, 5-membered heterocycloalkyl spiro 5-membered heterocycloalkyl, 5-membered heterocycloalkyl spiro 6-membered heterocycloalkyl, 4-membered heterocycloalkyl spiro 6-membered heterocycloalkyl, 5-membered heterocycloalkyl fused 5-membered cycloalkyl, 6-membered heterocycloalkyl fused 5-membered cycloalkyl, 4-membered cycloalkyl spiro 6-membered heterocycloalkyl, 5-membered cycloalkyl spiro 6-membered heterocycloalkyl, or 10- to 14-membered partially unsaturated tricyclic heterocycloalkyl; in some embodiments, ring A is selected from 5- to 6-membered cycloalkyl, 4- to 7-membered monocyclic heterocycloalkyl, 5-membered heterocycloalkyl fused 5-membered heterocycloalkyl, 5-membered heterocycloalkyl fused 6-membered heterocycloalkyl, 5-membered heterocycloalkyl spiro 5-membered heterocycloalkyl, 5-membered heterocycloalkyl spiro 6-membered heterocycloalkyl, 4-membered heterocycloalkyl spiro 6-membered heterocycloalkyl, 5-membered heterocycloalkyl fused 5-membered cycloalkyl, or 6-membered heterocycloalkyl fused 5-membered cycloalkyl;
ring C is selected from 5- to 7-membered heterocycloalkyl fused phenyl, 5- to 6-membered heterocycloalkyl fused 5- to 6-membered heteroaryl, 5- to 6-membered heterocycloalkyl fused 3- to 6-membered cycloalkyl, or 9- to 12-membered tricyclic heterocycloalkyl; in some embodiments, ring C is selected from 5- to 7-membered heterocycloalkyl fused phenyl, 5- to 6-membered heterocycloalkyl fused 5- to 6-membered heteroaryl, or 5- to 6-membered heterocycloalkyl fused 3- to 6-membered cycloalkyl;
each $R^A$ is independently selected from halogen, =O, CN, COOH, $C_{1-4}$ alkyl, $C_{2-4}$ alkenyl, $C_{2-4}$ alkynyl, $-OC_{1-4}$ alkyl, $-S(O)_2-C_{1-4}$ alkyl, $-S(O)_2-(CH_2)_{1-3}-R^a$, $-C(O)-O-(CH_2)_p-R^a$, $-(CH_2)_{1-2}-C(O)-R^a$, $-C(O)-(CH_2)_{1-2}-R^a$, -C(O)-O-halo $C_{1-4}$ alkyl, $-NH-R^a$, -C(O)-(6- to 9-membered bicyclic heterocycloalkyl), $-N(CH_3)-C(O)-(CH_2)_p-R^a$, $-NH-C(O)-O-R^a$, $-C(O)-(CH_2)_p-$(4- to 6-membered monocyclic heterocycloalkyl), - $C(O)-(CH_2)_p-$(4- to 6-membered monocyclic cycloalkyl), $-NH-C(O)-NH-R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), $-C(O)-NH-S(O)_2-C_{1-4}$ alkyl, -NH-C(O)deuterated $C_{1-2}$ alkyl, $-NH-C(O)-NH-C_{1-2}$ alkyl or $-NH-C(O)-C_{1-2}$ alkyl, wherein the alkyl, alkenyl, alkynyl, cycloalkyl, and heterocycloalkyl are optionally further substituted with 1-4 groups selected from halogen, OH, $NH_2$, CN, $-S(O)_2-CH_3$, or $-NH-S(O)_2-CH_3$; in some embodiments, each $R^A$ is independently selected from halogen, =O, CN, COOH, $C_{1-4}$ alkyl, $C_{2-4}$ alkenyl, $C_{2-4}$ alkynyl, $-OC_{1-4}$ alkyl, - $S(O)_2-C_{1-4}$ alkyl, $-S(O)_2-(CH_2)_{1-3}-R^a$, $-C(O)-O-(CH_2)_p-R^a$, $-(CH_2)_{1-2}-C(O)-R^a$, - $C(O)-(CH_2)_{1-2}-R^a$, -C(O)-O-halo $C_{1-4}$ alkyl, -C(O)-(6- to 9-membered bicyclic heterocycloalkyl), $-N(CH_3)-C(O)-(CH_2)_p-R^a$, $-NH-C(O)-O-R^a$, $-C(O)-(CH_2)_p-$(4- to 6-membered monocyclic heterocycloalkyl), $-C(O)-(CH_2)_p-$(4- to 6-membered monocyclic cycloalkyl), $-NH-C(O)-NH-R^a$, -C(O)-deuterated $C_{1-4}$ alkyl or -C(O)-NH-(4- to 6-membered monocyclic cycloalkyl), -C(O)-NH-(4- to 6-membered monocyclic heterocycloalkyl), $-C(O)-NH-S(O)_2-C_{1-4}$ alkyl, -NH-C(O)deuterated $C_{1-2}$ alkyl, $-NH-C(O)-NH-C_{1-2}$ alkyl or $-NH-C(O)-C_{1-2}$ alkyl, wherein the alkyl, alkenyl, alkynyl, cycloalkyl, and heterocycloalkyl are optionally further substituted with 1-4 groups selected from halogen, OH, $NH_2$, CN, $-S(O)_2-CH_3$, or $-NH-S(O)_2-CH_3$; in some embodiments, each $R^A$ is independently selected from halogen, CN, COOH, $C_{2-4}$ alkenyl, $C_{2-4}$ alkynyl, $-OC_{1-4}$ alkyl, $-S(O)_2-C_{1-4}$ alkyl, $-S(O)_2-(CH_2)_{1-3}-R^a$, $-C(O)-O-(CH_2)_p-R^a$, $-(CH_2)_{1-2}-C(O)-R^a$, $-C(O)-(CH_2)_{1-2}-R^a$, -C(O)-O-halo $C_{1-4}$ alkyl, - C(O)-6- to 8-membered bicyclic heterocycloalkyl, or $-N(CH_3)-C(O)-(CH_2)_p-R^a$, wherein the alkyl, alkenyl, alkynyl, and heterocycloalkyl are optionally further substituted with 1-4 groups selected from halogen, OH, $NH_2$, CN, or $-S(O)_2-CH_3$; in some embodiments, each $R^A$ is independently selected from halogen, CN, COOH, $C_{2-4}$ alkenyl, $C_{2-4}$ alkynyl, $-OC_{1-4}$ alkyl, $-S(O)_2-C_{1-4}$ alkyl, $-S(O)_2-(CH_2)_{1-3}-R^a$, $-C(O)-O-(CH_2)_p-R^a$, $-(CH_2)_{1-2}-C(O)-R^a$, or $-C(O)-(CH_2)_{1-2}-R^a$, or is selected from -C(O)-O-halo $C_{1-4}$ alkyl, wherein the alkyl, alkenyl, and alkynyl are optionally further substituted with 1-4 groups selected from

halogen, OH, $NH_2$, or CN; in some embodiments, each $R^A$ is independently selected from halogen, CN, COOH, $C_{2-4}$ alkenyl, $C_{2-4}$ alkynyl, $-OC_{1-4}$ alkyl, $-S(O)_2-C_{1-4}$ alkyl, $-S(O)_2-(CH_2)_{1-3}-R^a$, $-C(O)-O-(CH_2)_p-R^a$, $-(CH_2)_{1-2}-C(O)-R^a$, or $-C(O)-(CH_2)_{1-2}-R^a$, wherein the alkyl, alkenyl, and alkynyl are optionally further substituted with 1-4 groups selected from halogen, OH, $NH_2$, or CN;

$R^a$ is selected from deuterated $C_{1-2}$ alkyl, ethynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, OH, =O, $C_{1-2}$ alkyl, deuterated $C_{1-2}$ alkyl, $C_{1-2}$ alkoxy, $-S(O)_2-CH_3$ or $=CH_2$; in some embodiments, $R^a$ is selected from deuterated $C_{1-2}$ alkyl, ethynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, OH, $C_{1-2}$ alkyl, $C_{1-2}$ alkoxy, or $-S(O)_2-CH_3$; in some embodiments, $R^a$ is selected from ethynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, OH, $C_{1-2}$ alkyl, $C_{1-2}$ alkoxy, or $-S(O)_2-CH_3$; in some embodiments, $R^a$ is selected from 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, OH, $C_{1-2}$ alkyl, or $-S(O)_2-CH_3$; in some embodiments, $R^a$ is selected from 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, OH, or $C_{1-2}$ alkyl;

each p is independently selected from 0, 1, or 2;

m is selected from 1, 2, or 3;

n is selected from 1, 2, or 3;

the remaining groups are as described in the preceding second technical solution.

More specifically, as a fourth technical solution of the present invention, provided is a compound as represented by formula (I), or a stereoisomer, tautomer, deuterated compound, solvate, co-crystal or pharmaceutically acceptable salt, wherein

ring A has one of the following structures:

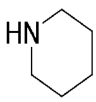 , 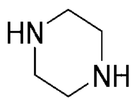 , 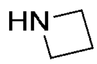 ,

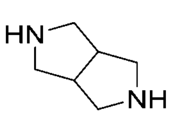 , 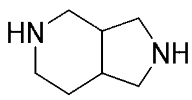 , 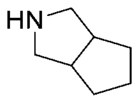 , 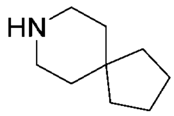 , 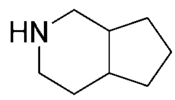 ,

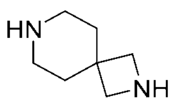 , 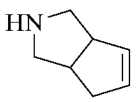 , 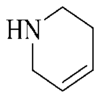 , 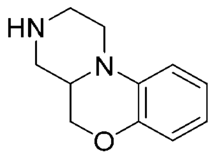 , or 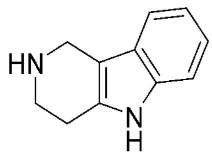 ,

or is selected from

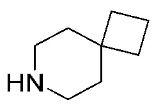 ,

or is selected from

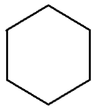, 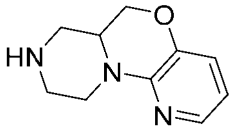,

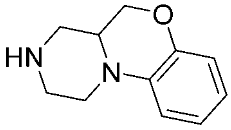, 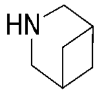, 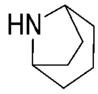, 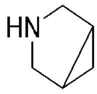, or 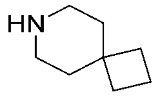;

or is selected from

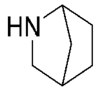;

or is selected from

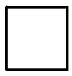, 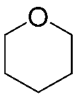, 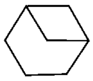, 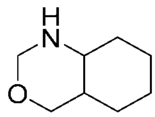, 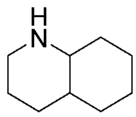,

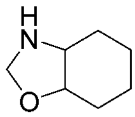, or 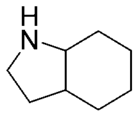;

or is selected from

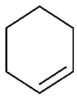;

ring C has one of the following structures:

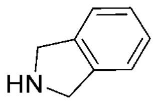, 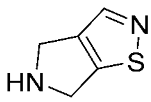,

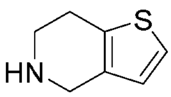, 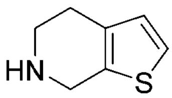, 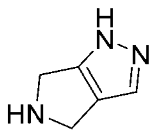, 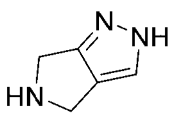, 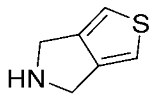, 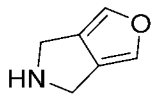,

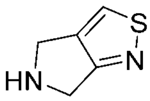, 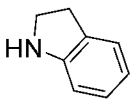, 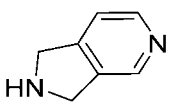, 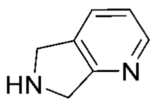, 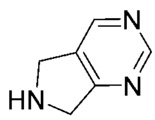, 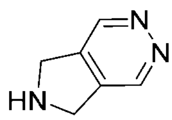,

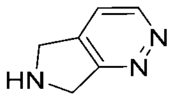, 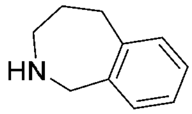, 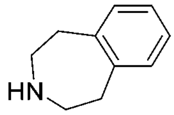, 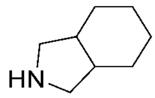, 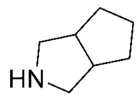,

or

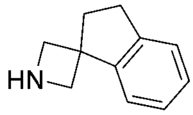,

or is selected from

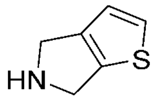;

or is selected from

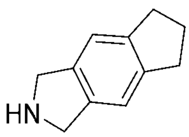

or

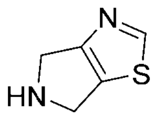;

or is selected from

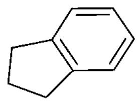;

$L_1$ is selected from a bond, -($C_{1-2}$ alkylene)-O-, -($C_{1-2}$ alkylene)-Se-, $C_{1-2}$ alkylene, -O-($C_{1-2}$ alkylene)-, $C_{2-4}$ alkenylene, -C(=O)-N($C_{1-2}$ alkylene)-, -O-, - C(=O)-O- or $C_{2-4}$ alkynylene, wherein the alkylene and alkenylene are optionally further substituted with 1-3 $R^{L1}$; in some embodiments, in some embodiments, $L_1$ is selected from a bond, -($C_{1-2}$ alkylene)-O-, -($C_{1-2}$ alkylene)-Se-, $C_{1-2}$ alkylene, -O-($C_{1-2}$ alkylene)-, $C_{2-4}$ alkenylene, -O- or $C_{2-4}$ alkynylene, wherein the alkylene and alkenylene are optionally further substituted with 1-3 $R^{L1}$; in some embodiments, $L_1$ is selected from a bond, -($C_{1-2}$ alkylene)-O-, $C_{1-2}$ alkylene, -O-($C_{1-2}$ alkylene)-, $C_{2-4}$ alkenylene, -C(=O)-N($C_{1-2}$ alkylene)-, -O-, or -C(=O)-O-, wherein the alkylene and alkenylene are optionally substituted with 1-3 $R^{L1}$;

$L_2$ is selected from a bond, $C_{1-2}$ alkylene, -C(=O)-, -NH-, or -O-, wherein the alkylene is optionally further substituted with 1-3 $R^{L1}$; in some embodiments, $L_2$ is selected from a bond, $C_{1-2}$ alkylene, -NH-, or -O-, wherein the alkylene is optionally further substituted with 1-3 $R^{L1}$;

each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl, or two $R^{L1}$ on the same carbon atom or two adjacent carbon atoms, together with the atom to which they are connected, form 3- to 6-membered cycloalkyl; in some embodiments, each $R^{L1}$ is independently selected from halogen, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl, or two $R^{L1}$ on the same carbon atom or two adjacent carbon atoms, together with the atom to which they are connected, form 3- to 6-membered cycloalkyl; in some embodiments, each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered

cycloalkyl;

each $R^A$ is independently selected from =O, $C_{1-4}$ alkyl, -S(O)$_2$-$C_{1-4}$ alkyl, - S(O)$_2$-(CH$_2$)$_{1-3}$-$R^a$, -C(O)-O-(CH$_2$)$_p$-$R^a$, -(CH$_2$)$_{1-2}$-C(O)-$R^a$, -C(O)-(CH$_2$)$_{1-2}$-$R^a$, - C(O)-O-halo $C_{1-3}$ alkyl, -C(O)-(6- to 9-membered bicyclic heterocycloalkyl), -NH-C(O)-(CH$_2$)$_p$-$R^a$, -NH-$R^a$, -N(CH$_3$)-C(O)-(CH$_2$)$_p$-$R^a$, -NH-SO$_2$-(CH$_2$)$_{1-2}$-$R^a$, -NH-C(O)-O-$R^a$, -C(O)-(CH$_2$)$_p$-(4- to 6-membered monocyclic heterocycloalkyl), -C(O)-(CH$_2$)$_p$-(4- to 6-membered monocyclic cycloalkyl), -NH-C(O)-NH-$R^a$, -C(O)-deuterated $C_{1-2}$ alkyl, -C(O)-NH-(4- to 6-membered monocyclic cycloalkyl), -C(O)-NH-(4- to 6-membered monocyclic heterocycloalkyl), -C(O)-NH-S(O)$_2$-$C_{1-4}$ alkyl, -NH-C(O)deuterated $C_{1-2}$ alkyl, -NH-C(O)-NH-$C_{1-2}$ alkyl or -NH-C(O)-$C_{1-2}$ alkyl, wherein the alkyl, cycloalkyl, and heterocycloalkyl are optionally further substituted with 1-4 groups selected from halogen, OH, NH$_2$, CN, -S(O)$_2$-CH$_3$, or -NH-S(O)$_2$-CH$_3$; in some embodiments, each $R^A$ is independently selected from =O, $C_{1-4}$ alkyl, -S(O)$_2$-$C_{1-4}$ alkyl, -S(O)$_2$-(CH$_2$)$_{1-3}$-$R^a$, -C(O)-O-(CH$_2$)$_p$-$R^a$, -(CH$_2$)$_{1-2}$-C(O)-$R^a$, - C(O)-(CH$_2$)$_{1-2}$-$R^a$, -C(O)-O-halo $C_{1-3}$ alkyl, -C(O)-(6- to 9-membered bicyclic heterocycloalkyl), -NH-C(O)-(CH$_2$)$_p$-$R^a$, -N(CH$_3$)-C(O)-(CH$_2$)$_p$-$R^a$, -NH-SO$_2$-(CH$_2$)$_{1-2}$-$R^a$, -NH-C(O)-O-$R^a$, -C(O)-(CH$_2$)$_p$-(4- to 6-membered monocyclic heterocycloalkyl), -C(O)-(CH$_2$)$_p$-(4- to 6-membered monocyclic cycloalkyl), -NH-C(O)-NH-$R^a$, -C(O)-deuterated $C_{1-2}$ alkyl or -C(O)-NH-(4- to 6-membered monocyclic cycloalkyl), -C(O)-NH-(4- to 6-membered monocyclic heterocycloalkyl), -C(O)-NH-S(O)$_2$-$C_{1-4}$ alkyl, -NH-C(O)deuterated $C_{1-2}$ alkyl, - NH-C(O)-NH-$C_{1-2}$ alkyl or -NH-C(O)-$C_{1-2}$ alkyl, wherein the alkyl, cycloalkyl, and heterocycloalkyl are optionally further substituted with 1-4 groups selected from halogen, OH, NH$_2$, CN, -S(O)$_2$-CH$_3$, or -NH-S(O)$_2$-CH$_3$; in some embodiments, each $R^A$ is independently selected from -S(O)$_2$-$C_{1-4}$ alkyl, -S(O)$_2$-(CH$_2$)$_{1-3}$-$R^a$, -C(O)-O-(CH$_2$)$_p$-$R^a$, -(CH$_2$)$_{1-2}$-C(O)-$R^a$, -C(O)-(CH$_2$)$_{1-2}$-$R^a$, -C(O)-O-halo $C_{1-3}$ alkyl, - C(O)-6- to 8-membered bicyclic heterocycloalkyl, -NH-C(O)-(CH$_2$)$_p$-$R^a$, -N(CH$_3$)-C(O)-(CH$_2$)$_p$-$R^a$, or -NH-SO$_2$-(CH$_2$)$_{1-2}$-$R^a$, wherein the alkyl and heterocycloalkyl are optionally further substituted with 1-4 groups selected from halogen, OH, NH$_2$, CN, or -S(O)$_2$-CH$_3$; in some embodiments, each $R^A$ is independently selected from -S(O)$_2$-$C_{1-4}$ alkyl, -S(O)$_2$-(CH$_2$)$_{1-3}$-$R^a$, -C(O)-O-(CH$_2$)$_p$-$R^a$, -(CH$_2$)$_{1-2}$-C(O)-$R^a$, - C(O)-(CH$_2$)$_{1-2}$-$R^a$, -C(O)-O-halo $C_{1-3}$ alkyl, -NH-C(O)-(CH$_2$)$_p$-$R^a$, or -NH-SO$_2$-(CH$_2$)$_{1-2}$-$R^a$, wherein the alkyl is optionally further substituted with 1-4 groups selected from halogen, OH, NH$_2$, or CN; in some embodiments, each $R^A$ is independently selected from -S(O)$_2$-$C_{1-4}$ alkyl, -S(O)$_2$-(CH$_2$)$_{1-3}$-$R^a$, -C(O)-O-(CH$_2$)$_p$-$R^a$, -(CH$_2$)$_{1-2}$-C(O)-$R^a$, -C(O)-(CH$_2$)$_{1-2}$-$R^a$, or -C(O)-O-halo $C_{1-3}$ alkyl, wherein the alkyl is optionally further substituted with 1-4 groups selected from halogen, OH, NH$_2$, or CN; in some embodiments, each $R^A$ is independently selected from -S(O)$_2$-$C_{1-4}$ alkyl, -S(O)$_2$-(CH$_2$)$_{1-3}$-$R^a$, -C(O)-O-(CH$_2$)$_p$-$R^a$, -(CH$_2$)$_{1-2}$-C(O)-$R^a$, or -C(O)-(CH$_2$)$_{1-2}$-$R^a$, wherein the alkyl is optionally further substituted with 1-4 groups selected from halogen, OH, NH$_2$, or CN;

$R^a$ is selected from deuterated $C_{1-2}$ alkyl, ethynyl, 3- to 5-membered cycloalkyl, 4- to 6-membered heterocycloalkyl, or 5- to 6-membered heteroaryl, wherein the cycloalkyl, heterocycloalkyl, and heteroaryl are optionally further substituted with groups selected from halogen, methyl, deuterated methyl, methoxy, ethoxy, OH, =O, -S(O)$_2$-CH$_3$ or =CH$_2$; in some embodiments, $R^a$ is selected from deuterated $C_{1-2}$ alkyl, ethynyl, 3- to 5-membered cycloalkyl, 4- to 6-membered heterocycloalkyl, or 5- to 6-membered heteroaryl, wherein the cycloalkyl, heterocycloalkyl, and heteroaryl are optionally further substituted with groups selected from halogen, methyl, methoxy, ethoxy, OH, or -S(O)$_2$-CH$_3$; in some embodiments, $R^a$ is selected from ethynyl, 3-to 5-membered cycloalkyl, or 4- to 6-membered heterocycloalkyl, wherein the cycloalkyl and heterocycloalkyl are optionally further substituted with groups selected from halogen, methyl, methoxy, ethoxy, OH, or -S(O)$_2$-CH$_3$; in some embodiments, $R^a$ is selected from 3- to 5-membered cycloalkyl, or 4- to 6-membered heterocycloalkyl, wherein the cycloalkyl and heterocycloalkyl are optionally further substituted with groups selected from halogen, methyl, OH or -S(O)$_2$-CH$_3$; in some embodiments, $R^a$ is selected from 3- to 5-membered cycloalkyl, or 4- to 6-membered heterocycloalkyl, wherein the cycloalkyl and heterocycloalkyl are optionally further substituted with groups selected from halogen, methyl, or OH;

each $R^C$ is independently selected from H, CN, F, Cl, Br, OH, -SF$_5$, methyl, difluoromethyl, trifluoromethyl, ethyl, ethynyl, cyclopropyl, -O-cyclopropyl, -O-CH$_2$-cyclopropyl, cyclobutyl, -O-cyclobutyl, -O-CH$_2$-cyclobutyl, or -Se-trifluoromethyl, or any two $R^C$, together with the atom to which they are connected, form 3- to 6-membered cycloalkyl; in some embodiments, each $R^C$ is independently selected from H, F, Cl, Br, OH, -SF$_5$, methyl, difluoromethyl, trifluoromethyl, ethyl, ethynyl, cyclopropyl, -O-cyclopropyl, -O-CH$_2$-cyclopropyl, cyclobutyl, -O-cyclobutyl, or -O-CH$_2$-cyclobutyl, or any two $R^C$, together with the atom to which they are connected, form 3- to 6-membered cycloalkyl; in some embodiments, each $R^C$ is independently selected from H, F, Cl, Br, OH, methyl, ethyl, cyclopropyl, cyclobutyl, trifluoromethyl, or ethynyl, or any two $R^C$, together with the atom to which they are connected, form 3- to 6-membered cycloalkyl; in some embodiments, each $R^C$ is independently selected from H, F, Cl, Br, OH, methyl, ethyl, cyclopropyl, or cyclobutyl, or any two $R^C$, together with the atom to which they are connected, form 3- to 6-membered cycloalkyl;

each p is independently selected from 0, 1, or 2;

m is selected from 1 or 2;

n is selected from 1 or 2;

the remaining groups are as described in the preceding third technical solution.

More specifically, as a fifth technical solution of the present invention, provided is a compound as represented by

formula (I), or a stereoisomer, tautomer, deuterated compound, solvate, co-crystal or pharmaceutically acceptable salt, wherein

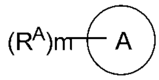

has one of the following structures:

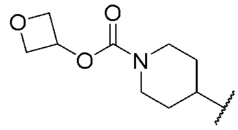,

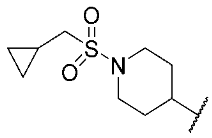, 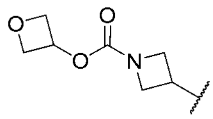, 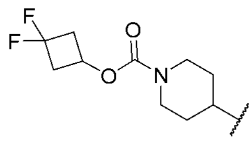, 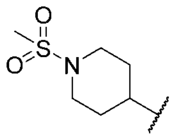,

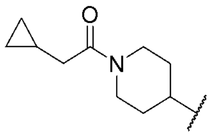, or 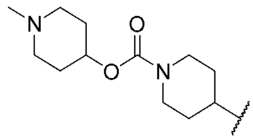,

or is selected from

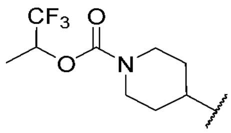,

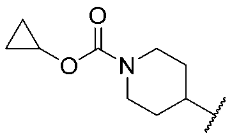, 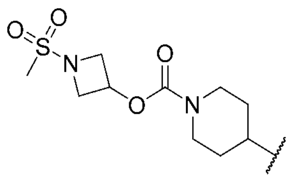, 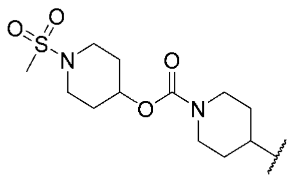,

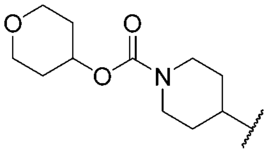, 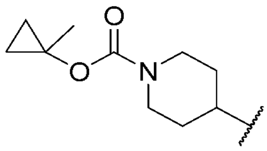, 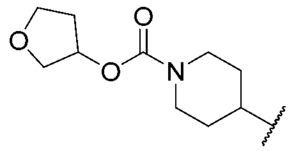, 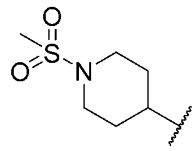,

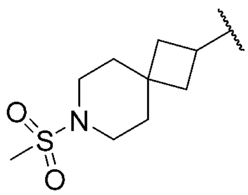, or 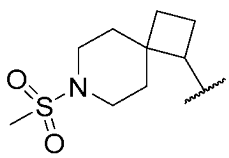,

or is selected from

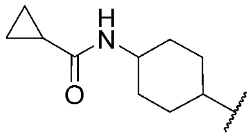,

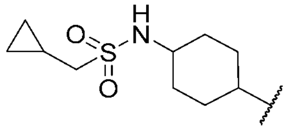, 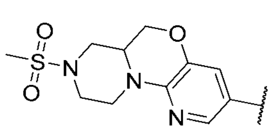, 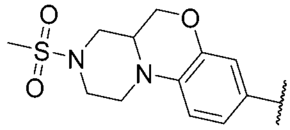,

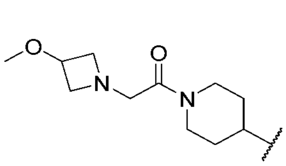, 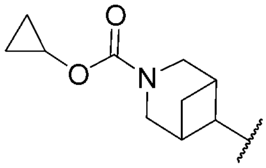, 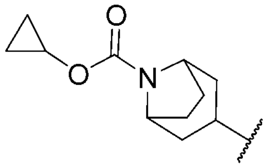,

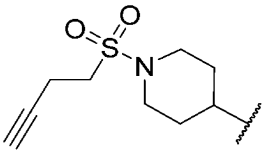, 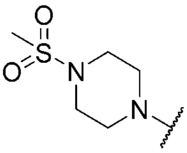, 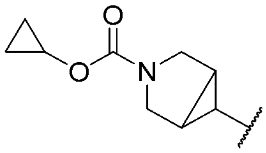,

or

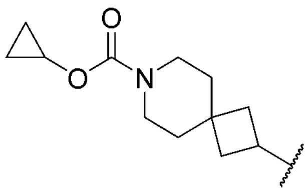;

or is selected from

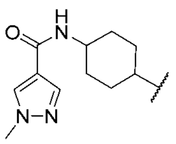, 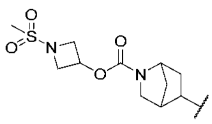,

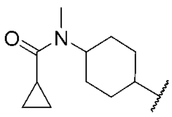, 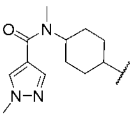, 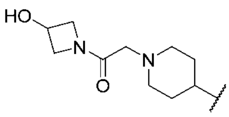, or 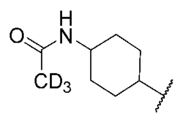;

or is selected from

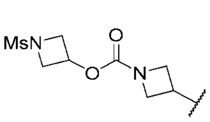, 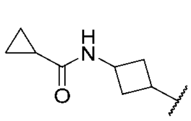, 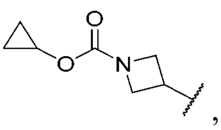, 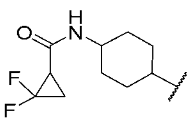, 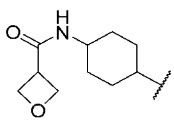,

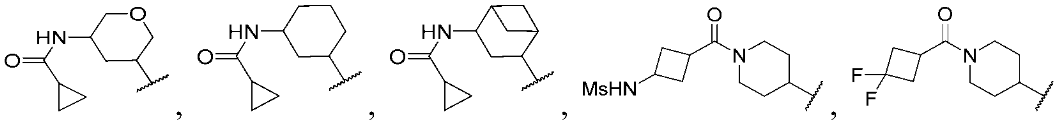 , , , , ,

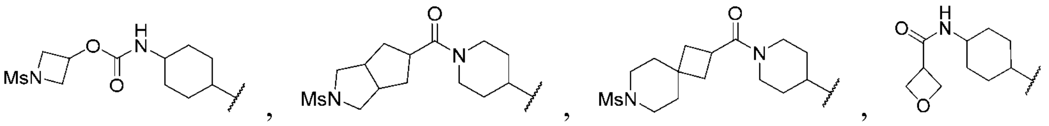 , , , ,

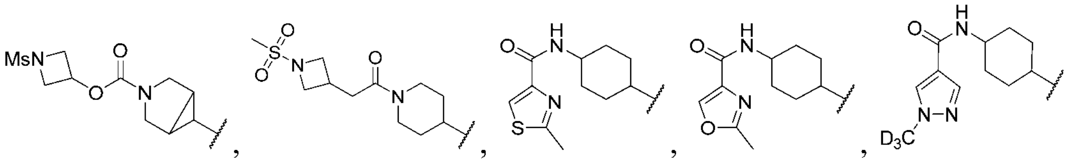 , , , , ,

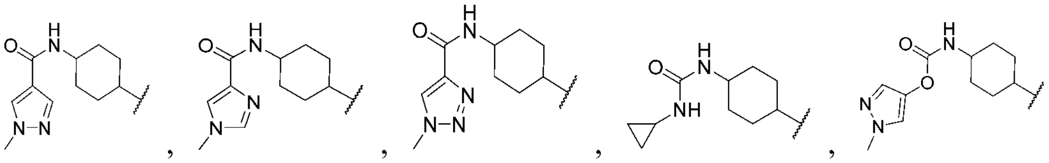 , , , , ,

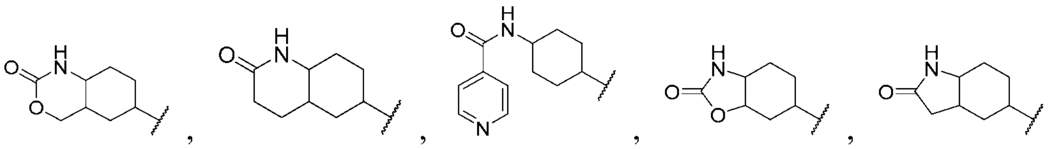 , , , , ,

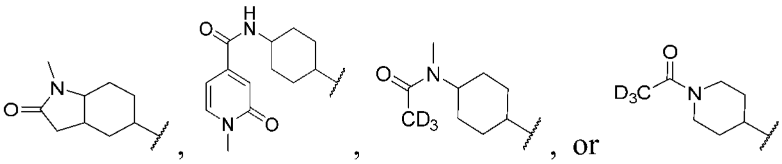 , , , or ;

or is selected from

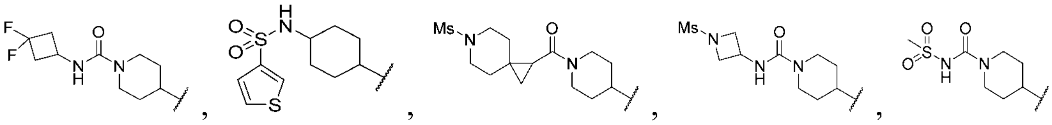 , , , , ,

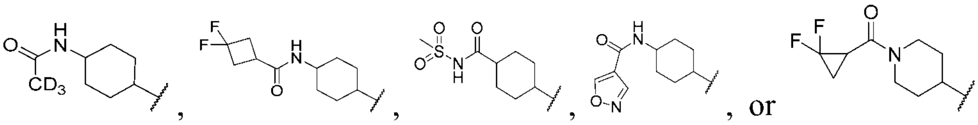 , , , , or ;

or is selected from

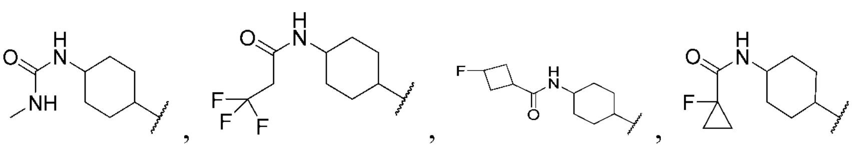 , , , ,

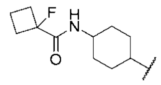, 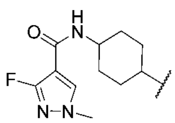, 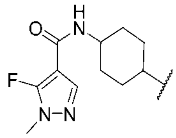;

or is selected from

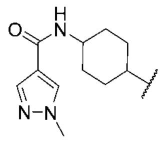,

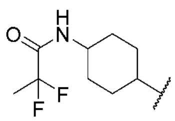, 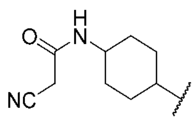, 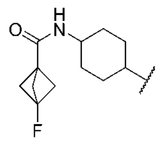, 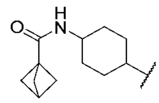, 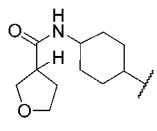, 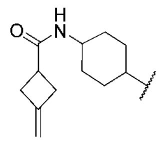,

or

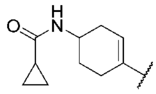;

or is selected from

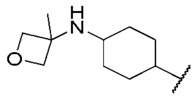 or 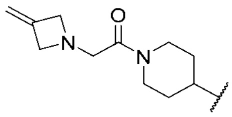;

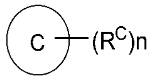

has one of the following structures:

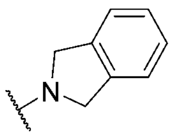, 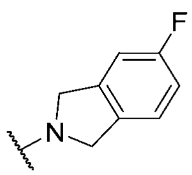,

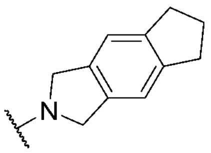, 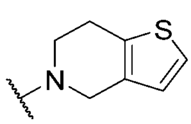, 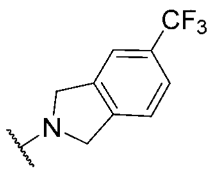, 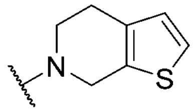,

or

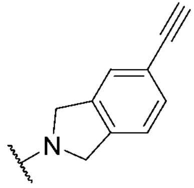,

or is selected from

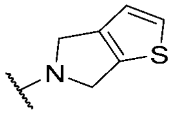, 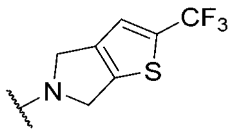, 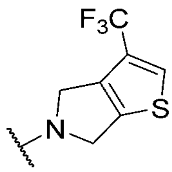,

or

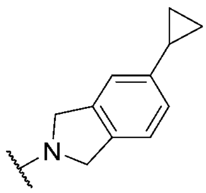,

or is selected from

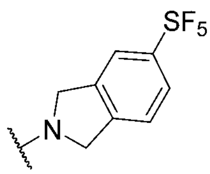 or 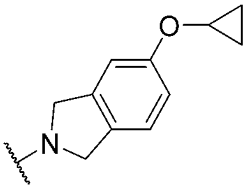;

or is selected from

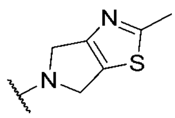, 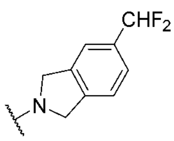, 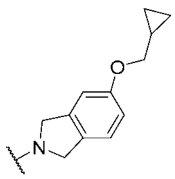, or 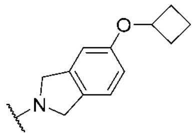;

or is selected from

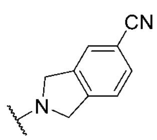 or 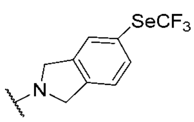;

or is selected from

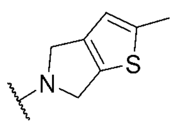

or

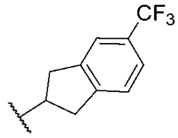

;

$L_1$ is selected from a bond, $-CH_2-O-$, $-CH_2-Se-$, -CH=CH-, -CF=CH-, $-CH_2-$, - $CH_2CH_2-$,

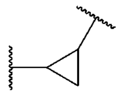

,

-O- or $-C(CH_3)=CH-$, $-CH=C(CH_3)-$ or

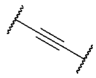

;

in some embodiments, $L_1$ is selected from a bond, $-CH_2-O-$, -CH=CH-, $-CH_2-$, $-CH_2CH_2-$,

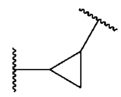

,

or -O-; in some embodiments, $L_1$ is $-CH_2-O-$, -CH=CH-, a bond, $-CH_2-$,

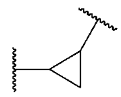

;

in some embodiments, $L_1$ is selected from $-CH_2-O-$ or -CH=CH-;
$L_2$ is $-CH_2-$;
the remaining groups are as described in the preceding fourth technical solution.

More specifically, as a sixth technical solution of the present invention, provided is a compound as represented by formula (I), or a stereoisomer, tautomer, deuterated compound, solvate, co-crystal or pharmaceutically acceptable salt, wherein the compound has a structure of formula (I-a) or (I-c):

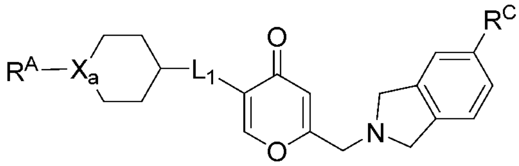

(I-a)

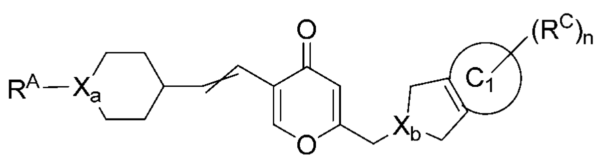

(I-c)

wherein

Xa is selected from CH or N;

$X_b$ is selected from CH or N;
ring $C_1$ is selected from 5- to 6-membered heteroaryl, phenyl or benzo 5- to 6-membered cycloalkyl;
the remaining groups are as described in any of the preceding technical solutions.

More specifically, as a seventh technical solution of the present invention, provided is a compound as represented by formula (I), or a stereoisomer, tautomer, deuterated compound, solvate, co-crystal or pharmaceutically acceptable salt, wherein the compound has a structure of formula (I-a):

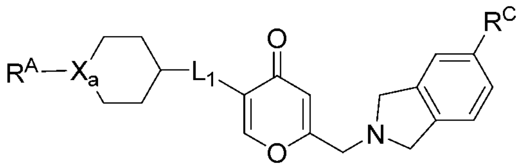
(I-a)

wherein

Xa is selected from CH or N;
the remaining groups are as described in any of the preceding technical solutions.

More specifically, as an eighth technical solution of the present invention, provided is a compound as represented by formula (I), or a stereoisomer, tautomer, deuterated compound, solvate, co-crystal or pharmaceutically acceptable salt, wherein the compound has a structure of formula (I-c):

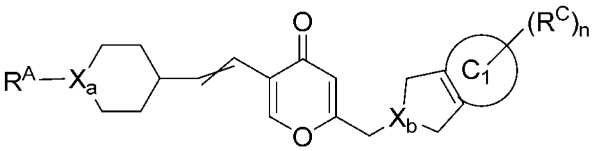
(I-c)

wherein

Xa is selected from CH or N;
$X_b$ is selected from CH or N;
ring $C_1$ is selected from 5- to 6-membered heteroaryl, phenyl or benzo 5- to 6-membered cycloalkyl;
the remaining groups are as described in any of the preceding technical solutions.

More specifically, as a ninth technical solution of the present invention, provided is a compound as represented by formula (I), (I-a) or (I-c), or a stereoisomer, tautomer, deuterated compound, solvate, co-crystal or pharmaceutically acceptable salt,

wherein $R^A$ is selected from $-S(O)_2-(CH_2)_{1-3}-R^a$, $-C(O)-O-(CH_2)_p-R^a$, $-(CH_2)_{1-2}-C(O)-R^a$, $-C(O)-(CH_2)_{1-2}-R^a$, -C(O)-O-halo $C_{1-4}$ alkyl, -C(O)-(6- to 9-membered bicyclic heterocycloalkyl), $-NH-C(O)-(CH_2)_p-R^a$, $-N(CH_3)-C(O)-(CH_2)_p-R^a$, $-NH-SO_2-(CH_2)_{1-2}-R^a$, $-NH-C(O)-O-R^a$, $-NH-R^a$, $-C(O)-(CH_2)_p$-(4- to 6-membered monocyclic heterocycloalkyl), $-C(O)-(CH_2)_p$-(4- to 6-membered monocyclic cycloalkyl), $-NH-C(O)-NH-R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), $-C(O)-NH-S(O)_2-C_{1-4}$ alkyl, -NH-C(O)deuterated $C_{1-2}$ alkyl, - NH-C(O)-NH-$C_{1-2}$ alkyl or -NH-C(O)-$C_{1-2}$ alkyl, wherein the alkyl, cycloalkyl, and heterocycloalkyl are optionally further substituted with 1-3 groups selected from halogen, OH, $NH_2$, CN, $-S(O)_2-CH_3$, -O-halo $C_{1-4}$ alkyl, or $-NH-S(O)_2-CH_3$;
$R^a$ is selected from deuterated $C_{1-4}$ alkyl, $C_{2-4}$ alkynyl, 3- to 5-membered cycloalkyl, 3- to 6-membered heterocycloalkyl, or 5- to 6-membered heteroaryl, wherein the alkynyl, cycloalkyl, heterocycloalkyl, and heteroaryl are optionally further substituted with 1-4 groups selected from halogen, OH, =O, $C_{1-4}$ alkyl, deuterated $C_{1-4}$ alkyl, $C_{1-4}$ alkoxy, $C_{2-4}$ alkynyl, -O-halo $C_{1-4}$ alkyl, $-S(O)_2-CH_3$ or $=CH_2$;
$R^C$ is selected from F, Cl, halo $C_{1-2}$ alkyl, $C_{2-4}$ alkynyl, 3-4-membered cycloalkyl, or -O-$C_{3-4}$ cycloalkyl;
$L_1$ is selected from -($C_{1-2}$ alkylene)-O-, $C_{1-2}$ alkylene, $C_{2-4}$ alkenylene, or -C(O)-NH-;
n is selected from 1 or 2;
p is selected from 0, 1 or 2;
the remaining groups are as described in any of the preceding technical solutions.

More specifically, as a tenth technical solution of the present invention, provided is a compound as represented by formula (I), (I-a) or (I-c), or a stereoisomer, tautomer, deuterated compound, solvate, co-crystal or pharmaceutically acceptable salt,

wherein $R^A$ is selected from $-C(O)-O-(CH_2)_p-R^a$, $-C(O)-(CH_2)_{1-2}-R^a$, $-NH-C(O)-(CH_2)_p-R^a$, $-N(CH_3)-C(O)-(CH_2)_p-R^a$, or $-NH-C(O)-O-R^a$;
$R^a$ is selected from 3- to 5-membered cycloalkyl, 3- to 6-membered heterocycloalkyl, or 5- to 6-membered heteroaryl, wherein the cycloalkyl, heterocycloalkyl, and heteroaryl are optionally further substituted with 1-4 groups selected from halogen, $C_{1-4}$ alkyl, deuterated $C_{1-4}$ alkyl, or $-S(O)_2-CH_3$;
$R^C$ is selected from F, Cl, $CHF_2$, $CF_3$, ethynyl, cyclopropyl, cyclobutyl, -O-cyclopropyl, or -O-cyclobutyl;
the remaining groups are as described in any of the preceding technical solutions.

More specifically, as an eleventh technical solution of the present invention, provided is a compound as represented by formula (I) or (I-a), or a stereoisomer, tautomer, deuterated compound, solvate, co-crystal or pharmaceutically acceptable salt, wherein

$L_1$ is -CH=CH-;
the remaining groups are as described in any of the preceding technical solutions.

Specifically, as a technical solution of the present invention, provided is the compound, or the stereoisomer, tautomer, deuterated compound, solvate, co-crystal or pharmaceutically acceptable salt thereof described in the present invention, wherein the compound is selected from one of the following structures:

Table 1

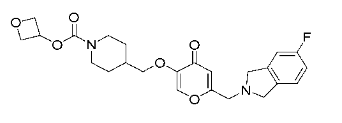
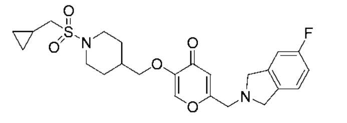
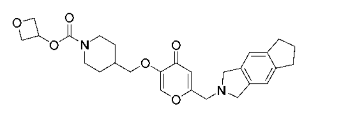

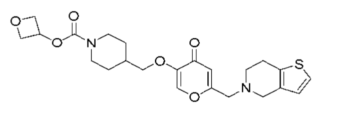
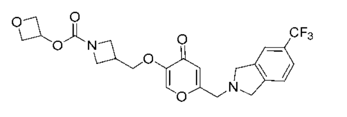
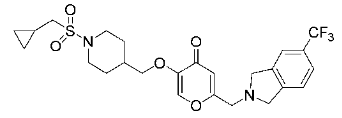

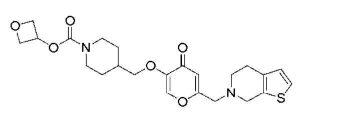
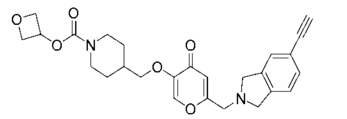
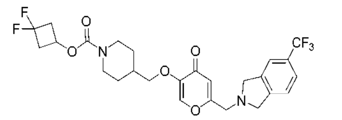

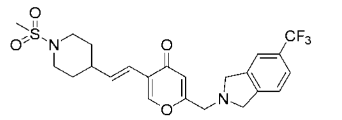
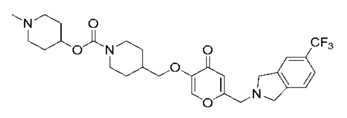
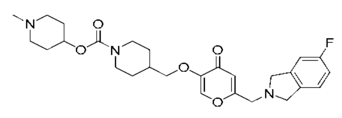

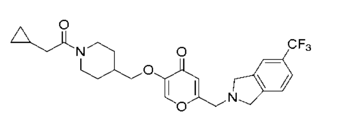
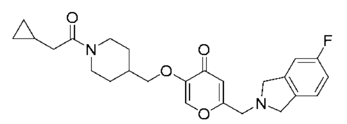
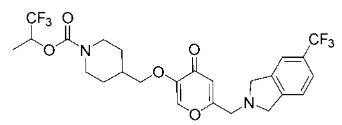

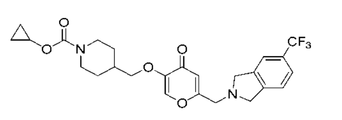
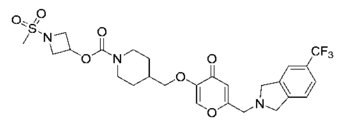
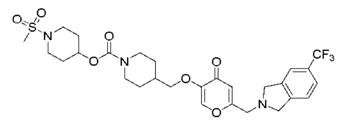

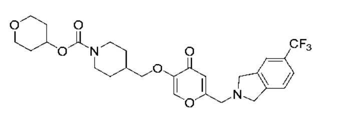
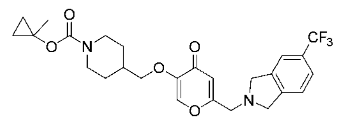
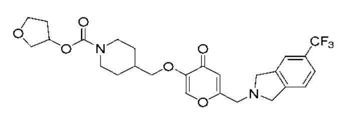

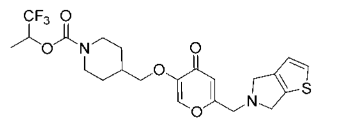
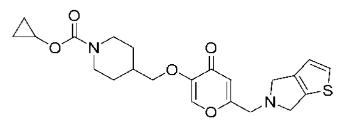
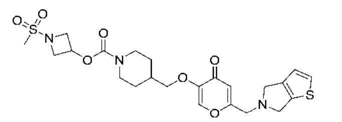

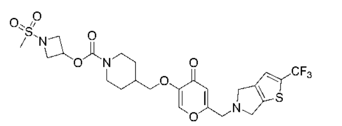
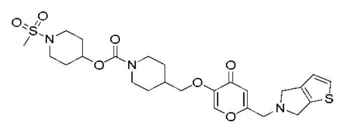
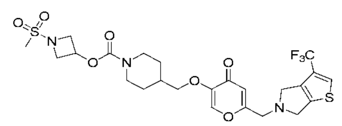

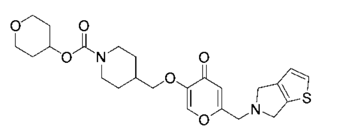
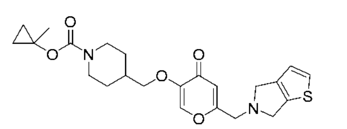
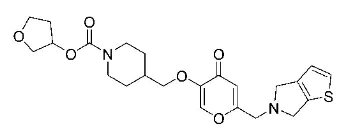

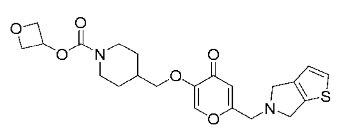
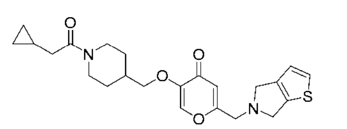
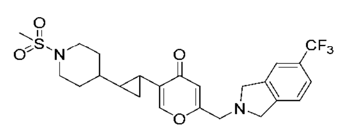

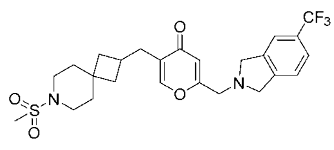
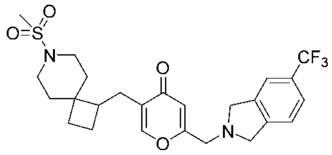
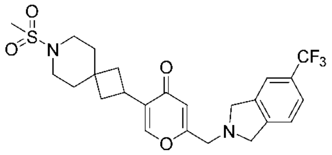
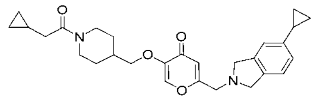
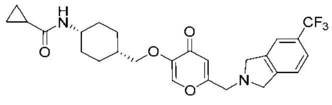
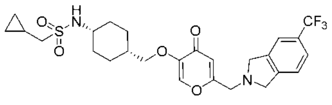
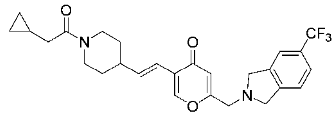
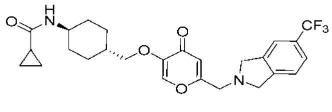
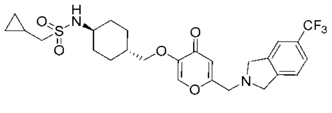
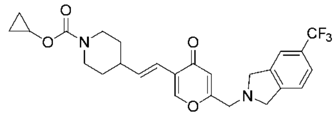
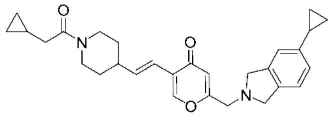
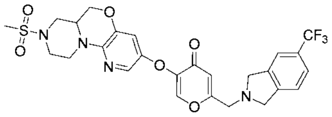
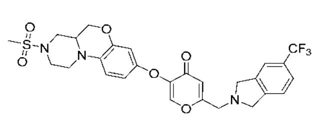
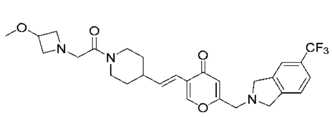
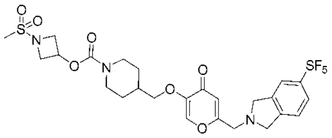
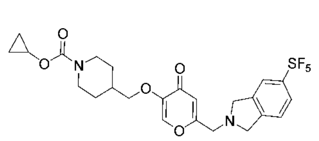
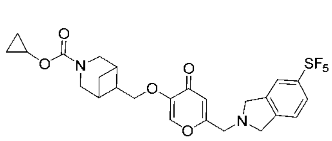
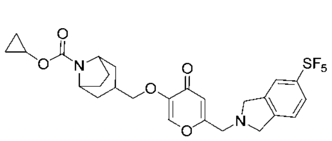
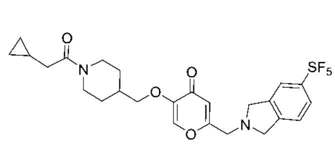
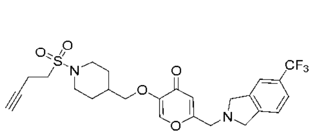
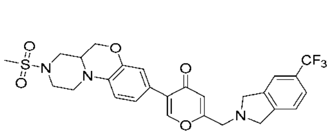
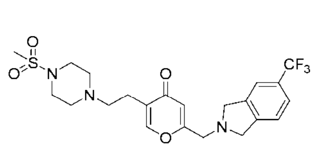
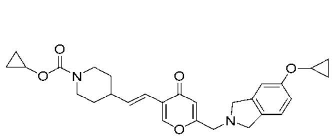
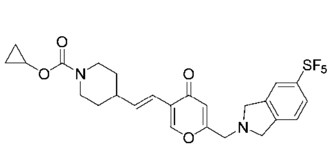
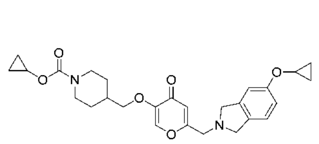
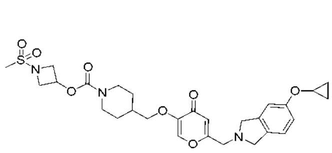
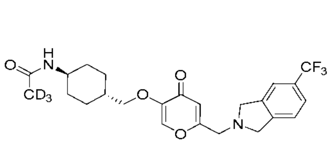
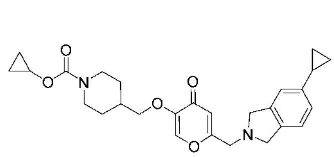
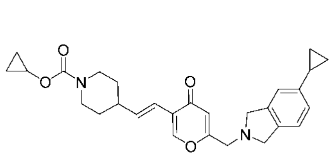
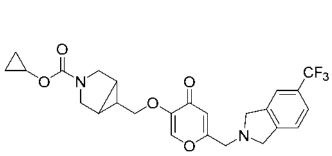
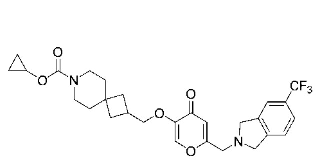
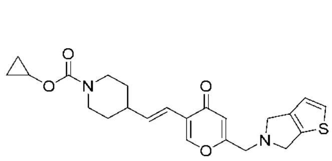
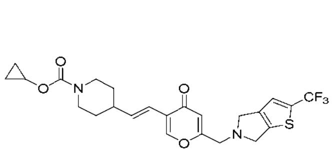

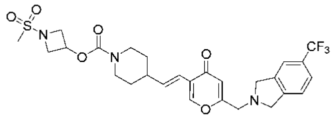
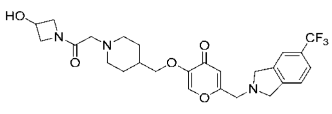
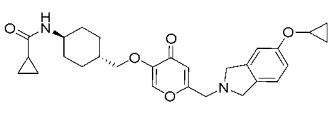
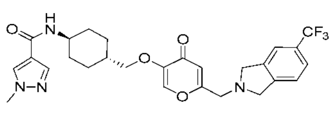
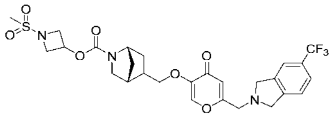
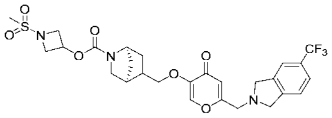
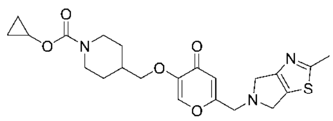
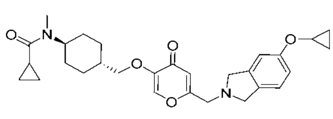
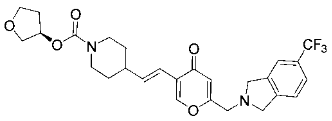
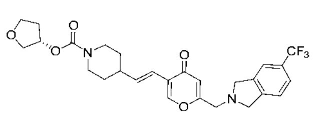
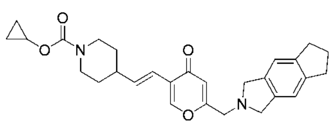
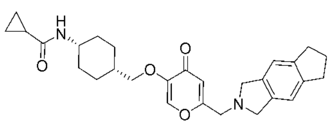
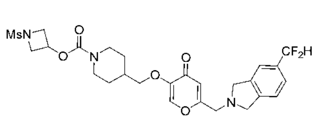
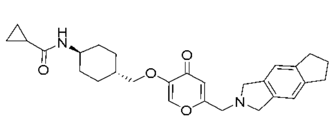
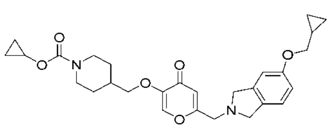
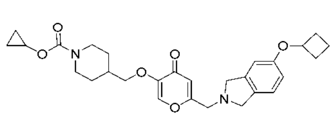
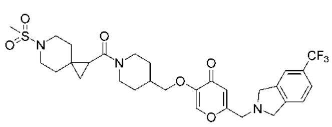
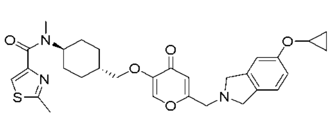
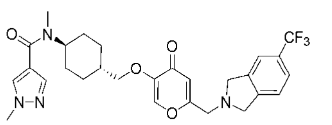
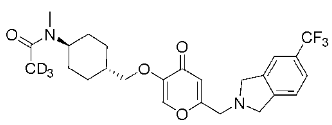
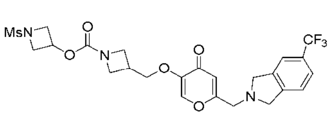
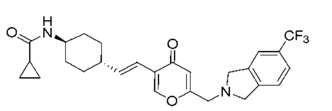
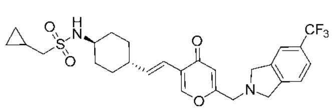
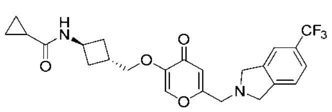
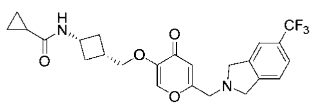
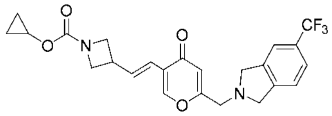
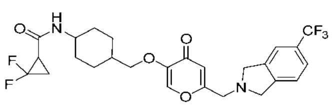
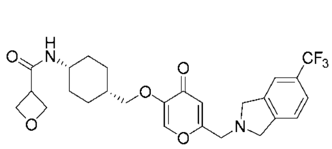
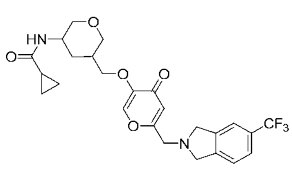
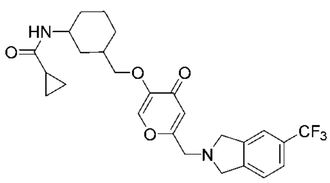
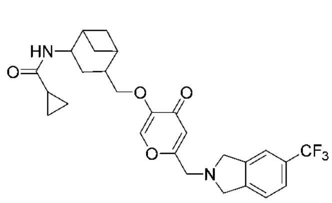
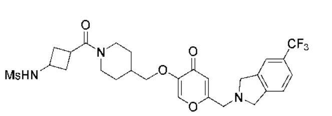
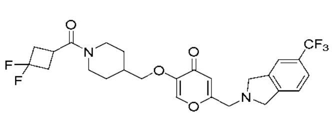

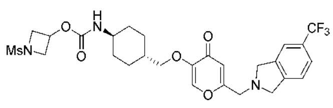
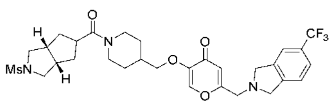
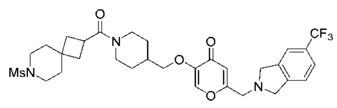
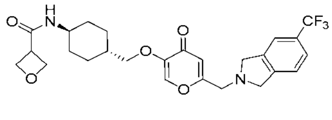
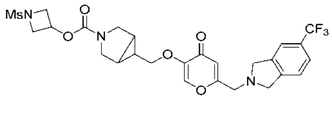
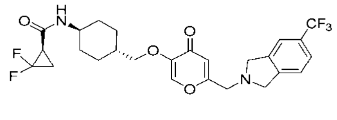
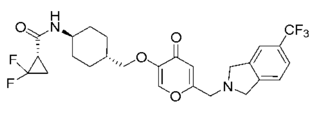
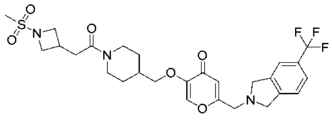
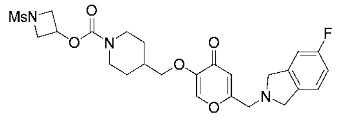
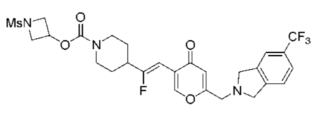
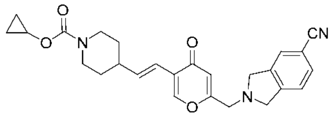
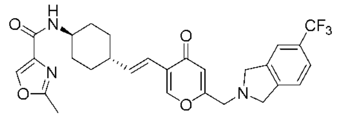
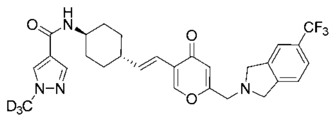
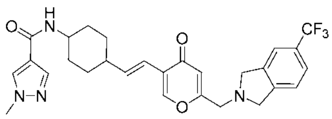
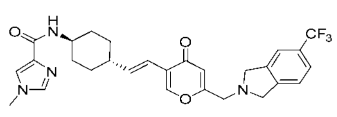
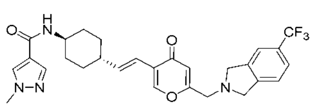
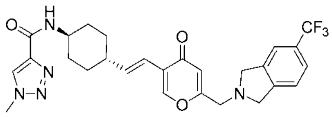
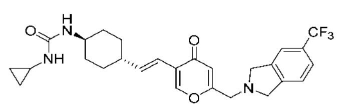
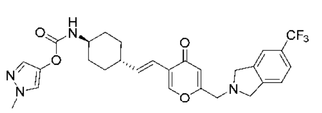
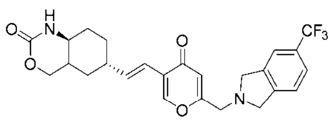
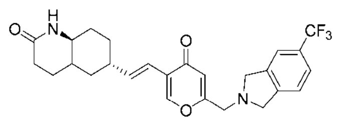
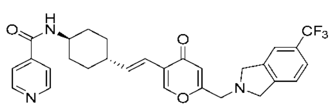
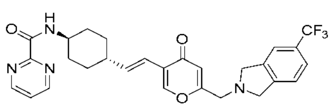
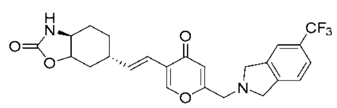
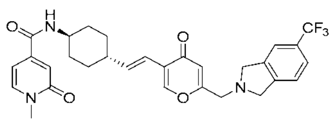
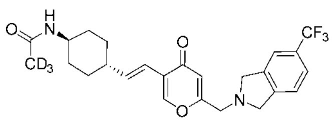
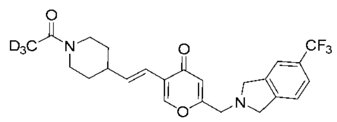
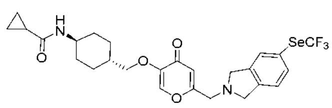
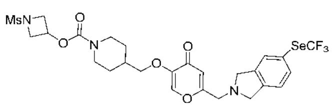
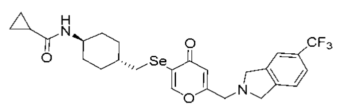
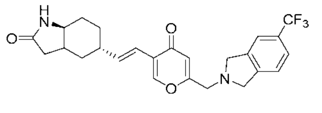
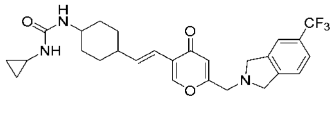
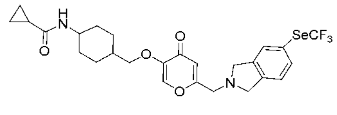
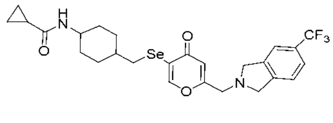
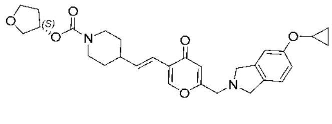
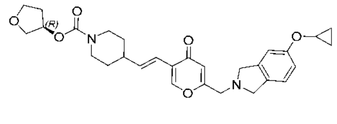

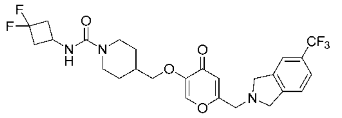
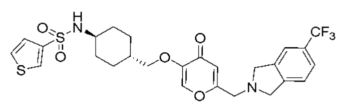
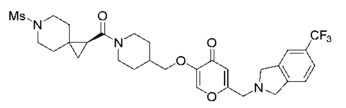

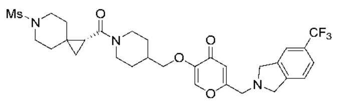
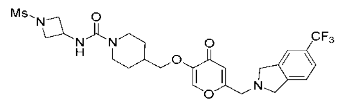
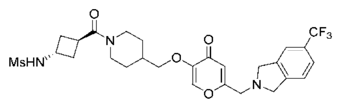

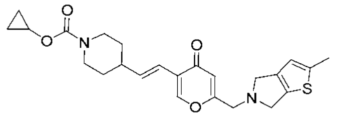
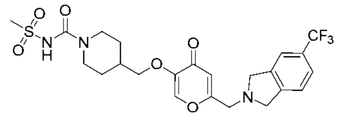
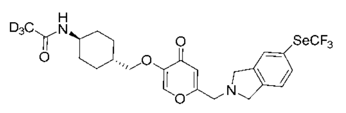

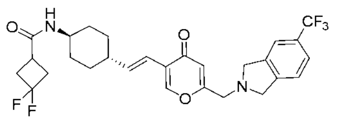
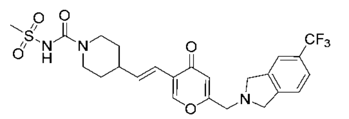
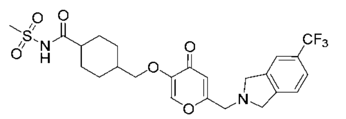

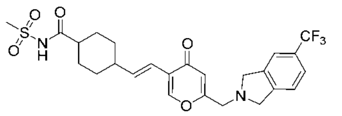
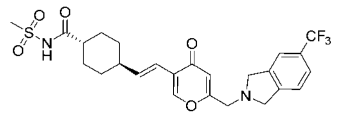
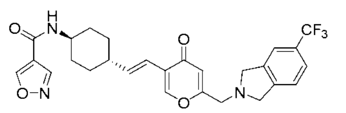

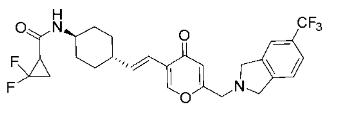
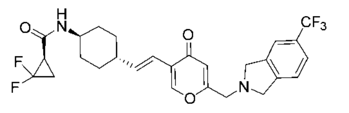
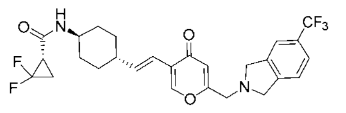

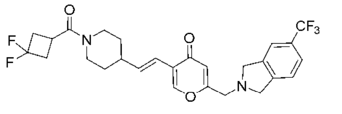
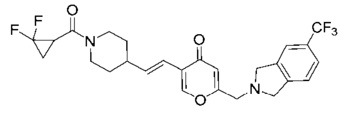
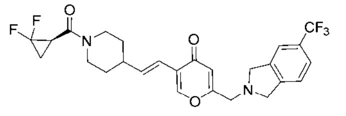

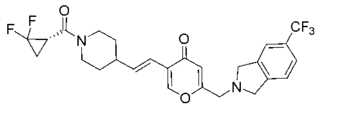
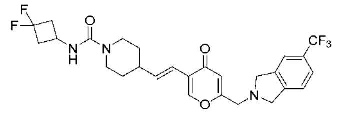
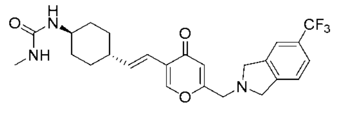

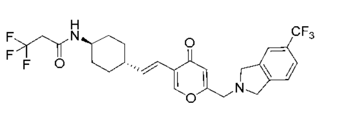
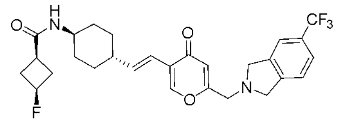
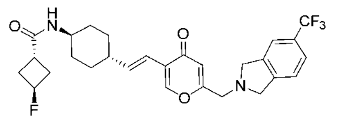

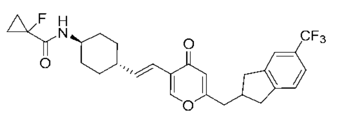
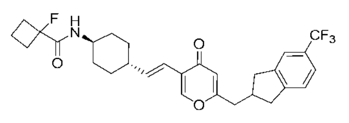
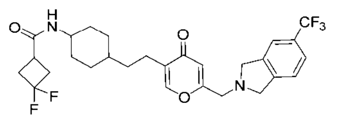

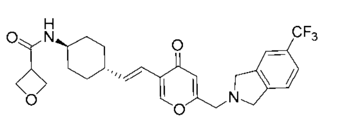
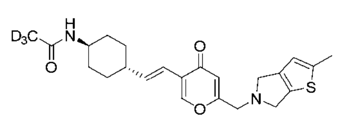
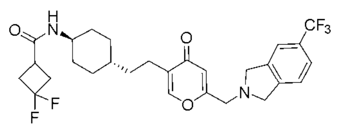

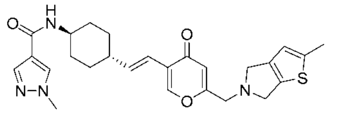
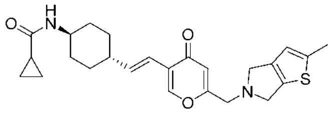
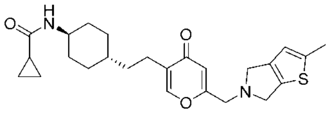
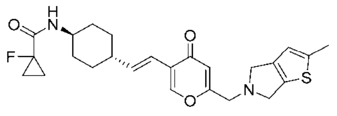
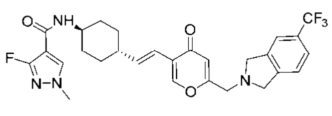
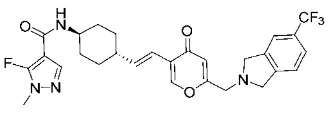
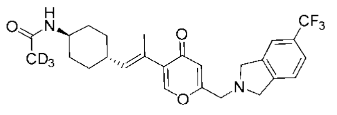
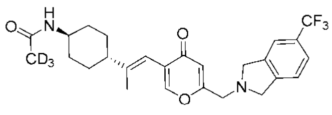
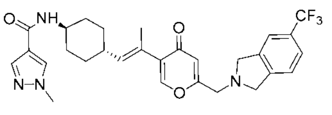
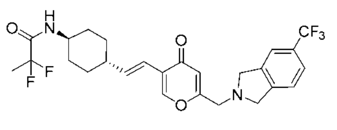
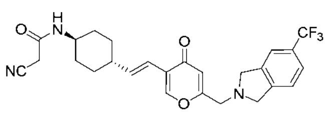
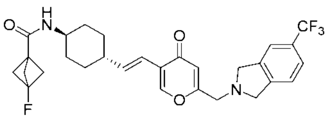
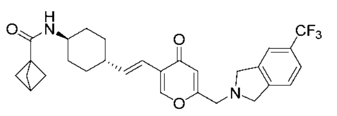
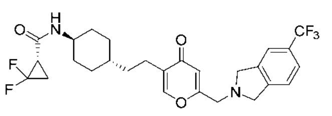
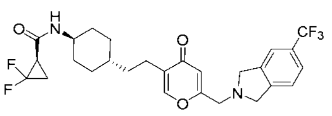
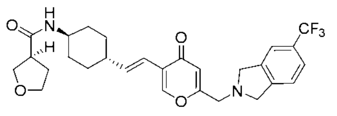
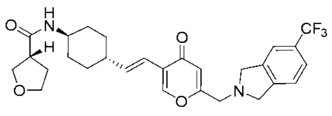
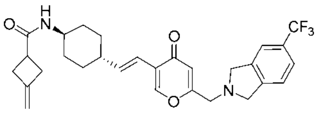
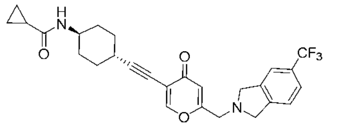
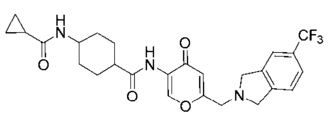
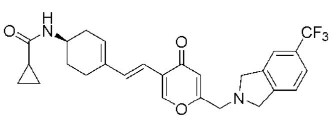
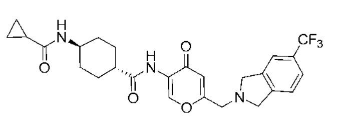
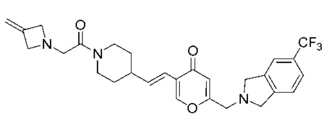
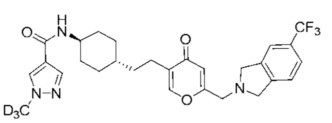
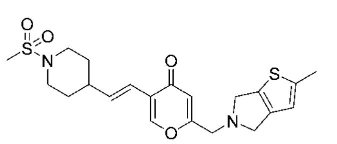
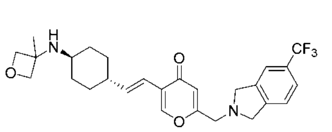
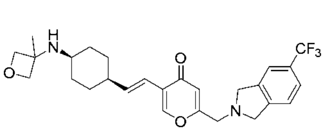
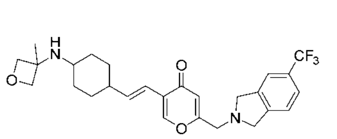
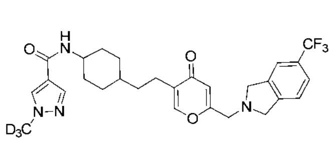
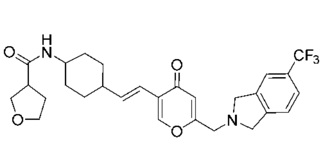
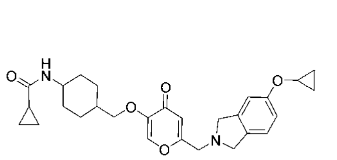
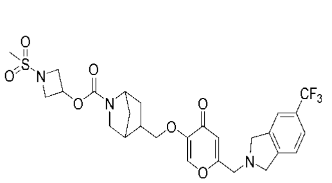
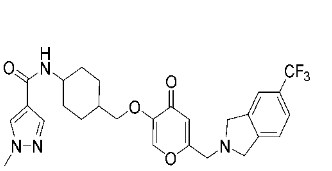

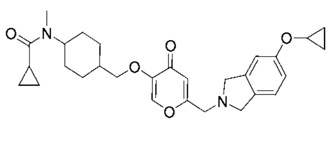
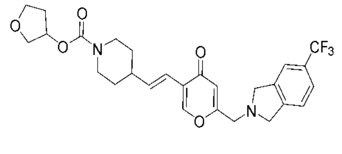
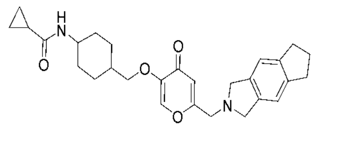
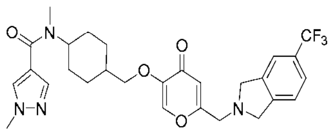
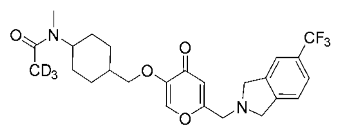
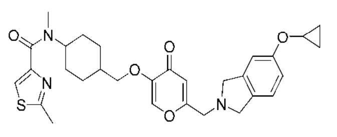
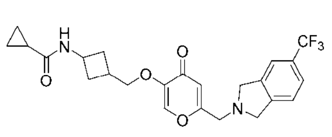
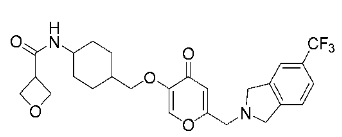
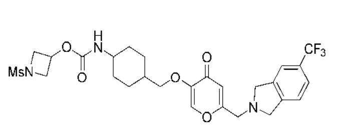
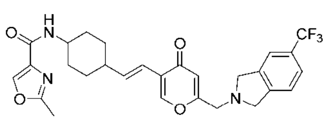
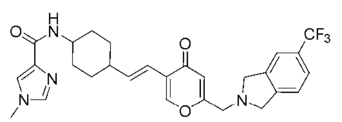
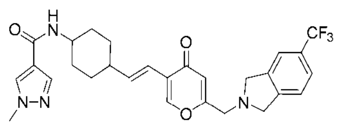
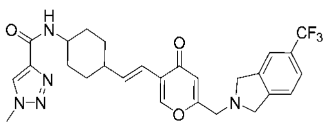
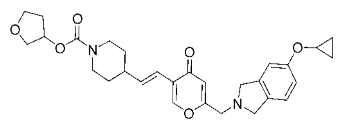
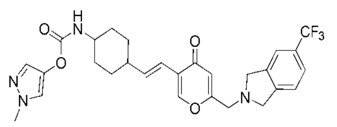
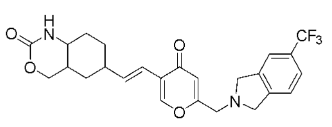
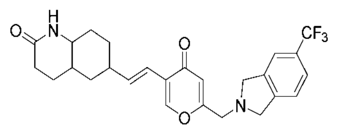
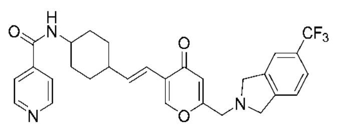
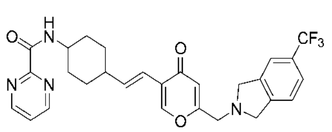
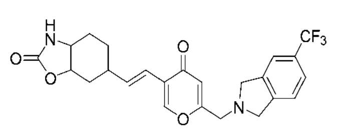
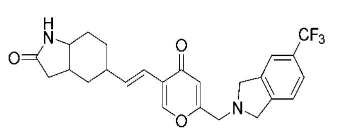
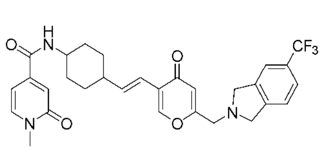
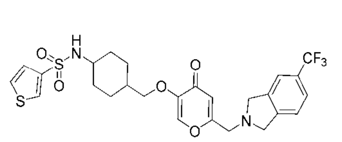
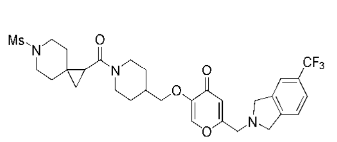
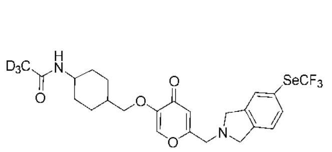
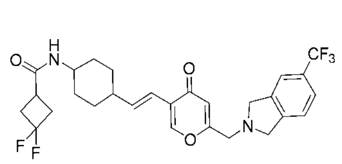
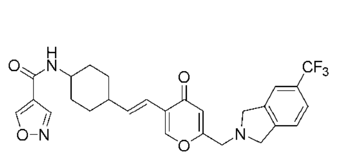
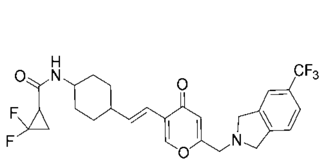
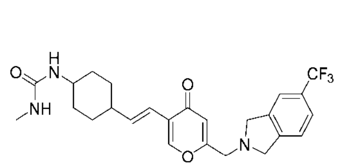
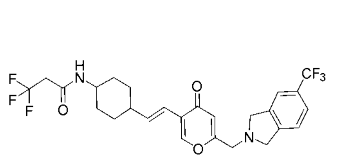
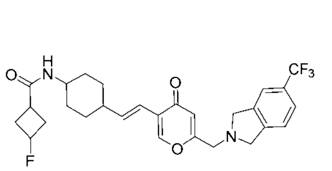
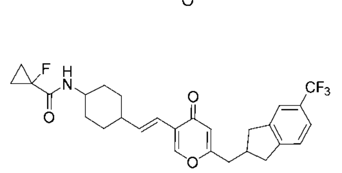
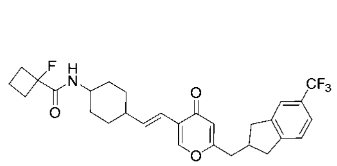

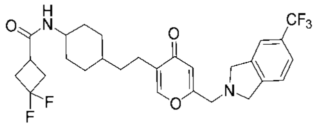
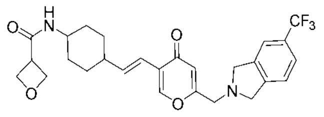
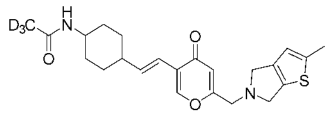
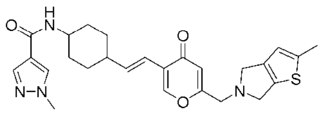
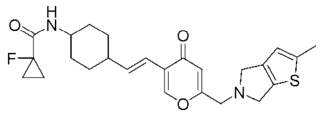
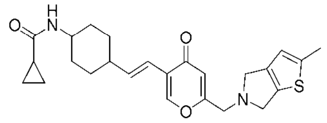
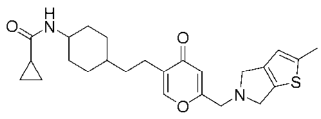
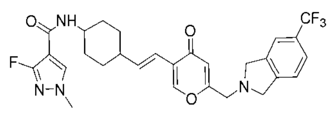
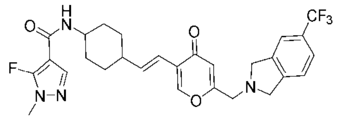
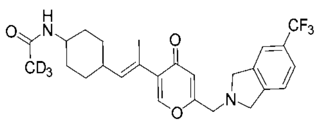
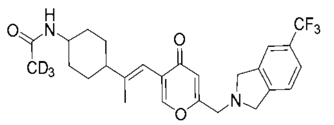
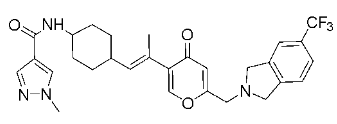
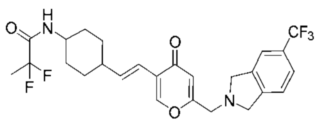
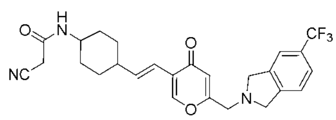
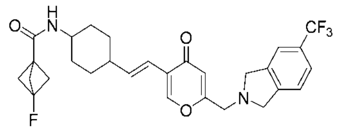
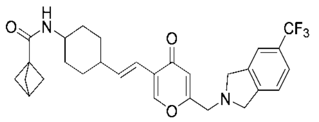
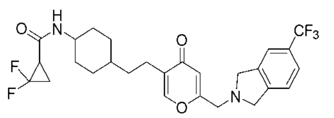
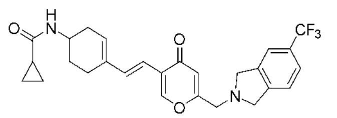
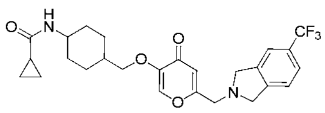
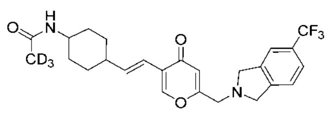
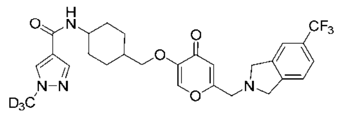
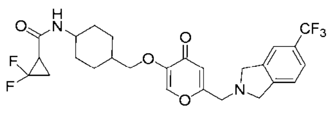
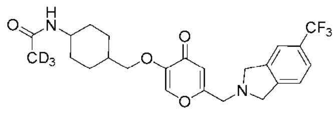
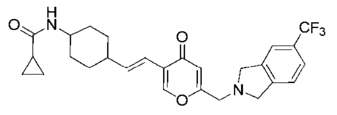
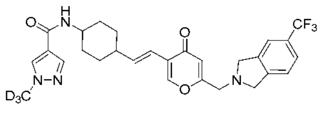
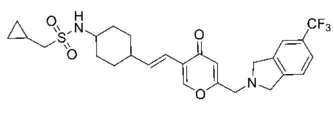
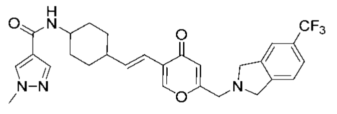
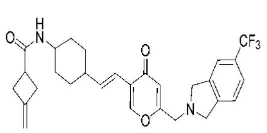
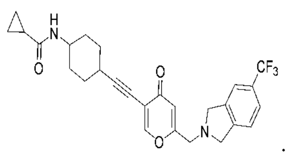

The present invention further provides a pharmaceutical composition or pharmaceutical preparation comprising the compound, or the stereoisomer, tautomer, deuterated compound, solvate, co-crystal or pharmaceutically acceptable salt thereof according to any one of the preceding solutions, and a pharmaceutically acceptable carrier and/or excipient.

Further, the composition or pharmaceutical preparation of the present invention contains 1-1500 mg of the compound, or the stereoisomer, tautomer, deuterated compound, solvate, co-crystal or pharmaceutically acceptable salt

thereof according to any one of the preceding solutions, and a carrier and/or excipient.

The present invention further provides the use of the compound, or the stereoisomer, tautomer, deuterated compound, solvate, co-crystal or pharmaceutically acceptable salt thereof according to any one of the preceding solutions, or the composition according to any one of the preceding solutions in the preparation of a drug for treating/preventing a CYP11A1-mediated disease; further, the CYP11A1-mediated disease is steroid hormone-dependent cancer, further preferably, the cancer is prostate cancer.

The present invention further provides a method for treating a disease in a mammal, the method comprising administering to a subject a therapeutically effective amount of the compound, or the stereoisomer, the tautomer, the deuterated compound, the solvate, or the pharmaceutically acceptable salt thereof, wherein the therapeutically effective amount is preferably 1-1500 mg, and the disease is preferably prostate cancer. In some embodiments, the mammal mentioned in the present invention includes human.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, or 100-200 mg.

The present invention relates to a pharmaceutical composition or pharmaceutical preparation, wherein the pharmaceutical composition or pharmaceutical preparation comprises a therapeutically effective amount of the compound, or the stereoisomer, tautomer, deuterated compound, solvate, co-crystal or pharmaceutically acceptable salt thereof described in the present invention, as well as a pharmaceutically acceptable carrier and/or excipient. The pharmaceutical composition can be in a unit preparation form (the amount of the drug substance in the unit preparation is also referred to as the "preparation strength"). In some embodiments, the pharmaceutical composition comprises the compound, or the stereoisomer, tautomer, deuterated compound, solvate, co-crystal or pharmaceutically acceptable salt thereof according to the present invention in an amount including but not limited to 1-1500 mg, 5-1500 mg, 5-1000 mg, 10-800 mg, 20-600 mg, 25-500 mg, 40-200 mg, 50-100 mg, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, and 1500 mg.

The present invention further provides a method for treating a disease in a mammal, the method comprising administering to a subject a therapeutically effective amount of the compound, or the stereoisomer, tautomer, deuterated compound, solvate, co-crystal or pharmaceutically acceptable salt according to the present invention as well as a pharmaceutically acceptable carrier and/or excipient, wherein the therapeutically effective amount is preferably 1-1500 mg, and the disease is preferably prostate cancer.

The present invention further provides a method for treating a disease in a mammal, the method comprising administering to a subject the compound, or the stereoisomer, tautomer, deuterated compound, solvate, co-crystal or pharmaceutically acceptable salt according to the present invention, as well as a pharmaceutically acceptable carrier and/or excipient at a daily dose of 1-1500 mg/day, wherein the daily dose can be a single dose or divided doses; in some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400

mg/day, or 1500 mg/day.

The present invention relates to a kit, which may comprise a composition in a single-dose or multi-dose form, wherein the kit comprises the compound or the stereoisomer, the deuterated compound or the pharmaceutically acceptable salt thereof according to the present invention, and the amount of the compound or the stereoisomer, the deuterated compound or the pharmaceutically acceptable salt thereof according to the present invention is the same as that in the above-mentioned pharmaceutical composition.

In the present invention, the amount of the compound, or the stereoisomer, or the pharmaceutically acceptable salt thereof according to the present invention is calculated in the form of a free base in each case.

The term "preparation strength" refers to the weight of the active drug contained in each vial, tablet or other unit preparation.

**Synthetic route**

Patent documents such as WO 2018115591 A1 have introduced a method for preparing a CYP11A1 inhibitor, and those skilled in the art would have been able to prepare the compounds of the present invention in conjunction with this document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalogue are optionally prepared by custom chemical synthesis plants, wherein many of standard chemical supply plants (such as those listed above) provide custom synthesis services.

**Terms**

Unless otherwise specified in the present invention, the terms of the present invention have the following meanings.

The term "halogen" herein refers to F, Cl, Br, I, or isotopes thereof.

The term "halo" or "substituted with halogen" means that a hydrogen atom is substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen.

The term "deuterated" or "deuterated compound" refers to the case where a hydrogen atom on alkyl, cycloalkyl, alkylene, aryl, heteroaryl, mercapto, heterocycloalkyl, alkenyl, alkynyl and other groups is substituted with at least one isotope deuterium, wherein the upper limit of the number of deuterium substituents is equal to the sum of the number of hydrogens that can be substituted in the group to be substituted. Without particular limitation, the number of deuterium substituents is any integer between 1 and the upper limit, preferably 1-20 deuterium atoms, more preferably 1-10 deuterium atoms, more preferably 1-6 deuterium atoms, and further preferably 1-3 deuterium atoms.

The term "alkyl" refers to a monovalent linear or branched saturated aliphatic hydrocarbon group. Unless otherwise specially specified, the alkyl refers to an alkyl group comprising 1 to 20 carbon atoms, preferably an alkyl group comprising 1 to 8 carbon atoms, more preferably an alkyl group comprising 1 to 6 carbon atoms, further preferably an alkyl group comprising 1 to 4 carbon atoms, and further preferably an alkyl group comprising 1-2 carbon atoms. Non-limiting examples include methyl, ethyl, n-propyl, isopropyl, n-butyl, sec-butyl, neobutyl, tert-butyl, n-pentyl, isoamyl, neopentyl, n-hexyl, and various branched isomers thereof.

The term "alkylene" refers to a divalent linear or branched saturated alkyl, and examples of alkylene include, but are not limited to, methylene, ethylene, propylene, butylene, etc.

The term "cycloalkyl" refers to a monovalent non-aromatic, partially unsaturated or fully saturated, substituted or unsubstituted carbocyclic hydrocarbon group, which generally has 3 to 12 carbon atoms, preferably 3-10 carbon atoms, more preferably 3-6 carbon atoms, and further preferably 3-4 carbon atoms, unless otherwise specially specified. Non-limiting examples include cyclopropyl, cyclobutyl, cyclopentyl, cyclohexyl,

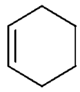,

cycloheptyl, etc.

The term "cycloalkylene" refers to a divalent group of "cycloalkyl", and non-limiting examples include cyclopropylene, cyclobutylene, etc.

The term "heterocycle" or "heterocyclyl" refers to a substituted or unsubstituted, saturated or unsaturated, aromatic or non-aromatic ring comprising 1 to 4 heteroatoms selected from N, O, or S unless otherwise specifically defined, including monocyclic heterocycles, bridged bicyclic heterocycles, fused bicyclic heterocycles, spiro bicyclic heterocycles, etc., which are 3- to 14-membered heterocycles, more preferably 4- to 12-membered heterocycles, more preferably 4-to 10-membered heterocycles, and further preferably 4- to 7-membered heterocycles unless otherwise specifically defined. The definition thereof comprises heterocycloalkyl and heteroaryl. The N and S in the heterocyclyl ring may be oxidized into various oxidation states. Heterocyclyl can be connected to a heteroatom or a carbon atom, and non-limiting examples of heterocyclyl include oxiranyl, aziridinyl, oxetanyl, azetidinyl, 1,3-dioxolanyl, 1,4-dioxolanyl, 1,3-dioxanyl, azepanyl, pyridinyl, furyl, thienyl, pyranyl, N-alkylpyrrolyl, pyrimidinyl, pyrazinyl, pyrazolyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzimidazolyl, benzopyridinyl, pyrrolopyridinyl, benzodihydrofuryl, azabicyclo[3.2.1]octyl, azabicyclo[5.2.0]nonanyl, oxatricyclo[5.3.1.1]dodecyl, azaadamantyl, oxaspiro[3.3]heptyl,

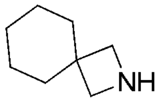, 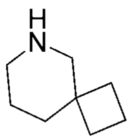, 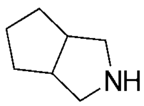, 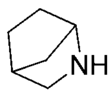,

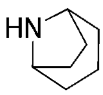, 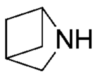, 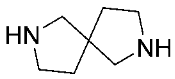, 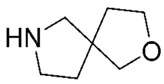, 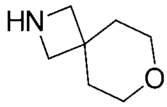, 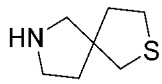,

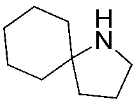, 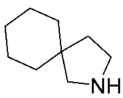, 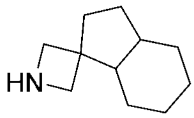, 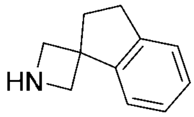, 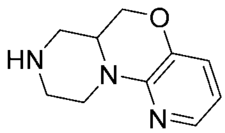,

etc.

The term "heterocyclylene" is a divalent group corresponding to "heterocyclyl", and non-limiting examples include imidazolylene, piperidinylene, aziridinylene, etc.

The term "carbocyclic" or "carbocyclyl" refers to a substituted or unsubstituted, saturated or unsaturated, aromatic or non-aromatic carbocyclic group, including monocyclic carbocyclic rings, bridged bicyclic rings, fused bicyclic rings, spiro bicyclic rings, etc., which have 3 to 12 carbon atoms, preferably 3-10 carbon atoms, and further preferably 3-6 carbon atoms unless otherwise specially specified. The definition thereof comprises cycloalkyl and aryl. In non-limiting embodiments, monocyclic carbocyclic rings include cyclopropyl, cyclobutyl, cyclopentyl, cyclohexyl, cycloheptyl or phenyl,

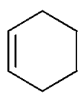,

etc., bridged bicyclic rings include

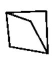,

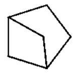, 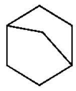,

etc., fused bicyclic rings include

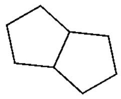, 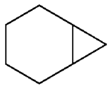, 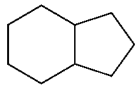,

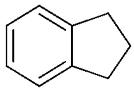, 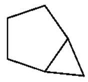,

etc., and spiro bicyclic rings include

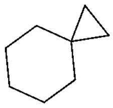, 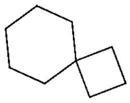,

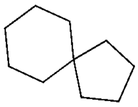, 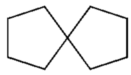, 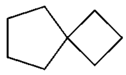, 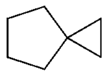,

etc.

The term "aryl" refers to an aromatic carbocyclic ring. Non-limiting examples include phenyl, naphthyl, etc.

The term "alkynyl" refers to a monovalent linear or branched unsaturated hydrocarbon group containing one or more carbon-carbon triple bonds. Unless otherwise specifically specified, the alkynyl contains 2-6 carbon atoms, preferably 2-4 carbon atoms, and non-limiting examples include ethynyl, propynyl, propargyl, etc.

The term "alkenyl" refers to a linear or branched monovalent unsaturated hydrocarbon group containing one or more carbon-carbon double bonds. Unless otherwise specifically specified, the alkynyl contains 2-6 carbon atoms, preferably contains 2-4 carbon atoms, and non-limiting examples include vinyl, propenyl, allyl, 2-butenyl, 1-butenyl, etc.

The term "alkoxy" or "alkyloxy" refers to -O-alkyl. Without particular limitation, alkoxy or alkyloxy is -O-$C_{1-8}$ alkyl, preferably -O-$C_{1-6}$ alkyl, more preferably -O-$C_{1-4}$ alkyl, and further preferably -O-$C_{1-2}$ alkyl. Non-limiting examples include methoxy, ethoxy, n-propoxy, isopropoxy, n-butoxy, secbutoxy, tert-butoxy, n-pentoxy, n-hexyloxy, cyclopropoxy, cyclobutoxy, etc.

The term "haloalkoxy" refers to -O-haloalkyl. Without particular limitation, the haloalkoxy is -O-halo $C_{1-8}$ alkyl, preferably -O-halo $C_{1-6}$ alkyl, more preferably -O-halo $C_{1-4}$ alkyl, and further preferably -O-halo $C_{1-2}$ alkyl. Non-limiting examples of haloalkoxy include monofluoromethoxy, difluoromethoxy, trifluoromethoxy, difluoroethyloxy, *etc.*

The term "$C_{1-4}$ alkylacyl" refers to $C_{1-4}$ alkyl-C(O)-. Non-limiting examples include formyl, acetyl, and propionyl.

The term "$C_{1-4}$ alkylsulphonyl" refers to $C_{1-4}$ alkyl-$S(O)_2$-. Non-limiting examples include methylsulphonyl, ethylsulphonyl, and propylsulphonyl.

The term "heteroaromatic ring" or "heteroaryl" refers to an aromatic heterocycle. Non-limiting examples include pyrazolyl, pyrimidinyl, thiazolyl, pyridinyl, furyl, pyrone, pyridone, etc.

The term "heterocycloalkyl" refers to a non-aromatic, partially unsaturated or fully saturated heterocycle, which generally has 4 to 12 ring members, preferably 4 to 10 ring members, more preferably 4 to 7 ring members, and further preferably 5 or 6 ring members. In addition to carbon atoms, heterocycloalkyl also comprises 1-3 heteroatoms selected from N, S and O as ring members. Non-limiting examples include azetidinyl, morpholinyl, piperazinyl, piperidyl, tetrahydropyranyl, oxetanyl, etc.

The term "alkyl$NH_2$" or "alk$NH_2$" refers to $NH_2$ substituted with one or two alkyl, and is also written as -N-$(alkyl)_2$ or -NH-alkyl, wherein the latter is also known as monoalkyl$NH_2$. Non-limiting examples include dimethyl $NH_2$, monomethyl $NH_2$, diethyl $NH_2$, monoethyl $NH_2$, etc.

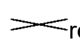represents vinyl (-CH=CH-), including cis, trans isomers or a mixture of cis and trans isomers.

The term "optional" or "optionally" refers to that the events or circumstances subsequently described may but not

necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or stereoisomers, solvates, pharmaceutically acceptable salts or co-crystals thereof as described herein and other components including physiologically/pharmaceutically acceptable carriers and/or excipients.

The term "carrier" refers to a system that does not cause significant irritation to the organism and does not eliminate the biological activity and characteristics of the administered compound and can change the way the drug enters the human body and the distribution of the drug in the body, control the release rate of the drug and deliver the drug to targeted organs. Non-limiting examples of the carrier include microcapsule, microsphere, nanoparticle, liposome, etc.

The term "excipient" refers to a substance that is not a therapeutic agent per se, but used as a diluent, adjuvant, binder and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrant, an absorbent, a preservative, a surfactant, a colorant, a flavouring agent, and a sweetening agent. Examples of pharmaceutically acceptable excipients include, but are not limited to: (1) sugars, such as lactose, glucose, and sucrose; (2) starches, such as corn starch and potato starch; (3) cellulose and derivatives thereof, such as sodium carboxymethyl cellulose, ethyl cellulose, cellulose acetate, hydroxypropyl methylcellulose, hydroxypropyl cellulose, microcrystalline cellulose, and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil, and soybean oil; (10) diols, such as propylene glycol; (11) polyols, such as glycerol, sorbitol, mannitol, and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffer solutions; (21) polyesters, polycarbonates and/or polyanhydrides; and (22) other non-toxic compatible substances used in a pharmaceutical preparation.

"Isomer" includes "stereoisomer" and "tautomer". "Stereoisomer" refers to isomers produced as a result of different spatial arrangement of atoms or <u>atomic groups</u> in molecules that are connected to each other in the same sequence. Stereoisomers include cis-trans isomers, optical isomers and conformational isomers. The term "tautomer" refers to isomers that can be transformed into each other by a reversible chemical reaction called tautomerization, which is a transformation between functional groups usually caused by the accompanying migration of a hydrogen atom and a $\pi$ bond (a double bond or a triple bond); such as, the following paired compounds: aldehyde/ketone-enol and imine-enamine.

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

## <u>Detailed Description of Embodiments</u>

The content of the present invention is described in detail by means of the following examples. In examples in which no specific conditions are indicated, experimental methods are carried out under conventional conditions. The listed examples are intended to better illustrate the content of the present invention but should not be construed as limiting the content of the present invention. Non-essential improvements and adjustments made to the embodiments by a person of ordinary skill in the art according to the above Summary of the Invention still fall within the scope of protection of the present invention.

Dess-Martin reagent: 1,1,1-Triacetoxy-1,1-dihydro-1,2-benziodoxol-3-(1H)-one
DIPEA: N,N-diisopropylethylamine
NMP: N-methylpyrrolidone

TBDMSCl: Tert-butyl dimethylsilyl chloride
DMAP: 4-Dimethylaminopyridine
HATU: 2-(7-azabenzotriazole)-N,N,N',N'-tetramethyluronium hexafluorophosphate
$T_3P$: Propylphosphonic cyclic anhydride

**Intermediate 1:**

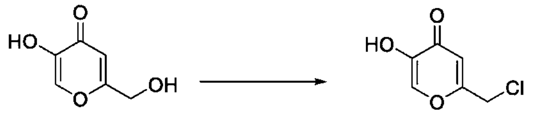

Intermediate 1

Kojic acid (20.0 g, 140.7 mmol) was dissolved in thionyl chloride (40 ml), and the resulting mixture was reacted at room temperature for 2 h. After the completion of the reaction, the reaction solution was filtered, and the filter cake was slurried with petroleum ether (100 mL) and filtered. The resulting filter cake was dried to afford **intermediate 1** (29.8 g, yield: 90%).

LCMS (ESI): m/z= 161.2 [M+H]
$^1$H NMR (400 MHz, DMSO) δ 8.12 (s, 1H), 6.57(s, 1H), 4.66 (s, 2H).

**Intermediate 2:**

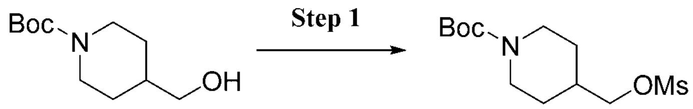


**2a** **Intermediate 2**

Step 1:

Compound **2a** (2 g, 9.30 mmol) and triethylamine (2.82 g, 27.9 mmol) were dissolved in dichloromethane (8 mL), methanesulphonyl chloride (1.28, 11.16 mmol) was slowly added dropwise in an ice bath. After the dropwise addition was completed, the mixture was allowed to naturally warm to room temperature. After 3 h, the reaction was completed as monitored by TLC. Water (50 mL) was added to the reaction solution. The mixture was extracted with dichloromethane (20 mL×3), and the organic layers were combined, washed with saturated sodium chloride aqueous solution (40 mL), dried over anhydrous sodium sulphate, and concentrated to obtain the target compound, i.e., intermediate 2 (2.74 g).
LC-MS (ESI): m/z =294.1 [M+H]$^+$.

**Example 1:**

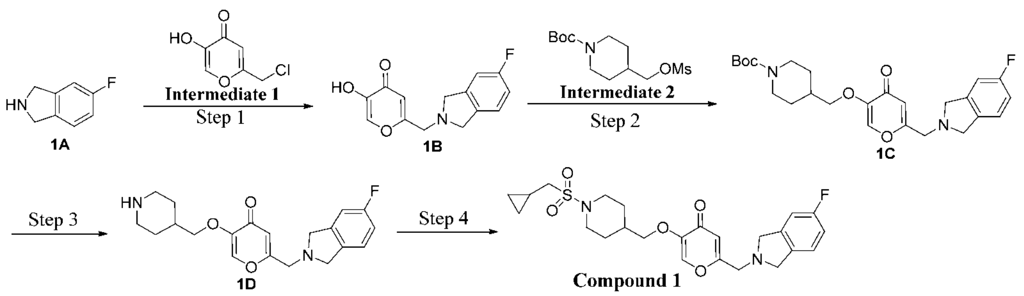


1A 1B 1C 1D Compound 1

Step 1:

**1A** (1 g, 7.29 mmol), **intermediate 1** (1.29 g, 8.02 mmol) and DIPEA (2.83 g, 21.87 mmol) were dissolved in

acetonitrile (15 mL), and the mixture was reacted at room temperature overnight. After the reaction was completed, the reaction solution was concentrated, water (25 mL) was added to the residue, the resulting mixture was extracted with ethyl acetate (15 mL×3), and the organic layers were combined, dried over anhydrous sodium sulphate, filtered and concentrated. The residue was separated and purified by silica gel column chromatography (dichloromethane: methanol (v/v) = 40:1) to obtain **1B** (1.08 g, yield: 58%).

LC-MS (ESI): m/z = 262.2 $[M+H]^+$.

Step 2:

**1B** (1.08 g, 4.13 mmol), **intermediate 2** (1.33 g, 4.54 mmol) and potassium carbonate (1.71 g, 12.39 mmol) were dissolved in DMF (25 mL), and the resulting mixture was reacted at 70°C under nitrogen atmosphere for 3 h. After the reaction was completed, the reaction mixture was cooled to room temperature, water (40 mL) was added to the reaction solution, and the resulting mixture was extracted with ethyl acetate (20 mL×3). The organic layers were combined, washed with saturated sodium chloride aqueous solution (30 mL), dried over anhydrous sodium sulphate, filtered, and concentrated. The residue was separated and purified by silica gel column chromatography (dichloromethane: methanol (v/v) = 20:1) to obtain **1C** (1.47 g, yield: 78%).

LC-MS (ESI): m/z = 459.6 $[M+H]^+$.

Step 3:

**1C** (1.47 g, 3.21 mmol) was dissolved in 4 M hydrogen chloride 1,4-dioxane solution (10 mL), and the mixture was reacted at room temperature for 3 h. After the reaction was completed, the reaction solution was concentrated, and the residue was separated and purified by silica gel column chromatography (dichloromethane: methanol (v/v) = 10:1) to obtain **1D** (1.01 g, yield: 88%).

LC-MS (ESI): m/z = 359.4 $[M+H]^+$.

Step 4:

**1D** (200 mg, 0.56 mmol) was dissolved in dichloromethane (10 mL), and triethylamine (227 mg, 2.24 mmol) was added. Under nitrogen atmosphere, cyclopropylmethanesulphonyl chloride (130 mg, 0.84 mmol) was added dropwise in an ice bath, and the resulting mixture was reacted at room temperature overnight. After the reaction was completed, water (25 mL) was added to the reaction solution, and the resulting mixture was extracted with ethyl acetate (15 mL×3). The organic layers were combined, washed with saturated sodium chloride aqueous solution (20 mL), dried over anhydrous sodium sulphate, filtered, and concentrated. The residue was separated and purified by silica gel column chromatography (dichloromethane: methanol (v/v) = 20:1) to obtain **compound 1** (58 mg, yield: 22%).

$^1H$ NMR (400 MHz, $CDCl_3$) δ 7.53 (s, 1H), 7.08-7.04 (m, 1H), 6.87-6.81 (m, 2H), 6.41 (s, 1H), 3.96-3.92 (m, 4H), 3.84-3.78 (m, 2H), 3.69 (s, 2H), 3.68-3.66 (m, 2H), 2.80-2.71 (m, 4H), 2.01-1.86 (m, 3H), 1.39-1.27 (m, 2H), 1.10-0.99 (m, 1H), 0.67-0.60 (m, 2H), 0.31-0.26 (m, 2H).

LC-MS (ESI): m/z = 477.5 $[M+H]^+$.

**Example 2:**

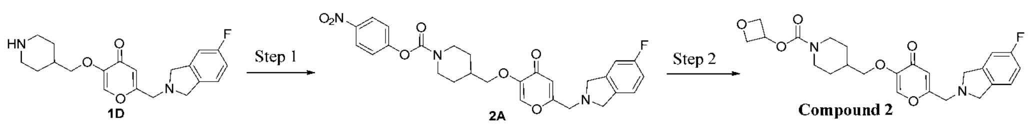

1D 2A Compound 2

Step 1:

**1D** (200 g, 0.56 mmol) and triethylamine (227 mg, 2.24 mmol) were dissolved in dichloromethane (1 mL). Under nitrogen atmosphere, p-nitrophenyl chloroformate (124 mg, 0.62 mmol) was added dropwise in an ice bath, and the resulting mixture was reacted at room temperature overnight. After the reaction was completed, water (25 mL) was added to the reaction solution, and the resulting mixture was extracted with ethyl acetate (15 mL×3). The organic layers were combined, washed with saturated sodium chloride aqueous solution (20 mL), dried over anhydrous sodium sulphate, filtered, and concentrated. The residue was separated and purified by silica gel column chromatography (dichloromethane: methanol (v/v) = 20:1) to obtain **2A** (178 mg, yield: 61%).

LC-MS (ESI): m/z = 524.6 $[M+H]^+$.

Step 2:

Oxetan-3-ol (101 mg, 1.36 mmol) was dissolved in tetrahydrofuran (4 mL). Under nitrogen atmosphere, 60% sodium hydride (27 mg, 0.68 mmol) was added in an ice bath. After the mixture was stirred for 15 min, a solution of **2A** (178 mg, 0.34 mmol) in DMF (3 mL) was added dropwise and the resulting mixture was reacted at room temperature for 2 h. After the reaction was completed, water (15 mL) was added to the reaction solution, and the resulting mixture was extracted with ethyl acetate (10 mL×3). The organic layers were combined, washed with saturated sodium chloride aqueous solution (15 mL), dried over anhydrous sodium sulphate, filtered, and concentrated. The residue was separated and purified by silica gel column chromatography (dichloromethane: methanol (v/v) = 20:1) to obtain **compound 2** (47 mg, yield: 30%).

$^1H$ NMR (400 MHz, $CDCl_3$) δ 7.53 (s, 1H), 7.09-7.04 (m, 1H), 6.87-6.81 (m, 2H), 6.41 (s, 1H), 5.36-5.28 (m, 1H), 4.83-4.79 (m, 2H), 4.61-4.56 (m, 2H), 4.15-4.09 (m, 2H), 3.96-3.92 (m, 4H), 3.71-3.63 (m, 4H), 2.88-2.66 (m, 2H), 2.04-1.96 (m, 1H), 1.86-1.77 (m, 2H), 1.25-1.14 (m, 2H).

LC-MS (ESI): m/z = 459.1 $[M+H]^+$.

**Example 3:**

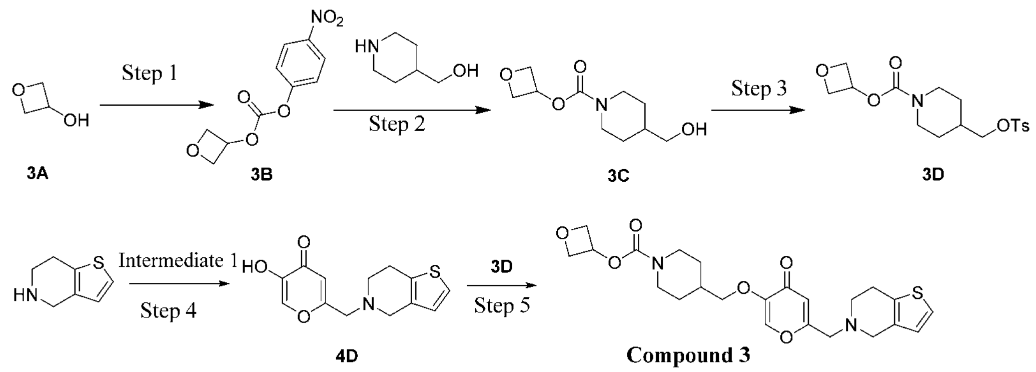

3A 3B 3C 3D
4D Compound 3

Step 1:

In a 1000 mL single-neck flask, **3A** (12 g, 161.99 mmol) was dissolved in dry dichloromethane (480 mL), and in an ice bath, triethylamine (40.98 g, 404.98 mmol) was added. After the addition, p-nitrophenyl chloroformate (39.18 g, 194.39 mmol) was slowly added in an ice bath and the resulting mixture was stirred at room temperature for 2 h. After the reaction was completed, the reaction solution was washed twice with saturated sodium carbonate aqueous solution (300 mL), dried over anhydrous sodium sulphate, and filtered. The filtrate was concentrated and purified by column chromatography (PE:EA=5:1) to obtain compound **3B** (18 g, yield: 46%).

Step 2:

In a 500 mL single-neck flask, compound **3B** (18 g, 75.26 mmol) was dissolved in dry dichloromethane (360 mL), and in an ice bath, triethylamine (26.65 g, 263.41 mmol) was added. After the addition, 4-hydroxymethylpiperidine (13.00 g, 122.89 mmol) was slowly added in an ice bath and the resulting mixture was stirred at room temperature for 2 h. After the reaction was completed, the reaction solution was diluted with dichloromethane (300 mL), and the organic phase was washed twice with water (300 mL), dried over anhydrous sodium sulphate, and filtered. The filtrate was concentrated to obtain compound **3C** (14.4 g).

Step 3:

In a 500 mL single-neck flask, compound **3C** (14.4 g, 66.90 mmol) was dissolved in dry dichloromethane (250 mL), and in an ice bath, triethylamine (23.70 g, 234.17 mmol) was added. After the addition, p-toluenesulphonyl chloride (14.03 g, 73.59 mmol) was slowly added in an ice bath and the resulting mixture was stirred at room temperature for 2 h. After the reaction was completed, the reaction solution was diluted with dichloromethane (300 mL), and the organic phase

was washed twice with water (200 mL), dried over anhydrous sodium sulphate and filtered. The filtrate was concentrated and purified by column chromatography (PE:EA=2:1) to obtain compound **3D** (20 g, yield: 81%).

Step 4:

4,5,6,7-tetrahydrothieno[3.2-c]pyridine (0.15 g, 1.1 mmol) and **intermediate 1** (0.2 g, 1.25 mmol) were dissolved in acetonitrile (20 mL), DIPEA (1 mL) was added, and the resulting mixture was reacted at room temperature for 3 h. Upon complete depletion of raw materials as monitored by LCMS, the system was concentrated and separated by column chromatography (ethyl acetate:petroleum ether =0:1-1:1) to obtain the target compound **4D** (0.3 g, yield: 91%).
LC-MS (ESI): m/z= 264.1 $[M+H]^+$.

Step 5:

Compound 4D (150 mg, 0.57 mmol) was dissolved in DMF (10 mL), 3D (211 mg, 0.57 mmol) and potassium carbonate (158 mg, 1.14 mmol) were added, and the resulting mixture was stirred at 80°C overnight. When the reaction was completed as monitored by TLC, water (20 mL) was poured into the reaction solution, the resulting mixture was extracted with ethyl acetate (30 mL×2), and the combined organic phases were dried over anhydrous sodium sulphate, filtered, and concentrated to obtain a crude, which was separated and purified by silica gel column chromatography (DCM: MeOH (v/v)= 10:1) to obtain **compound 3** (48 mg, yield: 18.3%).
$^1H$ NMR (400 MHz, $CDCl_3$) δ 7.59 (s, 1H), 7.11 - 7.07 (m, 1H), 6.72 - 6.69 (m, 1H), 6.48 (s, 1H), 5.43 - 5.35 (m, 1H), 4.91 - 4.84 (m, 2H), 4.68 - 4.63 (m, 2H), 4.23 - 4.14 (m, 2H), 3.76 - 3.70 (m, 2H), 3.65 (s, 2H), 3.58 (s, 2H), 2.96 - 2.89 (m, 4H), 2.10 - 2.00 (m, 1H), 1.94 - 1.83 (m, 2H), 1.36 - 1.20 (m, 4H).
LC-MS (ESI): m/z = 461.2 $[M+H]^+$.

**Example 4:**

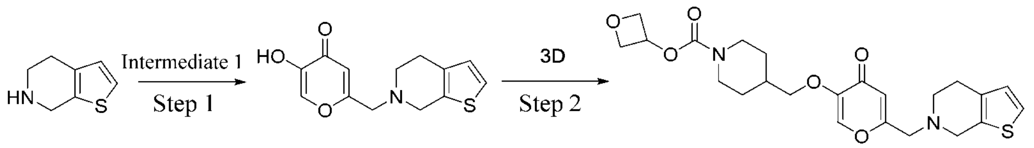


4A

Compound 4

Step 1:

4,5,6,7-tetrahydrothieno[2.3-c]pyridine (0.15 g, 1.1 mmol) and **intermediate 1** (0.2 g, 1.25 mmol) were dissolved in acetonitrile (20 mL), DIPEA (1 mL) was added, and the resulting mixture was reacted at room temperature for 3 h. Upon complete depletion of raw materials as monitored by LCMS, the system was concentrated and separated by column chromatography (ethyl acetate:petroleum ether =0:1-1:1) to obtain the target compound **4A** (0.3 g, yield: 91%).
LC-MS (ESI): m/z= 264.1 $[M+H]^+$.

Step 2:

Compound **4A** (150 mg, 0.57 mmol) was dissolved in DMF (10 mL), 3D (211 mg, 0.57 mmol) and potassium carbonate (158 mg, 1.14 mmol) were added, and the resulting mixture was stirred at 80°C overnight. When the reaction was completed as monitored by TLC, water (20 mL) was poured into the reaction solution, the resulting mixture was extracted with ethyl acetate (30 mL×2), and the combined organic phases were dried over anhydrous sodium sulphate, filtered, and concentrated to obtain a crude, which was separated and purified by silica gel column chromatography (DCM: MeOH (v/v)= 10:1) to obtain compound **4** (56 mg, yield: 21.3%).
$^1H$ NMR (400 MHz, $CDCl_3$) δ 7.59 (s, 1H), 7.13 - 7.09 (m, 1H), 6.81 - 6.76 (m, 1H), 6.48 (s, 1H), 5.44 - 5.34 (m, 1H), 4.92 - 4.83 (m, 2H), 4.69 - 4.61 (m, 2H), 4.24 - 4.14 (m, 2H), 3.80 (s, 2H), 3.77 - 3.70 (m, 2H), 3.59 (s, 2H), 2.91 - 2.87 (m, 2H), 2.79 - 2.75 (m, 2H), 2.10 - 2.00 (m, 1H), 1.96 - 1.80 (m, 2H), 1.35 - 1.20 (m, 4H).
LC-MS (ESI): m/z = 461.60 $[M+H]^+$.

**Example 5:**

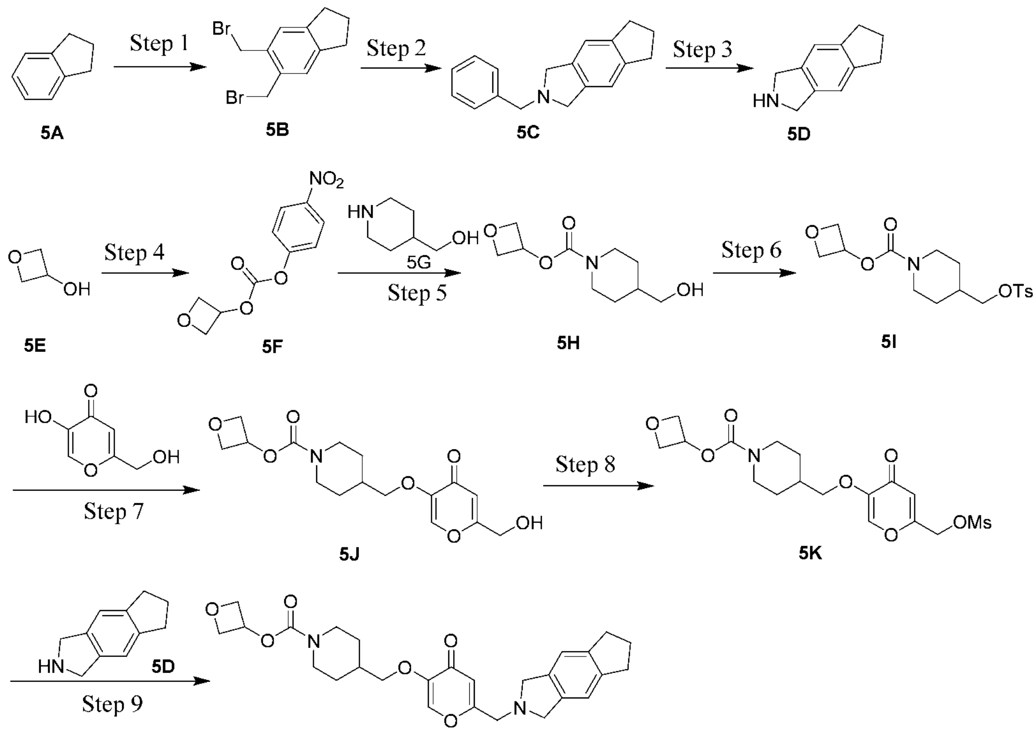


**Compound 5**

Step 1:

In a 500 mL single-neck flask, **5A** (20 g, 169.21 mmol) was dissolved in glacial acetic acid (200 mL). In an ice bath, paraformaldehyde (5.08 g, 169.21 mmol) was added. After the addition, 40% hydrobromic acid-glacial acetic acid solution (100 ml, 169.21 mmol) was slowly added dropwise in an ice bath, and the resulting mixture was stirred at room temperature for 30 min, slowly heated to 80°C and reacted overnight. After the reaction was completed, the reaction solution was diluted with ethyl acetate (500 mL), the organic phase was washed with water (300 mL) three times, washed twice with saturated sodium bicarbonate solution (100 mL), and washed once with saturated sodium chloride aqueous solution (100 mL), dried over anhydrous sodium sulphate, and filtered, and the filtrate was concentrated and purified by column chromatography (PE:EA=1:0) to obtain compound **5B** (28 g, yield: 54%).

Step 2:

Compound **5B** (28 g, 92.10 mmol) was dissolved in ethanol (280 ml), and potassium carbonate (44.55 g, 322.82 mmol) was added. Benzylamine (24.67 g, 230.25 mmol) was slowly added dropwise in an ice bath. After the addition, the resulting mixture was reacted at 80°C for 4 hours. After the reaction was completed, the reaction solution was filtered through diatomaceous earth pad, and the filtrate was concentrated and purified by column chromatography (PE:EA=40:1) to obtain compound **5C** (9.4 g, yield: 40%).

Step 3:

Compound **5C** (9.40 g, 92.10 mmol) was dissolved in methanol (940 ml), palladium on carbon (0.8 g, 7.52 mmol) was added, and the resulting mixture was subjected to hydrogen replacement three times and reacted at 40°C overnight. After the reaction was completed, the reaction solution was filtered through diatomaceous earth pad, and the filtrate was concentrated to obtain compound **5D** (4.6 g).

Step 4:

In a 1000 mL single-neck flask, **5E** (12 g, 161.99 mmol) was dissolved in dry dichloromethane (480 mL), and in an ice bath, triethylamine (40.98 g, 404.98 mmol) was added. After the addition, p-nitrophenyl chloroformate (39.18 g, 194.39 mmol) was slowly added in an ice bath and the resulting mixture was stirred at room temperature for 2 h. After the reaction was completed, the reaction solution was washed twice with saturated sodium carbonate aqueous solution (300 mL), dried over anhydrous sodium sulphate, and filtered. The filtrate was concentrated and purified by column chromatography (PE:EA=5:1) to obtain compound **5F** (18 g, yield: 46%).

Step 5:

In a 500 mL single-neck flask, compound **5F** (18 g, 75.26 mmol) was dissolved in dry dichloromethane (360 mL), and in an ice bath, triethylamine (26.65 g, 263.41 mmol) was added. After the addition, 4-hydroxymethylpiperidine **(5G)** (13.00 g, 122.89 mmol) was slowly added in an ice bath and the resulting mixture was stirred at room temperature for 2 h. After the reaction was completed, the reaction solution was diluted with dichloromethane (300 mL), and the organic phase was washed twice with water (300 mL), dried over anhydrous sodium sulphate, and filtered. The filtrate was concentrated to obtain compound **5H** (14.4 g).

Step 6:

In a 500 mL single-neck flask, compound **5H** (14.4 g, 66.90 mmol) was dissolved in dry dichloromethane (250 mL), and in an ice bath, triethylamine (23.70 g, 234.17 mmol) was added. After the addition, p-toluenesulphonyl chloride (14.03 g, 73.59 mmol) was slowly added in an ice bath and the resulting mixture was stirred at room temperature for 2 h. After the reaction was completed, the reaction solution was diluted with dichloromethane (300 mL), and the organic phase was washed twice with water (200 mL), dried over anhydrous sodium sulphate and filtered. The filtrate was concentrated and purified by column chromatography (PE:EA=2:1) to obtain compound **5I** (20 g, yield: 81%).

Step 7:

In a 250 mL single-neck flask, compound **5I** (9.5 g, 25.72 mmol) was dissolved in dry N,N-dimethylformamide (125 mL), and potassium carbonate (12.44 g, 90.02 mmol) and kojic acid (14.03 g, 73.59 mmol) were added. The resulting mixture was stirred at room temperature for 30 min, slowly heated to 100°C and reacted overnight. After the reaction was completed, the reaction solution was diluted with ethyl acetate (500 mL). The organic phase was washed three times with water (375 mL), washed once with saturated sodium chloride aqueous solution (100 mL), dried over anhydrous sodium sulphate and filtered. The filtrate was concentrated and purified by column chromatography (PE:EA=1:1) to obtain compound **5J** (8.4 g, yield: 96%).

Step 8:

In a 100 mL single-neck flask, compound **5J** (250 mg, 0.74 mmol) was dissolved in dry dichloromethane (15 mL), and in an ice bath, triethylamine (262 mg, 2.59 mmol) was added. After the addition, methanesulphonyl chloride (127 mg, 1.11 mmol) was slowly added in an ice bath and the resulting mixture was stirred at room temperature for 2 h. After the reaction was completed, the reaction solution was diluted with dichloromethane (100 mL), and the organic phase was washed twice with water (20 mL), dried over anhydrous sodium sulphate, and filtered. The filtrate was concentrated to obtain compound **5K** (310 mg).

Step 9:

In a 100 mL single-neck flask, compound **5K** (310 mg, 0.74 mmol) was dissolved in dry dichloromethane (15 mL), and in an ice bath, triethylamine (260 mg, 2.59 mmol) was added. After the addition, compound **5D** (140 mg, 0.89 mmol) was slowly added in an ice bath and the resulting mixture was stirred at room temperature for 2 h. After the reaction was completed, the reaction solution was diluted with dichloromethane (100 mL), and the organic phase was washed twice with water (20 mL), dried over anhydrous sodium sulphate, and filtered. The filtrate was concentrated and separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; and mobile phase B: water (containing 0.1% trifluoroacetic acid); gradient: isocratic elution using 30%-80% acetonitrile; cycle time: 15 minutes) to obtain compound 5.

LCMS m/z = 481.2 $[M+H]^+$.

**Example 6:**

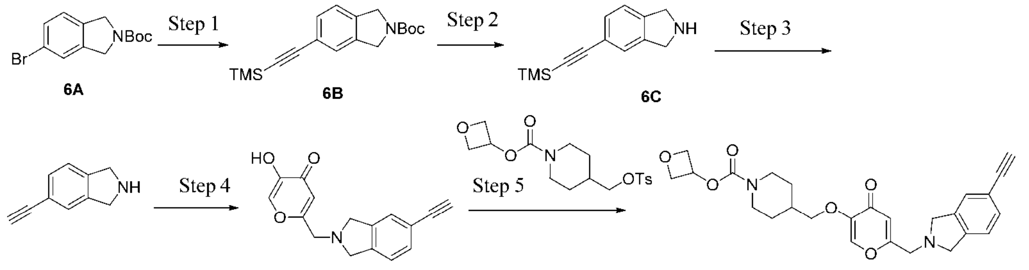


6A 6B 6C

6D 6E Compound 6

Step 1:

Compound **6A** (4.0 g, 13.4 mmol) and trimethylsilylacetylene (13.2 g, 134 mmol) were dissolved in tetrahydrofuran (50 mL), and bis(triphenylphosphine)palladium dichloride (941 mg, 1.34 mmol) and triethylamine (5.5 g, 53.6 mmol) were reacted at 50°C in a sealed tube for 4 h. After the reaction was completed as monitored by TLC, the reaction solution was dropwise added to ice water (100 mL), and the mixture was extracted with dichloromethane (200 mL x 3). The organic phases were combined, concentrated, and purified by silica gel column chromatography (PE:EA=15:1) to obtain the target compound **6B** (3.6 g, yield: 85%).
LCMS (ESI): m/z= 260.1 [M+H-tBu]

Step 2:

Compound **6B** (3.60 g, 11.4 mmol) was dissolved in anhydrous dioxane solution (10 mL), 4M hydrogen chloride dioxane solution (10 mL) was added, and the resulting mixture was reacted at room temperature for 8 h. The reaction was completed as monitored by LCMS and no hydrochloride adduct appeared. The reaction solution was spun to remove excess solvent, and the residue **6C** (some of the trimethylsilyl (TMS) protective groups were already removed in this step) was directly used in the next step (2.38 g).
LCMS (ESI): m/z= 216.2 [M+H]

Step 3:

Crude **6C** (2.38 g) and potassium carbonate (2.38 g, 17.2 mmol) were suspended in methanol (20 mL) and the resulting mixture was reacted at room temperature for 2 h. After the completion of the reaction, the reaction mixture was filtered, and the filtrate was concentrated, and separated by silica gel column chromatography (PE:EA = 1:1) to obtain the target compound **6D** (1.39 g, total yield over two steps: 83%).
LCMS (ESI): m/z= 144.2 [M+H]

Step 4:

Compound **6D** (1.39 g, 9.71 mmol) and intermediate **1** (1.56 g, 9.71 mmol) were dissolved in acetonitrile (15 mL), DIPEA (3.76 g, 29.1 mmol) was added, and the resulting mixture was reacted at room temperature for 16 h. After the completion of the reaction, the reaction solution was dropwise added to ice water (20 mL), and the resulting mixture was extracted with dichloromethane (50 mL x 3). The organic phases were combined and concentrated, and the resulting mixture was separated by silica gel column chromatography to obtain the target compound **6E** (DCM:MeOH =15:1) (620 mg, yield: 24%).
LCMS (ESI): m/z= 268.3 [M+H]

Step 5:

With reference to the synthetic step in step 7 of example 5, **compound 6** was obtained using **6E** and **5I** as the raw materials.
LC-MS (ESI): m/z =465.3 $[M+H]^+$.
$^1H$ NMR (400 MHz, $CDCl_3$): δ 7.60 (s, 1H), 7.25-7.16 (m, 4H), 6.49 (s, 1H), 5.16-5.08 (m, 1H), 4.25-4.12 (m, 4H),

4.07 (s, 4H), 4.02-3.97 (m, 2H), 3.79 (s, 2H), 3.75-3.71 (m, 2H), 2.89 (s, 5H), 2.09-2.02 (m, 1H), 1.93-7.84 (m, 2H), 1.33-1.21 (m, 2H).

**Example 7:**

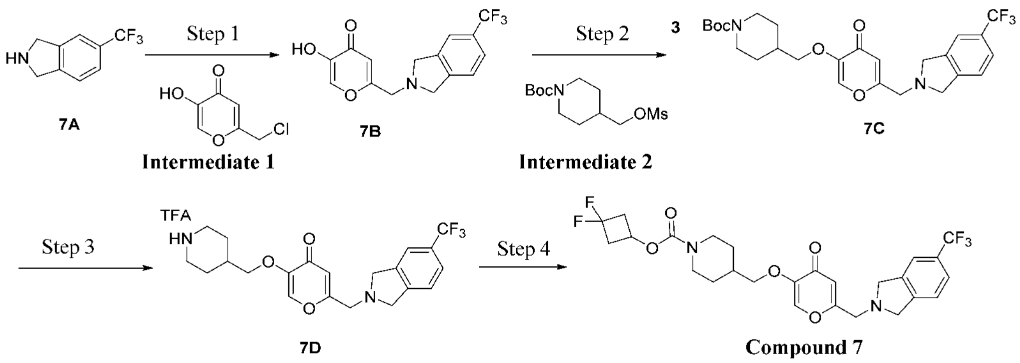


Step 1:

5-(Trifluoromethyl)isoindole (0.45 g, 2.42 mmol) and **intermediate 1** (0.47 g, 2.93 mmol) were dissolved in acetonitrile (20 mL), DIPEA (0.47 g, 3.63 mmol) was added, and the resulting mixture was reacted at room temperature for 3 hours. The reaction solution was concentrated under reduced pressure, and the residue was purified using a medium-pressure preparation instrument Biotage Isolera One (12 g silica gel column, eluents: 0-8% MeOH/DCM) to obtain compound **7B** (0.38 g, yield: 50.4%).

LC-MS (ESI): m/z= 312.3 $[M+H]^+$.

Step 2:

Compound **7B** (0.37 g, 1.19 mmol) and **intermediate 2** (0.42 g, 1.43 mmol) were dissolved in DMF (10 mL), potassium carbonate (0.21 g, 1.55 mmol) was added, and the resulting mixture was reacted at 80°C overnight. Upon complete depletion of raw materials as monitored by LCMS, the reaction solution was poured into ice water and quenched, and the resulting mixture was extracted with ethyl acetate (20 mL×3). The combined organic phases were washed with saturated sodium chloride aqueous solution (20 mL×2), dried over anhydrous sodium sulphate, filtered and concentrated. The residue was purified using a medium-pressure preparation instrument Biotage Isolera One (12 g silica gel column, eluents: 0-5% MeOH/DCM) to obtain compound **7C** (0.26 g, yield: 42.9%).

LC-MS (ESI): m/z= 409.2 $[M+H-100]^+$.

Step 3:

Compound **7C** (0.26 g, 0.51 mmol) was dissolved in DCM (10 mL), trifluoroacetic acid (1 mL) was added, and the resulting mixture was reacted at room temperature for 1 hour. Upon complete depletion of raw materials as monitored by LCMS, the reaction solution was concentrated to obtain compound **7D** (0.20 g), which was directly used in the next step without purification.

LC-MS (ESI): m/z= 409.1 $[M+H]^+$.

Step 4:

In a 50 mL single-neck flask, sodium hydride (0.02 g, 0.48 mmol), dry tetrahydrofuran (10 mL) and 3,3-difluorocyclobutanol (0.078 g, 0.72 mmol) were added at 0°C, and the resulting mixture was stirred for 15 minutes. Carbonyldiimidazole (0.078 g, 0.48 mmol) was added and the mixture was stirred for another 1 hour. Compound **7D** (0.13 g, 0.24 mmol) and a solution of triethylamine (0.025 g, 0.24 mmol) in tetrahydrofuran were added, and the resulting mixture was warmed to room temperature, stirred and reacted for 2 hours. The reaction solution was poured into water and the resulting mixture was extracted with ethyl acetate (20 mL×3). The combined organic phases were washed with saturated sodium chloride aqueous solution (20 mL×2), dried over anhydrous sodium sulphate, filtered and concentrated. The residue was purified using a medium-pressure preparation instrument Biotage Isolera One (12 g silica gel column, eluents:

0-5% MeOH/DCM) to obtain compound 7 (40 mg, yield: 30.7%).

LC-MS (ESI): m/z= 543.2 $[M+H]^+$.

$^1H$ NMR (400 MHz, $CDCl_3$): δ 7.59 (s, 1H), 7.52 - 7.49 (m, 2H), 7.46 - 7.30 (m, 1H), 6.49 (s, 1H), 4.92 - 4.85 (m, 1H), 4.21 - 4.14 (m, 2H), 4.09 (s, 4H), 3.79 (s, 2H), 3.73 - 3.68 (m, 2H), 3.03 - 2.99 (m, 2H), 2.90 - 2.82 (m, 2H), 2.68 - 2.63 (m, 2H), 2.09 - 2.03 (m, 1H), 1.90 - 1.87 (m, 2H), 1.30 - 1.21 (m, 2H).

**Example 8:**

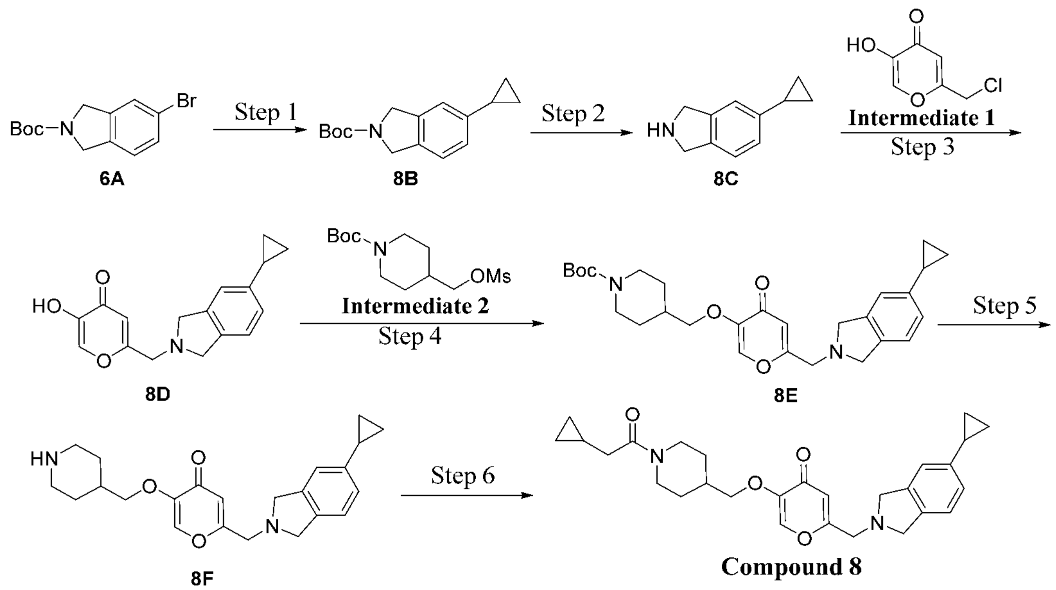


Step 1:

**6A** (1 g, 3.36 mmol), cyclopropyl boronic acid (576 mg, 6.72 mmol), bis(triphenylphosphine)palladium dichloride (236 mg, 0.34 mmol) and potassium phosphate (2.14 g, 10.08 mmol) were dissolved in mixed solvents of toluene (20 mL) and water (2 mL), and the resulting mixture was reacted at 100°C under nitrogen atmosphere overnight. After the reaction was completed, the reaction mixture was cooled to room temperature, water (50 mL) was added to the reaction solution, and the resulting mixture was extracted with ethyl acetate (30 mL×3). The organic layers were combined, washed with saturated sodium bicarbonate solution (40 mL), dried over anhydrous sodium sulphate, filtered, and concentrated. The residue was separated and purified by silica gel column chromatography (petroleum ether: ethyl acetate (v/v) = 20:1) to obtain **8B** (570 mg, yield: 66%).

LC-MS (ESI): m/z = 204.1 $[M+H-56]^+$.

Step 2:

**8B** (570 mg, 2.20 mmol) was dissolved in 4 M hydrogen chloride 1,4-dioxane solution (8 mL), and the resulting mixture was reacted at room temperature overnight. After 16 h, the reaction mixture was subjected to rotary evaporation under reduced pressure to remove the solvent. Saturated sodium bicarbonate solution (25 mL) was added to the residue and the mixture was extracted with ethyl acetate (20 mL X 3). The organic layers were combined, dried over anhydrous sodium sulphate and concentrated. The residue was separated and purified by silica gel column chromatography (dichloromethane: methanol (v/v) = 20:1) to obtain **8C** (335 mg, yield: 96%).

LC-MS (ESI): m/z = 160.1 $[M+H]^+$.

Step 3:

**8C** (335 mg, 2.11 mmol), **intermediate 1** (370 mg, 2.32 mmol) and N,N-diisopropylethylamine (639 mg, 6.33 mmol) were dissolved in acetonitrile (15 mL), and the resulting mixture was reacted at room temperature overnight. After the reaction was completed, the reaction solution was concentrated, and the residue was separated and purified by silica gel column chromatography (dichloromethane: methanol (v/v) = 40:1) to obtain **8D** (382 mg, yield: 64%).

LC-MS (ESI): m/z= 283.3 [M+H]+.

Step 4:

**8D** (200 mg, 0.71 mmol), **intermediate 2** (229 mg, 0.78 mmol) and potassium carbonate (294 mg, 2.31 mmol) were dissolved in DMF (10 mL), and the resulting mixture was reacted at 70°C under nitrogen atmosphere for 3 h. After the reaction was completed, the reaction mixture was cooled to room temperature, water (25 mL) was added, and the resulting mixture was extracted with ethyl acetate (15 mL×3). The organic layers were combined, washed with saturated sodium chloride aqueous solution (30 mL), dried over anhydrous sodium sulphate, filtered, and concentrated. The residue was separated and purified by silica gel column chromatography (dichloromethane: methanol (v/v)= 20:1) to obtain **8E** (268 mg, yield: 79%).

LC-MS (ESI): m/z= 481.3 [M+H]+.

Step 5:

**8E** (268 mg, 0.56 mmol) was dissolved in 4 M hydrogen chloride 1,4-dioxane solution (5 mL), and the mixture was reacted at room temperature for 1 h. After the reaction was completed, the reaction mixture was subjected to rotary evaporation under reduced pressure to remove the solvent and then concentrated. The residue was separated and purified by silica gel column chromatography (dichloromethane: methanol (v/v)= 20:1) to obtain **8F** (200 mg, yield: 94%).

Step 6:

**8F** (200 mg, 0.53 mmol) was dissolved in dichloromethane (10 mL), N,N-diisopropylethylamine (210 mg, 1.59 mmol) and HATU (264 mg, 0.69 mmol) were added, and the resulting mixture was stirred at room temperature for 10 min. Cyclopropyl acetic acid (53 mg, 0.53 mmol) was added and the mixture was reacted at room temperature for 1 h. After the reaction was completed, water (25 mL) was added to the reaction solution, and the resulting mixture was extracted with ethyl acetate (15 mL×3). The organic layers were combined, washed with saturated sodium chloride aqueous solution (25 mL), dried over anhydrous sodium sulphate, filtered, and concentrated. The residue was separated and purified by silica gel column chromatography (dichloromethane: methanol (v/v) = 20:1) to obtain **compound 8** (60 mg, yield: 25%).

$^1$H NMR (400 MHz, $CD_3Cl$) δ 7.59 (s, 1H), 7.09-7.06 (m, 1H), 6.96-6.92 (m, 1H), 6.90 (s, 1H), 6.48 (s, 1H), 4.73-4.67 (m, 1H), 3.99 (s, 4H), 3.90-3.85 (m, 1H), 3.78-3.73 (m, 3H), 3.71-3.65 (m, 1H), 3.09-3.01 (m, 1H), 2.63-2.55 (m, 1H), 2.30-2.73 (, 2H), 2.19-2.08 (m, 1H), 2.04-1.97 (m, 1H), 1.92-1.81 (m, 2H), 1.28-1.18 (m, 2H), 1.09-0.99 (m, 1H), 0.97-0.91 (m, 2H), 0.68-0.63 (m, 2H), 0.59-0.53 (m, 2H), 0.20-0.15 (m, 2H).

LC-MS (ESI): m/z = 463.2 [M+H]+.

**Example 9:**

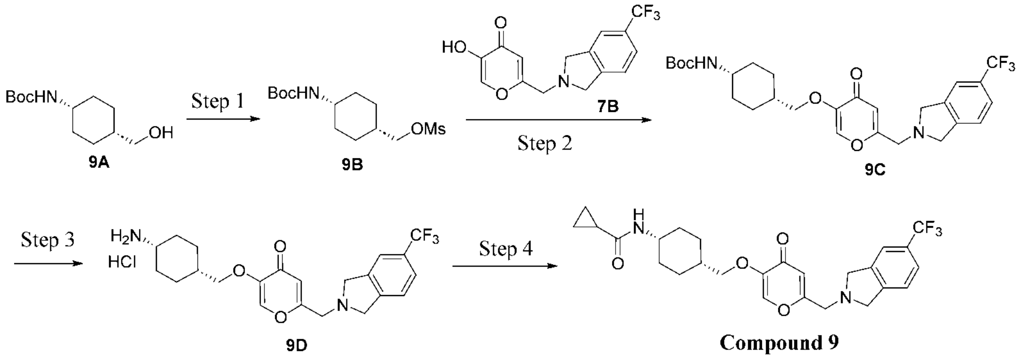


Step 1:

**9A** (500 mg, 2.18 mmol) and N,N-diisopropylethylamine (845 mg, 6.54 mmol) were dissolved in dichloromethane (10 mL). Under nitrogen atmosphere, methanesulphonyl chloride (374 mg, 3.27 mmol) was added dropwise in an ice bath and the mixture was reacted at room temperature overnight. After the reaction was completed, saturated sodium bicarbonate solution (30 mL) was added to the reaction solution to quench the reaction, and the resulting mixture was

extracted with dichloromethane (20 mL×3). The organic layers were combined, washed with 1 M diluted aqueous hydrochloric acid solution (25 mL), dried over anhydrous sodium sulphate, filtered and concentrated to obtain **9B** (580 mg).
$^1H$ NMR (400 MHz, $CD_3Cl$) δ 4.61 (s, 1H), 4.08-4.00 (m, 2H), 3.69 (s, 1H), 2.95 (s, 3H), 1.85-1.75 (m, 1H), 1.67-1.50 (m, 6H), 1.38 (s, 9H), 1.30-1.16 (m, 2H).
LC-MS (ESI): m/z= 252.2 $[M+H-56]^+$.

Step 2:

**9B** (436 mg, 1.42 mmol), **7B** (400 mg, 1.29 mmol) and potassium carbonate (535 mg, 3.87 mmol) were dissolved in DMF (10 mL), and the resulting mixture was reacted at 60°C under nitrogen atmosphere overnight. After the reaction was completed, the reaction mixture was cooled to room temperature, water (30 mL) was added, and the resulting mixture was extracted with ethyl acetate (20 mL×3). The organic layers were combined, washed with saturated sodium chloride aqueous solution (30 mL), dried over anhydrous sodium sulphate, filtered, and concentrated. The residue was separated and purified by silica gel column chromatography (dichloromethane: methanol (v/v) = 20:1) to obtain **9C** (470 mg, yield: 70%).
LC-MS (ESI): m/z= 423.1 $[M+H-100]^+$.

Step 3:

**9C** (470 mg, 0.90 mmol) was dissolved in 4 M hydrogen chloride 1,4-dioxane solution (8 mL), and the mixture was reacted at room temperature for 1 h. After the reaction was completed, the reaction mixture was subjected to rotary evaporation under reduced pressure to remove the solvent and concentrated to obtain crude **9D** (350 mg, 92%), which was directly used in the next reaction without further purification.
LC-MS (ESI): m/z = 423.4 $[M+H]^+$.

Step 4:

Cyclopropanecarboxylic acid (48 mg, 0.56 mmol) was dissolved in DMF (6 mL), N-methylimidazole (69 mg, 0.84 mmol) and TCFH (86 mg, 0.31 mmol) were added, and the resulting mixture was stirred at room temperature for 10 min. **9D** (120 mg, 0.28 mmol) was subsequently added and the mixture was reacted at room temperature for 1 h. After the reaction was completed, water (25 mL) was added to the reaction solution, and the resulting mixture was extracted with ethyl acetate (15 mL×3). The organic layers were combined, washed with saturated sodium chloride aqueous solution (25 mL), dried over anhydrous sodium sulphate, filtered, and concentrated. The residue was separated and purified by silica gel column chromatography (dichloromethane: methanol (v/v) = 25:1) to obtain **compound 9** (33 mg, yield: 24%).
$^1H$ NMR (400 MHz, $CD_3Cl$) δ 7.61 (s, 1H), 7.51-7.48 (m, 1H), 7.46 (s, 1H), 7.33-7.30 (m, 1H), 6.49 (s, 1H), 6.00 (s, 1H), 4.14-4.08 (m, 5H), 3.81-3.77 (m, 4H), 1.97-1.90 (m, 1H), 1.76-1.59 (m, 6H), 1.55-1.44 (m, 2H), 1.42-1.33 (m, 1H), 0.98-0.93 (m, 2H), 0.74-0.68 (m, 2H).
LC-MS (ESI): m/z = 491.3 $[M+H]^+$.

**Example 10:**

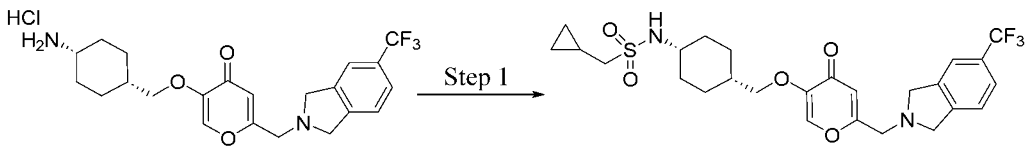


9D

Compound 10

Step 1:

**9D** (100 mg, 0.24 mmol) and N,N-diisopropylethylamine (93 mg, 0.72 mmol) were dissolved in dichloromethane (8 mL). Under nitrogen atmosphere, cyclopropanemethanesulphonyl chloride (56 mg, 0.36 mmol) was added dropwise in an ice bath, and the mixture was reacted at room temperature overnight. After the reaction was completed, water (25 mL) was added to the reaction solution, and the resulting mixture was extracted with ethyl acetate (15 mL×3). The organic layers were combined, washed with saturated sodium chloride aqueous solution (25 mL), dried over anhydrous sodium sulphate, filtered, and concentrated. The residue was separated and purified by silica gel column chromatography

(dichloromethane: methanol (v/v)= 25:1) to obtain **compound 10** (25 mg, yield: 19%).

$^1$H NMR (400 MHz, $CD_3Cl$) δ 7.55-7.52 (m, 2H), 7.48 (s, 1H), 7.35-7.33 (m, 1H), 6.49 (s, 1H), 4.68 (s, 1H), 4.43 (s, 4H), 4.04 (s, 2H), 3.69-3.61 (m, 3H), 2.89-2.86 (m, 2H), 1.90-1.83 (m, 1H), 1.78-1.73 (m, 2H), 1.69-1.59 (m, 4H), 1.45-1.35 (m, 2H), 1.12-1.03 (m, 1H), 0.66-0.60 (m, 2H), 0.34-0.30 (m, 2H).

LC-MS (ESI): m/z = 541.3 [M+H]$^+$.

**Example 11:**

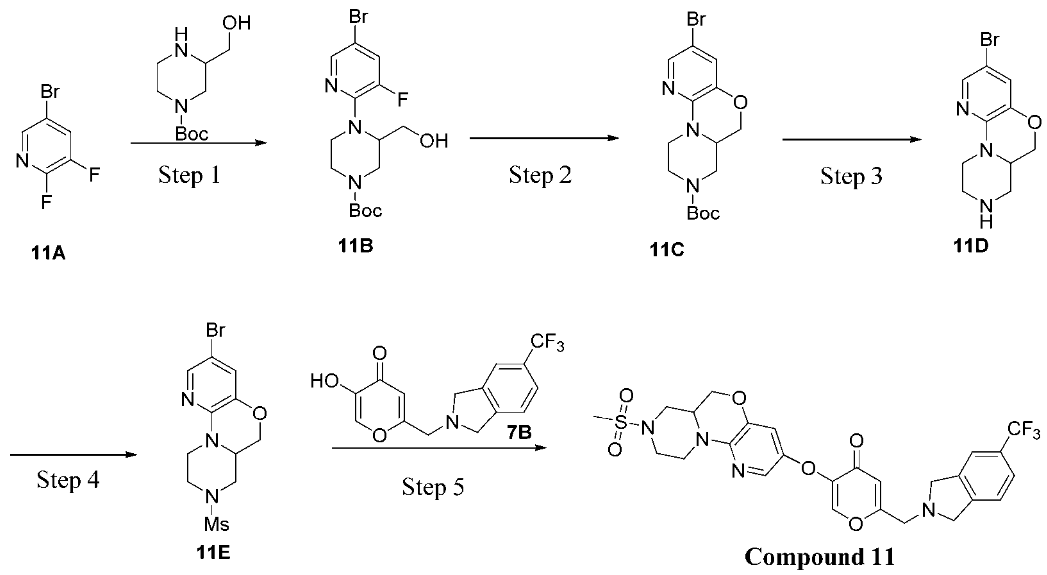


11A 11B 11C 11D 11E 7B Compound 11

Step 1:

Compound **11A** (20.0 g, 103 mmol), tert-butyl 3-(hydroxymethyl)piperazine-1-carboxylate (24.6 g, 113 mmol) and N, N-diisopropylethylamine (26.6 g, 206 mmol) were added to a 500 mL round bottom flask, N-methylpyrrolidone (150 mL) was then added, and the mixture was heated to 130°C and reacted overnight. The reaction was cooled to room temperature. The reaction solution was poured into water (600 mL), and the mixture was extracted with ethyl acetate (100 mL x 5). The organic phases were combined and washed three times with water (150 mL). The organic phase was dried over anhydrous sodium sulphate, filtered and concentrated. The resulting crude was separated and purified by silica gel column chromatography (petroleum ether/ethyl acetate (v/v) = 100/0-50/50) to obtain the target compound **11B** (21.3 g, yield: 52.9%).
LC-MS (ESI): m/z =390.1 [M+H]$^+$

Step 2:

Compound **11B** (21.3 g, 54.6 mmol) and potassium tert-butoxide (12.2 g, 109 mmol) were dissolved in tert-butanol (150 mL), and the resulting mixture was heated to 80°C and continuously stirred for 3 h. After the reaction was completed, the system was cooled to room temperature, and the reaction was quenched by adding water (400 mL). The system was extracted with ethyl acetate (100 mL x 5), and the organic phases were combined, washed with saturated sodium chloride aqueous solution, and dried over anhydrous sodium sulphate. After filtration, the reaction solution was concentrated under reduced pressure and then separated and purified by silica gel column chromatography (petroleum ether/ethyl acetate (v/v) = 100/0-70/30) to obtain the target compound **11C** (19.4 g, yield: 96.0%).
LC-MS (ESI): m/z =370.1 [M+H]$^+$

Step 3:

At room temperature, compound **11C** (1.80 g, 4.86 mmol) was dissolved in dichloromethane (24 mL), trifluoroacetic acid (8 mL) was added, and the resulting mixture was continuously stirred at room temperature for 1 h. After the disappearance of the raw materials as monitored by LCMS, the reaction mixture was directly concentrated under reduced

pressure to obtain the trifluoroacetate of compound **11D** (crude, 2.45 g), which was directly used in the next reaction without purification.

LC-MS (ESI): m/z =270.1 $[M+H]^+$.

Step 4:

In an ice-water bath, the trifluoroacetate of compound **11D** (crude, 2.45 g) obtained in the previous step was dissolved in dichloromethane (60 mL), triethylamine (2.95 g, 29.2 mmol) and methanesulphonyl chloride (836 mg, 7.30 mmol) were successively added, and the mixture was reacted at 0°C for 2 h. After the reaction was completed as monitored by LCMS, saturated aqueous ammonium chloride solution (50 mL) was added to quench the reaction, and the reaction mixture was extracted with dichloromethane (50 mL x 5). The organic phases were combined, dried over anhydrous sodium sulphate, filtered and concentrated, and the crude was separated and purified by silica gel column chromatography (dichloromethane/methanol (v/v) = 100/0-90/10) to obtain compound **11E** (1.58 g, two-step yield: 93.5%).

LC-MS (ESI): m/z =348.1 $[M+H]^+$.

Step 5:

Under nitrogen protection, compound **11E** (400 mg, 1.15 mmol), **7B** (536 mg, 1.72 mmol), X-Phos-Pd-G2 (181 mg, 0.230 mmol), caesium carbonate (749 mg, 2.30 mmol) and potassium iodide (381 mg, 2.30 mmol) were all dissolved in 1, 4-dioxane (30 mL), and the resulting mixture was warmed to 100°C and reacted overnight. After the reaction was completed as monitored by LCMS, the reaction was directly concentrated under reduced pressure, and the crude was preliminarily separated and purified by silica gel column chromatography (dichloromethane/methanol (v/v) =100/0-85/15), and then separated and purified by preparative HPLC to obtain the racemate compound 11 (24 mg, yield: 3.6%).

Preparative HPLC separation and purification method: 1. Instrument: waters 2767 preparative liquid chromatographic instrument; chromatographic column: SunFire @ Prep C18 (19 mm x 250 mm). 2. The sample was filtered with a 0.45 μm filter to prepare a sample solution. 3. Preparative chromatography conditions: a. composition of mobile phases A and B: mobile phase A: acetonitrile; and mobile phase B: water (containing 0.1% ammonium acetate); b. gradient elution, mobile phase A: 5% - 50%; c. flow rate: 12 mL/min; d. elution time: 30 min.

$^1H$ NMR (400 MHz, DMSO-d6) δ 9.68 - 9.21 (m, 1H), 8.44 (d, 1H), 7.63 (d, 1H), 7.61 - 7.55 (m, 2H), 7.48 (d, 1H), 6.45 (s, 1H), 4.69 - 4.62 (m, 1H), 4.42 (dd, 1H), 4.11 - 4.05 (m, 4H), 4.02 (dd, 1H), 3.92 (s, 2H), 3.72 - 3.55 (m, 3H), 2.98 - 2.88 (m, 4H), 2.83 (td, 1H), 2.65 (t, 1H).

LC-MS (ESI): m/z =579.3 $[M+H]^+$.

**Example 12:**

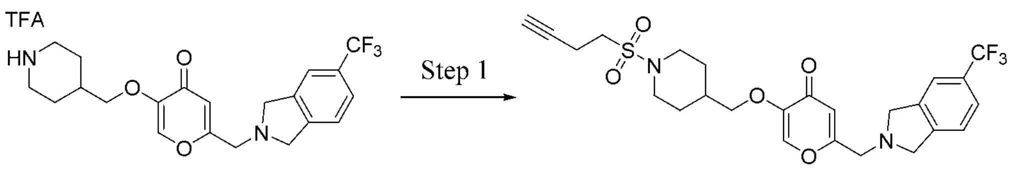


7D

Compound 12

Step 1:

**7D** (300 mg, 0.67 mmol) and triethylamine (203 mg, 2.01 mmol) were dissolved in dichloromethane (5 ml), and in an ice bath, 3-butyne-1-sulphonyl chloride (152 mg, 1.00 mmol) was added, and the resulting mixture was reacted for 1 h. After the completion of the reaction, the excess solvent was removed from the reaction solution using a rotary evaporator, and the resulting mixture was purified by silica gel column chromatography (DCM: MeOH (v/v) = 20:1) to obtain the target compound **12** (142 mg, yield: 39%).

LCMS(ESI): m/z = 525.3 $[M+H]^+$

$^1H$ NMR (400 MHz, DMSO) δ 8.14 (s, 1H), 7.63 (s, 1H), 7.59-7.55 (m, 1H), 7.49-7.45 (m, 1H), 6.39 (s, 1H), 4.03 (s, 4H), 3.81 (s, 2H), 3.72 (d, 2H), 3.62 (d, 2H), 3.27-3.20 (m, 2H), 3.00-2.96 (m, 1H), 2.96-2.82 (m, 2H), 2.64-2.55 (m, 2H), 1.88-1.78 (d, 3H), 1.35 - 1.21 (m, 2H).

**Example 13:**

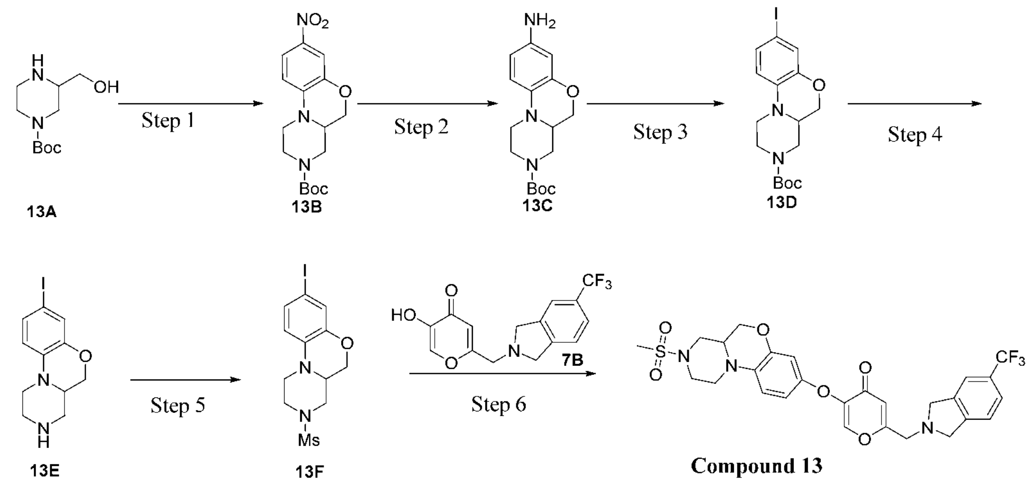


13A 13B 13C 13D 13E 13F 7B **Compound 13**

Step 1:

To a 250 mL single-mouth flask, **13A** (10.0 g, 46.3 mmol), 1,2-difluoro-4-nitrobenzene (7.3 g, 46.3 mmol), potassium hydroxide (7.8 g, 138.9 mmol), and N, N-dimethylformamide (100 mL) were successively added. The mixture was reacted at room temperature for 6 hours, then warmed to 60°C and reacted for 24 hours. After filtration, ethyl acetate (500 mL) was added to the filtrate, the mixture was washed with water (100 mL×3), and the organic phase was dried over anhydrous sodium sulphate and filtered. The filtrate was concentrated under reduced pressure and separated and purified by silica gel column chromatography (ethyl acetate/petroleum ether (v/v) = 20/100) to obtain **13B** (10.0 g, yield: 64.5%).
LC-MS (ESI): m/z = 336.2 $[M+H]^+$.

Step 2:

To a 250 mL single-neck flask, **13B** (10.0 g, 29.9 mmol), ethyl acetate (100 mL) and palladium on carbon (2 g) were successively added, and the mixture was hydrogenated and reacted at room temperature for 12 hours. After filtration, the filtrate was concentrated under reduced pressure to obtain crude **13C** (7.0 g).
LC-MS (ESI): m/z = 306.2 $[M+H]^+$.

Step 3:

To a 100 mL single-mouth flask, acetonitrile (30 mL), tert-butyl nitrite (1.5 g, 14.7 mmol), and cuprous iodide (2.24 g, 11.8 mmol) were successively added and heated to 65°C, and a solution **of 13C** (3.0 g, 9.8 mmol) in acetonitrile (10 mL) was dropwise added. After the dropwise addition was completed, the system was reacted at 65°C for 4 hours, and after heating was turned off, the system was further reacted for 12 hours, concentrated under reduced pressure, and then separated and purified by silica gel column chromatography (ethyl acetate/petroleum ether (v/v) = 20/100) to obtain **13D** (0.4 g, yield: 12.6%).
LC-MS (ESI): m/z = 361.2 $[M-56+H]^+$.

Step 4:

At room temperature, compound **13D** (2.0 g, 4.80 mmol) was dissolved in dichloromethane (40 mL), trifluoroacetic acid (10 mL) was added, and the resulting mixture was continuously stirred at room temperature for 1 h. After the disappearance of the raw materials as monitored by LCMS, the reaction mixture was directly concentrated at 30°C under reduced pressure to obtain the trifluoroacetate of compound **13E** (crude, 2.98 g), which was directly used in the next reaction without purification.
LC-MS (ESI): m/z =317.1 $[M+H]^+$.

Step 5:

In an ice-water bath, the trifluoroacetate of compound 13E (crude, 2.98 g) obtained in the previous step was dissolved in dichloromethane (80 mL), triethylamine (2.92 g, 28.8 mmol) and methanesulphonyl chloride (1.10 g, 9.61 mmol) were successively added, and the mixture was warmed to room temperature and reacted for 4 h. After the reaction was completed as monitored by LCMS, saturated aqueous ammonium chloride solution (50 mL) was added to quench the reaction, and the reaction mixture was extracted with dichloromethane (50 mL x 5). The organic phases were combined, dried over anhydrous sodium sulphate, filtered and concentrated, and the crude was separated and purified by silica gel column chromatography (petroleum ether/ethyl acetate (v/v) = 100/0-65/35) to obtain compound **13F** (1.46 g, yield: 77.1%).
LC-MS (ESI): m/z =395.1 $[M+H]^+$.

Step 5:

Under nitrogen protection, compound **13F** (300 mg, 0.761 mmol), **7B** (355 mg, 1.14 mmol), X-Phos-Pd-G2 (120 mg, 0.152 mmol), and caesium carbonate (496 mg, 1.52 mmol) were all dissolved in 1, 4-dioxane (30 mL), and the resulting mixture was warmed to 100°C and reacted overnight. After the reaction was completed as monitored by LCMS, the reaction was directly concentrated under reduced pressure, and the crude was preliminarily separated and purified by silica gel column chromatography (dichloromethane/methanol (v/v) = 100/0-85/15), and then separated and purified by preparative HPLC to obtain the racemate compound **13** (27 mg, yield: 6.1%).
Preparative HPLC separation and purification method: 1. Instrument: waters 2767 preparative liquid chromatographic instrument; chromatographic column: SunFire @ Prep C18 (19 mm x 250 mm). 2. The sample was filtered with a 0.45 μm filter to prepare a sample solution. 3. Preparative chromatography conditions: a. composition of mobile phases A and B: mobile phase A: acetonitrile; and mobile phase B: water (containing 0.1% trifluoroacetic acid); b. gradient elution, mobile phase A: 5% - 50%; c. flow rate: 12 mL/min; d. elution time: 30 min. After the preparation, the mixture was adjusted to about pH 8 with saturated aqueous sodium bicarbonate solution and extracted with ethyl acetate to obtain the product.
$^1H$ NMR (400 MHz, DMSO-$d_6$) δ 9.58 - 9.02 (m, 1H), 7.66 - 7.51 (m, 4H), 7.48 (d, 1H), 7.03 (d, 1H), 6.39 (s, 1H), 4.36 (dd, 1H), 4.07 (s, 3H), 4.06 - 3.93 (m, 2H), 3.90 (s, 2H), 3.62 (t, 2H), 3.45 - 3.18 (m, 2H, overlapped), 2.97 - 2.78 (m, 5H), 2.61 (t, 1H).
LC-MS (ESI): m/z =578.2 $[M+H]^+$.
Chromatographic analytical conditions: 1. Instrument: Shimadzu LC-20AT; 2. chromatographic column: Xtimate C18 4.6×50mm, 3 μm; 3. mobile phase system: A for 0.05% TFA in H2O; B for ACN; 4. gradient: B 5%-95%; 5. flow rate: 1.0 mL/min, run time: 10 min. retention time: 3.650 min.

**Example 14:**

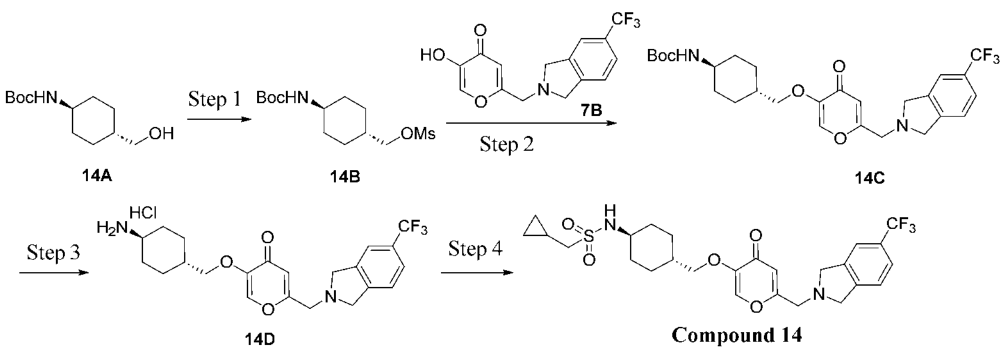

14A 14B 14C 14D Compound 14

Step 1:

**14A** (1.02 g, 4.45 mmol) and N-methylmorpholine (900 mg, 8.90 mmol) were dissolved in dichloromethane (20 mL). Under nitrogen atmosphere, methanesulphonyl chloride (610 mg, 5.34 mmol) was added dropwise in an ice bath, and the resulting mixture was reacted at room temperature overnight. After the reaction was completed, saturated sodium bicarbonate solution (30 mL) was added to the reaction solution to quench the reaction, and the resulting mixture was extracted with dichloromethane (20 mL×3). The organic layers were combined, washed with 1 M diluted aqueous hydrochloric acid solution (25 mL), dried over anhydrous sodium sulphate, filtered and concentrated to obtain **14B** (1.05

mg, yield: 76.7%).

$^1H$ NMR (400 MHz, $CDCl_3$) δ 4.38 (s, 1H), 4.04 - 3.99 (m, 2H), 3.37 (s, 1H), 2.99 (s, 3H), 2.13 - 2.00 (m, 2H), 1.92 - 1.80 (m, 2H), 1.76 - 1.63 (m, 1H), 1.43 (s, 9H), 1.18 - 1.03 (m, 4H).

LC-MS (ESI): m/z= 252.2 $[M+H-56]^+$.

Step 2:

With reference to the operations (step 2) in **Example 9, compound 14C** (400 mg, yield: 50%) was obtained using **14B** (470 mg, 1.53 mmol) as the raw material.

LC-MS (ESI): m/z = 467.50 $[M+H-56]^+$.

Step 3:

With reference to the operation (step 3) in **example 9,** the hydrochloride of **compound 14D** (crude, 472 mg) was obtained using **14C** (400 mg, 0.77 mmol) as the raw material, and the crude was directly used in the next reaction without further purification.

LC-MS (ESI): m/z = 423.2 $[M+H]^+$.

Step 4:

**14D** (220 mg, 0.52 mmol) and triethylamine (158 mg, 1.56 mmol) were dissolved in dichloromethane (10 mL). Under nitrogen atmosphere, cyclopropanemethanesulphonyl chloride (96 mg, 0.62 mmol) was added dropwise in an ice bath, and the mixture was reacted at room temperature overnight. After the reaction was completed, water (25 mL) was added to the reaction solution, and the resulting mixture was extracted with ethyl acetate (15 mL×3). The organic layers were combined, washed with saturated sodium chloride aqueous solution (25 mL), dried over anhydrous sodium sulphate, filtered, and concentrated. The residue was separated and purified by silica gel column chromatography (dichloromethane: methanol (v/v)= 20:1) to obtain **compound 14** (51 mg, yield: 18.1%).

LC-MS (ESI): m/z = 541.60 $[M+H]^+$.

$^1H$ NMR (400 MHz, $CDCl_3$) δ 7.57 (s, 1H), 7.52 - 7.48 (m, 1H), 7.46 (s, 1H), 7.33 - 7.28 (m, 1H), 6.48 (s, 1H), 4.10 (s, 4H), 4.08 - 4.03 (m, 1H), 3.79 (s, 2H), 3.68 - 3.64 (m, 2H), 3.35 - 3.24 (m, 1H), 2.97 - 2.91 (m, 2H), 2.18 - 2.10 (m, 2H), 2.00 - 1.94 (m, 2H), 1.87 - 1.77 (m, 1H), 1.30 - 1.25 (m, 2H), 1.18 - 1.13 (m, 2H), 0.74 - 0.66 (m, 2H), 0.43 - 0.37 (m, 2H).

**Example 15:**

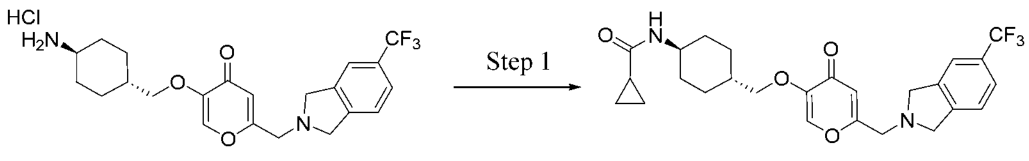


14D

Compound 15

Step 1:

**14D** (220 mg, 0.52 mmol) and triethylamine (158 mg, 1.56 mmol) were dissolved in dichloromethane (8 mL). Under nitrogen atmosphere, cyclopropanecarbonyl chloride (65 mg, 0.62 mmol) was added dropwise in an ice bath, and the mixture was reacted at room temperature overnight. After the reaction was completed, water (25 mL) was added to the reaction solution, and the resulting mixture was extracted with ethyl acetate (15 mL×3). The organic layers were combined, washed with saturated sodium chloride aqueous solution (25 mL), dried over anhydrous sodium sulphate, filtered, and concentrated. The residue was separated and purified by silica gel column chromatography (dichloromethane: methanol (v/v) = 25:1) to obtain **compound 15** (72 mg, yield: 28.2%).

LC-MS (ESI): m/z = 491.50 $[M+H]^+$.

$^1H$ NMR (400 MHz, $CDCl_3$) δ 7.57 (s, 1H), 7.52 - 7.47 (m, 1H), 7.46 (s, 1H), 7.33 - 7.29 (m, 1H), 6.48 (s, 1H), 5.45 - 5.40 (m, 1H), 4.12 (s, 4H), 3.80 (s, 2H), 3.78 - 3.72 (m, 1H), 3.67 - 3.61 (m, 2H), 2.10 - 1.94 (m, 4H), 1.91 - 1.81 (m, 1H), 1.31 - 1.27 (m, 1H), 1.20 - 1.07 (m, 4H), 0.99 - 0.93 (m, 2H), 0.74 - 0.67 (m, 2H).

**Example 16:**

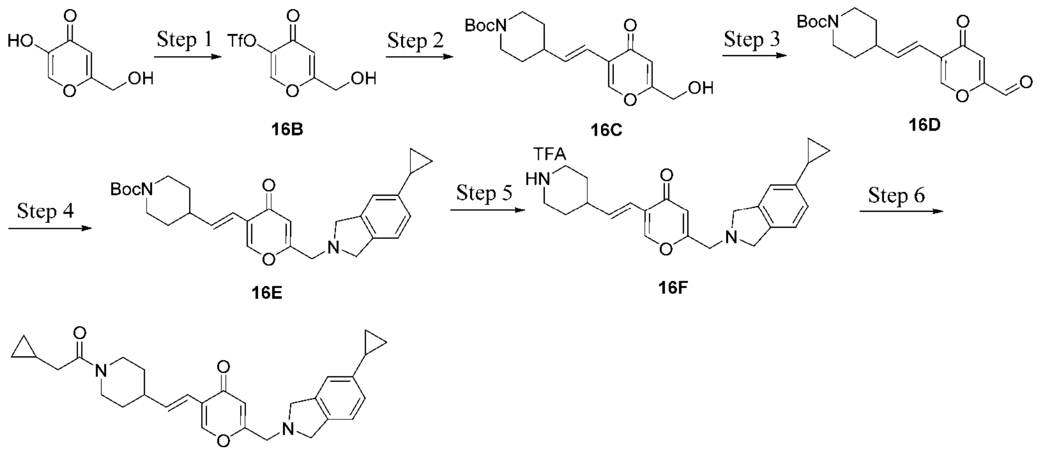


16B 16C 16D 16E 16F

**Compound 16**

Step 1:

Kojic acid (10.0 g, 70.3 mmol) was dissolved in DMF (120 mL), potassium carbonate (11.7 g, 84.4 mmol) and N,N-bis(trifluoromethylsulphonyl)aniline (30.2 g, 84.4 mmol) were added, and the resulting mixture was reacted at room temperature for 14 h. After the reaction was completed, the reaction solution was diluted with water, and extracted with ethyl acetate. The organic layers were combined, washed with saturated sodium chloride aqueous solution, dried over anhydrous $Na_2SO_4$, filtered, and concentrated. The residue was separated and purified by silica gel column chromatography (PE/EA=1/1) to obtain **16B** (17.0 g, yield: 88.2%).

$^1H$ NMR (400 MHz, $CDCl_3$) $\delta$ 8.08 (s, 1H), 6.69 (s, 1H), 4.53 (s, 2H).

Step 2:

**16B** (8.13 g, 29.7 mmol) and tert-butyl (E)-4-(2-(4,4,5,5-tetramethyl-1,3,2-dioxaborolan-2-yl)vinyl)piperidine-1-carboxylate (10.0 g, 29.7 mmol) were dissolved in 1,4-dioxane (300 mL), 1,1-bis(diphenylphosphino)ferrocene palladium dichloride (2.2 g, 2.9 mmol), potassium carbonate (8.2 g, 59.3 mmol) and water (60 mL) were added, and the system was subjected to nitrogen replacement three times, heated to 90°C and reacted 3 h. The reaction mixture was passed through a short silica gel column, rinsed with ethyl acetate, concentrated, and subjected to column chromatography (petroleum ether/ethyl acetate = 1 / 1) to obtain compound **16C** (5.2 g, yield: 52%).

LC-MS (ESI): m/z = 280.2 $[M+H-56]^+$.

Step 3:

**16C** (1.5g, 4.5 mmol) was dissolved in DCM (50 mL), Dess-Martin oxidant (2.28g, 5.4 mmol) was added in portions at room temperature, and after the addition, the resulting mixture was reacted at room temperature for two hours. The reaction was quenched with saturated aqueous sodium thiosulphate solution, the aqueous phase was extracted with dichloromethane, and the organic layers were combined and washed with saturated NaCl solution. The organic layers were combined, dried over $Na_2SO_4$, filtered and subjected to rotary evaporation. The residue was subjected to silica gel column chromatography (dichloromethane: methanol (v/v) = 10:1) to obtain **16D** (1.4 g, yield: 94%).

LC-MS (ESI): m/z = 278.2 $[M+H-56]^+$.

Step 4:

**16D** (500 mg, 1.5 mmol) was dissolved in 1,2-dichloroethane (10 mL) and acetic acid (1 mL), then 5-cyclopropyl-2,3-dihydro 1H-isoindole (0.29 g, 1.8 mmol) was added, and the mixture was stirred at room temperature for 1 hour. Sodium triacetoxyborohydride (0.41g, 1.9 mmol) was added and the resulting mixture was reacted at room temperature for an hour. The reaction solution was quenched with saturated aqueous sodium bicarbonate solution, the aqueous phase was extracted with dichloromethane, and the organic layers were combined and washed with saturated

NaCl solution. The organic layers were combined, dried over $Na_2SO_4$, filtered and subjected to rotary evaporation. The residue was subjected to silica gel column chromatography (dichloromethane: methanol (v/v) = 10:1) to obtain **16E** (490 mg, yield: 69%).

LC-MS (ESI): m/z = 421.3 $[M+H-56]^+$.

Step 5:

**16E** (490 mg, 1.0 mmol) was dissolved in DCM (5 mL), then trifluoroacetic acid (5 mL) was added, and the resulting mixture was reacted at room temperature for 1 hour. After the reaction was completed, the reaction solution was concentrated, and the crude product **16F** was directly used in the next reaction without purification.

Step 6:

The crude product **16F** obtained in the previous step was dissolved in DCM (20 mL), triethylamine (0.31 g, 3.1 mmol) was added, and the resulting mixture was stirred at room temperature for five minutes. Then 2-cyclopropylacetyl chloride (0.15g, 1.3 mmol) was added, and the resulting mixture was reacted at room temperature for 1 hour. The reaction solution was quenched with water, the aqueous phase was extracted with dichloromethane, and the organic layers were combined and washed with saturated NaCl solution. The organic layers were combined, dried over $Na_2SO_4$, filtered and subjected to rotary evaporation. The residue was subjected to silica gel column (rinsed with ethyl acetate) chromatography to obtain compound **16** (200 mg, yield: 38%).

LC-MS (ESI): m/z = 459.3 $[M+H]^+$.

$^1H$ NMR (400 MHz, DMSO-$d_6$) δ 8.31 (s, 1H), 7.09 (d, 1H), 6.95 - 6.89(m, 2H), 6.70 - 6.61 (m, 1H), 6.34 (s, 1H), 6.13 (d, 1H), 4.39 (d, 1H), 3.95 - 3.71 (m, 7H), 3.04 (t, 1H), 2.59 (t, 1H), 2.38 - 2.28 (m, 1H), 2.25 (d, 2H), 1.93 - 1.85 (m, 1H), 1.77 - 1.65 (m, 2H), 1.30 - 1.21 (m, 1H), 1.19 - 1.12 (m, 1H), 0.97 - 0.86 (m, 3H), 0.65 - 0.56 (m, 2H), 0.46 - 0.40 (m, 2H), 0.13 - 0.09 (m, 2H).

**Example 17:**

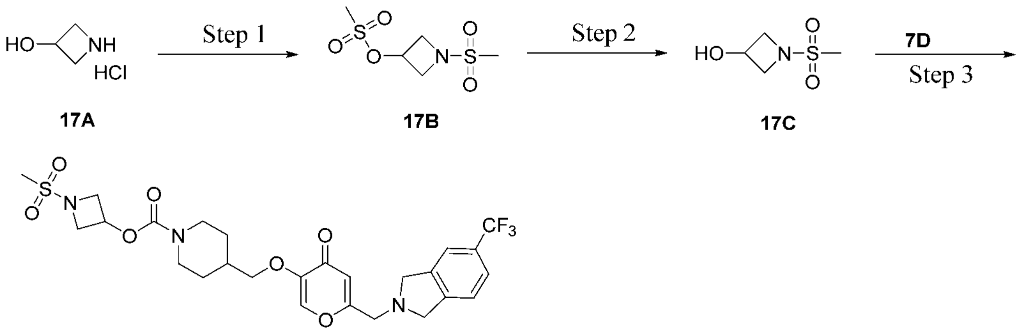


17A 17B 17C

Compound 17

Step 1:

**17A** (1 g, 9.13 mmol) was dissolved in THF (15 mL), and the system was cooled to 0°C. Methanesulphonyl chloride (2.61 g, 22.82 mmol) was slowly added dropwise. After the dropwise addition was completed, the mixture was reacted at this temperature for 3 hours. After the completion of the reaction, the reaction solution was poured into water, and the mixture was extracted with ethyl acetate. The organic phase was washed with saturated sodium chloride aqueous solution, dried over anhydrous sodium sulphate, and concentrated to obtain crude **17B** (1.2 g), which was directly used in the next reaction.

Step 2:

Crude **17B** (1.2 g) was added to 2 M sodium hydroxide solution (10 mL), and the mixture was stirred at room temperature for 2 hours. The system was adjusted to a neutral pH, and extracted with ethyl acetate, and the organic phase was washed with saturated sodium chloride aqueous solution, dried over anhydrous sodium sulphate, and concentrated to obtain a crude. The crude was purified with a medium-pressure preparation instrument Biotage Isolera One (12 g silica gel

column, eluents: 0-5% MeOH/DCM) to obtain the target compound **17C** (0.52 g, two-step yield: 41.8%).

Step 3:

**17C** (120 mg, 0.78 mmol) and CDI (84 mg, 0.6 mmol) were dissolved in THF (10 mL), and the mixture was stirred at room temperature for half an hour. Then TEA (350 mg, 3.46 mmol) and **7D** (160 mg, 0.4 mmol) were added and the resulting mixture was warmed to 60°C and reacted overnight. After the completion of the reaction, the reaction solution was poured into water. The system was extracted with ethyl acetate, and the organic phase was washed with saturated sodium chloride aqueous solution, dried over anhydrous sodium sulphate, and concentrated to obtain a crude. The crude was purified with a medium-pressure preparation instrument Biotage Isolera One (12 g silica gel column, eluents: 0-5% MeOH/DCM) to obtain the target compound **17** (80 mg, yield: 34.4%).
LC-MS (ESI): m/z =586.1 $[M+H]^+$.
$^1$H NMR (400 MHz, $CDCl_3$): δ 7.60 (s, 1H), 7.52-7.44 (m, 2H), 7.33-7.28 (m, 1H), 6.49 (s, 1H), 5.17-5.09 (m, 1H), 4.23-4.13 (m, 4H), 4.10 (s, 4H), 4.02-3.97 (m, 2H), 3.79 (s, 2H), 3.76-3.71 (m, 2H), 2.89 (s, 5H), 1.93-1.85 (m, 2H), 1.33-1.21 (m, 3H).

**Example 18:**

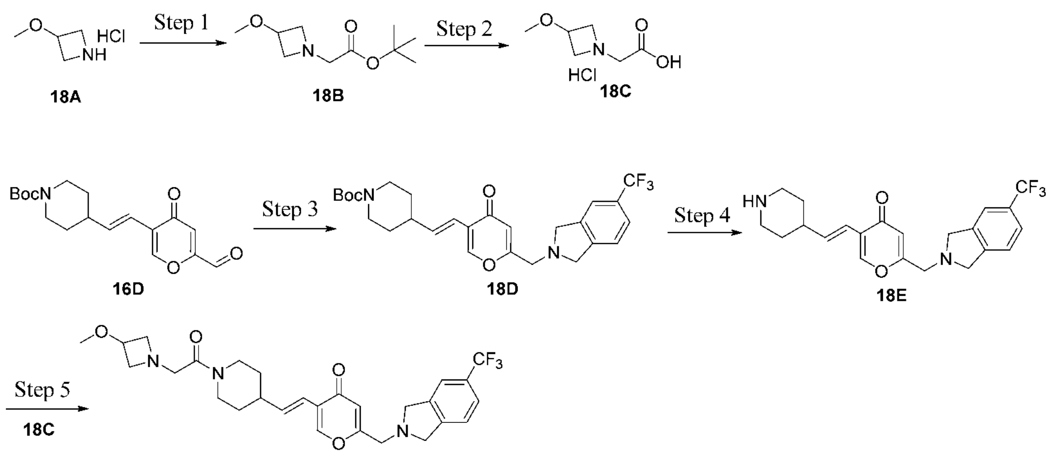


18A 18B 18C 16D 18D 18E

Compound 18

Step 1:

**18A** (1.2 g, 10 mmol) was dissolved in 1,4-dioxane (40 mL), N,N-diisopropylethylamine (3.9 g, 30 mmol) and tert-butyl bromoacetate (2.3 g, 12 mmol) were added, and the mixture was reacted at room temperature for 18 h. After the reaction was completed, water (25 mL) was added to the reaction solution, and the resulting mixture was extracted with ethyl acetate (20 mL×3). The organic layers were combined, washed with saturated sodium chloride aqueous solution (25 mL), dried over anhydrous sodium sulphate, filtered, and concentrated. The residue was separated and purified by silica gel column chromatography (PE/EA=4/1) to obtain **18B** (1.0 g, yield: 50%).
$^1$H NMR (400 MHz, $CDCl_3$) δ 4.11 - 4.05 (m, 1H), 3.84 - 3.72 (m, 2H), 3.25 (s, 3H), 3.20 (s, 2H), 3.04 - 2.94 (m, 2H), 1.46 (s, 9H).

Step 2:

To **18B** (1.0 g, 5 mmol) was added diluted hydrochloric acid (10 mL, 6 M), and the mixture was stirred at room temperature for 1 h. The solvent was removed using a rotary evaporator at 60°C to obtain the product **18C** (400 mg).
LC-MS (ESI): m/z = 145.2 $[M+H]^+$.

Step 3:

**16D** (1.5 g, 74.5 mmol) was dissolved in DCM (20 mL), 5-trifluoromethylisoindoline hydrochloride (1.1 g, 5.0 mmol), triethylamine (0.5 g, 5.0 mmol) and sodium borohydride acetate (1.4 g, 6.8 mmol) were added, and the resulting

mixture was reacted at room temperature for 2 h. After the reaction was completed, water (10 mL) was added to the reaction solution, and the system was extracted with DCM (20 mL×2). The organic layers were combined, washed with saturated sodium chloride aqueous solution (25 mL×1), dried over anhydrous sodium sulphate, filtered, and concentrated. The residue was purified by silica gel column chromatography (PE/EA=1/1) to obtain **18D** (1.8 g, yield: 79.3%).
LC-MS (ESI): m/z = 505.5 $[M+H]^+$.

Step 4:

Compound **18D** (1.5 g, 3.0 mmol) was dissolved in dichloromethane (20 mL). Trifluoroacetic acid (6 mL) was then added, and the mixture was reacted at room temperature for half an hour and then concentrated to obtain compound **18E** (1.3 g), which was directly used in the next step.

Step 5:

**18E** (202 mg, 0.50 mmol) was dissolved in DMF (10 mL), and **18C** (86.4 mg, 0.6 mmol) and N-methylimidazole (164 mg, 2 mmol) were added. At 0°C, N,N,N',N'-tetramethylchloroformamidinium hexafluorophosphate (168 mg, 0.6 mmol) was added and the resulting mixture was stirred at room temperature for 20 min. After the reaction was completed, water (20 mL) was added to the reaction solution, and the resulting mixture was extracted with ethyl acetate (15 mL×3). The organic layers were combined, washed with saturated sodium chloride aqueous solution (25 mL×3), dried over anhydrous sodium sulphate, filtered, and concentrated. The residue was separated and purified by silica gel column chromatography (dichloromethane: methanol (v/v) = 20:1) to obtain **compound 18** (40 mg, yield: 15%).
$^1H$ NMR (400 MHz, $CD_3OD$) δ 8.23 (s, 1H), 7.77 - 7.65 (m, 2H), 7.58 (d, 1H), 6.67 - 6.61 (m, 2H), 6.24 (d, 1H), 4.78 (s, 4H), 4.59 - 3.90 (m, 10H), 3.67 (d, 1H), 3.33 (d, 3H), 3.17 (t, 1H), 2.80 (t, 1H), 2.42 (s, 1H), 1.85 (t, 2H), 1.51 - 1.31 (m, 2H).
LC-MS (ESI): m/z = 531.2 $[M+H]^+$.

**Example 19:**

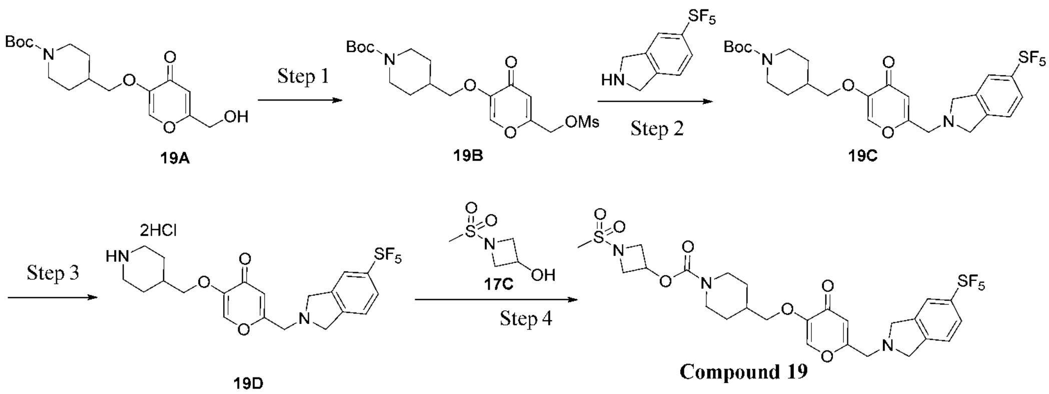


19A 19B 19C 19D Compound 19

Step 1:

Compound 19A (0.60 g, 1.77 mmol) and TEA (0.54 g, 5.33 mmol) were dissolved in DCM (15 mL), then methanesulphonyl chloride (0.16 mL, 2.09 mmol) was added, and the resulting mixture was reacted at room temperature for 0.5 hours. After the completion of the reaction, the reaction solution was directly used in the next step without further treatment.

Step 2:

To the reaction solution obtained in the previous step was added compound 5-sulphur pentafluoride isoindoline hydrochloride (470 mg, 1.68 mmol), and the mixture was dissolved in dichloromethane (15 mL). Then triethylamine (0.51 mg, 5.04 mmol) was added and the resulting mixture was reacted at room temperature for 16 h. After the completion of the reaction, the mixture was concentrated, and the resulting crude was purified by column chromatography (DCM:MeOH = 10:1) to obtain compound 19C (550 mg, two-step yield: 57.7%).
LC-MS (ESI): m/z=467.5 $[M+H-100]^+$.

Step 3:

Compound 19C (0.55 g, 0.98 mmol) was dissolved in DCM (10 mL), and at room temperature, hydrogen chloride dioxane solution (4 mol/L, 5 mL) was added. Then the mixture was stirred at room temperature for 1 hour. The reaction solution was directly subjected to rotary evaporation to obtain compound 19D (530 mg).
LC-MS (ESI): m/z= 467.1 $[M+H]^+$.

Step 4:

17C (0.14 g,0.93 mmol) was dissolved in tetrahydrofuran (15 mL), N,N'-carbonyldiimidazole (0.15 g, 0.93 mmol) was added at room temperature, and the resulting mixture was stirred at room temperature for 0.5 hours. Then triethylamine (0.25 g, 2.47 mmol) was added and the mixture was stirred for another 0.5 hours. Then 19D (330 mg, 0.61 mmol) was added to the reaction system. The mixture was warmed to 60°C and continuously reacted for 16 hours. The reaction solution was directly subjected to rotary evaporation. The resulting crude was purified by column chromatography (DCM:MeOH = 20:1) to obtain compound 19 (180 mg, yield: 48.4%).
LC-MS (ESI): m/z=644.9 $[M+H]^+$.
$^1H$ NMR (400 MHz, $CDCl_3$) δ 7.63 (d, 1H), 7.60 (s, 2H), 7.28 (d, 1H), 6.48 (s, 1H), 5.19 - 5.07 (m, 1H), 4.23 - 4.05 (m, 8H), 4.00 (dd, 2H), 3.78 (s, 2H), 3.73 (d, 2H), 2.84 (d, 5H), 2.14 - 2.01 (m, 1H), 1.89 (d, 2H), 1.27 (qd, 2H).

**Example 20:**

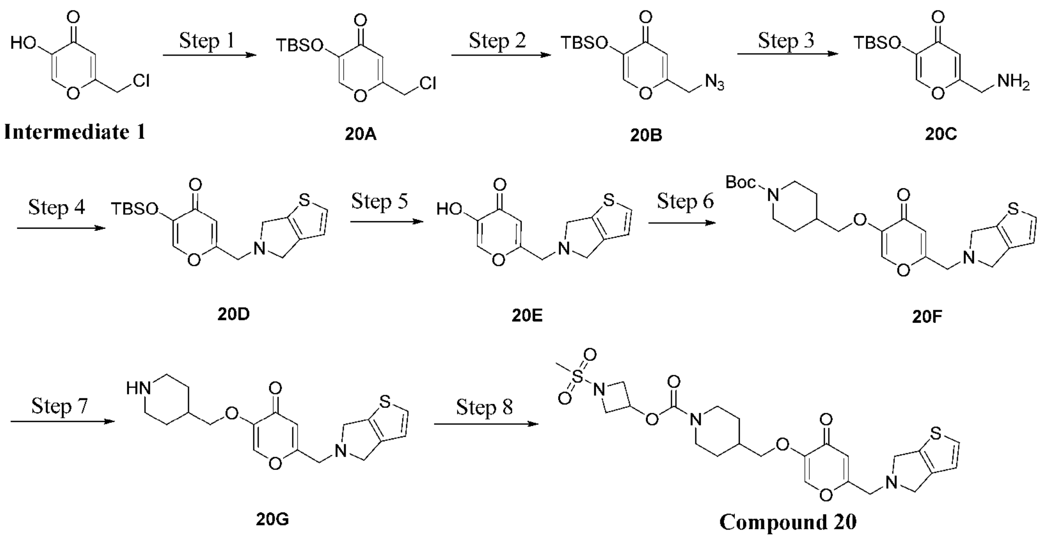


Intermediate 1, 20A, 20B, 20C, 20D, 20E, 20F, 20G, Compound 20

Step 1:

**Intermediate 1** (15.0 g, 93.43 mmol) and imidazole (12.7 g, 187 mmol) were dissolved in DCM (100 mL), tert-butyldimethylsilyl chloride (15.49 g, 102.77 mmol) was added dropwise in an ice bath, and the mixture was reacted at room temperature for 1 h. After the reaction was completed, water (100 mL) was added to the reaction solution, and the resulting mixture was extracted with DCM (50 mL×2). The organic layers were combined, washed with saturated sodium chloride aqueous solution (100 mL×2), dried over anhydrous sodium sulphate, filtered, and concentrated. The residue was separated and purified by silica gel column chromatography (petroleum ether: ethyl acetate (v/v) = 20:1) to obtain **20A** (20.0 g, yield: 77.9%).
LC-MS (ESI): m/z = 275.1 $[M+H]^+$.

Step 2:

**20A** (20.0 g, 72.78 mmol) was dissolved in DMF (30 mL), sodium azide (5.2 g, 80.06 mmol) was subsequently added, and the mixture was reacted at room temperature overnight. After 16 h, water (50 mL) was added to the reaction solution, and the resulting mixture was extracted with ethyl acetate (30 mL×3). The organic layers were combined, washed with saturated sodium chloride aqueous solution (40 mL), dried over anhydrous sodium sulphate, filtered, and

concentrated. The residue was separated and purified by silica gel column chromatography (petroleum ether: ethyl acetate (v/v) = 20:1) to obtain **20B** (19.0 g, yield: 92.7%).

LC-MS (ESI): m/z = 282.3 $[M+H]^+$.

Step 3:

**20B** (19.0 g, 67.52 mmol) was dissolved in tetrahydrofuran (100 mL), and in an ice bath, triphenylphosphine (26.61 g, 101.47 mmol) was added in portions. The mixture was stirred for 10 min, then pure water (2.5 mL) was slowly added dropwise and the resulting mixture was reacted at 50°C overnight. After the reaction was completed, the reaction mixture was cooled to room temperature, water (50 mL) was added to the reaction solution, and the resulting mixture was extracted with ethyl acetate (50 mL×3). The organic layers were combined, washed with saturated sodium chloride aqueous solution (100 mL), dried over anhydrous sodium sulphate, filtered, and concentrated. The residue was separated and purified by silica gel column chromatography (dichloromethane: methanol (v/v) = 20:1) to obtain **20C** (12 g, yield: 69.6%).

LC-MS (ESI): m/z = 256.3 $[M+H]^+$.

Step 4:

**20C** (1.2 g, 4.7 mmol), 2,3-bis(chloromethyl)thiophene (847 mg, 4.7 mmol), potassium iodide (156 mg, 0.94 mmol) and potassium carbonate (1.95 g, 14.1 mmol) were dissolved in acetonitrile (60 mL), and under nitrogen atmosphere, the mixture was reacted at 80°C for 30 min. After the reaction was completed, the reaction mixture was cooled to room temperature, water (40 mL) was added to the reaction solution, and the resulting mixture was extracted with ethyl acetate (30 mL×3). The organic layers were combined, washed with saturated sodium chloride aqueous solution (50 mL), dried over anhydrous sodium sulphate, filtered, and concentrated. The residue was separated and purified by silica gel column chromatography (dichloromethane: methanol (v/v) = 40:1) to obtain **20D** (300 mg, yield: 17.5%).

LC-MS (ESI): m/z = 364.2 $[M+H]^+$.

Step 5:

**20D** (300 mg, 0.83 mmol) was dissolved in methanol (10 mL), potassium carbonate (343 mg, 2.49 mmol) was added, and the resulting mixture was reacted at room temperature overnight. After the reaction was completed, the reaction solution was concentrated, and the residue was dissolved in EA (20 mL). The organic phase was washed with water (15 mL×3) and saturated sodium chloride aqueous solution (20 mL), dried over anhydrous sodium sulphate, filtered, and concentrated. The residue was separated and purified by silica gel column chromatography (dichloromethane: methanol (v/v) = 25:1) to obtain **20E** (200 mg, yield: 97.2%).

LC-MS (ESI): m/z = 250.2 $[M+H]^+$.

Step 6:

**20E** (200 mg, 0.80 mmol) was dissolved in dry DMF (10 mL), tert-butyl 4-(((methylsulphonyl)oxy)methyl) piperidine-1-carboxylate (259 mg, 0.88 mmol) was added, and the mixture was stirred evenly. Potassium carbonate (331 mg, 2.4 mmol) was added, and the resulting mixture was reacted at 80°C for 16 hours. Upon complete depletion of raw materials as monitored by TLC, the reaction was stopped. The reaction mixture was cooled to room temperature, water (20 mL) was added to the reaction solution, and the resulting mixture was extracted with ethyl acetate (15 mL×3). The organic layers were combined, washed with saturated sodium chloride aqueous solution (20 mL), dried over anhydrous sodium sulphate, filtered, and concentrated. The residue was separated and purified by silica gel column chromatography (DCM:MeOH (v/v) = 15:1) to obtain **20F** (100 mg, yield: 27.9%).

LC-MS (ESI): m/z = 447.2 $[M+H]^+$.

Step 7:

At room temperature, **20F** (100 mg, 0.22 mmol) was dissolved in DCM (10 mL), hydrogen chloride dioxane solution (0.5 mL, 2 mmol, 4M) was added, and the mixture was reacted at room temperature for 1 hour. Upon complete depletion of raw materials as monitored by TLC, the reaction was stopped. The reaction solution was concentrated to obtain **20G** (70 mg) without purification.

LC-MS (ESI): m/z = 347.2 $[M+H]^+$.

Step 8:

At room temperature, 1-(methylsulphonyl)azetidin-3-ol (61 mg, 0.40 mmol) was dissolved in dry THF (10 mL), CDI (64.8 mg, 0.40 mmol) was added, and the mixture was stirred at room temperature for 30 min. Triethylamine (0.5 mL) was added, followed by **20G** (70 mg, 0.20 mmol), and the resulting mixture was reacted at 80°C for 16 hours. Upon complete depletion of raw materials as monitored by TLC, the reaction was stopped. The reaction mixture was cooled to room temperature, water (20 mL) was added to the reaction solution, and the resulting mixture was extracted with ethyl acetate (15 mL×3). The organic layers were combined, washed with saturated sodium chloride aqueous solution (20 mL), dried over anhydrous sodium sulphate, filtered, and concentrated. The residue was separated and purified by silica gel column chromatography (DCM:MeOH (v/v) = 15:1) to obtain compound **20** (5 mg, yield: 4.7%).

$^1H$ NMR (400 MHz, $CDCl_3$) δ 7.59-7.58 (d, 1H), 7.24-7.23 (d, 1H), 6.82-6.80 (d, 1H), 6.48 (s, 1H), 5.16-5.10 (m, 1H), 4.19-4.10 (m, 6H), 4.02-3.97 (m, 4H), 3.83 (s, 2H), 3.75-3.69 (m, 2H), 2.89 (s, 3H), 2.82-2.76 (t, 2H), 2.08-2.01 (m, 1H), 1.90-1.73 (t, 4H).

LC-MS (ESI): m/z = 524.1 $[M+H]^+$.

**Example 21:**

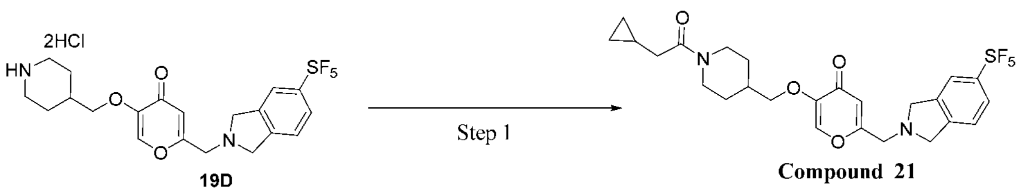


19D

Compound 21

Step 1:

Compound 19D (200 mg, 0.37 mmol) and triethylamine (150 mg, 1.48 mmol) were dissolved in dichloromethane (15 mL). In an ice-water bath, cyclopropylacetyl chloride (0.066 mg, 0.55 mmol) was added dropwise. After the addition, the mixture was reacted at room temperature for 2 h. After the completion of the reaction, the mixture was concentrated and then purified by silica gel column chromatography (dichloromethane: methanol (v/v) = 20:1) to obtain compound 21 (62 mg, yield: 30.5%).

LC-MS (ESI): m/z= 549.9 $[M+H]^+$.

$^1H$ NMR (400 MHz, $CDCl_3$) δ 7.63 (d, 1H), 7.59 (s, 2H), 7.29 (s, 1H), 6.48 (s, 1H), 4.70 (d, 1H), 4.08 (d, 4H), 3.88 (d, 1H), 3.82 - 3.65 (m, 4H), 3.05 (t, 1H), 2.59 (t, 1H), 2.28 (d, 2H), 2.19 - 2.06 (m, 1H), 2.00 (d, 1H), 1.84 (d, 1H), 1.30 - 1.18 (m, 2H), 1.09 - 0.98 (m, 1H), 0.56 (dt, 2H), 0.18 (q, 2H).

**Example 22:**

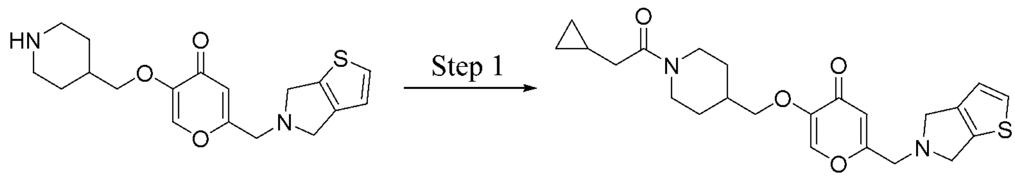


20G

Compound 22

Step 1:

At room temperature, **20G** (70 mg, 0.20 mmol), 2-cyclopropyl acetic acid (40 mg, 0.40 mmol) and NMI (20 mg, 0.24 mmol) were dissolved in dry DCM (10 mL), and the mixture was stirred at room temperature for 30 min. TCFH (67.2 mg, 0.24 mmol) was added, and the mixture was reacted for another 1 hour. Upon complete depletion of raw materials as monitored by TLC, the reaction was stopped. Water (20 mL) was added to the reaction solution, and the resulting mixture was extracted with dichloromethane (15 mL×3). The organic layers were combined, washed with saturated sodium

chloride aqueous solution (20 mL), dried over anhydrous sodium sulphate, filtered, and concentrated. The residue was separated and purified by silica gel column chromatography (DCM:MeOH (v/v) = 15:1) to obtain compound **22** (15 mg, yield: 17.3%).

$^1H$ NMR (400 MHz, $CDCl_3$) δ 7.63-7.62 (t, 1H), 7.35-7.32 (m, 1H), 7.02-6.97 (m, 1H), 6.72-6.67 (t, 1H), 3.90-3.83 (m, 3H), 3.80-3.69 (m, 4H), 3.48 (s, 2H), 3.08-3.02 (t, 1H), 2.63-2.57 (t, 1H), 2.29-2.27 (d, 2H), 2.25-2.20 (m, 1H), 2.18-2.10 (m, 1H), 2.03-1.99 (m, 2H), 1.89-1.83 (m, 1H), 1.68-1.60 (m, 1H), 1.09-1.01 (d, 2H), 0.58-0.54 (m, 2H), 0.20-0.15 (m, 2H).

LC-MS (ESI): m/z = 429.2 $[M+H]^+$.

**Example 23:**

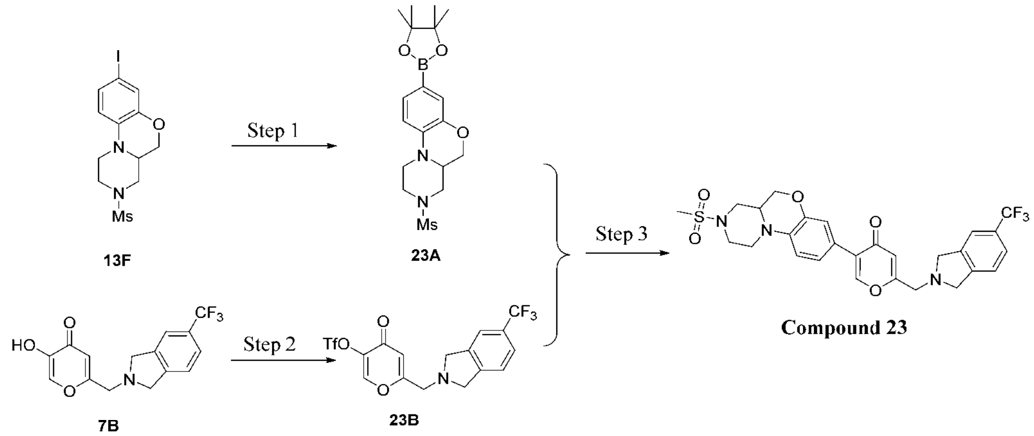


Step 1:

To a 50 mL single-neck flask, compound **13F** (600 mg, 1.52 mmol), bis(pinacolato)diboron (1.16 g, 4.56 mmol), bis(triphenylphosphine)palladium dichloride (214 mg, 0.304 mmol) and potassium acetate (298 mg, 3.04 mmol) were successively added. After nitrogen replacement, N, N-dimethylformamide (30 mL) was added by injection, and the system was warmed to 100°C and reacted overnight. Upon complete depletion of raw materials as monitored by TLC, the reaction mixture was cooled to room temperature and directly concentrated under reduced pressure. The resulting residue was separated and purified by silica gel column chromatography (dichloromethane/ethyl acetate (v/v) = 93/7-75/25) to obtain **23A** (309 mg, yield: 51.5%).

LC-MS (ESI): m/z = 395.2 $[M+H]^+$.

Step 2:

Compound **7B** (650 mg, 2.09 mmol) and potassium carbonate (866 mg, 6.26 mmol) were dissolved in N, N-dimethylformamide (20 mL), N-phenylbis(trifluoromethanesulphonyl)imide (1.49 g, 4.18 mmol) was added under stirring, and the mixture was reacted at room temperature for about 4 hours. Upon complete depletion of raw materials as monitored by TLC, water (100 mL) was added to quench the reaction. The reaction mixture was extracted with ethyl acetate (50 mL x 5), and the organic phases were combined, dried over anhydrous sodium sulphate, filtered, and concentrated. The crude was separated and purified by silica gel column chromatography (petroleum ether/ethyl acetate (v/v) = 100/0-65/35) to obtain compound **23B** (908 mg, yield: 98.1%).

LC-MS (ESI): m/z = 444.1 $[M+H]^+$.

Step 3:

Under nitrogen protection, compound **23A** (270 mg, 0.685 mmol), **23B** (364 mg, 0.822 mmol), bis(triphenylphosphine)palladium dichloride (96.1 mg, 0.137 mmol) and potassium carbonate (189 mg, 1.37 mmol) were all dissolved in 1, 4-dioxane (20 mL), water (10 drops) was added, and the mixture was warmed to 100°C and reacted overnight. After the reaction was completed as monitored by LCMS, the reaction mixture was cooled to room temperature and directly concentrated under reduced pressure. The crude was preliminarily separated and purified by silica gel column chromatography (dichloromethane/methanol (v/v) = 100/0-85/15), and then separated and purified by SFC to obtain the racemate compound **23** (65.2 mg, yield: 17.0%).

SFC purification method: 1. Instrument: Waters 150 Prep-SFC F; 2. chromatographic column: Chiralcel AD column 50×4.6mm I.D., 3μm; 3. mobile phase system: A for $CO_2$; B for 0.1%NH3•$H_2O$ in IPA and ACN; 4. gradient: B 5%-50%; 5. Flow rate: 100 mL/min, cycle time: 3.60 min.

$^1$H NMR (400 MHz, DMSO-*d6*) δ 8.34 (s, 1H), 7.64 (s, 1H), 7.58 (d, 1H), 7.48 (d, 1H), 7.06 (dd, 1H), 7.03 - 6.94 (m, 2H), 6.42 (s, 1H), 4.36 (dd, 1H), 4.07 (s, 4H), 4.02 - 3.91 (m, 2H), 3.85 (s, 2H), 3.66 - 3.57 (m, 2H), 3.24 - 3.16 (m, 1H), 2.93 (s, 4H), 2.78 (td, 1H), 2.60 (t, 1H).

LC-MS (ESI): m/z =562.3 [M+H]$^+$.

Chromatographic analytical conditions: 1. Instrument: Shimadzu LC-20AT; 2. chromatographic column: Xtimate C18 4.6×50 mm, 3 μm; 3. mobile phase system: A for 0.05% TFA in $H_2O$; B for ACN; 4. gradient: B 5%-95%; 5. Flow rate: 1.0 mL/min, run time: 10 min. retention time: 3.573 min.

**Example 24:**

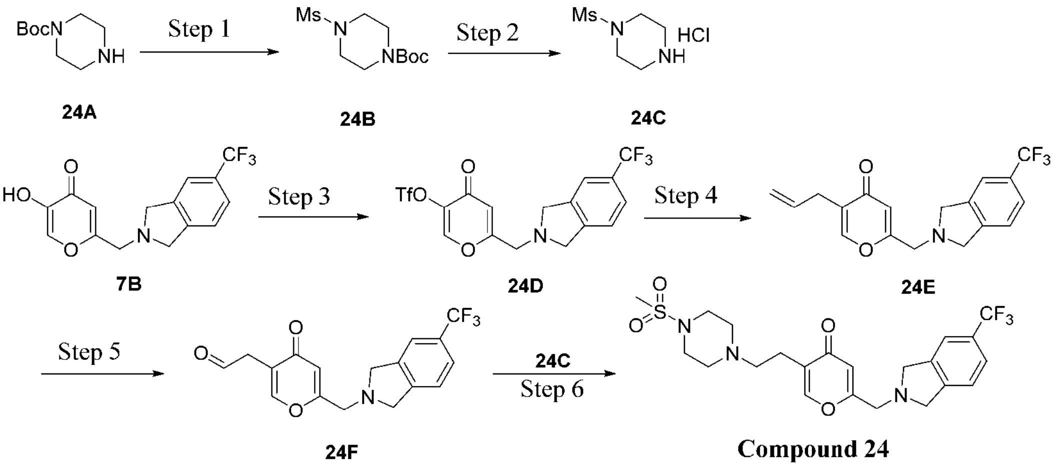


24A 24B 24C 7B 24D 24E 24F Compound 24

Step 1:

**24A** (1 g, 5.36 mmol) and triethylamine (1.63 g, 16.08 mmol) were dissolved in dichloromethane (20 mL), methanesulphonyl chloride (798 mg, 6.97 mmol) was added dropwise in an ice bath under nitrogen atmosphere, and the mixture was reacted at room temperature for 1 h. After the reaction was completed, saturated sodium bicarbonate solution (30 mL) was added to the reaction solution to quench the reaction, and the resulting mixture was extracted with ethyl acetate (25 mL×3). The organic layers were combined, washed with saturated sodium chloride aqueous solution (25 mL), dried over anhydrous sodium sulphate, filtered, and concentrated. The residue was separated and purified by silica gel column chromatography (petroleum ether: ethyl acetate (v/v) = 10:1) to obtain **24B** (1.18 g, yield: 83%).

LC-MS (ESI): m/z = 165.2 [M+H-100]$^+$.

Step 2:

**24B** (1.18 g, 4.46 mmol) was dissolved in dichloromethane, 4 M hydrogen chloride 1,4-dioxane solution (4 mL) was added in an ice bath, and the mixture was reacted at room temperature for 30 min. After the reaction was completed, the reaction mixture was subjected to rotary evaporation under reduced pressure to remove the solvent. The residue was dried to obtain **24C** (705 mg), which was directly used in the next reaction without further purification.

Step 3:

**7B** (1 g, 3.21 mmol) and triethylamine (0.97 g, 9.67 mmol) were dissolved in tetrahydrofuran (15 mL), trifluoromethanesulphonic anhydride (1.09 g, 3.86 mmol) was added dropwise in an ice bath under nitrogen atmosphere, and the mixture was reacted at room temperature for 4 h. After the reaction was completed, saturated sodium bicarbonate solution (30 mL) was added to the reaction solution to quench the reaction, and the resulting mixture was extracted with ethyl acetate (20 mL×3). The organic layers were combined, washed with saturated sodium chloride aqueous solution (25 mL), dried over anhydrous sodium sulphate, filtered, and concentrated. The residue was separated and purified by silica gel column chromatography (petroleum ether: ethyl acetate (v/v) = 5:1) to obtain **24D** (850 mg, yield: 60%).

LC-MS (ESI): m/z= 444.0 $[M+H]^+$.

Step 4:

**24D** (300 mg, 0.68 mmol), XPhos-Pd-G2 (54 mg, 0.068 mmol) and lithium chloride (35 mg, 0.82 mmol) were dissolved in 1,4-dioxane (8 mL), propenyl tributyl stannane (338 mg, 1.02 mmol) was added dropwise at room temperature under nitrogen atmosphere, and the mixture was reacted at 100°C overnight. After the reaction was completed, the reaction mixture was cooled to room temperature, water (25 mL) was added, and the resulting mixture was extracted with ethyl acetate (15 mL×3). The organic layers were combined, washed with saturated sodium chloride aqueous solution (30 mL), dried over anhydrous sodium sulphate, filtered, and concentrated. The residue was separated and purified by silica gel column chromatography (dichloromethane: methanol (v/v) = 40:1) to obtain 24E (188 mg, yield: 82%).
LC-MS (ESI): m/z= 336.2 $[M+H]^+$.

Step 5:

**24E** (188 mg, 0.56 mmol) was dissolved in mixed solvents of tetrahydrofuran (5 mL) and water (5 mL), then potassium osmate dihydrate (21 mg, 0.056 mmol) and sodium periodate (300 mg, 1.40 mmol) were added, and the mixture was reacted at room temperature for 5 h. After the reaction was completed, water (25 mL) was added to the reaction solution, and the resulting mixture was extracted with ethyl acetate (15 mL×5). The organic layers were combined, washed with saturated sodium chloride aqueous solution (25 mL), dried over anhydrous sodium sulphate, filtered, and concentrated. The residue was separated and purified by silica gel column chromatography (dichloromethane: methanol (v/v) = 40:1) to obtain **24F** (140 mg, yield: 74%).
LC-MS (ESI): m/z= 338.1 $[M+H]^+$.

Step 6:

**24F** (140 mg, 0.42 mmol) and 24C (69 mg, 0.42 mmol) were dissolved in methanol (8 mL), sodium triacetoxyborohydride (356 mg, 1.68 mmol) was added in portions, and the mixture was reacted at room temperature overnight. After the reaction was completed, saturated sodium bicarbonate solution (30 mL) was added to the reaction solution, the mixture was extracted with ethyl acetate (15 mL×4), and the organic layers were combined, dried over anhydrous sodium sulphate, filtered, and concentrated. The residue was separated and purified by silica gel column chromatography (dichloromethane: methanol (v/v) = 25:1) to obtain **compound 24** (35 mg, yield: 17%).
$^1H$ NMR (400 MHz, DMSO-$d_6$) $\delta$ 8.14 (s, 1H), 7.10-7.07 (m, 1H), 6.93-6.91 (m, 2H), 6.37 (s, 1H), 3.89 (s, 4H), 3.78-3.69 (m, 4H), 3.60-3.55 (m, 2H), 2.85 (s, 3H), 2.76-2.69 (m, 2H), 1.93-1.80 (m, 5H), 1.35-1.24 (m, 2H).
LC-MS (ESI): m/z = 486.1 $[M+H]^+$.

**Example 25:**

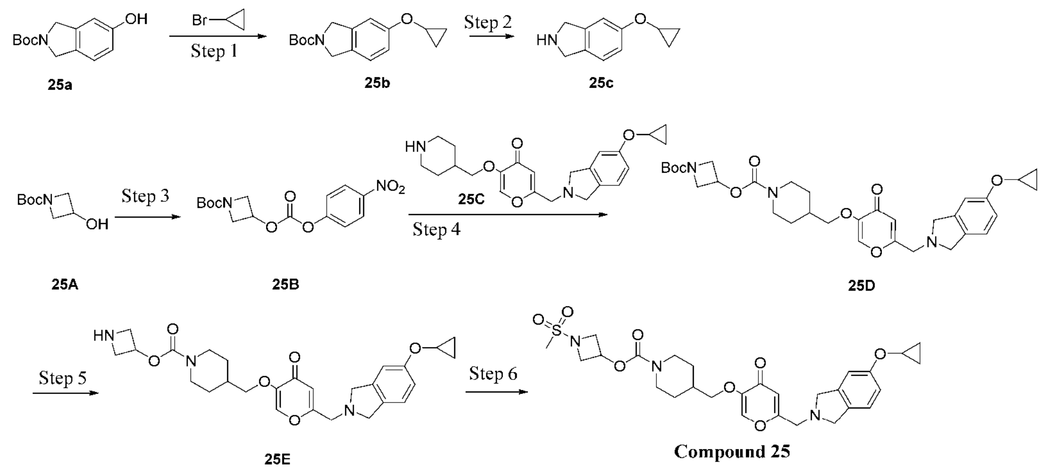

25a 25b 25c
25A 25B 25C 25D
25E Compound 25

Step 1: To a 100 mL single-neck flask was added compound **25a** (1.8 g, 7.6 mmol), which was then dissolved in N-methylpyrrolidinone (10 mL), and then caesium carbonate (5.4 g, 16.7 mmol) was added. The mixture was heated to

130°C and reacted overnight. Saturated ammonium chloride solution was added to quench the reaction, and the mixture was extracted with ethyl acetate, dried, and concentrated. The residue was subjected to column chromatography (petroleum ether/ethyl acetate = 10/1) to obtain compound **25b** (0.89 g, 40%).

LC-MS (ESI): m/z = 220.1 $[M+H-56]^+$.

Step 2: To a 100 mL single-neck flask was added compound **25b** (0.89 g, 3.2 mmol), which was dissolved in dichloromethane (15 mL), and then trifluoroacetic acid (3.0 mL) was added. The mixture was reacted at room temperature for 2 h, and concentrated to obtain compound **25c** (1.0 g, 100%).

LC-MS (ESI): m/z = 176.1 $[M+H]^+$.

Step 3:

**25A** (173 mg, 1.0 mmol) was dissolved in dichloromethane (10 mL), DIEPA (154 mg, 1.2 mmol) and bis(4-nitrophenyl)carbonate (365 mg, 1.2 mmol) were added, and the resulting mixture was stirred at room temperature for 30 min to obtain the dichloromethane solution of **25B** for later use.

Step 4:

**25C** (396 mg, 1.0 mmol, synthesized using 25c as the raw material with reference to the synthetic route of **8F**) was dissolved in dichloromethane (10 mL), DIEPA (258 mg, 2.0 mmol) and **25B** solution were added, and the resulting mixture was stirred at room temperature for 30 min. After the reaction was completed, the solvent was concentrated and the residue was separated and purified by silica gel column chromatography (PE/EA=1/1) to obtain **25D** (350 mg, yield: 59%).

LC-MS (ESI): m/z = 596.7 $[M+H]^+$.

Step 5:

Compound **25D** (350 mg, 0.6 mmol) was dissolved in dichloromethane (10 mL). Trifluoroacetic acid (1 mL) was then added, and the mixture was reacted at room temperature for half an hour and then concentrated to obtain compound **25E** (295 mg, yield: 98%), which was directly used in the next step.

Step 6:

**25E** (295 mg, 0.6 mmol) was dissolved in DCM (10 mL), triethylamine (182 mg, 1.8 mmol) and trifluoromethanesulphonyl chloride (118 mg, 0.7 mmol) were added, and the resulting mixture was stirred at room temperature for 20 min. After concentration, the residue was separated and purified by silica gel column chromatography (dichloromethane: methanol (v/v)=10:1) to obtain **compound 25** (100 mg, yield: 29%).

LC-MS (ESI): m/z = 574.6 $[M+H]^+$.

$^1$H NMR (400 MHz, $CDCl_3$) δ 7.60 (s, 1H), 7.09 (d, 1H), 6.89 (d, 2H), 6.48 (s, 1H), 5.18 - 5.06 (m, 1H), 4.19 - 4.15 (m, 4H), 4.04 - 3.96 (m, 6H), 3.81 - 3.72 (m, 4H), 2.89 - 2.72 (m, 6H), 2.07 - 2.03 (m, 1H), 1.89 (d, 2H), 1.32 - 1.22 (m, 2H), 0.82 - 0.70 (m, 4H).

**Example 26:**

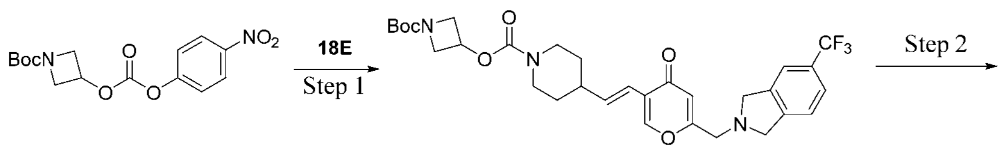


25B 26A

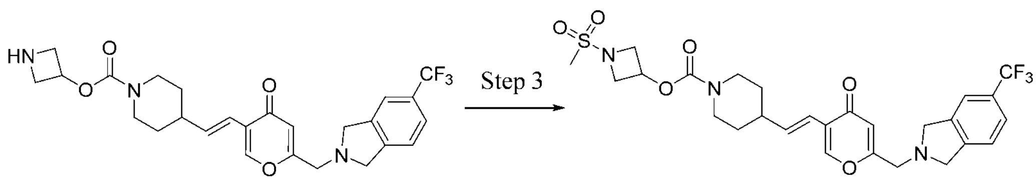


26B **Compound 26**

Step 1:

**18E** (808 mg, 2.0 mmol) was dissolved in dichloromethane (20 mL), DIEPA (387 mg, 3.0 mmol) and **25B** solution (1.0 g, 3.0 mmol) were added, and the mixture was stirred at room temperature for 30 min. After the reaction was completed, the solvent was concentrated and the residue was separated and purified by silica gel column chromatography (PE/EA=1/2) to obtain **26A** (920 mg, yield: 77%).
LC-MS (ESI): m/z = 603.6 $[M+H]^+$.

Step 2:

Compound **26A** (920 mg, 1.5 mmol) was dissolved in dichloromethane (10 mL). Trifluoroacetic acid (3 mL) was then added, and the mixture was reacted at room temperature for half an hour and then concentrated to obtain compound **26B** (890 mg), which was directly used in the next step.

Step 3:

**26B** (890 mg, 1.4 mmol) was dissolved in DCM (10 mL), triethylamine (565 mg, 5.6 mmol) and methanesulphonyl chloride (171 mg, 1.5 mmol) were added, and the resulting mixture was stirred at room temperature for 20 min. After concentration, the residue was separated and purified by silica gel column chromatography (dichloromethane: methanol (v/v)=10:1) to obtain **compound 25** (300 mg, yield: 37%).
LC-MS (ESI): m/z = 581.6 $[M+H]^+$.
$^1$H NMR (400 MHz, CDCl3) δ 7.80 (s, 1H), 7.50 (d, 1H), 7.46 (s, 1H), 7.31 (d, 1H), 6.58 - 6.52 (m, 1H), 6.42 (s, 1H), 6.18 (d, 1H), 5.20 - 5.07 (m, 1H), 4.22 - 4.05 (m, 8H), 4.01 - 3.96 (m, 2H), 3.79 (s, 2H), 2.97 - 2.73 (m, 5H), 2.39 - 2.21 (m, 1H), 1.79 (d, 2H), 1.45 - 1.34 (m, 2H).
$^{19}$F NMR (377 MHz, CDCl3) δ -60.07 (s).

**Example 27:**

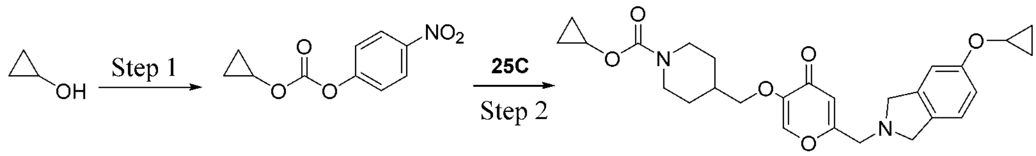

27B
Compound 27

Step 1:

Cyclopropanol (116 mg, 2.0 mmol) was dissolved in dichloromethane (10 mL), DIEPA (308 mg, 2.4 mmol) and bis(4-nitrophenyl)carbonate (730 mg, 2.4 mmol) were added, and the resulting mixture was stirred at room temperature for 30 min to obtain the dichloromethane solution of **27B** for later use.

Step 2:

**25C** (792 mg, 2.0 mmol) was dissolved in dichloromethane (20 mL), DIEPA (516 mg, 4.0 mmol) and **27B** solution (446 mg, 2.0 mmol) were added, and the mixture was stirred at room temperature for 30 min. After the reaction was completed, the solvent was concentrated and the residue was separated and purified by silica gel column chromatography (PE/EA=1/1) to obtain **27** (400 mg, yield: 42%).
LC-MS (ESI): m/z = 480.6 $[M+H]^+$.
$^1$H NMR (400 MHz, $CDCl_3$) δ 7.59 (s, 1H), 7.12 - 7.05 (m, 1H), 6.92 - 6.85 (m, 2H), 6.47 (s, 1H), 4.16 (s, 1H), 4.09 - 4.03 (m, 1H), 3.99 (d, 4H), 3.75 (s, 2H), 3.74 - 3.66 (m, 3H), 6.92 - 6.85 (m, 2H), 2.10 - 1.92 (m, 1H), 1.85 (d, 2H), 1.73 (s, 1H), 1.22 (d, 2H), 0.80 - 0.70 (m, 4H), 0.70 - 0.59 (m, 4H).

**Example 28:**

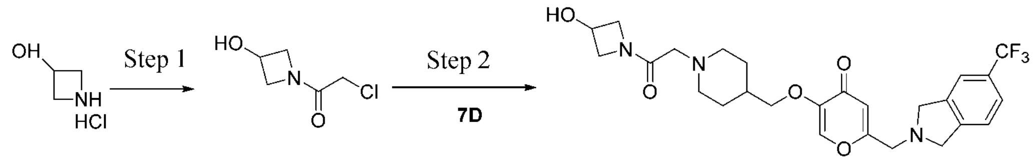


17A 28B Compound 28

Step 1:

17A (0.30 g, 4.10 mmol) was added to dichloromethane (10 mL) and saturated sodium bicarbonate (10 mL), chloroacetyl chloride (0.55 g, 4.93 mmol) was added dropwise in an ice bath, and the resulting mixture was continuously stirred and reacted for 2 hours. The reaction solution was extracted with dichloromethane, and the organic phase was concentrated under reduced pressure. The residue was purified using a medium-pressure preparation instrument Biotage Isolera One (12 g silica gel column, eluents: 0-30% EA/PE) to obtain compound **28B** (0.41 g, yield: 66.8%).
LC-MS (ESI): m/z= 150.2 $[M+H]^+$.
$^1H$ NMR (400 MHz, $CD_3OD$-$d_4$): δ 4.63 - 4.57 (m, 1H), 4.51 - 4.46 (m, 1H), 4.26 - 4.21 (m, 1H), 4.08 - 4.04 (m, 1H), 4.02 (s, 2H), 3.82 - 3.78 (m, 1H).

Step 2:

Compound **28B** (0.054 g, 0.36 mmol) and **compound 7D** (0.1 g, 0.24 mmol) were dissolved in DMF (10 mL), potassium carbonate (0.049 g, 0.35 mmol) was added, and the resulting mixture was reacted at 70°C overnight. Upon complete depletion of raw materials as monitored by LCMS, the reaction solution was poured into ice water and quenched, and the resulting mixture was extracted with ethyl acetate (20 mL×3). The combined organic phases were washed with saturated sodium chloride aqueous solution (20 mL×2), dried over anhydrous sodium sulphate, filtered and concentrated. The residue was purified using a medium-pressure preparation instrument Biotage Isolera One (12 g silica gel column, eluents: 0-5% MeOH/DCM) to obtain compound **28** (0.050 g, yield: 39.9%).
LC-MS (ESI): m/z= 522.2 $[M+H]^+$.
$^1H$ NMR (400 MHz, $CDCl_3$): δ 7.58 (s, 1H), 7.50 - 7.48 (m, 1H), 7.47 - 7.45 (m, 1H), 7.31 - 7.29 (m, 1H), 6.49 (s, 1H), 4.70 - 4.64 (m, 1H), 4.47 - 4.43 (m, 1H), 4.30 - 4.25 (m, 1H), 4.11 - 4.08 (m, 5H), 3.90 - 3.86 (m, 1H), 3.78 (s, 2H), 3.71 - 3.70 (m, 2H), 3.02 (s, 2H), 2.93 - 2.90 (m, 2H), 2.10 - 2.05 (m, 2H), 1.89 - 1.82 (m, 3H), 1.44 - 1.35 (m, 2H).

**Example 29:**

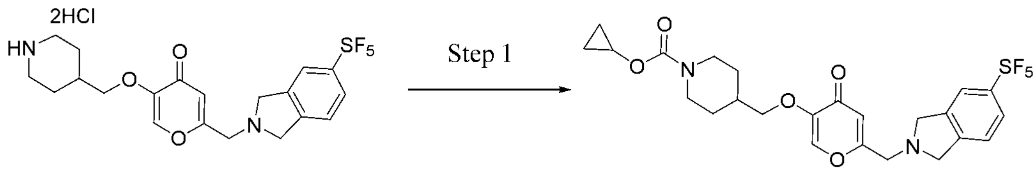


19D Compound 29

Step 1:

Cyclopropanol (0.045 g,0.78 mmol) was dissolved in tetrahydrofuran (15 mL), CDI (0.13 g, 0.78 mmol) was added at room temperature, and the resulting mixture was stirred at room temperature for 0.5 hours. Then triethylamine (0.21 g, 2.08 mmol) was added and the mixture was stirred for another 0.5 hours. Then 19D (280 mg, 0.52 mmol) was added to the reaction system. The mixture was warmed to 60°C and continuously reacted for 16 hours. The reaction solution was directly subjected to rotary evaporation. The resulting crude was purified by column chromatography (DCM:MeOH=20:1) to obtain compound 19 (60 mg, yield: 20.9%).
LC-MS (ESI): m/z= 551.2 $[M+H]^+$.

**Example 30:**

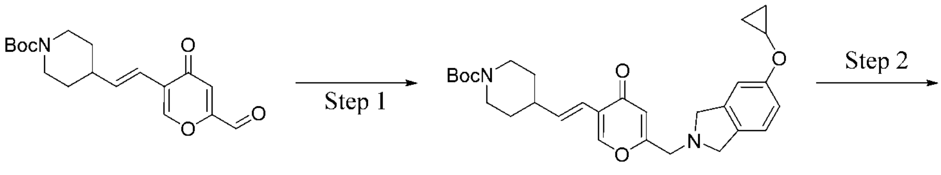


16D 30A

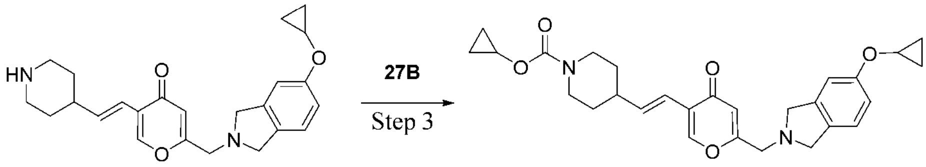


30B **Compound 30**

Step 1:

**16D** (750 mg, 2.25 mmol) was dissolved in 1,2-dichloroethane (20 mL), then 5-cyclopropoxyisoindoline trifluoroacetate (780 mg, 2.70 mmol) was added, and the resulting mixture was stirred at room temperature for 1 hour. Subsequently, sodium triacetoxyborohydride (572 mg, 2.7 mmol) was added, and the mixture was reacted at room temperature for an hour. The reaction solution was quenched with saturated aqueous sodium bicarbonate solution, the aqueous phase was extracted with dichloromethane, and the organic layers were combined and washed with saturated NaCl solution. The organic layers were combined, dried over $Na_2SO_4$, filtered and subjected to rotary evaporation. The residue was subjected to silica gel column chromatography (dichloromethane: methanol (v/v)=10:1) to obtain **30A** (482 mg, yield: 43%).

LC-MS (ESI): m/z = 437.3 $[M+H-56]^+$.

Step 2:

**30A** (482 mg, 0.98 mmol) was dissolved in DCM (5 mL), then trifluoroacetic acid (5 mL) was added, and the resulting mixture was reacted at room temperature for 1 hour. After the reaction was completed, the reaction solution was concentrated, and the crude product **30B** was directly used in the next reaction without purification.

Step 3:

The crude product **30B** obtained in the previous step was dissolved in DCM (20 mL), N,N-diisopropylethylamine (379 mg, 2.94 mmol) was added, and the mixture was stirred at room temperature for five minutes. Subsequently, **27B** (328 mg, 1.47 mmol) was added, and the mixture was reacted at room temperature for 1 hour. The reaction solution was quenched with water, the aqueous phase was extracted with dichloromethane, and the organic layers were combined and washed with saturated NaCl solution. The organic layers were combined, dried over $Na_2SO_4$, filtered and subjected to rotary evaporation. The residue was subjected to silica gel column (rinsed with ethyl acetate) chromatography to obtain compound **30** (200 mg, yield: 43%).

LC-MS (ESI): m/z = 477.7 $[M+H]^+$.

$^1$H NMR (400 MHz, $CDCl_3$) δ 7.79 (s, 1H), 7.08 (d, 1H), 6.91 - 6.86 (m, 2H), 6.56 - 6.46 (m, 1H), 6.41 (s, 1H), 6.20 - 6.13 (m, 1H), 4.25 - 3.93 (m, 6H), 3.75 (s, 2H), 3.73 - 3.67 (m, 1H), 2.85 - 2.74 (m, 2H), 2.33 - 2.19 (m, 1H), 1.74 (d, 2H), 1.45 - 1.25 (m, 3H), 0.79 - 0.71 (m, 4H), 0.72 - 0.64 (m, 4H).

**Example 31:**

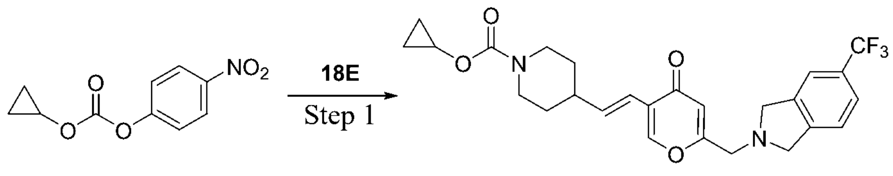


27B

Compound 31

Step 1

**18E** (808 mg, 2.0 mmol) was dissolved in dichloromethane (20 mL), DIEPA (516 mg, 4.0 mmol) and **27B** solution (446 mg, 2.0 mmol) were added, and the mixture was stirred at room temperature for 30 min. After the reaction was completed, the solvent was concentrated and the residue was separated and purified by silica gel column chromatography (PE/EA=1/2) to obtain compound **31** (360 mg, yield: 37%).

LC-MS (ESI): m/z = 488.5 $[M+H]^+$.

$^1H$ NMR (400 MHz, $CDCl_3$) δ 7.79 (s, 1H), 7.49 (d, 1H), 7.45 (s, 1H), 7.30 (d, 1H), 6.55 - 6.50 (m, 1H), 6.41 (s, 1H), 6.17 (d, 1H), 4.33 - 3.89 (m, 7H), 3.78 (s, 2H), 2.82 - 2.76 (m, 2H), 2.27 - 2.23 (m, 1H), 1.74 (d, 2H), 1.36 (d, 2H), 0.69 - 0.67 (m, 4H).

$^{19}F$ NMR (376 MHz, CDCl3) δ -60.07 (s).

**Example 32:**

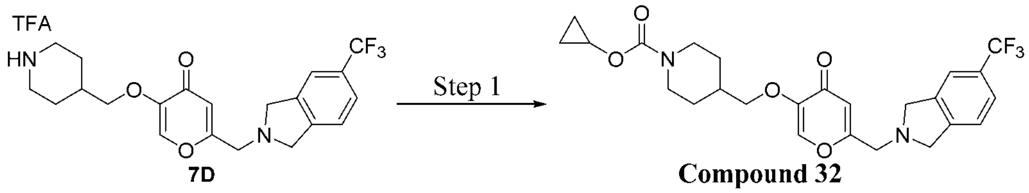


7D

Compound 32

Step 1:

Cyclopropanol (57 mg, 0.98 mmol) and N,N'-carbonyldiimidazole (119 mg, 0.73 mmol) were dissolved in tetrahydrofuran (10 mL), and the mixture was stirred at room temperature for 30 min. Subsequently, **7D** (200 mg, 0.49 mmol) and triethylamine (198 mg, 1.96 mmol) were added, and the mixture was reacted at 70°C under nitrogen atmosphere overnight. After the reaction was completed, the reaction mixture was cooled to room temperature, water (30 mL) was added to the reaction solution, and the resulting mixture was extracted with ethyl acetate (15 mL×3). The organic layers were combined, washed with saturated sodium chloride aqueous solution (25 mL), dried over anhydrous sodium sulphate, filtered, and concentrated. The residue was separated and purified by silica gel column chromatography (dichloromethane: methanol (v/v) = 25:1) to obtain **compound 32** (68 mg, yield: 28%).

$^1H$ NMR (400 MHz, $CDCl_3$) δ 7.59 (s, 1H), 7.52-7.49 (m, 1H), 7.46 (s, 1H), 7.32-7.30 (m, 1H), 6.49 (s, 1H), 4.29-3.96 (m, 7H), 3.80 (s, 2H), 3.73-3.70 (m, 2H), 2.81-2.73 (m, 2H), 2.09-1.98 (m, 1H), 1.89-1.81 (m, 2H), 1.28-1.17 (m, 2H), 0.70-0.64 (m, 4H).

LC-MS (ESI): m/z = 493.2 $[M+H]^+$.

**Example 33:**

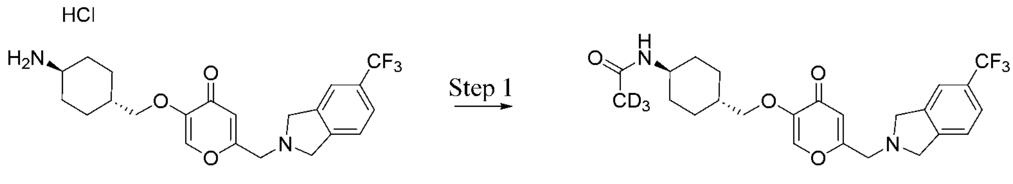


14D Compound 33

Step 1:

**14D** (550 mg, 1.30 mmol) and triethylamine (526 mg, 5.20 mmol) were dissolved in dichloromethane (10 mL). Under nitrogen atmosphere, deuterated acetyl chloride (117 mg, 1.43 mmol) was added dropwise in an ice bath, and the mixture was reacted at room temperature for 1 hour. After the reaction was completed, water (25 mL) was added to the reaction solution, and the resulting mixture was extracted with ethyl acetate (15 mL×3). The organic layers were combined, washed with saturated sodium chloride aqueous solution (25 mL), dried over anhydrous sodium sulphate, filtered, and concentrated. The residue was separated and purified by silica gel column chromatography (dichloromethane: methanol (v/v) = 20:1) to obtain **compound 33** (287 mg, yield: 47.2%).
LC-MS (ESI): m/z = 467.2 $[M+H]^+$.
$^1H$ NMR (400 MHz, DMSO-$d_6$) δ 8.12 (s, 1H), 7.73 - 7.67 (m, 1H), 7.65 - 7.61 (m, 1H), 7.60 - 7.54 (m, 1H), 7.50 - 7.43 (m, 1H), 6.38 (s, 1H), 4.02 (s, 4H), 3.80 (s, 2H), 3.66 - 3.60 (m, 2H), 3.51 - 3.42 (m, 1H), 1.89 - 1.77 (m, 4H), 1.71 - 1.60 (m, 1H), 1.23 - 1.00 (m, 4H).

**Example 34:**

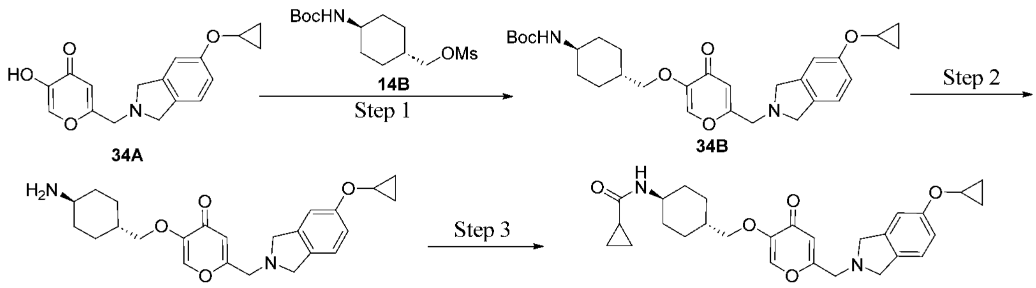


34A 14B 34B 34C Compound 34

Step 1:

With reference to the synthetic step of step 2 in **example 9, 34B** (573 mg, yield: 73%) was synthesized with **34A** (460 mg, 1.54 mmol, synthesized using 25c as the raw material with reference to the method of intermediate 8D) and **14B** (520 mg, 1.69 mmol) as the raw materials.
LC-MS (ESI): m/z= 511.2 $[M+H]^+$.

Step 2:

With reference to the synthetic step of step 3 in **example 9, 34C** (420 mg, yield: 91%) was synthesized using **34B** (570 mg) as the raw material.
LC-MS (ESI): m/z = 411.8 $[M+H]^+$.

Step 3:

With reference to the synthetic step of step 4 in **example 9, compound 34** (340 mg, yield: 70%) was synthesized using **34C** (420 mg, 1.02 mmol) as the raw material.
$^1H$ NMR (400 MHz, $CDCl_3$) δ 7.56 (s, 1H), 7.10-7.07 (m, 1H), 6.91-6.75 (m, 2H), 6.47 (s, 1H), 4.01-3.97 (m, 4H), 3.81-3.68 (m, 4H), 3.66-3.63 (m, 2H), 2.07-2.04 (m, 2H), 2.00-1.96 (m, 2H), 1.89-1.81 (m, 1H), 1.31-1.06 (m, 6H), 0.97-0.92 (m, 2H), 0.78-0.67 (m, 5H).

LC-MS (ESI): m/z = 479.2 $[M+H]^+$.

**Example 35:**

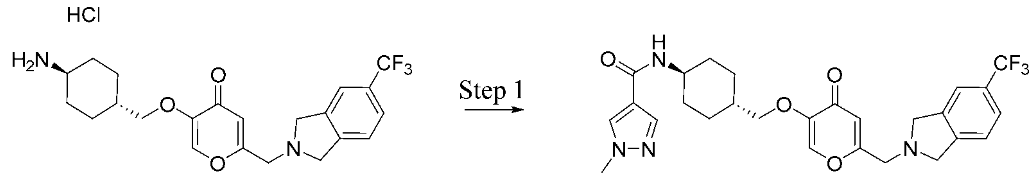


14D

Compound 35

Step 1:

1-methylpyrazole-4-carboxylic acid (179 mg, 1.42 mmol) and triethylamine (478 mg, 4.72 mmol) were dissolved in dichloromethane (20 mL), HATU (673 mg, 1.77 mmol) was added in an ice bath under nitrogen atmosphere, and the mixture was stirred for 5 minutes. **Compound 14D** (500 mg, 1.18mmol) was added and the mixture was reacted at room temperature for 2 hours. After the reaction was completed, water (25 mL) was added to the reaction solution, and the resulting mixture was extracted with ethyl acetate (15 mL×3). The organic layers were combined, washed with saturated sodium chloride aqueous solution (25 mL), dried over anhydrous sodium sulphate, filtered, and concentrated. The residue was separated and purified by silica gel column chromatography (dichloromethane: methanol (v/v) = 20:1) to obtain **compound 35** (398 mg, yield: 63.6%).

LC-MS (ESI): m/z = 531.1 $[M+H]^+$.

$^1H$ NMR (400 MHz, DMSO-$d_6$) δ 8.13 (s, 1H), 8.09 (s, 1H), 7.83 - 7.80 (m, 1H), 7.81 - 7.75 (m, 1H), 7.65 - 7.61 (m, 1H), 7.61 - 7.55 (m, 1H), 7.50 - 7.45 (m, 1H), 6.39 (s, 1H), 4.03 (s, 4H), 3.83 (s, 3H), 3.81 (s, 2H), 3.73 - 3.66 (m, 1H), 3.66 - 3.63 (m, 2H), 1.91 - 1.82 (m, 4H), 1.72 - 1.64 (m, 1H), 1.38 - 1.25 (m, 2H), 1.16 - 1.03 (m, 2H).

**Example 36 and example 37:**

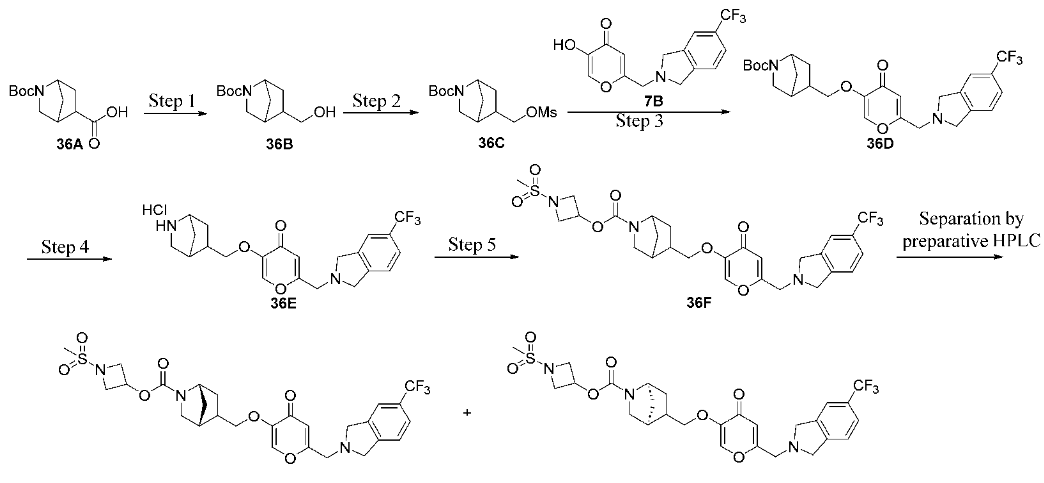


Compound 36

Compound 37

Step 1:

**36A** (400 mg, 1.66 mmol) was dissolved in tetrahydrofuran (10 mL), a borane tetrahydrofuran complex (5 mL, 1 M in THF) was slowly added dropwise in an ice bath, and the mixture was reacted in an ice bath for another 2 h. After the reaction was completed, 1 M aqueous hydrogen chloride solution (2 mL) was added to the reaction solution to quench the

reaction, then water (20 mL) was added, and the mixture was extracted with ethyl acetate (15 mL×3). The organic layers were combined, dried over anhydrous sodium sulphate, filtered, and concentrated. The residue was separated and purified by silica gel column chromatography (dichloromethane: methanol (v/v) = 25:1) to obtain **36B** (310 mg, yield: 82%).

LC-MS (ESI): m/z= 172.1 $[M+H-56]^+$.

Step 2:

**36B** (310 mg, 1.36 mmol) and triethylamine (410 mg, 4.08 mmol) were dissolved in dichloromethane (10 mL), methanesulphonyl chloride (187 mg, 1.63 mmol) was added dropwise in an ice bath under nitrogen atmosphere, and the mixture was reacted at room temperature for 1 h. After the reaction was completed, saturated sodium bicarbonate solution (30 mL) was added to the reaction solution to quench the reaction, and the resulting mixture was extracted with dichloromethane (20 mL×3). The organic layers were combined, dried over anhydrous sodium sulphate, filtered, and concentrated. The residue was separated and purified by silica gel column chromatography (petroleum ether: ethyl acetate (v/v) = 25:1) to obtain **36C** (295 mg, yield: 71%).

LC-MS (ESI): m/z= 250.1 $[M+H-56]^+$.

Step 3:

**36C** (295 mg, 0.97 mmol), **7B** (302 mg, 0.97 mmol) and potassium carbonate (402 mg, 2.91 mmol) were dissolved in DMF (8 mL), and the resulting mixture was reacted at 70°C under nitrogen atmosphere overnight. After the reaction was completed, the reaction mixture was cooled to room temperature, water (30 mL) was added, and the resulting mixture was extracted with ethyl acetate (15 mL×3). The organic layers were combined, washed with saturated sodium chloride aqueous solution (30 mL), dried over anhydrous sodium sulphate, filtered, and concentrated. The residue was separated and purified by silica gel column chromatography (dichloromethane: methanol (v/v) = 40:1) to obtain **36D** (380 mg, yield: 75%).

LC-MS (ESI): m/z= 465.5 $[M+H-56]^+$.

Step 4:

**36D** (380 mg, 0.73 mmol) was dissolved in dichloromethane (10 mL), 4 M hydrogen chloride 1,4-dioxane solution (2 mL) was added dropwise in an ice bath, and the mixture was reacted at room temperature for 2 h. After the reaction was completed, the reaction mixture was subjected to rotary evaporation under reduced pressure to remove the solvent and concentrated to obtain crude **36E** (300 mg, yield: 97%), which was directly used in the next reaction without further purification.

LC-MS (ESI): m/z = 421.1 $[M+H]^+$.

Step 5:

1-(methylsulphonyl)azetidin-3-ol (73 mg, 0.48 mmol) and N,N'-carbonyldiimidazole (117 mg, 0.72 mmol) were dissolved in tetrahydrofuran (6 mL), and the mixture was stirred at room temperature for 30 min. Then **36E** (200 mg, 0.48 mmol) and triethylamine (194 mg, 1.92 mmol) were added, and the mixture was reacted at 70°C under nitrogen atmosphere overnight. After the reaction was completed, the reaction mixture was cooled to room temperature, water (25 mL) was added to the reaction solution, and the resulting mixture was extracted with ethyl acetate (15 mL×3). The organic layers were combined, washed with saturated sodium chloride aqueous solution (25 mL), dried over anhydrous sodium sulphate, filtered, and concentrated. The residue was separated and purified by silica gel column chromatography (dichloromethane: methanol (v/v) = 25:1) to obtain **36F** (121 mg, yield: 43%).

**36F** was separated and purified by preparative HPLC to obtain **compound 36** (41 mg) and **compound 37** (21 mg).

Preparative HPLC separation and purification method: 1. Instrument: waters 2767 preparative liquid chromatographic instrument; chromatographic column: SunFire @ Prep C18 (19 mm x 250 mm). 2. The sample was filtered with a 0.45 μm filter to prepare a sample solution. 3. Preparative chromatography conditions: a. composition of mobile phases A and B: mobile phase A: acetonitrile; and mobile phase B: water (containing 0.1% ammonium acetate); b. gradient elution, mobile phase A: 5% - 50%; c. flow rate: 12 mL/min; d. elution time: 30 min.

HPLC analytical method: 1. Instrument: Shimadzu LC-20AT; 2. chromatographic column: Xtimate C18 4.6×50mm, 3 μm; 3. mobile phase system: A for 0.05% TFA in H2O; B for ACN; 4. gradient: B 5%-95%; 5. acquisition time: 10 min; 6. flow rate: 1.0 mL/min. retention time: **Compound 36:** tR=3.327 min; **Compound 37:** tR=3.318 min.

**Compound 36:** $^1$H NMR (400 MHz, $CDCl_3$) δ 7.68-7.62 (m, 1H), 7.51-7.48 (m, 1H), 7.46 (s, 1H), 7.32-7.30 (m, 1H), 6.49-6.49 (m, 1H), 5.18-5.09 (m, 1H), 4.27-4.23 (m, 1H), 4.19-4.12 (m, 2H), 4.09 (s, 4H), 4.03-3.94 (m, 3H), 3.88-3.75

(m, 3H), 3.43-3.31 (m, 1H), 3.26-3.21 (m, 1H), 2.89 (s, 3H), 2.82 (s, 1H), 2.67-2.56 (m, 1H), 1.98-1.88 (m, 1H), 1.83-1.74 (m, 1H), 1.63-1.58 (m, 2H).

LC-MS (ESI): m/z = 598.9 $[M+H]^+$.

**Compound 37:** $^1H$ NMR (400 MHz, $CDCl_3$) δ 7.63 (s, 1H), 7.51-7.48 (m, 1H), 7.46 (s, 1H), 7.32-7.29 (m, 1H), 6.48 (s, 1H), 5.18-5.11 (m, 1H), 4.30-4.25 (m, 1H), 4.19-4.14 (m, 2H), 4.09 (s, 4H), 4.04-3.96 (m, 2H), 3.81-3.68 (m, 4H), 3.37-3.31 (m, 1H), 3.12-3.07 (m, 1H), 2.89 (s, 3H), 2.75-2.71 (m, 1H), 2.28-2.18 (m, 1H), 1.98-1.86 (m, 1H), 1.63-1.59 (m, 3H), 1.35-1.28 (m, 1H).

LC-MS (ESI): m/z = 598.9 $[M+H]^+$.

**Example 38:**

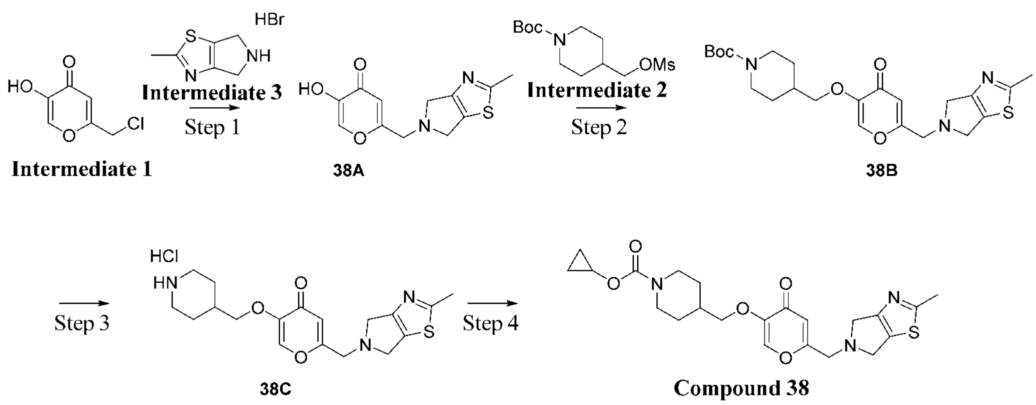


Intermediate 1, 38A, 38B, 38C, Compound 38

Step 1:

With reference to the operations in **example 1** (step 1), **compound 38A** (820 mg, yield: 49.4%) was obtained using **intermediate 1** (1 g, 6.28 mmol) and **intermediate 3** (synthesized with reference to document WO 2007087231 A2) as the raw materials.

LC-MS (ESI): m/z = 265.1 $[M+H]^+$.

Step 2:

With reference to the operations (step 2) in **example 1, compound 38B** (544 mg, yield: 38.0%) was obtained using **38A** (820 mg, 3.10 mmol) as the raw material.

LC-MS (ESI): m/z = 462.2 $[M+H]^+$.

Step 3:

With reference to the operation (step 3) in **example 1,** the hydrochloride of **compound 38C** (crude, 873 mg) was obtained using **38B** (544 mg, 1.18 mmol) as the raw material, and the crude was directly used in the next step without further purification.

Step 4:

With reference to the operations (step 8) in **example 20, compound 38** (65 mg, yield: 11.8%) was obtained using **38C** (450 mg) as the raw material.

LC-MS (ESI): m/z = 446.40 $[M+H]^+$.

$^1H$ NMR (400 MHz, $CDCl_3$) δ 7.58 (s, 1H), 6.46 (s, 1H), 4.32 - 4.06 (m, 4H), 4.06 - 4.04 (m, 1H), 4.04 - 4.01 (m, 2H), 3.84 (s, 2H), 3.75 - 3.68 (m, 2H), 2.83 - 2.72 (m, 2H), 2.71 (s, 3H), 2.09 - 2.00 (m, 1H), 1.89 - 1.81 (m, 2H), 1.28 - 1.17 (m, 2H), 0.72 - 0.64 (m, 4H).

**Example 39:**

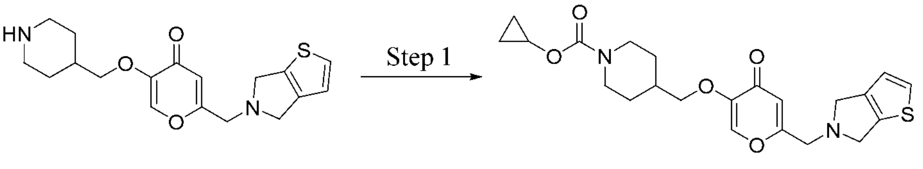


20G

Compound 39

Step 1:

At room temperature, cyclopropanol (17 mg, 0.28 mmol) and triphosgene (83 mg, 0.28 mmol) were dissolved in dry DCM (10 mL), and in an ice bath, triethylamine (85 mg, 0.84 mmol) was added dropwise. At 0°C, the mixture was stirred for 30 min, **20G** (50 mg, 0.14 mmol) was added, and the mixture was stirred evenly and then reacted at room temperature for 1 hour. Upon complete depletion of raw materials as monitored by TLC, the reaction was stopped. Water (20 mL) was added to the reaction solution, and the resulting mixture was extracted with dichloromethane (15 mL×3). The organic layers were combined, washed with saturated sodium chloride aqueous solution (20 mL), dried over anhydrous sodium sulphate, filtered, and concentrated. The residue was separated and purified by silica gel column chromatography (DCM:MeOH (v/v)= 15:1) to obtain compound **39** (10 mg, yield: 16.1%).

$^1$H NMR (400 MHz, $CDCl_3$) δ 7.59 (s, 1H), 7.24 (s, 1H), 6.82-6.81 (d, 1H), 6.48 (s, 1H), 4.15-4.01 (m, 7H), 3.86 (s, 2H), 3.72-3.70 (d, 2H), 2.80-2.73 (m, 2H), 2.08-1.98 (m, 3H), 1.86-1.83 (d,, 2H), 0.68-0.67 (d, 4H).

LC-MS (ESI): m/z = 431.1 $[M+H]^+$.

**Example 40:**

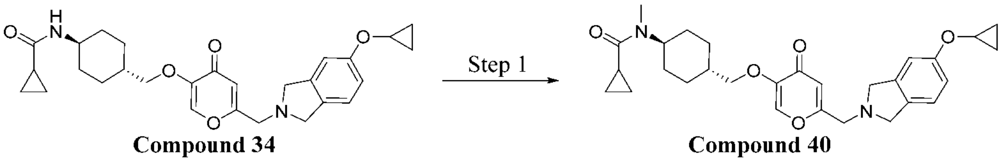


Compound 34

Compound 40

Step 1:

**Compound 34** (200 mg, 0.42 mmol) was dissolved in tetrahydrofuran (6 mL). Under nitrogen atmosphere, 60% sodium hydride (34 mg, 0.63 mmol) was added in portions in an ice bath, the mixture was stirred at room temperature for 30 min, and then iodomethane (118 mg, 0.63 mmol) was slowly added dropwise. The resulting mixture was reacted at room temperature overnight. After the reaction was completed, the reaction solution was slowly added dropwise to 1 M aqueous hydrogen chloride solution (10 mL) to quench the reaction, then water (10 mL) was added, and the mixture was extracted with ethyl acetate (15 mL×3). The organic layers were combined, washed with saturated sodium chloride aqueous solution (20 mL), dried over anhydrous sodium sulphate, filtered, and concentrated. The residue was separated and purified by silica gel column chromatography (dichloromethane: methanol (v/v) = 25:1) to obtain **compound 40** (55 mg, yield: 27%).

$^1$H NMR (400 MHz, $CDCl_3$) δ 7.56 (s, 1H), 7.11-7.08 (m, 1H), 6.91-6.76 (m, 2H), 6.48 (s, 1H), 4.03-3.99 (m, 4H), 3.82-3.69 (m, 4H), 3.67-3.63 (m, 2H), 3.21 (s, 3H), 2.08-2.05 (m, 2H), 2.01-1.96 (m, 2H), 1.89-1.82 (m, 1H), 1.33-1.07 (m, 6H), 0.98-0.92 (m, 2H), 0.79-0.69 (m, 5H).

LC-MS (ESI): m/z = 493.4 $[M+H]^+$.

**Example 41:**

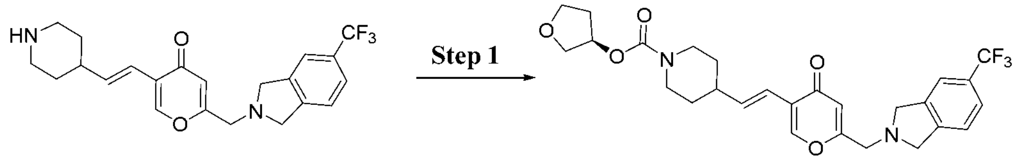


18E　　Compound 41

Step 1:

(R)-(-)-3-hydroxytetrahydrofuran (70.49 mg, 0.80 mmol) was dissolved in dichloromethane (5 mL), and N,N'-carbonyldiimidazole (0.13 g, 0.80 mmol) was added at room temperature. After the addition, the mixture was reacted at room temperature for 4.5 hrs, and triethylamine (0.16 g, 1.58 mmol) was added dropwise to the reaction system at room temperature. After the addition, the mixture was reacted at room temperature for 2 hrs, and 18E (160 mg, 0.40 mmol) was added dropwise at 0°C-5°C. After the addition, the mixture was reacted at 60°C overnight. After the reaction was completed, the reaction solution was concentrated and purified by preparative HPLC to obtain compound 41 (85 mg, yield: 40%).

LC-MS (ESI): *m*/*z*= 519.7 $[M+H]^+$.

$^1H$ NMR (400 MHz, DMSO-$d_6$) δ 8.32 (s, 1H), 7.63 (s, 1H), 7.58-7.56 (d, 1H), 7.48-7.46 (d, 1H), 6.69-6.63 (dd, 1H), 6.36 (s, 1H), 6.15-6.11 (d, 1H), 5.13-5.11 (m, 1H), 4.03 - 3.95 (m, 6H), 3.81 - 3.65 (m, 6H), 2.83 (s, 2H), 2.28-2.25 (m, 1H), 2.17 -2.05 (m, 1H), 1.91 - 1.87 (m, 1H), 1.71-1.68 (d, 2H), 1.27-1.17 (m, 2H).

**Example 42:**

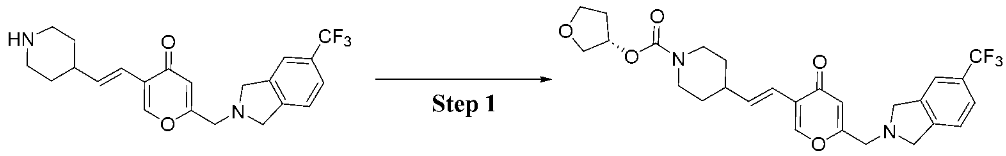


18E　　Compound 42

Step 1:

(S)-(-)-3-hydroxytetrahydrofuran (70.49 mg, 0.80 mmol) was dissolved in dichloromethane (5 mL), and N,N'-carbonyldiimidazole (0.13 g, 0.80 mmol) was added at room temperature. After the addition, the mixture was reacted at room temperature for 4.5 hrs, and triethylamine (0.16 g, 1.58 mmol) was added dropwise to the reaction system at room temperature. After the addition, the mixture was reacted at room temperature for 2 hrs, and 18E (160 mg, 0.40 mmol) was added dropwise at 0°C-5°C. After the addition, the mixture was reacted at 60°C overnight. After the reaction was completed, the reaction solution was concentrated and purified by preparative HPLC to obtain compound 42 (90 mg, yield: 40%).

LC-MS (ESI): *m*/*z*= 519.7 $[M+H]^+$.

$^1H$ NMR (400 MHz, DMSO-$d_6$) δ8.32 (s, 1H), 7.63 (s, 1H), 7.58-7.56 (d, 1H), 7.48-7.46 (d, 1H), 6.69-6.63 (dd, 1H), 6.36 (s, 1H), 6.15-6.11 (d, 1H), 5.13 - 5.11 (m, 1H), 4.03 - 3.95 (m, 6H), 3.82 - 3.65 (m, 6H), 2.84 (s, 2H), 2.28-2.25 (m, 1H), 2.13 - 2.06 (m, 1H), 1.92 - 1.86 (m, 1H), 1.71-1.68 (d, 2H), 1.27-1.17 (m, 2H).

**Example 43:**

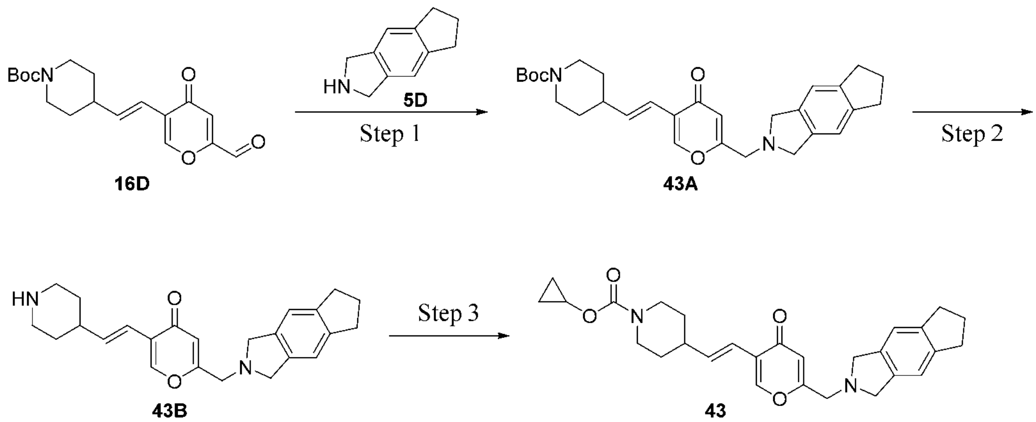


Step 1

**16D** (333.0 mg, 2.25 mmol) was dissolved in 1,2-dichloroethane (20 mL), then 5D (174.9 mg, 1.1 mmol) was added, and the resulting mixture was stirred at room temperature for 1 hour. Subsequently, sodium triacetoxyborohydride (466.0 mg, 2.2 mmol) was added, and the mixture was reacted at room temperature overnight. The reaction solution was quenched with saturated aqueous sodium bicarbonate solution, the aqueous phase was extracted with dichloromethane, and the organic layers were combined and washed with saturated NaCl solution. The organic layers were combined, dried over $Na_2SO_4$, filtered and subjected to rotary evaporation. The residue was subjected to silica gel column chromatography (dichloromethane: methanol (v/v) = 10:1) to obtain 43A (300.0 mg, yield: 63.0%).

LC-MS (ESI): *m/z* = 477.2 $[M+H]^+$.

Step 2

Compound 43A (300.0 mg, 0.62 mmol) was dissolved in dichloromethane (3.0 mL). Trifluoroacetic acid (2.0 mL) was then added, and the mixture was reacted at room temperature for half an hour and then concentrated to obtain compound 43B (310.0 mg), which was directly used in the next step.

LC-MS (ESI): *m/z* = 377.2 $[M+H]^+$.

Step 3

Cyclopropanol (44.0 mg, 0.75 mmol) was dissolved in tetrahydrofuran (5.0 mL), then CDI (120.0 mg, 0.75 mmol) was added, and the mixture was stirred and reacted at room temperature for 0.5 h. A solution of 43B (310.0 mg, 0.62 mmol) in tetrahydrofuran (5.0 mL), and triethylamine (191.0 mg, 1.9 mmol) were added, and the mixture was heated to 60°C and reacted overnight. After the reaction was completed as monitored by TLC, the reaction mixture was concentrated and the residue was separated and purified by silica gel column chromatography (petroleum ether: ethyl acetate (v/v) = 10:1) to obtain compound 43 (102.0 mg, yield: 35%).

LC-MS (ESI): m/z = 461.2 $[M+H]^+$.

$^1H$ NMR (400 MHz, $CDCl_3$) δ 7.79 (s, 1H), 7.04 (s, 2H), 6.55 - 6.49 (m, 1H), 6.42 (d, 1H), 6.17 (d, 1H), 4.45 - 3.86 (m, 7H), 3.78 (d, 2H), 2.94 - 2.71 (m, 6H), 2.32 - 2.15 (m, 1H), 2.11 - 2.04 (m, 2H), 1.74 (d, 2H), 1.36 (d, 2H), 0.69 - 0.67 (m, 4H).

**Example 44:**

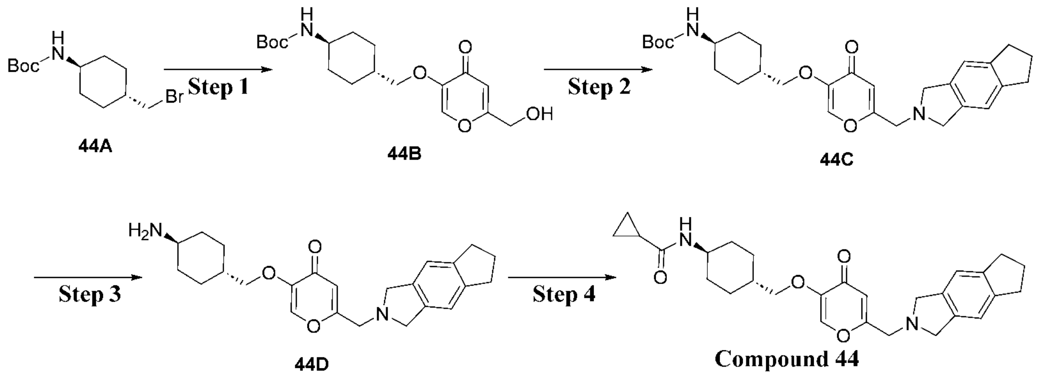


Step 1:

Kojic acid (1.00 g, 7.04 mmol) was dissolved in N,N-dimethylformamide (20 mL), and potassium carbonate (1.95 g, 14.08 mmol), 44A (2.47 g, 8.45 mmol) and potassium iodide (0.12 g, 0.70 mmol) were added at room temperature. After the addition, the mixture was reacted at 85°C overnight. After the reaction was completed, to the reaction solution was added water (50 mL) to quench the reaction, and the resulting mixture was extracted with ethyl acetate (50 mL×2). The organic phase was washed with water (50 mL) and saturated sodium chloride aqueous solution (50 mL), dried over anhydrous sodium sulphate, and concentrated. The residue was purified by column chromatography (petroleum ether/ethyl acetate =1/4, (v/v)) to obtain 44B (256 mg, yield: 10%).
LC-MS *(ESI): m/z*= 298.4 $[M-56+H]^+$.

Step 2:

44B (256 mg, 0.72 mmol) was dissolved in dichloromethane (10 mL), and triethylamine (0.36 g, 3.60 mmol) and methanesulphonyl chloride (0.99 g, 0.86 mmol) were added at 0°C-5°C. After the addition, the mixture was reacted at room temperature for 30 min, and 5D (197 mg, 0.86 mmol) was added at room temperature. After the addition, the mixture was reacted at room temperature overnight. After the reaction was completed, the reaction solution was concentrated and purified by column chromatography (petroleum ether/ethyl acetate =1/1, (v/v)) to obtain compound 44C (220 mg, yield: 61%).
LC-MS (ESI): *m/z*= 495.3 $[M+H]^+$.

Step 3:

44C (100 mg, 0.20 mmol) was dissolved in 1,4-dioxane (0.5 mL), and 4 N HCl 1,4-dioxane solution (10 mL) was added at room temperature. After the addition, the mixture was reacted at room temperature for 2.5 hrs. After the reaction was completed, the reaction mixture was concentrated to obtain the crude compound 44D, which was directly used in the next reaction.

Step 4:

Crude 44D was dissolved in dichloromethane (5 mL), and at room temperature, triethylamine (0.10 g, 1.00 mmol) and cyclopropanecarbonyl chloride (0.31 g, 0.30 mmol) were added dropwise to the reaction system. After the addition, the mixture was reacted at room temperature overnight. After the reaction was completed, the reaction solution was concentrated and purified by preparative HPLC to obtain compound 44 (15 mg, yield: 16%).
LC-MS (ESI): *m/z*= 463.5 $[M+H]^+$.
$^1$H NMR (400 MHz, DMSO-$d_6$) δ 8.11 (s, 1H), 7.91-7.89 (d, 1H), 7.06 (s, 2H), 6.36 (s, 1H), 3.87 (s, 4H), 3.75 (s, 2H), 3.64-3.62 (d, 2H), 3.51 (m, 1H), 2.82-2.78 (t, 4H), 2.04-1.96 (m, 2H), 1.83-1.81 (d, 4H), 1.66 (m, 1H), 1.52-1.47 (m, 1H), 1.24 - 1.14 (m, 2H), 1.09-1.00 (m, 2H), 0.64 - 0.59 (m, 4H).

**Example 45:**

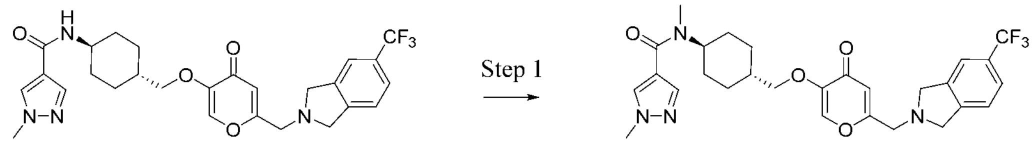


Compound 35 Compound 45

Step 1:

At room temperature, **compound 35** (281 mg, 0.53 mmol) was dissolved in dry DMF (5 mL). In an ice bath, NaH (42 mg, 1.06 mmol) was added, and the mixture was stirred for 10 minutes. Then to the reaction system was added iodomethane (113 mg, 0.79 mmol), and the resulting mixture was warmed to room temperature and reacted for another 6 hours. Then the reaction was stopped. Water (10 mL) was added to quench the reaction. The mixture was extracted with EA (15 mL) and subjected to liquid separation. The aqueous phase was washed twice with EA (10 mL), and the organic phases were combined, washed twice with saturated sodium chloride solution (20 mL), dried and concentrated. The residue was subjected to column chromatography (DCM:MeOH=15:1) to obtain **compound 45** (17 mg, yield: 6.1%).

LC-MS (ESI): m/z = 545.4 $[M+H]^+$.

$^1H$ NMR (400 MHz, DMSO-$d_6$) δ 8.12 - 8.08 (m, 2H), 7.83 - 7.81 (m, 1H), 7.80 - 7.76 (m, 1H), 7.69 - 7.66 (m, 1H), 7.54 - 7.49 (m, 1H), 7.08 - 7.03 (m, 1H), 5.32 (s, 1H), 4.40 - 4.30 (m, 1H), 3.83 (s, 3H), 3.72 - 3.67 (m, 2H), 3.67 - 3.65 (m, 2H), 2.35 (s, 3H), 2.04 - 1.98 (m, 1H), 1.97 (s, 2H), 1.92 - 1.83 (m, 4H), 1.71 (s, 1H), 1.36 - 1.27 (m, 2H), 1.19 - 1.08 (m, 2H).

**Example 46:**

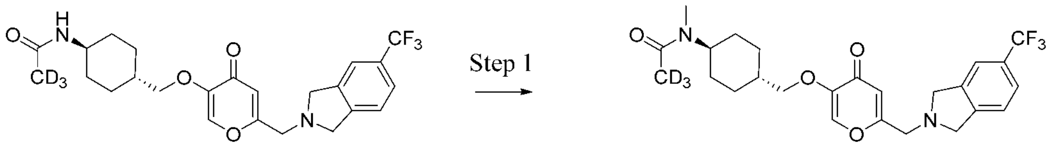


Compound 33 Compound 46

Step 1:

With reference to the operations in **example 45** (step 1), **compound 46** (20 mg, yield: 10.4%) was obtained using **compound 33** (187 mg, 0.40 mmol).

LC-MS (ESI): m/z = 481.3 $[M+H]^+$.

$^1H$ NMR (400 MHz, DMSO-$d_6$) δ 8.09 (s, 1H), 7.72 - 7.68 (m, 1H), 7.67 (s, 1H), 7.54 - 7.48 (m, 1H), 7.08 - 7.03 (m, 1H), 5.32 (s, 1H), 4.41 - 4.28 (m, 1H), 3.72 - 3.66 (m, 1H), 3.66 - 3.62 (m, 2H), 3.52 - 3.44 (m, 1H), 2.35 (s, 3H), 2.03 - 1.98 (m, 1H), 1.97 (s, 2H), 1.87 - 1.79 (m, 4H), 1.74 (s, 1H), 1.20 - 1.02 (m, 4H).

**Example 47:**

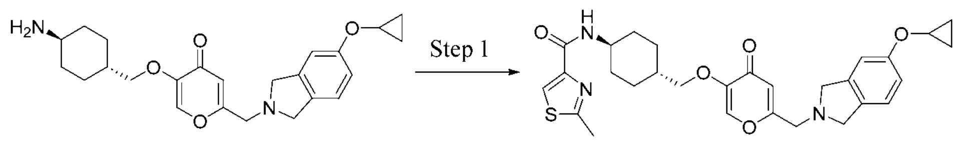


34B Compound 47

Step 1:

At room temperature, **34B** (160 mg, 0.39 mmol), 2-methylthiazole-4-carboxylic acid (61 mg, 0.43 mmol) and NMI (96 mg, 1.17 mmol) were dissolved in dry DCM (10 mL), and the mixture was stirred at room temperature for 10 min. TCFH (130 mg, 0.46 mmol) was added and the resulting mixture was reacted for another 1 hour. Upon complete depletion of raw

materials as monitored by TLC, the reaction was stopped. Water (20 mL) was added to the reaction solution, and the resulting mixture was extracted with dichloromethane (15 mL×3). The organic layers were combined, washed with saturated sodium chloride aqueous solution (20 mL), dried over anhydrous sodium sulphate, filtered, and concentrated. The residue was separated and purified by silica gel column chromatography (DCM:MeOH (v/v) = 15:1) to obtain compound **47** (20 mg, yield: 9.6%).

$^1$H NMR (400 MHz, $CDCl_3$) δ 7.92 (s, 1H), 7.58 (s, 1H), 7.18-7.16 (d, 1H), 7.10-7.08 (d, 1H), 6.90-6.88 (d, 1H), 6.48 (s, 1H), 4.03-4.00 (d, 4H), 3.76 (s, 2H), 3.69-3.67 (d, 2H), 2.71 (s, 3H), 2.15-2.12 (d, 2H), 2.04-2.01 (d, 2H), 1.37-1.14 (m, 8H), 0.75 (s, 4H).

LC-MS (ESI): m/z = 536.3 [M+H]$^+$.

**Example 48:**

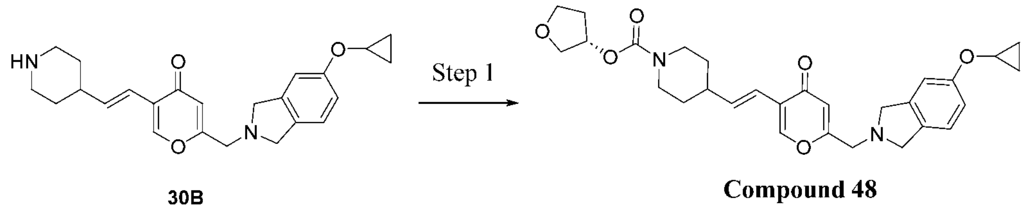


30B

Compound 48

Step 1:

(S)-(-)-3-hydroxytetrahydrofuran (68.00 mg, 0.77 mmol) was dissolved in tetrahydrofuran (5 mL), and then N,N'-carbonyldiimidazole (130 mg, 0.77 mmol) was added. After the addition, the mixture was reacted at room temperature for 4 h, then triethylamine (120 mg, 1.23 mmol) was added dropwise to the reaction system, and the mixture was reacted for 2 hrs. In an ice-water bath, compound 41E (120 mg, 0.31 mmol) was added dropwise. After the addition, the reaction system was warmed to 60°C and stirred overnight. Upon complete depletion of raw materials as monitored by LCMS, the reaction solution was concentrated and purified by preparative HPLC to obtain compound 48 (50 mg, yield: 32%).

LC-MS (ESI): *m/z* = 507.80 [M+H]$^+$.

$^1$H NMR (400 MHz, DMSO-d6) δ 8.31 (s, 1H), 7.13 (d, 2H), 6.99-6.91 (m, 1H), 6.89-6.82 (m, 1H), 6.69-6.61 (m, 1H), 6.34 (s, 1H), 6.13 (d, 1H), 5.15-5.08 (m, 1H), 4.00-3.86 (m, 6H), 3.80-3.64 (m, 7H), 2.94-2.76 (m, 2H), 2.31-2.21 (m, 1H), 2.15-2.05 (m, 1H), 1.94-1.84 (m, 1H), 1.72-1.64 (m, 2H), 1.28-1.16 (m, 2H), 0.79-0.71 (m, 2H), 0.65-0.57 (m, 2H).

**Example 49:**

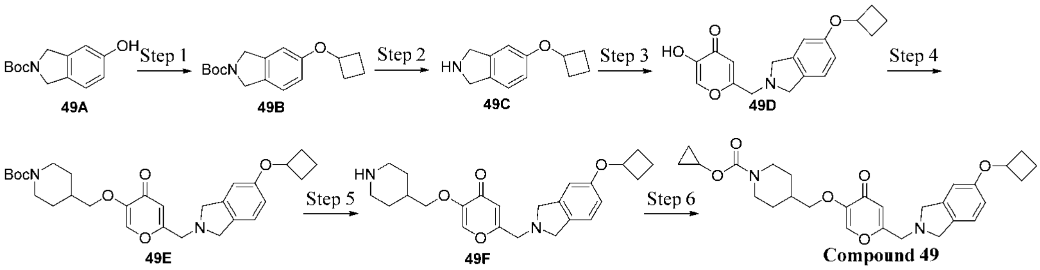


49A 49B 49C 49D 49E 49F Compound 49

Step 1:

Compound **49A** (1.00 g, 4.25 mmol) was dissolved in DMF (20 mL), bromocyclobutane (690 mg, 5.10 mmol) and caesium carbonate (3.46 g, 10.63 mmol) were respectively added in sequence, and the reaction solution was stirred at 90°C under nitrogen protection overnight. After the reaction was completed as monitored by TLC, the reaction solution was cooled to room temperature, water (40 mL) was added, and the mixture was extracted twice with dichloromethane (30 mL). The organic phases were combined, dried and concentrated. The residue was separated by silica gel column chromatography (petroleum ether:ethyl acetate = 10:1) to obtain compound **49B** (600 mg, yield: 48.8%).
LC-MS (ESI): m/z = 234.2 [M-56+H]$^+$

Step 2:

Compound **49B** (600 mg, 2.07 mmol) was dissolved in dichloromethane (8 mL), and then trifluoroacetic acid (2 mL) was added. The mixture was reacted at room temperature for half an hour, and the reaction solution was concentrated under reduced pressure to obtain the crude compound **49C** (800 mg), which was directly used in the next reaction.
LC-MS (ESI): m/z=190.2 $[M+H]^+$

Step 3:

Compound **49C** (540 mg, 2.39 mmol) was dissolved in acetonitrile (8 mL), and then (2-chloromethyl)-5-hydroxy-4H-pyran-4-one (365 mg, 2.27 mmol) and triethylamine (730 mg, 7.18 mmol) were added. The reaction solution was stirred at room temperature for 16 h, and concentrated under reduced pressure. The residue was separated by silica gel column chromatography (dichloromethane/methanol (v/v) = 95/5) to obtain compound **49D** (580 mg, yield: 77.4%).
LC-MS (ESI): m/z=314.1 $[M+H]^+$

Step 4:

Compound **49D** (580 mg, 1.85 mmol) was dissolved in DMF (15 mL), and then tert-butyl 4-bromopiperidine-1-carboxylate (540 mg, 2.05 mmol) and caesium carbonate (1.51 g, 4.63 mmol) were added. The reaction solution was stirred at 85°C for 16 h and cooled to room temperature. Water (60 mL) was added and the resulting mixture was extracted three times with dichloromethane (40 mL). The organic phases were combined, dried and concentrated. The residue was separated by silica gel column chromatography (dichloromethane/methanol (v/v) = 95/5) to obtain compound **49E** (900 mg, yield: 95.2%).
LC-MS (ESI): m/z = 511.5 $[M+H]^+$

Step 5:

Compound **49E** (900 mg, 1.76 mmol) was dissolved in dichloromethane (8 mL) and then hydrogen chloride dioxane solution (2 mL) was added. The mixture was reacted at room temperature for half an hour, and the reaction solution was concentrated under reduced pressure to obtain the crude compound **49F** (850 mg), which was directly used in the next reaction.
LC-MS (ESI): m/z=411.2 $[M+H]^+$

Step 6:

Cyclopropanol (210 mg, 3.58 mmol) was dissolved in DMF (10 mL), and then carbonyldiimidazole (580 mg, 3.58 mmol) and triethylamine (730 mg, 7.18 mmol) were added. The mixture was stirred at room temperature for 40 min. **49F** (850 mg, 1.76 mmol) was then added, and the reaction solution was transferred to an 85°C, nitrogen atmosphere and stirred for 16 h. The reaction solution was cooled to room temperature, and water (60 mL) was added. The mixture was extracted three times with dichloromethane (50 mL). The organic phases were combined, dried and concentrated. The residue was separated by silica gel column chromatography (dichloromethane/methanol (v/v) = 95/5), and then further subjected to reverse phase preparative chromatography (acetonitrile/water (v/v) = 5/95 - 95/5) to obtain compound **49** (80 mg, yield: 9.05%).

$^1$H NMR (400 MHz, DMSO-$d_6$) δ 8.12 (s, 1H), 7.11 - 7.08 (m, 1H), 6.72 (s, 1H), 6.69 - 6.64 (m, 1H), 6.36 (s, 1H), 4.66 - 4.59 (m, 1H), 4.00 - 3.95 (m, 2H), 3.89 - 3.86 (m, 5H), 3.75 (s, 2H), 3.69 - 3.68 (m, 2H), 2.81 - 2.75 (m, 2H), 2.43 - 2.36 (m, 2H), 2.05 - 1.96 (m, 2H), 1.93 - 1.87 (m, 1H), 1.77 - 1.70 (m, 3H), 1.66 - 1.59 (m ,1H), 1.16 - 1.07 (m, 2H), 0.63 - 0.60 (m, 4H).
LC-MS (ESI): m/z = 495.3 $[M+H]^+$

**Example 50:**

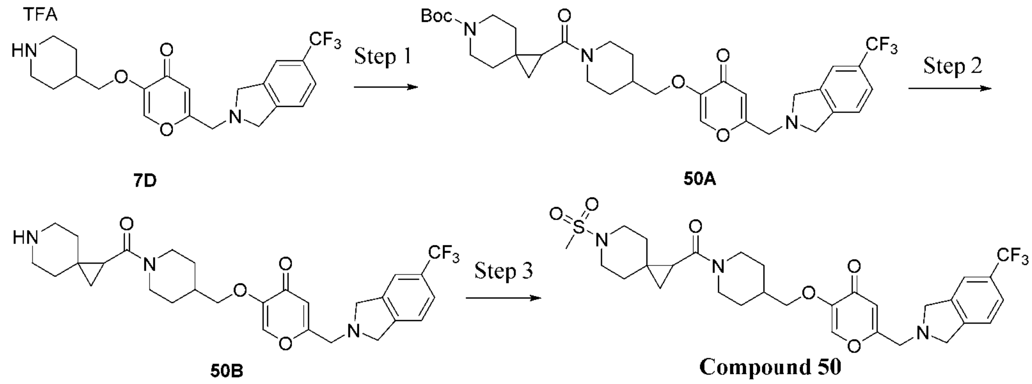


7D 50A 50B Compound 50

Step 1:

6-BOC-6-azaspiro[2.5]octane-1-carboxylic acid (0.14 g, 0.54 mmol), compound **7D** (0.22 g, 0.54 mmol) and methylimidazole (0.089 g, 1.08 mmol) were dissolved in DMF (10 mL) and the mixture was stirred for 10 minutes. N,N,N',N'-tetramethylchloroformamidinium hexafluorophosphate (0.23 g, 0.81 mmol) was added, and the mixture was stirred and reacted at room temperature for 1 hour. The reaction solution was poured into water and the resulting mixture was extracted with ethyl acetate (20 mL×3). The combined organic phases were washed with saturated sodium chloride aqueous solution (20 mL×2), dried over anhydrous sodium sulphate, filtered and concentrated under reduced pressure. The residue was purified using a medium-pressure preparation instrument Biotage Isolera One (12 g silica gel column, eluents: 0-50% EA/PE) to obtain compound **50A** (0.215 g, yield: 61.66%).
LC-MS (ESI): m/z= 646.3 $[M+H]^+$.

Step 2:

Compound **50A** (0.215 g, 0.33 mmol) was dissolved in DCM (10 mL), trifluoroacetic acid (2 mL) was added, and the mixture was stirred and reacted at room temperature for 1 hour. Upon complete depletion of raw materials as monitored by LCMS, the reaction solution was poured into ice water to quench the reaction, and the resulting mixture was extracted with ethyl acetate (20 mL×3). The combined organic phases were washed with saturated sodium chloride aqueous solution (20 mL×2), dried over anhydrous sodium sulphate, filtered and concentrated under reduced pressure to obtain compound **50B** (0.15 g), which was directly used in the next step without purification.
LC-MS (ESI): m/z= 546.1 $[M+H]^+$.

Step 3:

At 0°C, compound **50B** (0.15 g, 0.27 mmol) and triethylamine (0.082 g, 0.81 mmol) were dissolved in dichloromethane (10 mL), the mixture was stirred for 15 minutes, and then methanesulphonyl chloride (0.037 g, 0.32 mmol) was added dropwise. The resulting mixture was stirred for another 1 hour. The reaction solution was poured into water and the resulting mixture was extracted with ethyl acetate (20 mL×3). The combined organic phases were washed with saturated sodium chloride aqueous solution (20 mL×2), dried over anhydrous sodium sulphate, filtered and concentrated under reduced pressure. The residue was purified using a medium-pressure preparation instrument Biotage Isolera One (12 g silica gel column, eluents: 0-5% MeOH/DCM) to obtain compound **50** (40 mg, yield: 23.7%).
LC-MS (ESI): m/z= 624.7 $[M+H]^+$.
$^1$H NMR (400 MHz, $CDCl_3$): δ 7.61 (s, 1H), 7.51 - 7.49 (m, 1H), 7.46 (s, 1H), 7.32 - 7.30 (m, 1H), 6.49 (s, 1H), 4.69 - 4.60 (m, 1H), 4.12 (s, 5H), 3.81 - 3.66 (m, 4H), 3.60 - 3.37 (m, 2H), 3.21 - 2.93 (m, 4H), 2.80 (s, 3H), 2.67 - 2.62 (m, 1H), 2.15 - 1.68 (m, 7H), 1.43 - 1.13 (m, 4H).

**Example 51:**

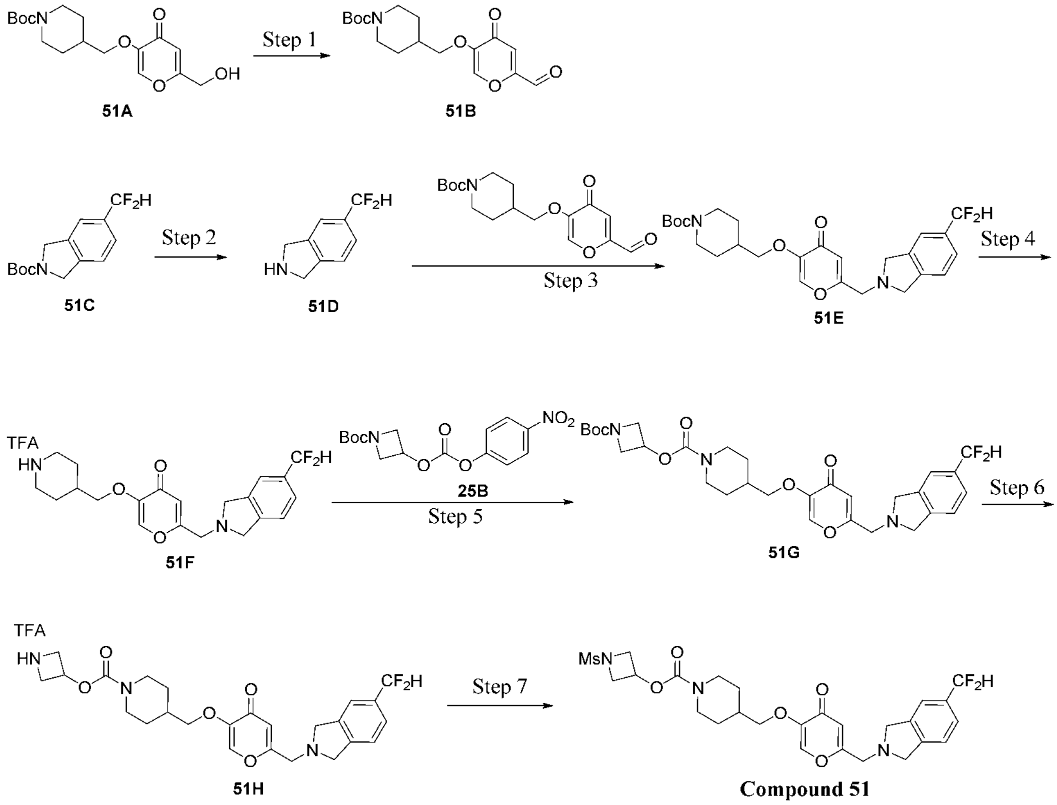


51A 51B 51C 51D 51E 51F 25B 51G 51H Compound 51

Step 1:

**51A** (3.4 g, 10 mmol) was dissolved in ethyl acetate (20 mL), IBX (5.6 g, 20 mmol) was added, and the mixture was reacted at 80°C for 12 h. After the reaction was completed, the reaction mixture was filtered and concentrated to obtain **51B** (2.1 g).

LC-MS (ESI): $m/z$ = 337.1 $[M+H]^+$.

Step 2:

Compound **51C** (540 mg, 2 mmol) was dissolved in dichloromethane (10 mL). Trifluoroacetic acid (2.0 mL) was then added, and the mixture was reacted at room temperature for 15 min and then concentrated to obtain compound **51D** (520 mg), which was directly used in the next step.

LC-MS (ESI): $m/z$ = 169.2 $[M+H]^+$.

Step 3:

**51B** (606 mg, 1.8 mmol) was dissolved in DCE (20 mL), 5-difluoromethylisoindoline hydrochloride (370 mg, 1.8 mmol), sodium borohydride acetate (763 mg, 3.6 mmol) and one drop of glacial acetic acid were successively added, and the mixture was reacted at room temperature for 1 h. After the reaction was completed, to the reaction solution was added water (10 mL), the mixture was extracted with DCM (20 mL×2), and the organic layers were combined, washed with saturated sodium chloride aqueous solution (20 mL×1), dried over anhydrous sodium sulphate, filtered, and concentrated. The residue was separated and purified by silica gel column chromatography (PE/EA=1/1) to obtain **51E** (735 mg, yield: 83%).

LC-MS (ESI): $m/z$ = 169.2 $[M+H]^+$.

Step 4:

Compound **51E** (735 mg, 1.5 mmol) was dissolved in dichloromethane (10 mL). Trifluoroacetic acid (3.0 mL) was then added, and the mixture was reacted at room temperature for 10 min and then concentrated to obtain compound **51F** (710 g), which was directly used in the next step.

Step 5:

**51F** (710 mg, 1.4 mmol) was dissolved in dichloromethane (20 mL), DIEPA (254 mg, 2.0 mmol) and **25B** solution (0.6 g, 2.0 mmol) were added, and the mixture was stirred at room temperature for 30 min. After the reaction was completed, the solvent was concentrated and the residue was separated and purified by silica gel column chromatography (PE/EA=1/2) to obtain **51G** (589 mg, yield: 72%).
LC-MS (ESI): m/z = 590.6 $[M+H]^+$.

Step 6:

Compound **51G** (590 mg, 1.0 mmol) was dissolved in dichloromethane (10 mL). Trifluoroacetic acid (3 mL) was then added, and the mixture was reacted at room temperature for half an hour and then concentrated to obtain compound **51H** (570 mg), which was directly used in the next step.

Step 7:

**51H** (570 mg, 0.9 mmol) was dissolved in DCM (10 mL), triethylamine (122 mg, 1.2 mmol) and methanesulphonyl chloride (137 mg, 1.2 mmol) were added, and the resulting mixture was stirred at room temperature for 20 min. After concentration, the residue was separated and purified by silica gel column chromatography (dichloromethane: methanol (v/v)=10:1) to obtain **compound 51** (120 mg, yield: 24%).

LC-MS (ESI): m/z = 568.6 $[M+H]^+$.
$^1$H NMR (400 MHz, $CDCl_3$) δ 7.60 (s, 1H), 7.36 (d, 2H), 7.29 (s, 1H), 6.78 - 6.41 (m, 2H), 5.29 - 5.00 (m, 1H), 4.19 - 4.15 (m, 4H), 4.08 (s, 4H), 4.02 - 3.98 (m, 2H), 3.78 (s, 2H), 3.73 (d, 2H), 2.89 (s, 3H), 2.79 (s, 2H), 2.12 - 2.00 (m, 1H), 1.89 (d, 2H), 1.32 - 1.22 (m, 2H),
$^{19}$F NMR (400 MHz, CDCl3) δ -107.63 (s).

**Example 52:**

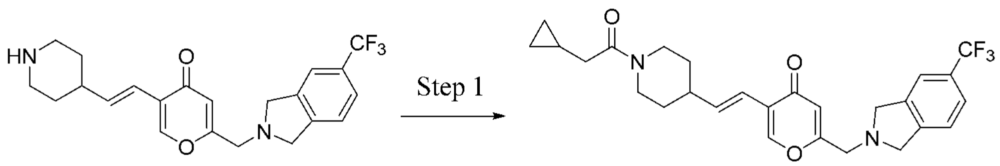


18E

Compound 52

Step 1:

Compound 18E (0.37 g, 0.91 mmol) was dissolved in dichloromethane (10 mL), and in an ice-water bath, triethylamine (0.28 g, 2.77 mmol) and cyclopropylacetyl chloride (0.22 g, 1.86 mmol) were successively added. After the dropwise addition was completed, the mixture was reacted for another 4 h in an ice-water bath. Upon complete depletion of raw materials as monitored by LCMS, the reaction solution was concentrated and purified by preparative HPLC to obtain compound 52 (40 mg, yield: 9%).
LC-MS (ESI): m/z = 487.30 $[M+H]^+$.
$^1$H NMR (400 MHz, DMSO-$d_6$) δ 8.32 (s, 1H), 7.62 (s, 1H), 7.57 (d, 1H), 7.46 (d, 1H), 6.76-6.60 (m, 1H), 6.36 (s, 1H), 6.13 (d, 1H), 4.38 (d, 1H), 4.03 (s, 4H), 3.90-3.74 (m, 3H), 3.10-2.98 (m, 1H), 2.64-2.54 (m, 1H), 2.36-2.19 (m, 3H), 1.77-1.62 (m, 2H), 1.31-1.09 (m, 2H), 1.00-0.87 (m, 1H), 0.48-0.38 (m, 2H), 0.15-0.06 (m, 2H).

**Example 53:**

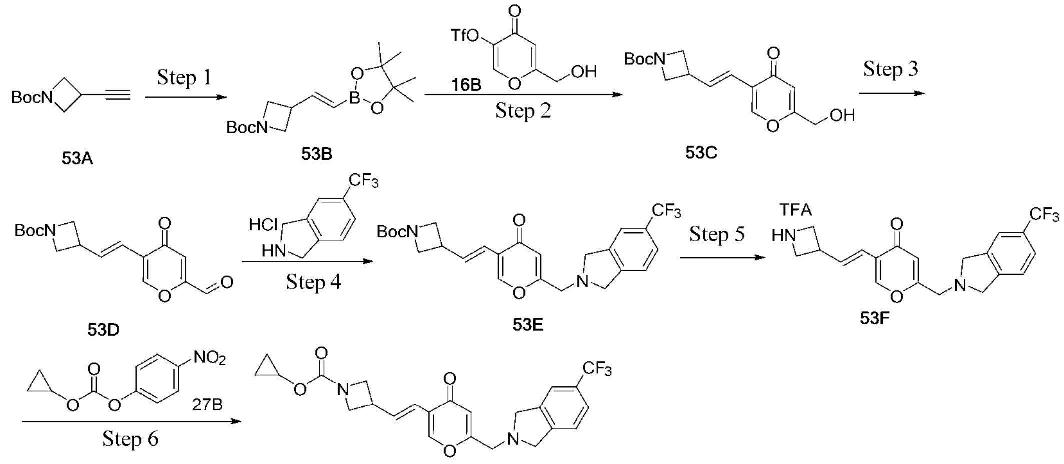


53A 53B 53C 53D 53E 53F

Compound 53

Step 1:

Compound **53A** (5.4 g, 30 mmol) was dissolved in toluene (50 mL), bis(cyclopentadienyl)zirconium chloride hydride (397 mg, 1.5 mmol) and pinacolborane (5.0 g, 39 mmol) were successively added, and the mixture was reacted at 60°C for 18 h. After the reaction was completed, the reaction mixture was washed with saturated sodium chloride aqueous solution (20 mL×3), dried over anhydrous sodium sulphate, filtered, and concentrated. The residue was separated and purified by silica gel column chromatography (PE/EA=2/1) to obtain **53B** (7.7 g, yield: 83%).

LC-MS (ESI): *m/z* = 310.2 $[M+H]^+$.

Step 2:

**53B** (7.7 g, 25 mmol) was dissolved in 1,4-dioxane (40 mL), and 1,1-bis(diphenylphosphino)ferrocene palladium dichloride (1.8 mg, 2.5 mmol), caesium carbonate (16 g, 50 mmol), **16B** (6.8 g, 25 mmol), and water (10 mL) were then added. After nitrogen displacement three times, the mixture was heated to 90°C and reacted for 3 h. The system was then passed through a short silica gel column, rinsed with ethyl acetate, concentrated, and subjected to column chromatography (petroleum ether/ethyl acetate = 9 / 1) to obtain compound 53C (4.6 g, yield: 60%).

LC-MS (ESI): *m/z* = 308.4 $[M+H]^+$.

Step 3:

**53C** (4.6 g, 15 mmol) was dissolved in ethyl acetate (50 mL), IBX (8.4 g, 30 mmol) was added, and the mixture was reacted at 80°C for 12 h. After the reaction was completed, the reaction mixture was filtered and concentrated to obtain **53D** (4.3 g).

Step 4:

**53D** (1.5 g, 5 mmol) was dissolved in DCE (20 mL), 5-trifluoromethylisoindoline hydrochloride (1.1 g, 5.0 mmol), sodium borohydride acetate (1.4 g, 6.8 mmol) and five drops of glacial acetic acid were successively added, and the mixture was reacted at room temperature for 1 h. After the reaction was completed, to the reaction solution was added water (10 mL), the mixture was extracted with DCM (20 mL×2), and the organic layers were combined, washed with saturated sodium chloride aqueous solution (25 mL×1), dried over anhydrous sodium sulphate, filtered, and concentrated. The residue was separated and purified by silica gel column chromatography (PE/EA=2/1) to obtain **53F** (1.8 g, yield: 78%).

Step 5:

Compound **53F** (1.8 g, 3.8 mmol) was dissolved in dichloromethane (10 mL). Trifluoroacetic acid (8.0 mL) was then added, and the mixture was reacted at room temperature for 10 min and then concentrated to obtain compound **53G** (1.6 g), which was directly used in the next step.

LC-MS (ESI): *m/z* = 377.2 $[M+H]^+$.

Step 6:

**53G** (1.1 g, 3.0 mmol) was dissolved in dichloromethane (20 mL), DIEPA (1.1 g, 8.0 mmol) and **27B** solution (1.4 g,6 mmol) were added, and the mixture was stirred at room temperature for 30 min. After the reaction was completed, the solvent was concentrated and the residue was separated and purified by silica gel column chromatography (PE/EA=1/1) to obtain **53** (320 mg, yield: 30%).
LC-MS (ESI): *m/z* = 461.1 $[M+H]^+$.
$^1H$ NMR (400 MHz, $CDCl_3$) δ 7.81 (s, 1H), 7.50 (d, 1H), 7.46 (s, 1H), 7.31 (d, 1H), 6.89 - 6.83 (m, 1H), 6.43 (s, 1H), 6.18 (d, 1H), 4.16 (t, 2H), 4.10 (s, 4H), 4.07 - 3.98 (m, 1H), 3.89 - 3.82 (m, 2H), 3.79 (s, 2H), 3.44 - 3.26 (m, 1H), 0.79 - 0.56 (m, 4H).
$^{19}F$ NMR (400 MHz, $CDCl_3$) δ -60.08 (s).

**Example 54:**

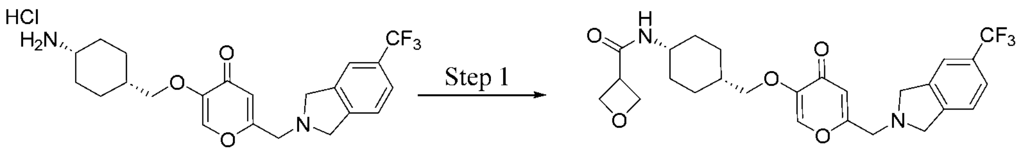

9D

Compound 54

Step 1:

With reference to the synthetic step of step 4 in **example 9, compound 54** (66 mg, yield: 36%) was synthesized using **9D** (150 mg, 0.36 mmol) and oxetane-3-carboxylic acid (44 mg, 0.43 mmol) as the raw materials.
$^1H$ NMR (400 MHz, $CDCl_3$) δ 7.60 (s, 1H), 7.51-7.48 (m, 1H), 7.46 (s, 1H), 7.32-7.29 (m, 1H), 6.48 (s, 1H), 6.17-6.14 (m, 1H), 4.89-4.85 (m, 2H), 4.81-4.77 (m, 2H), 4.19-4.14 (m, 1H), 4.09 (s, 4H), 3.82-3.70 (m, 5H), 1.93-1.88 (m, 1H), 1.78-1.61 (m, 6H), 1.57-1.46 (m, 2H).
LC-MS (ESI): m/z = 507.3 $[M+H]^+$.

**Example 55:**

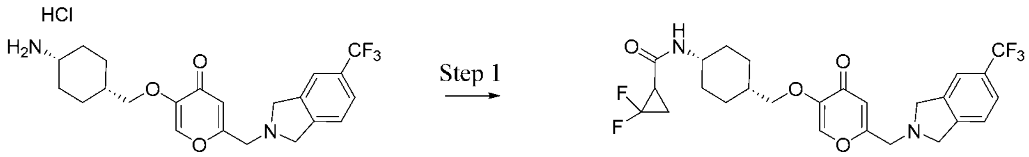

9D

Compound 55

Step 1:

2,2-difluorocyclopropanecarboxylic acid (80 mg, 0.65 mmol), DIPEA (305 mg, 2.36 mmol) and HATU (270 mg, 0.71 mmol) were dissolved in dichloromethane (10 mL), and in an ice water bath, the mixture was stirred under nitrogen atmosphere for 10 minutes. **9D** (250 mg, 0.59 mmol) was added and in an ice-water bath, the mixture was stirred for 5 minutes, warmed to room temperature and reacted for 1 hour. After the reaction was completed, the mixture was directly distilled under reduced pressure and concentrated. The residue was separated and purified by silica gel column chromatography (dichloromethane: methanol (v/v) = 15:1) to obtain **compound 55** (67 mg, 21.6%).
LC-MS (ESI): m/z = 527.2 $[M+H]^+$.
$^1H$ NMR (400 MHz, DMSO-$d_6$) δ 8.18 (s, 1H), 8.17 - 8.13 (m, 1H), 7.63 (s, 1H), 7.60 - 7.55 (m, 1H), 7.49 - 7.45 (m, 1H), 6.40 (s, 1H), 4.03 (s, 4H), 3.85 (s, 1H), 3.81 (s, 2H), 3.74 - 3.68 (m, 2H), 2.71 - 2.58 (m, 1H), 1.92 - 1.74 (m, 3H), 1.62 - 1.37 (m, 8H).

**Example 56 and example 57:**

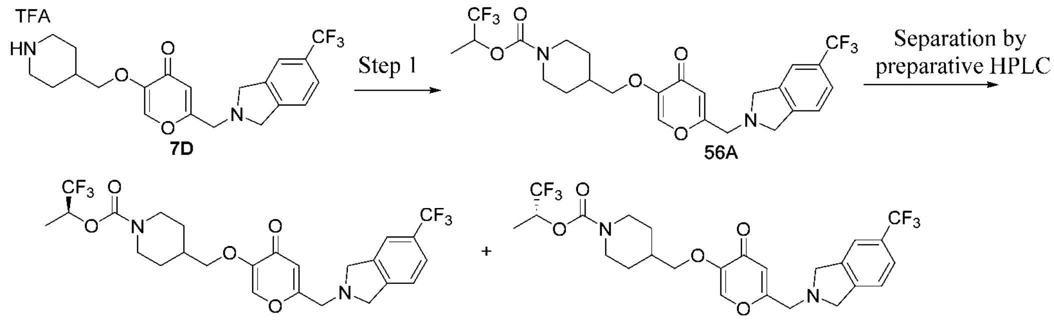


7D

56A

Compound 56

Compound 57

Step 1:

1,1,1-trifluoropropan-2-ol (223 mg, 1.96 mmol) and N,N'-carbonyldiimidazole (238 mg, 1.47 mmol) were dissolved in tetrahydrofuran (15 mL), and the mixture was stirred at room temperature for 30 min. Subsequently, **7D** (400 mg, 0.98 mmol) and triethylamine (397 mg, 3.92 mmol) were added, and the mixture was reacted at 70°C under nitrogen atmosphere overnight. After the reaction was completed, the reaction mixture was cooled to room temperature, water (30 mL) was added to the reaction solution, and the resulting mixture was extracted with ethyl acetate (15 mL×3). The organic layers were combined, washed with saturated sodium chloride aqueous solution (25 mL), dried over anhydrous sodium sulphate, filtered, and concentrated. The residue was separated and purified by silica gel column chromatography (dichloromethane: methanol (v/v) = 25:1) to obtain **56A** (120 mg, yield: 22%).

**56A** was separated and purified by preparative HPLC to obtain **compound 56** (36 mg, retention time: 3.940 min) and **compound 57** (32 mg, retention time: 3.930 min).

Preparative HPLC separation and purification method: 1. Instrument: waters 2767 preparative liquid chromatographic instrument; chromatographic column: SunFire @ Prep C18 (19 mm x 250 mm). 2. The sample was filtered with a 0.45 μm filter to prepare a sample solution. 3. Preparative chromatography conditions: a. composition of mobile phases A and B: mobile phase A: acetonitrile; and mobile phase B: water (containing 0.1% ammonium acetate); b. gradient elution, mobile phase A: 5% - 50%; c. flow rate: 12 mL/min; d. elution time: 30 min.

HPLC analytical method: 1. Instrument: Shimadzu LC-20AT; 2. chromatographic column: Xtimate C18 4.6×50 mm, 3 μm; 3. mobile phase system: A for 0.05% TFA in H2O; B for ACN; 4. gradient: B 5%-95%; 5. acquisition time: 10 min; 6. flow rate: 1.0 mL/min.

**Compound 56:** $^1$H NMR (400 MHz, $CDCl_3$) δ 7.60 (s, 1H), 7.50-7.47 (m, 1H), 7.25-7.21 (m, 2H), 6.49 (s, 1H), 5.28-5.21 (m, 1H), 4.31-4.13 (m, 2H), 4.04 (s, 4H), 3.77-3.72 (m, 4H), 2.87-2.82 (m, 2H), 2.07 (s, 1H), 1.91-1.87 (m, 2H), 1.41-1.39 (m, 3H), 1.34-1.19 (m, 2H).

LC-MS (ESI): m/z = 549.3 $[M+H]^+$.

**Compound 57:** $^1$H NMR (400 MHz, $CDCl_3$) δ 7.60 (s, 1H), 7.51-7.47 (m, 1H), 7.26-7.22 (m, 2H), 6.49 (s, 1H), 5.28-5.22 (m, 1H), 4.31-4.13 (m, 2H), 4.05 (s, 4H), 3.79-3.70 (m, 4H), 2.88-2.83 (m, 2H), 2.07 (s, 1H), 1.92-1.88 (m, 2H), 1.42-1.39 (m, 3H), 1.34-1.20 (m, 2H).

LC-MS (ESI): m/z = 549.3 $[M+H]^+$.

**Example 58:**

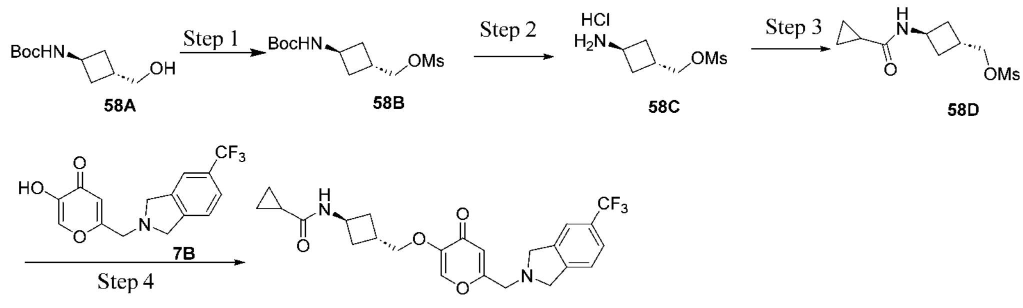


**Compound 58**

Step 1:

Compound **58A** (1.0 g, 4.97 mmol) and triethylamine (1.51 g, 14.91 mmol) were successively added to dichloromethane (30 mL), methanesulphonic acid anhydride (1.73 g, 9.94 mmol) was slowly added in an ice bath, and then the mixture was slowly warmed to room temperature and stirred overnight. To the reaction solution was added saturated sodium bicarbonate solution (30 mL), and the mixture was extracted with dichloromethane. The combined organic phases were washed with saturated sodium chloride aqueous solution, dried over anhydrous sodium sulphate, filtered, and concentrated to obtain the crude compound 58B (1.50 g), and the crude was directly used in the next reaction without purification.

LC-MS (ESI): m/z =280.1 $[M+H]^+$.

Step 2:

Compound 58B (0.5 g, 1.79 mmol) was dissolved in anhydrous methanol (2 mL), and then hydrogen chloride methanol solution (5.0 mL, 4M) was added. After the dropwise addition, the mixture was reacted at room temperature for 3 hours. The reaction solution was concentrated to obtain the crude compound **58C,** which was directly used in the next step.

LC-MS (ESI): m/z =180.1 $[M+H]^+$.

Step 3:

Compound **58C** (0.45 g, 2.51 mmol) and triethylamine (0.76 g, 7.53 mmol) were successively added to dichloromethane (30 mL), cyclopropanecarbonyl chloride (0.34 g, 3.26 mmol) was slowly added in an ice bath, and then the mixture was slowly warmed to room temperature and stirred overnight. To the reaction solution was added saturated sodium bicarbonate solution (30 mL), and the mixture was extracted with dichloromethane. The combined organic phases were washed with saturated sodium chloride aqueous solution, dried over anhydrous sodium sulphate, filtered, and concentrated to obtain the crude compound **58D** (0.4 g), and the crude was directly used in the next reaction without purification.

LC-MS (ESI): m/z =248.1 $[M+H]^+$.

Step 4:

Compound **58D** (0.4 g, 1.62 mmol), intermediate **7B** (0.50 g, 1.62 mmol) and potassium carbonate (0.45 g, 3.24 mmol) were successively added to DMF (20 ml), and the mixture was heated to 75°C, reacted overnight and filtered. The mixture was diluted with water, and extracted with ethyl acetate, and the combined organic phases were washed with saturated sodium chloride aqueous solution, dried over anhydrous sodium sulphate, filtered, and concentrated to obtain a crude. The crude was purified using Biotage Isolera One (20 g silica gel column, eluents: 0-10% dichloromethane/anhydrous methanol) to obtain **compound 58** (100 mg, yield: 13%).

LC-MS (ESI): m/z = 463.2$[M+H]^+$.

$^1$H NMR (400 MHz, DMSO-$d_6$) δ 8.34(d, 1H), 8.15 (s, 1H), 7.63 (s, 1H), 7.56-7.58 (m, 1H), 7.47 (d, 1H), 6.39 (s, 1H), 4.28-4.34 (m, 1H), 4.03 (s, 4H), 3.88 (d, 2H), 3.81 (s, 2H), 2.52-2.56 (m, 1H), 2.06-2.09 (m, 4H), 1.47-1.51 (m, 1H), 0.60-0.65 (m, 4H).

**Example 59:**

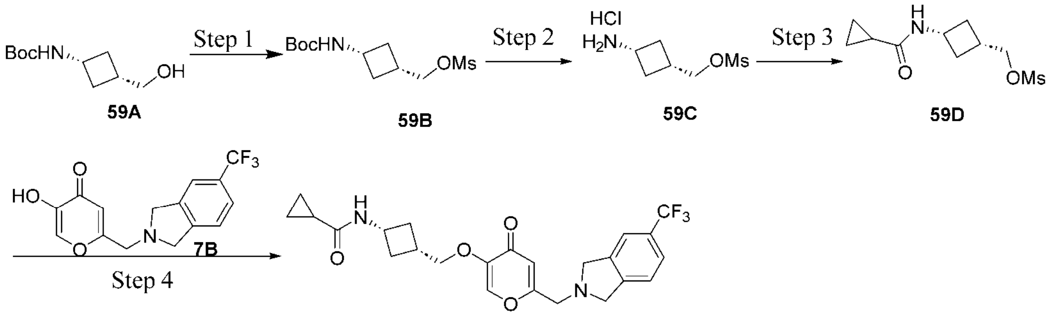


**Compound 59**

Step 1:

Compound **59A** (1.0 g, 4.97 mmol) and triethylamine (1.51 g, 14.91 mmol) were successively added to dichloromethane (30 mL), methanesulphonic acid anhydride (1.73 g, 9.94 mmol) was slowly added in an ice bath, and then the mixture was slowly warmed to room temperature and stirred overnight. To the reaction solution was added saturated sodium bicarbonate solution (30 mL), and the mixture was extracted with dichloromethane. The combined organic phases were washed with saturated sodium chloride aqueous solution, dried over anhydrous sodium sulphate, filtered, and concentrated to obtain the crude compound **59B** (1.50 g), and the crude was directly used in the next reaction without purification.

LC-MS (ESI): m/z =280.1 $[M+H]^+$.

Step 2:

Compound **59B** (0.5 g, 1.79 mmol) was dissolved in anhydrous methanol (2 mL), and then hydrogen chloride methanol solution (5.0 mL, 4M) was added. After the dropwise addition, the mixture was reacted at room temperature for 3 hours. The reaction solution was concentrated to obtain the crude compound **59C,** which was directly used in the next step.

LC-MS (ESI): m/z =180.1 $[M+H]^+$.

Step 3:

Compound **59C** (0.45 g, 2.51 mmol) and triethylamine (0.76 g, 7.53 mmol) were successively added to dichloromethane (30 mL), cyclopropanecarbonyl chloride (0.34 g, 3.26 mmol) was slowly added in an ice bath, and then the mixture was slowly warmed to room temperature and stirred overnight. To the reaction solution was added saturated sodium bicarbonate solution (30 mL), and the mixture was extracted with dichloromethane. The combined organic phases were washed with saturated sodium chloride aqueous solution, dried over anhydrous sodium sulphate, filtered, and concentrated to obtain the crude compound **59D** (0.5 g), and the crude was directly used in the next reaction without purification.

LC-MS (ESI): m/z =248.1 $[M+H]^+$.

Step 4:

Compound **59D** (0.5 g, 2.02 mmol), intermediate **7B** (0.63 g, 2.02 mmol) and potassium carbonate (0.56 g, 4.04 mmol) were successively added to DMF (20 ml), and the mixture was heated to 75°C, reacted overnight and filtered. The mixture was diluted with water, and extracted with ethyl acetate, and the combined organic phases were washed with saturated sodium chloride aqueous solution, dried over anhydrous sodium sulphate, filtered, and concentrated to obtain a crude. The crude was purified using Biotage Isolera One (20 g silica gel column, eluents: 0-10% dichloromethane/anhydrous methanol) to obtain **compound 59** (130 mg, yield: 14%).

LC-MS (ESI): m/z=463.2$[M+H]^+$.

$^1$H NMR (400 MHz, DMSO-$d_6$) δ 8.29(d, 1H), 8.15 (s, 1H), 7.63 (s, 1H), 7.56-7.58 (m, 1H), 7.47 (d, 1H), 6.41 (s, 1H), 4.12-4.17 (m, 1H), 4.03 (s, 4H), 3.79-3.82 (m, 4H), 2.29-2.36 (m, 3H), 1.68-1.74 (m, 2H), 1.50-1.54 (m, 1H), 0.59-0.66 (m, 4H).

**Example 60:**

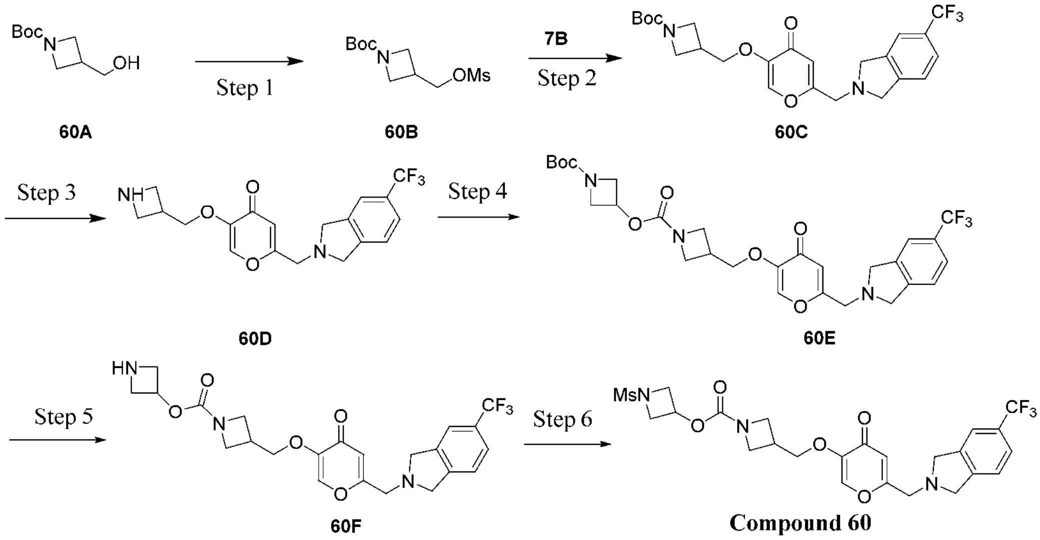


60A 60B 60C 60D 60E 60F Compound 60

Step 1:

Compound **60A** (1.0 g, 5.34 mmol) and triethylamine (1.62 g, 16.02 mmol) were weighed and added to a 100 mL single-neck flask, the mixture was dissolved in dichloromethane (20 mL), and methanesulphonic acid anhydride (1.12 g, 6.43 mmol) was added. After the addition, the resulting mixture was stirred at 25°C for 16 h. After the reaction was completed as monitored by TLC plate spotting (petroleum ether:ethyl acetate = 3:1), water (20 mL) was added and the mixture was stirred for 5 min, extracted with dichloromethane (20 mL), and separated. The resulting organic phase was dried over anhydrous sodium sulphate and concentrated under reduced pressure. The crude was purified by column chromatography (eluents: petroleum ether: ethyl acetate = 3:1) to obtain the target compound **60B** (1.2 g, yield: 90%), which was directly used in the next step.

Step 2:

To a 100 mL single-neck flask was added compound **7B** (0.5 g, 1.61 mmol) and **60B** (0.48 g, 1.93 mmol), and the mixture was dissolved in DMF (20 mL). Potassium carbonate (0.66 g, 4.75 mmol) was added. After the addition, the system was placed under nitrogen protection and stirred at 70°C for 16 h. Upon complete depletion of raw materials as monitored by LCMS, water (20 mL) was added, and the mixture was stirred for 5 min, extracted with ethyl acetate (20 mL), and separated. The organic phase was dried over anhydrous sodium sulphate, and concentrated under reduced pressure, and the crude was purified by column chromatography (eluents: petroleum ether: ethyl acetate =1:1) to obtain the target compound **60C** (0.5 g, yield: 64%).
LCMS m/z =481.1 $[M +1]^+$

Step 3:

To a 100 mL single-neck flask was added compound **60C** (0.5 g, 1.04 mmol), which was dissolved in dichloromethane (6 mL), and trifluoroacetic acid (3 mL) was added. After the addition was completed, the system was subjected to nitrogen protection and stirred at 20°C for 2 h. Upon complete depletion of raw materials as monitored by LCMS, the reaction solution was concentrated under reduced pressure to obtain the target compound **60D** (0.4 g, crude).
LCMS m/z =381.1 $[M +1]^+$

Step 4:

Compound carbonyldiimidazole (0.34 g, 2.1 mmol) and N-Boc-3-hydroxyazetidine (0.36 g, 2.1 mmol) were weighed and added to a 100 mL single-neck flask, and the mixture was dissolved in tetrahydrofuran (10 mL). The resulting mixture was stirred at 25°C for 1 h and then **60D** (0.4 g, 1.05 mmol) and triethylamine (0.32 g, 3.15 mmol) were added. After the addition, the mixture was stirred at 70°C for 16 h. After the reaction was completed as monitored by TLC plate spotting (petroleum ether:ethyl acetate =1:1), water (20 mL) was added, and the mixture was stirred for 5 min, extracted with ethyl acetate (20 mL), and separated. The organic phase was dried over anhydrous sodium sulphate and concentrated under reduced pressure. The crude was purified by column chromatography (eluents: petroleum ether: ethyl acetate = 1:1) to

obtain the target compound **60E** (0.40 g, yield: 66%).
LCMS m/z =524.1 [M -55]$^+$

Step 5:

To a 100 mL single-neck flask was added compound **60E** (0.4 g, 0.69 mmol), which was dissolved in dichloromethane (6 mL), and trifluoroacetic acid (3 mL) was added. After the addition was completed, the system was subjected to nitrogen protection and stirred at 20°C for 4 h. Upon complete depletion of raw materials as monitored by LCMS, the reaction solution was concentrated under reduced pressure to obtain the target compound **60F** (0.4 g, crude).
LCMS m/z =480.1 [M +1]$^+$

Step 6:

Compound **60F** (300 mg, 0.73 mmol) and triethylamine (0.16 g, 1.58 mmol) were weighed and added to a 100 mL single-neck flask. The mixture was dissolved in dichloromethane (6 mL), and at 0°C, methanesulphonyl chloride (89 mg, 0.77 mmol) was added. After the addition, the system was subjected to nitrogen protection and stirred at 0°C for 2 h. Upon complete depletion of raw materials as monitored by LCMS, water (20 mL) was added, and the mixture was stirred for 5 min, extracted with dichloromethane (20 mL), and separated. The organic phase was dried over anhydrous sodium sulphate, and concentrated under reduced pressure. The crude was purified by column chromatography (eluents: petroleum ether: ethyl acetate = 1:1) to obtain the target crude compound, which was subjected to preparative HPLC to obtain compound **60** (35 mg, yield: 12%).
Preparation method: instrument: waters 2767 preparative liquid chromatographic instrument; chromatography column: SunFire@ Prep C18 (19 mm × 250 mm); the sample was dissolved in DMF and filtered with a 0.45 μm filter to prepare a sample liquid; Preparative chromatography conditions: a. composition of mobile phases A and B: mobile phase A: acetonitrile, and mobile phase B: water (containing 5 mM aqueous ammonia); b. gradient elution, mobile phase A: 35%-70%; c. flow rate: 15 mL/min; d. elution time: 20 min.

LCMS m/z =558.2 [M +1]$^+$
$^1$H NMR (400 MHz, DMSO) δ8.20 (s, 1H), 7.63 (s, 1H), 7.57 (d, 1H), 7.47 (d, 1H),6.42 (s, 1H), 5.00-5.05 (m,2H), 4.12-4.16 (m, 3H), 4.00-4.03 (m, 7H), 3.82-3.86 (m,5H), 3.04 (s, 3H), 2.95-3.01 (m,1H).

**Example 61:**

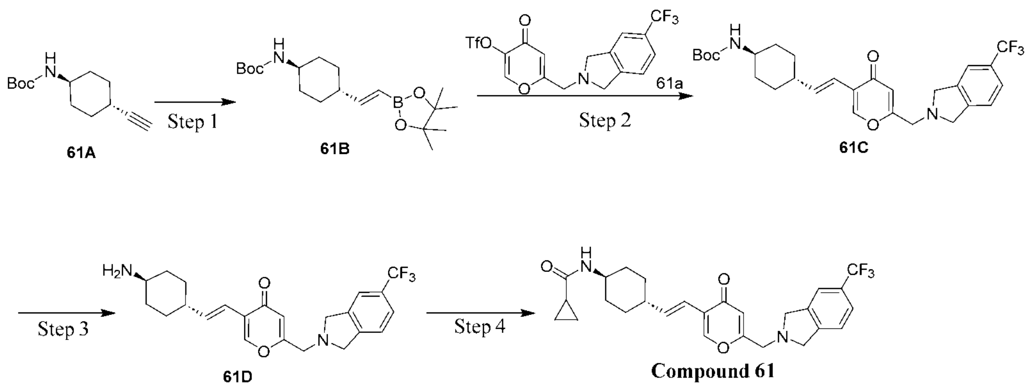

61A 61B 61C 61D Compound 61

Step 1:

61A (2.00 g, 8.96 mmol) was dissolved in dry toluene (60 mL), pinacolborane (2.00 g, 15.61 mmol) was added dropwise, and bis(cyclopentadienyl)zirconium chloride hydride (0.46 g, 1.79 mmol) was added. After the addition, the system was subjected to nitrogen replacement, and stirred and reacted at 65°C for 18 h. After the reaction was completed as monitored by TLC (petroleum ether: ethyl acetate =2:1 (v/v)), the reaction solution was concentrated, and the crude was directly used in the next step.

Step 2:

Compound 61B (3.14 g, 8.94 mmol) and 61a (3.96 g, 8.94 mmol, prepared using 7B as the raw material with reference to the synthesis method of 16B) were dissolved in 1,4-dioxane (60 mL), and then [1,1'-bis(diphenylphosphino) ferrocene]palladium dichloride (II) (0.65 g, 0.89 mmol), potassium carbonate (4.32 g, 31.29 mmol) and water (12 mL) were successively added. After the addition, the system was subjected to nitrogen replacement three times, and stirred and reacted at 85°C for 18 h. After the completion of the reaction, water (50 mL) was added, the resulting mixture was extracted with ethyl acetate (100 mL×3), and the organic phases were combined, washed with saturated sodium chloride aqueous solution (40 mL×1), dried over anhydrous sodium sulphate and filtered. The filtrate was concentrated under reduced pressure and purified by column chromatography (petroleum ether:ethyl acetate =2:1 (v/v)) to obtain compound 61C (1.4 g, yield: 30%).
LC-MS (ESI): *m/z* = 463.10 $[M-56+H]^+$.

Step 3:

Compound 61C (800 mg, 1.54 mmol) was dissolved in dichloromethane (20 mL), trifluoroacetic acid (5 mL) was added, and the mixture was reacted at room temperature for 2 h. After the reaction was completed as monitored by TLC (petroleum ether: ethyl acetate =1:1 (v/v)), the reaction solution was concentrated to obtain compound 61D (640 mg, crude), which was directly used in the next step without purification.
LC-MS (ESI): *m/z* = 419.10 $[M+H]^+$.

Step 4:

Compound 61D (640 mg, 1.53 mmol) was dissolved in dichloromethane (15 mL), and in an ice-water bath, triethylamine (542 mg, 5.35 mmol) and cyclopropanecarbonyl chloride (240 mg, 2.30 mmol) were successively added. After the dropwise addition was completed, the mixture was reacted for another 2 h in an ice-water bath. After the reaction was completed as monitored by TLC (dichloromethane: methanol =20:1 (v/v)), the reaction solution was concentrated, and the crude was purified by column chromatography (eluents: dichloromethane: methanol=65:1 (v/v)) to obtain the target compound 61 (87 mg, yield: 12%).

LCMS *m/z* = 487.7 $[M+1]^+$
$^1$H NMR (400 MHZ, DMSO-$d_6$) δ 8.30 (s, 1H), 7.89 (d, 1H), 7.62 (s, 1H), 7.57 (d, 1H), 7.47 (d, 1H), 6.63 (dd, 1H), 6.35 (s, 1H), 6.11 (d, 1H), 4.03 (s, 4H), 3.81 (s, 2H), 3.49 (s, 1H), 2.01 (dd, 1H), 1.79 (dd, 4H), 1.53 - 1.46 (m, 1H), 1.26 - 1.15 (m, 4H), 0.68 - 0.55 (m, 4H).

**Example 62:**

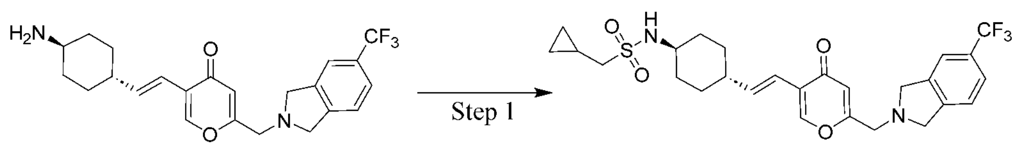

61D
Compound 62

Step 1:

Compound 61D (485 mg, 1.16 mmol) was dissolved in dichloromethane (15 mL), and in an ice-water bath, triethylamine (411 mg, 4.06 mmol) and cyclopropylmethanesulphonyl chloride (269 mg, 1.74 mmol) were successively added. After the dropwise addition was completed, the mixture was reacted for another 7 h in an ice-water bath. After the reaction was completed as monitored by TLC (dichloromethane: methanol =20:1 (v/v)), the reaction solution was concentrated, and the crude was purified by column chromatography (eluents: dichloromethane: methanol=65:1 (v/v)) to obtain the target compound 67 (68 mg, yield: 11%).

LCMS *m/z* = 537.8 $[M+1]^+$
$^1$H NMR (400 MHZ, DMSO-$d_6$) δ 8.30 (s, 1H), 7.62 (s, 1H), 7.57 (d, 1H), 7.47 (d, 1H), 6.99 (d, 1H), 6.62 (dd, 1H), 6.35 (s, 1H), 6.09 (d, 1H), 4.03 (s, 4H), 3.81 (s, 2H), 3.07 (s, 1H), 2.92 (d, 2H), 1.92 (d, 3H), 1.74 (d, 2H), 1.35 - 1.11 (m, 4H), 1.00

(qd, 1H), 0.59 - 0.52 (m, 2H), 0.37 - 0.29 (m, 2H).

Example **63**

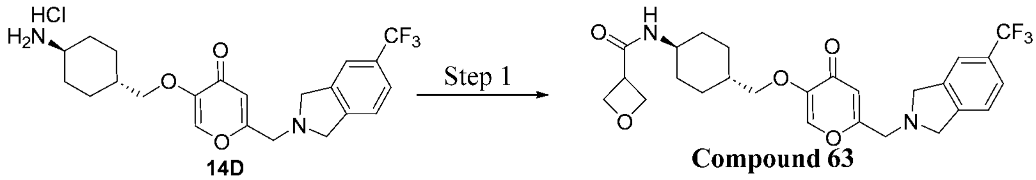


**14D** **Compound 63**

Step 1:

With reference to the synthetic step of step 4 in **example 9, compound 63** (46 mg, yield: 23%) was synthesized using **14D** (200 mg, 0.47 mmol) and oxetane-3-carboxylic acid (58 mg, 0.56 mmol) as the raw materials.
$^1$H NMR (400 MHz, $CDCl_3$) δ 7.58 (s, 1H), 7.51-7.48 (m, 1H), 7.46 (s, 1H), 7.32-7.29 (m, 1H), 6.48 (s, 1H), 5.68-5.65 (m, 1H), 4.87-4.83 (m, 2H), 4.79-4.75 (m, 2H), 4.09 (s, 4H), 3.83-3.75 (m, 3H), 3.72-3.63 (m, 3H), 2.08-1.96 (m, 4H), 1.88-1.82 (m, 1H), 1.26-1.08 (m, 4H).
LC-MS (ESI): m/z = 507.8 $[M+H]^+$.

**Example 64**

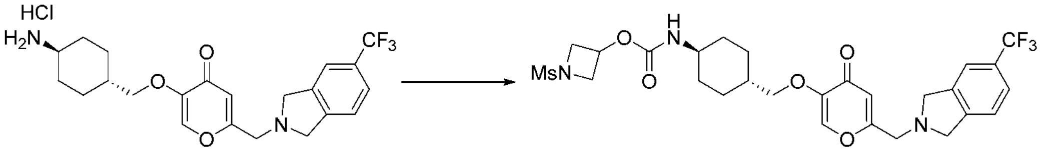

**14D** **Compound 64**

Step 1:

With reference to step 3 in compound example 17, compound **64** was synthesized using compound **14D** and compound **17C** as the raw materials.
LC-MS (ESI): m/z =600.6 $[M+H]^+$.
$^1$H NMR (400 MHz, $CDCl_3$): δ 7.57 (s, 1H), 7.50 (d, 1H), δ 7.46 (s, 1H), 7.31 (s, 1H), 6.48 (s, 1H), 5.17-5.11 (m, 1H), δ 4.68 (s, 1H), 4.16 (t, 2H), 4.10 (s, 4H), 3.98-3.94 (m, 2H), 3.79 (s, 2H), 3.67 (d, 2H), 3.48-3.40 (m, 1H), 2.89 (s, 3H), 2.08-1.97 (m, 4H), 1.89-1.80 (m, 1H), 1.23-1.10 (m, 4H).

**Example 65:**

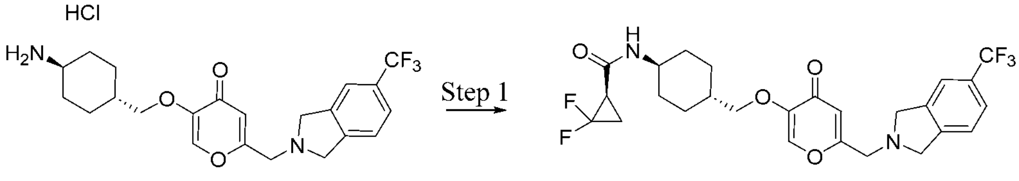


**14D** **Compound 65**

Step 1:

With reference to step 1 in example 55, **compound 65** (61 mg, yield: 19.6%) was obtained using (1R)-2,2-difluorocyclopropane-1-carboxylic acid (86 mg, 0.71 mmol) and **compound 14D** (250 mg, 0.54 mmol) as the raw materials.
LC-MS (ESI): *m/z* = 527.2 [M+H]$^+$.
$^1$H NMR (400 MHz, DMSO-$d_6$) δ 8.22 - 8.17 (m, 1H), 8.12 (s, 1H), 7.64 - 7.62 (m, 1H), 7.59 - 7.55 (m, 1H), 7.49 - 7.44 (m, 1H), 6.39 (s, 1H), 4.02 (s, 4H), 3.80 (s, 2H), 3.66 - 3.61 (m, 2H), 3.57 - 3.48 (m, 1H), 1.90 - 1.77 (m, 6H), 1.73 - 1.61 (m, 1H), 1.27 - 1.12 (m, 3H), 1.12 - 1.00 (m, 2H).

**Example 66**

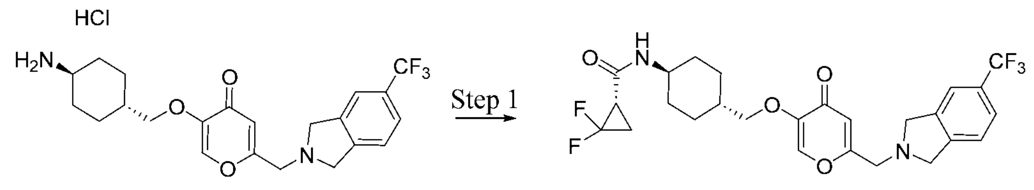


14D Compound 66

Step 1:

With reference to step 1 in example 55, **compound 66** (53 mg, yield: 17.1%) was obtained using (1S)-2,2-difluorocyclopropane-1-carboxylic acid (86 mg, 0.71 mmol) and **compound 14D** (250 mg, 0.54 mmol) as the raw materials.
LC-MS (ESI): m/z = 527.2 [M+H]$^+$.
$^1$H NMR (400 MHz, DMSO-$d_6$) δ 8.21 - 8.16 (m, 1H), 8.12 (s, 1H), 7.65 - 7.62 (m, 1H), 7.59 - 7.56 (m, 1H), 7.50 - 7.45 (m, 1H), 6.39 (s, 1H), 4.03 (s, 4H), 3.81 (s, 2H), 3.66 - 3.61 (m, 2H), 3.58 - 3.48 (m, 1H), 1.92 - 1.77 (m, 6H), 1.72 - 1.63 (m, 1H), 1.27 - 1.12 (m, 3H), 1.12 - 1.01 (m, 2H).

**Example 67:**

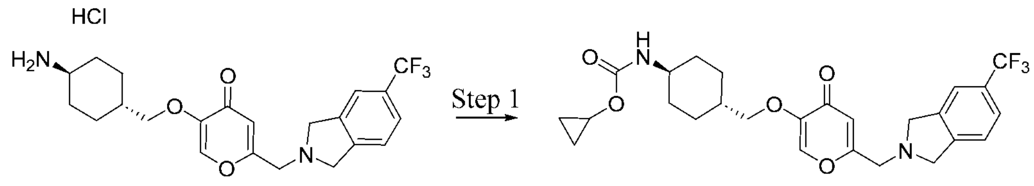


14D Compound 67

Step 1:

With reference to step 4 in example 19, **compound 67** (135 mg, yield: 37.5%) was obtained using cyclopropanol (83 mg, 1.42 mmol) and **compound 14D** (300 mg, 0.65 mmol) as the raw materials.
LC-MS (ESI): m/z = 507.1 [M+H]$^+$.
$^1$H NMR (400 MHz, DMSO-$d_6$) δ 8.11 (s, 1H), 7.64 - 7.61 (m, 1H), 7.59 - 7.55 (m, 1H), 7.49 - 7.45 (m, 1H), 7.06 - 6.99 (m, 1H), 6.38 (s, 1H), 4.03 (s, 4H), 3.94 - 3.88 (m, 1H), 3.80 (s, 2H), 3.65 - 3.59 (m, 2H), 3.25 - 3.17 (m, 1H), 1.86 - 1.76 (m, 4H), 1.67 - 1.56 (m, 1H), 1.22 - 0.98 (m, 4H), 0.63 - 0.57 (m, 2H), 0.57 - 0.50 (m, 2H).

**Example 68**

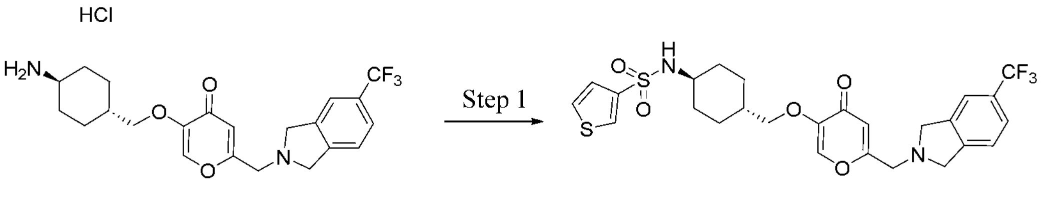


14D

Compound 68

Step 1:

With reference to the operations in **example 14** (step 4), **compound 68** (57 mg, yield: 25%) was obtained using **14D** (170 mg, 0.37 mmol) and 3-thiophenesulphonyl chloride (88 mg, 0.48 mmol) as the raw materials.

LC-MS (ESI): m/z = 569.1 $[M+H]^+$.

$^1H$ NMR (400 MHz, DMSO-$d_6$) δ 8.14 - 8.11 (m, 1H), 8.09 (s, 1H), 7.75 - 7.71 (m, 1H), 7.63 - 7.61 (m, 1H), 7.60 - 7.53 (m, 2H), 7.48 - 7.45 (m, 1H), 7.35 - 7.32 (m, 1H), 6.37 (s, 1H), 4.02 (s, 4H), 3.79 (s, 2H), 3.60 - 3.56 (m, 2H), 2.99 - 2.88 (m, 1H), 1.77 - 1.64 (m, 4H), 1.62 - 1.52 (m, 1H), 1.24 - 1.14 (m, 2H), 1.02 - 0.91 (m, 2H).

**Examples 69 and 70:**

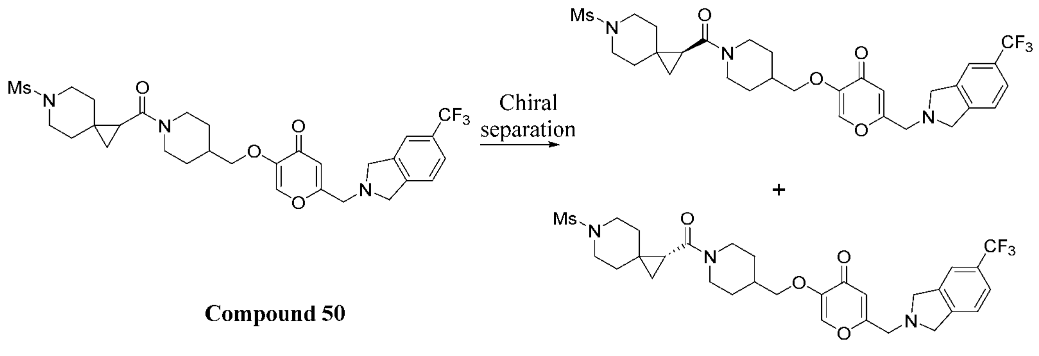


Compound 50

Compound 69 & Compound 70

Step 1:

**Isomer A** and **Isomer B** were prepared by SFC using compound **50** as the raw material.

SFC preparation method: instrument: Waters 150 Prep-SFC E preparative liquid phase: chromatographic column: Chiralcel AD column. The sample was dissolved in DMF and filtered with a 0.45 μm filter to prepare a sample liquid. Preparative chromatography conditions: composition of mobile phases A and B: mobile phase A: $CO_2$; mobile phase B: 0.1% $NH_3$•$H_2O$ in MeOH and ACN, gradient elution, mobile phase B: 70%, flow rate: 100 mL/min. Column pressure: 100 bar, column temperature: 25°C, absorption wavelength: 220 nm, cycle time: 3.0 min. **Isomer A** retention time: 1.247 min, assigned as compound 69. **Isomer B** retention time: 1.741 min, assigned as compound 70.

**Isomer A:**

LC-MS (ESI): m/z= 624.3 $[M+H]^+$.

$^1H$ NMR (400 MHz, $CDCl_3$): δ 7.60 (s, 1H), 7.51 - 7.49 (m, 1H), 7.46 (s, 1H), 7.32 - 7.30 (m, 1H), 6.49 (s, 1H), 4.69 - 4.61 (m, 1H), 4.12 (s, 5H), 3.81 - 3.66 (m, 4H), 3.60 - 3.37 (m, 2H), 3.22 - 2.93 (m, 4H), 2.80 (s, 3H), 2.67 - 2.62 (m, 1H), 2.15 - 1.68 (m, 7H), 1.43 - 1.15 (m, 4H).

**Isomer B:**

LC-MS (ESI): m/z= 624.7 $[M+H]^+$.

$^1H$ NMR (400 MHz, $CDCl_3$): δ 7.61 (s, 1H), 7.51 - 7.49 (m, 1H), 7.46 (s, 1H), 7.32 - 7.30 (m, 1H), 6.49 (s, 1H), 4.68 - 4.60 (m, 1H), 4.12 (s, 5H), 3.81 - 3.66 (m, 4H), 3.60 - 3.37 (m, 2H), 3.21 - 2.93 (m, 4H), 2.80 (s, 3H), 2.68 - 2.62 (m, 1H), 2.15 -

1.68 (m, 7H), 1.43 - 1.14 (m, 4H).

**Example 71**

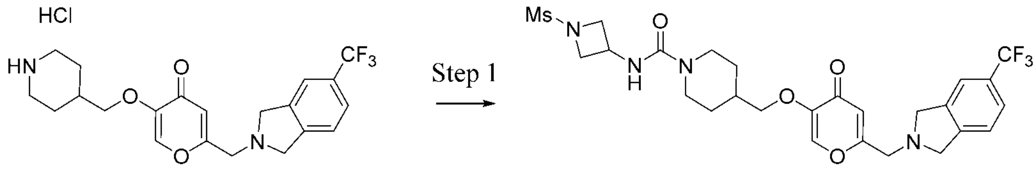


14D

Compound 71

Step 1:

With reference to the operations in **example 14** (step 4), **compound 71** (140 mg, yield: 48.8%) was obtained using **14D** (200 mg, 0.49 mmol) and 3-thiophenesulphonyl chloride (147 mg, 0.98 mmol) as the raw materials.

LC-MS (ESI): m/z = 585.2 $[M+H]^+$.

$^1$H NMR (400 MHz, DMSO-$d_6$) δ 8.13 (s, 1H), 7.65 - 7.61 (m, 1H), 7.60 - 7.54 (m, 1H), 7.49 - 7.45 (m, 1H), 6.98 - 6.94 (m, 1H), 6.39 (s, 1H), 4.42 - 4.33 (m, 1H), 4.05 - 3.93 (m, 8H), 3.83 - 3.76 (m, 4H), 3.72 - 3.67 (m, 2H), 2.99 (s, 3H), 2.77 - 2.65 (m, 2H), 1.96 - 1.84 (m, 1H), 1.75 - 1.66 (m, 2H), 1.18 - 1.05 (m, 2H).

**Example 72**

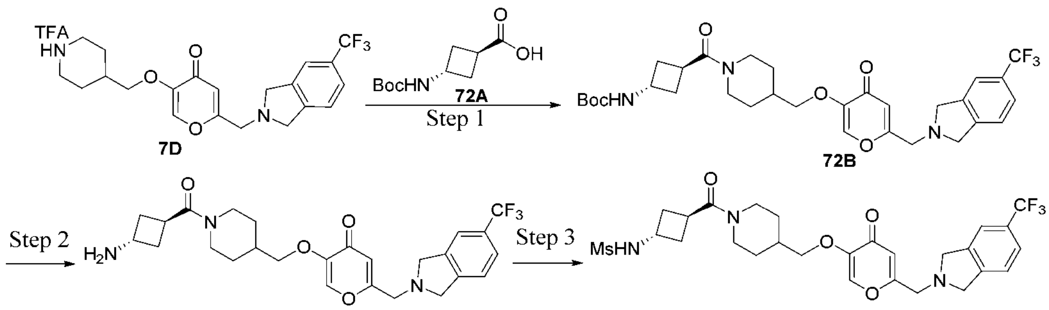


7D, 72B, 72C, Compound 72

Step 1:

With reference to the synthetic step of step 1 in **example 50, 72B** (161 mg, yield: 72%) was synthesized using **7D** (150 mg, 0.37 mmol) and **72A** (80 mg, 0.37 mmol) as the raw materials.

Step 2:

With reference to the synthetic step of step 2 in **example 50, 72C** (113 mg, yield: 83%) was synthesized using **72B** (161 mg, 0.27 mmol) as the raw material.

LC-MS (ESI): m/z = 506.2 $[M+H]^+$.

Step 3:

With reference to the synthetic step of step 3 in **example 50, compound 72** (34 mg, yield: 26%) was synthesized using **72C** (113 mg, 0.22 mmol) as the raw material.

$^1$H NMR (400 MHz, $CDCl_3$) δ 7.61 (s, 1H), 7.51-7.48 (m, 1H), 7.46 (s, 1H), 7.32-7.29 (m, 1H), 6.49 (s, 1H), 5.08-5.05 (m, 1H), 4.67-4.63 (m, 1H), 4.09-4.02 (m, 5H), 3.81-3.62 (m, 5H), 3.24-3.16 (m, 1H), 3.03-2.92 (m, 4H), 2.75-2.68 (m, 2H), 2.67-2.58 (m, 1H), 2.33-2.22 (m, 2H), 2.17-2.07 (m, 1H), 1.98-1.94 (m, 1H), 1.89-1.84 (m, 1H), 1.29-1.15 (m, 2H).

LC-MS (ESI): m/z = 584.7 $[M+H]^+$.

**Example 73**

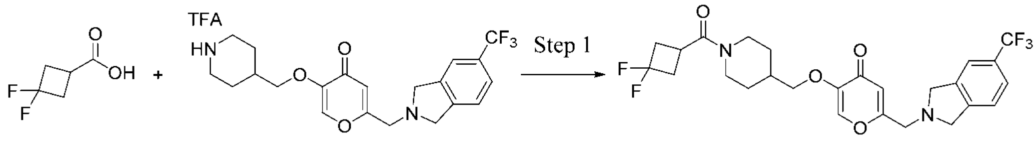


73A 7D Compound 73

Step 1:

With reference to the synthetic step of step 1 in **example 50, compound 73** was synthesized using **73A** and **7D** as the raw materials.

$^1H$ NMR (400 MHz, $CDCl_3$) δ 7.60 (s, 1H), 7.51-7.49 (m, 1H), 7.46 (s, 1H), 7.32 -7.30 (m, 1H), 6.49 (s, 1H), 4.66-4.63 (m, 1H), 4.11 (s, 4H), 3.80-3.68 (m, 5H), 3.08 -3.01 (m, 2H), 2.97-2.88 (m, 2H), 2.76-2.61 (m, 3H), 2.18-2.11 (m, 1H), 2.02-1.99 (m, 1H), 1.88-1.85 (m, 1H), 1.29-1.15 (m, 2H).

LC-MS (ESI): m/z = 527.2 $[M+H]^+$.

**Example 74**

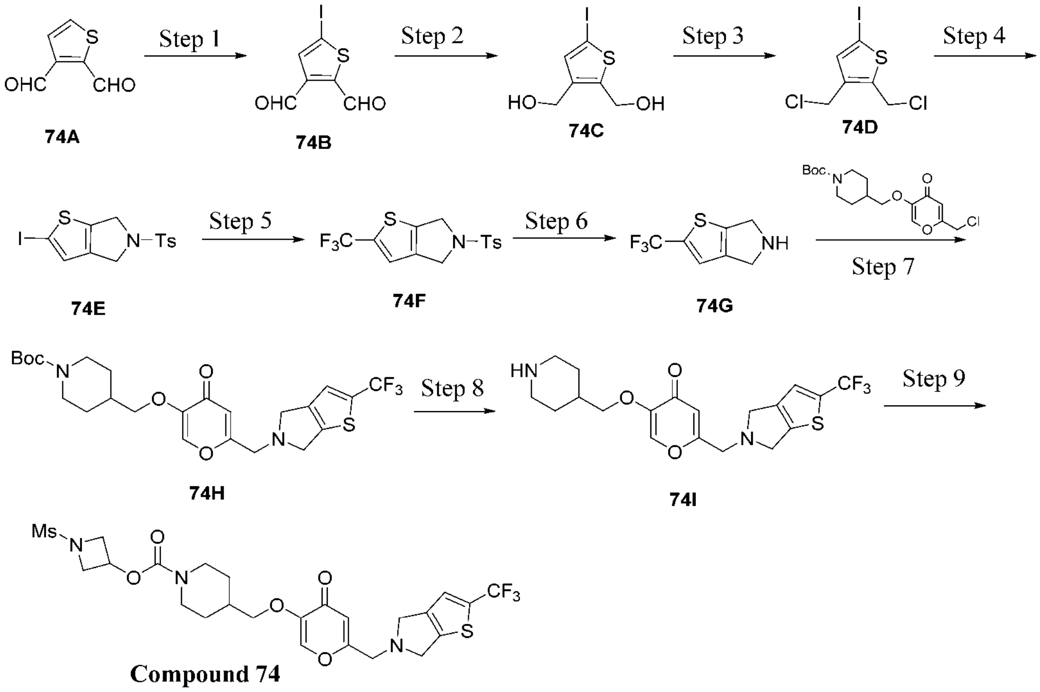


74A 74B 74C 74D 74E 74F 74G 74H 74I Compound 74

Step 1:

With reference to the document (Journal of Materials Chemistry C: Materials for Optical and Electronic Devices, Volume: 10, Issue: 42, Pages: 15861-15871, 2022), **74B** (5.1 g, yield: 76.8%) was synthesized using **74A** (3.5 g, 24.93 mmol) as the raw material.

Step 2:

At room temperature, **74B** (5.1 g, 19.17 mmol) was dissolved in methanol (50 mL), and in an ice bath, sodium borohydride (1.45 g, 38.34 mmol) was added in portions into the mixture. After the addition, the mixture was reacted at room temperature for 3 hours. Upon complete depletion of raw materials as monitored by TLC, the reaction was stopped. The reaction solution was concentrated, the residue was redissolved in EA (50 mL) and the organic phase was washed with water (50 mL×2) and saturated sodium chloride aqueous solution (50 mL×2), dried over anhydrous sodium sulphate, filtered, and concentrated. The residue was separated and purified by silica gel column chromatography (DCM:MeOH=10:1) to obtain **74C** (4.5 g, yield: 86.9%).

Step 3:

At room temperature, **74C** (4.5 g, 16.66 mmol) was dissolved in dry DCM (50 mL), and in an ice bath, thionyl chloride (3.96 g, 33.36 mmol) was added dropwise to the mixture. After the dropwise addition, the mixture was reacted at room temperature for 2 hours. Upon complete depletion of raw materials as monitored by TLC, the reaction was stopped. The reaction solution was concentrated, the residue was redissolved in DCM (50 mL) and the organic phase was washed with water (50 mL×2) and saturated sodium chloride aqueous solution (50 mL×2), dried over anhydrous sodium sulphate, filtered, and concentrated. The residue was separated and purified by silica gel column chromatography (PE:EA=10:1) to obtain **74D** (3.5 g, yield: 68.4%).

Step 4:

At room temperature, **74D** (3.5 g, 11.40 mmol) was dissolved in dry DMF (20 mL), and in an ice bath, sodium hydride (1.0 g, 24.97 mmol) was added, and the mixture was stirred evenly, and then p-toluenesulphonamide (1.95 g, 11.4 mmol) was added. The mixture was reacted at 0°C for 30 min, warmed to room temperature, and reacted for another 2 hours. Upon complete depletion of raw materials as monitored by TLC, the reaction was stopped. To the reaction solution was added EA (40 mL), and the organic phase was washed with water (50 mL×2) and saturated sodium chloride aqueous solution (50 mL×2), dried over anhydrous sodium sulphate, filtered and concentrated. The residue was separated and purified by silica gel column chromatography (PE:EA=4:1) to obtain **74E** (2.5 g, yield: 54.1%).

LC-MS (ESI): m/z = 405.9 $[M+H]^+$.

Step 5:

At room temperature, **74E** (2.5 g, 6.17 mmol) was dissolved in dry DMF (20 mL), copper iodide (1.53 g, 8.03 mmol) and HMPA (5.53 g, 30.85 mmol) were added, and the mixture was stirred at room temperature under nitrogen atmosphere for 10 min. Then methyl fluorosulphonyldifluoroacetate (5.93 g, 30.81 mmol) was added, and the mixture was reacted at 100°C under nitrogen atmosphere for 3 hours. Upon complete depletion of raw materials as monitored by TLC, the reaction was stopped. The reaction mixture was cooled to room temperature. To the reaction solution was added EA (30 mL), and the organic phase was washed with water (60 mL×2) and saturated sodium chloride aqueous solution (60 mL×2), dried over anhydrous sodium sulphate, filtered and concentrated. The residue was separated and purified by silica gel column chromatography (PE:EA=4:1) to obtain **74F** (1.7 g, yield: 79.3%).

LC-MS (ESI): m/z = 348.3 $[M+H]^+$.

Step 6:

At room temperature, **74F** (1.7 g, 4.89 mmol), phenol (0.92 g, 9.78 mmol), and propanoic acid (1.49 g, 1.5 mL, 20.05 mmol) were successively added to a round bottom flask, 48% aqueous hydrobromic acid solution (20 mL) was then added, and under nitrogen atmosphere, the mixture was reacted at 100°C for 16 hours. Upon complete depletion of raw materials as monitored by TLC, the reaction was stopped. The reaction mixture was cooled to room temperature, and to the reaction solution was added water (10 mL). The aqueous phase was washed with diethyl ether (30 mL×3), and the aqueous layer was concentrated to obtain product **74G** (1.2 g, yield: 89.9%) without purification.

LC-MS (ESI): m/z = 194.1 $[M+H]^+$.

Step 7:

With reference to the synthesis method of step 1 in **example 1,** compound **74H** (100 mg, yield: 55.5%) was synthesized using **74G** (74.38 mg, 0.39 mmol) as the raw material.

LC-MS (ESI): m/z = 415.1 $[M+H-100]^+$.

Step 8:

With reference to the synthesis method of step 3 in **example 1,** compound **74I** (75 mg, yield: 95.25%) was synthesized using **74H** (100 mg, 0.19 mmol) as the raw material.

LC-MS (ESI): m/z = 415.2 [M+H]$^+$.

Step 9:

With reference to the synthesis method of step 8 in **example 20,** the target compound **74** (20 mg, yield: 18.8%) was synthesized using **74I** (75 mg, 0.18 mmol) as the raw material.

LC-MS (ESI): m/z = 592.0 [M+H]$^+$.

$^1$H NMR (400 MHz, $CDCl_3$) δ 7.59 (s, 1H), 7.18 (s, 1H), 6.47 (s, 1H), 5.16-5.10 (m, 1H), 4.21-4.15 (m, 6H), 4.02-3.98 (m, 4H), 3.83 (s, 2H), 3.74-3.72 (d, 2H), 2.89 (s, 3H), 2.84-2.75 (m, 2H), 2.12-2.00 (d, 1H), 1.90-1.87 (d, 2H), 1.29-1.28 (d, 2H).

**Example 75**

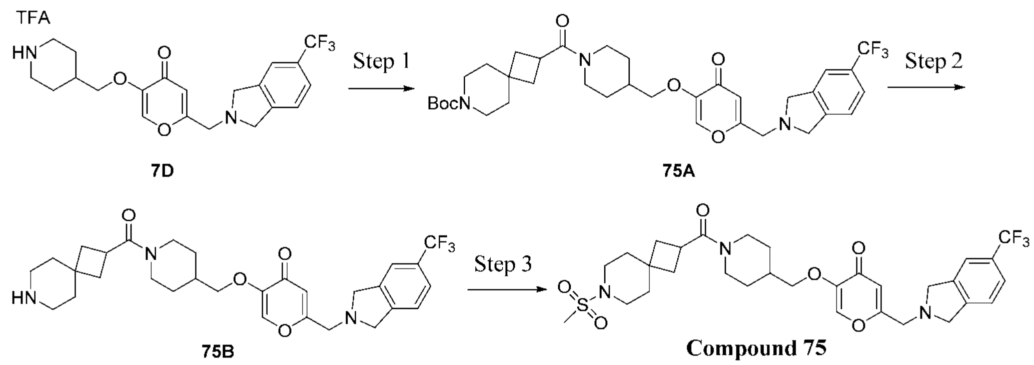


7D 75A 75B Compound 75

Step 1:

With reference to the synthetic step of step 1 in **example 50, compound 75A** was synthesized using **7D** and 7-(tert-butoxycarbonyl)-7-azaspiro[3.5]nonane-2-carboxylic acid as the raw materials.

LC-MS (ESI): m/z = 660.9 [M+H]$^+$.

Step 2:

With reference to the synthetic step of step 2 in **example 50, compound 75B** was synthesized using **75A** as the raw material.

LC-MS (ESI): m/z =560.7 [M+H]$^+$.

Step 3:

With reference to the synthetic step of step 3 in **example 50, compound 75** was synthesized using **75B** as the raw material.

$^1$H NMR (400 MHz, $CDCl_3$) δ 7.59 (s, 1H), 7.49 (d, *J* = 8.0 Hz, 1H), 7.45 (s, 1H), 7.31 (d, *J* = 8.0 Hz, 1H), 6.48 (s, 1H), 4.63 (d, *J* = 13.6 Hz, 1H), 4.09 (s, 4H), 3.79 (s, 2H), 3.77 - 3.67 (m, 3H), 3.24 - 3.15 (m, 3H), 3.12 - 3.09 (m, 2H), 2.97 (td, *J* = 13.2, 2.4 Hz, 1H), 2.75 (s, 3H), 2.61 (td, *J* = 12.8, 2.8 Hz, 1H), 2.17 - 2.08 (m, 3H), 2.05 - 1.96 (m, 3H), 1.84 (d, *J* = 12.8 Hz, 1H), 1.77 - 1.75 (m, 2H), 1.69 - 1.66 (m, 2H), 1.24 - 1.14 (m, 2H).

LC-MS (ESI): m/z = 638.3 [M+H]$^+$.

**Example 76**

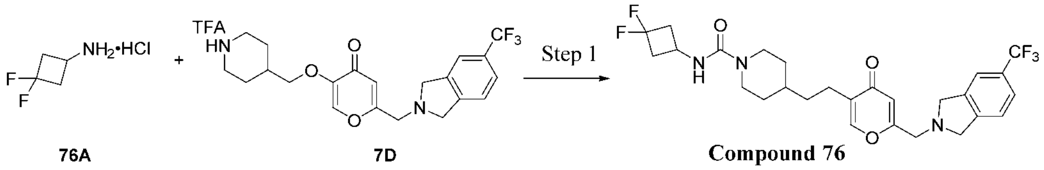


76A 7D Compound 76

Step 1:

With reference to the synthetic step of step 1 in **example 71, compound 76** was synthesized using **76A** and **7D** as the raw materials.

LC-MS (ESI): m/z = 542.2 $[M+H]^+$.

$^1H$ NMR (400 MHz, $CDCl_3$) δ 7.59 (s, 1H), 7.51-7.48 (m, 1H), 7.46 (s, 1H), 7.32 -7.30 (m, 1H), 6.48 (s, 1H), 4.66-4.65 (m, 1H), 4.18-4.13 (m, 1H), 4.10 (s, 4H), 3.98-3.95 (m, 2H), 3.79 (s, 2H), 3.73 -3.71 (m, 2H), 3.04 -2.93 (m, 2H), 2.85-2.78 (m, 2H), 2.50-2.37 (m, 2H), 2.08-2.04 (m, 1H), 1.91-1.88 (m, 2H), 1.32-1.22 (m, 4H).

**Example 77**

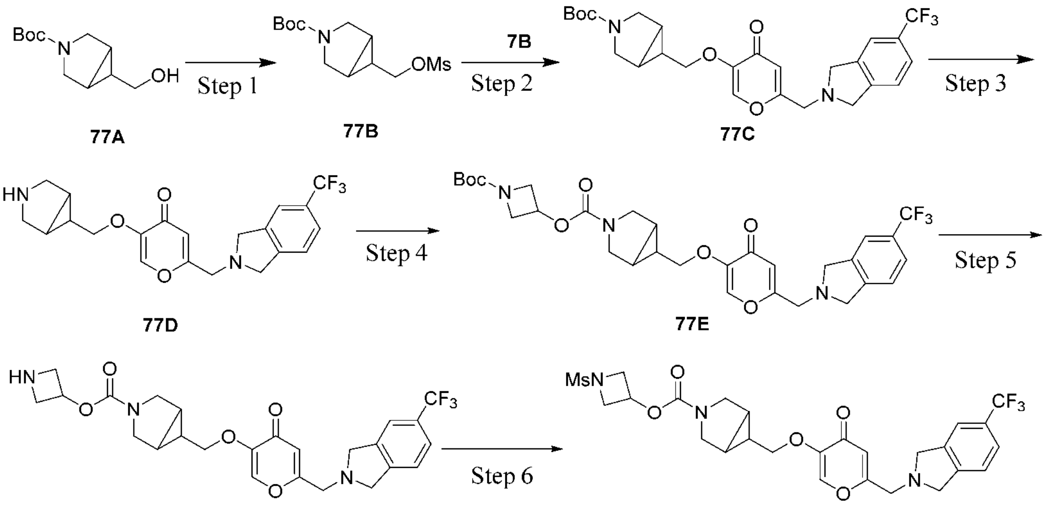


77A 77B 77C 77D 77E 77F Compound 77

Step 1:

Compound **77A** (1.20 g, 5.63 mmol) and triethylamine (1.71 g, 16.89 mmol) were weighed and added to a 100 mL single-neck flask, the mixture was dissolved in dichloromethane (20 mL), and methanesulphonic acid anhydride (1.18 g, 6.76 mmol) was added. After the addition, the resulting mixture was stirred at 25°C for 16 h. After the reaction was completed as monitored by TLC plate spotting (petroleum ether:ethyl acetate =3:1), water (20 mL) was added and the mixture was stirred for 5 min, extracted with dichloromethane (20 mL), and separated. The resulting organic phase was dried over anhydrous sodium sulphate and concentrated under reduced pressure. The crude was purified by column chromatography (eluents: petroleum ether: ethyl acetate =3:1) to obtain the target compound **77B** (1.5 g, yield: 91%), which was directly used in the next step.

Step 2:

To a 100 mL single-neck flask was added compound **7B** (1.50 g, 4.81 mmol) and **77B** (1.40 g, 4.81 mmol), and the mixture was dissolved in DMF (20 mL). Potassium carbonate (1.99 g, 14.43 mmol) was added. After the addition, the system was placed under nitrogen protection and stirred at 70°C for 16 h. Upon complete depletion of raw materials as monitored by LCMS, water (40mL) was added, and the mixture was stirred for 5 min, extracted with ethyl acetate (40mL), and separated. The organic phase was dried over anhydrous sodium sulphate, and concentrated under reduced pressure, and the crude was purified by column chromatography (eluents: petroleum ether: ethyl acetate =1:1) to obtain the target compound **77C** (0.90 g, yield: 37%).

LCMS m/z =451.2 [M - 55]$^+$

Step 3:

To a 100 mL single-neck flask was added compound **77C** (1.0 g, 1.97 mmol), which was dissolved in dichloromethane (10 mL), and trifluoroacetic acid (10 mL) was added. After the addition was completed, the system was subjected to nitrogen protection and stirred at 20°C for 2 h. Upon complete depletion of raw materials as monitored by LCMS, the reaction solution was concentrated under reduced pressure to obtain the target compound **77D** (0.8 g, crude).
LCMS m/z =407.1 [M +1]$^+$

Step 4:

Compound carbonyldiimidazole (0.40 g, 2.47 mmol) and N-Boc-3-hydroxyazetidine (0.43 g, 2.47 mmol) were weighed and added to a 100 mL single-neck flask, and the mixture was dissolved in tetrahydrofuran (20 mL). The resulting mixture was stirred at 25°C for 1 h and then **77D** (0.50 g, 1.23 mmol) and triethylamine (0.37 g, 3.65 mmol) were added. After the addition, the mixture was stirred at 70°C for 16 h. After the reaction was completed, as found by TLC plate spotting (petroleum ether:ethyl acetate =1:1), water (20 mL) was added, and the mixture was stirred for 5 min, extracted with ethyl acetate (20 mL), and separated. The organic phase was dried over anhydrous sodium sulphate and concentrated under reduced pressure. The crude was purified by column chromatography (eluents: petroleum ether: ethyl acetate = 1:1) to obtain the target compound **77E** (0.40 g, yield: 53%).
LCMS m/z =550.1 [M -55]$^+$

Step 5:

To a 100 mL single-neck flask was added compound **77E** (0.4 g, 0.66 mmol), which was dissolved in dichloromethane (6 mL), and trifluoroacetic acid (3 mL) was added. After the addition was completed, the system was subjected to nitrogen protection and stirred at 20°C for 4 h. Upon complete depletion of raw materials as monitored by LCMS, the reaction solution was concentrated under reduced pressure to obtain the target compound **77F** (0.3 g, crude).
LCMS m/z =506.1 [M +1]$^+$

Step 6:

Compound **77F** (300 mg, 0.59 mmol) and triethylamine (0.18 g, 1.78 mmol) were weighed and added to a 100 mL single-neck flask. The mixture was dissolved in dichloromethane (6 mL), and at 0°C, methanesulphonyl chloride (81 mg, 0.71 mmol) was added. After the addition, the system was subjected to nitrogen protection and stirred at 0°C for 2 h. Upon complete depletion of raw materials as monitored by LCMS, water (20 mL) was added, and the mixture was stirred for 5 min, extracted with dichloromethane (20 mL), and separated. The organic phase was dried over anhydrous sodium sulphate, and concentrated under reduced pressure. The crude was purified by column chromatography (eluents: petroleum ether: ethyl acetate = 1:1) to obtain the target crude compound, which was subjected to **preparative** HPLC **to obtain compound 77** (35 mg, yield: 12%).

Preparation method: instrument: waters 2767 preparative liquid chromatographic instrument; chromatography column: SunFire@ Prep C18 (19 mm × 250 mm); the sample was dissolved in DMF and filtered with a 0.45 μm filter to prepare a sample liquid; Preparative chromatography conditions: a. composition of mobile phases A and B: mobile phase A: acetonitrile, and mobile phase B: water (containing 5 mM aqueous ammonia); b. gradient elution, mobile phase A: 40%-80%; c. flow rate: 15 mL/min; d. elution time: 20 min.

LCMS m/z =584.2 [M +1]$^+$
$^1$H NMR (400 MHZ, DMSO-$d_6$) δ8.12 (s, 1H), 7.63 (s, 1H), 7.57 (d, 1H), 7.47 (d, 1H),6.40 (s, 1H), 4.99-5.05 (m,1H), 4.10-4.16 (m, 2H), 4.02 (s, 4H), 3.81-3.87 (m,4H), 3.77 (d, 2H), 3.58 (d, 1H), 3.49 (d, 1H), 3.41-3.45 (m, 1H), 3.33-3.38 (m, 1H), 3.04 (s, 3H), 1.58-1.65 (m,2H), 1.00-1.05 (m,1H).

**Example 78:**

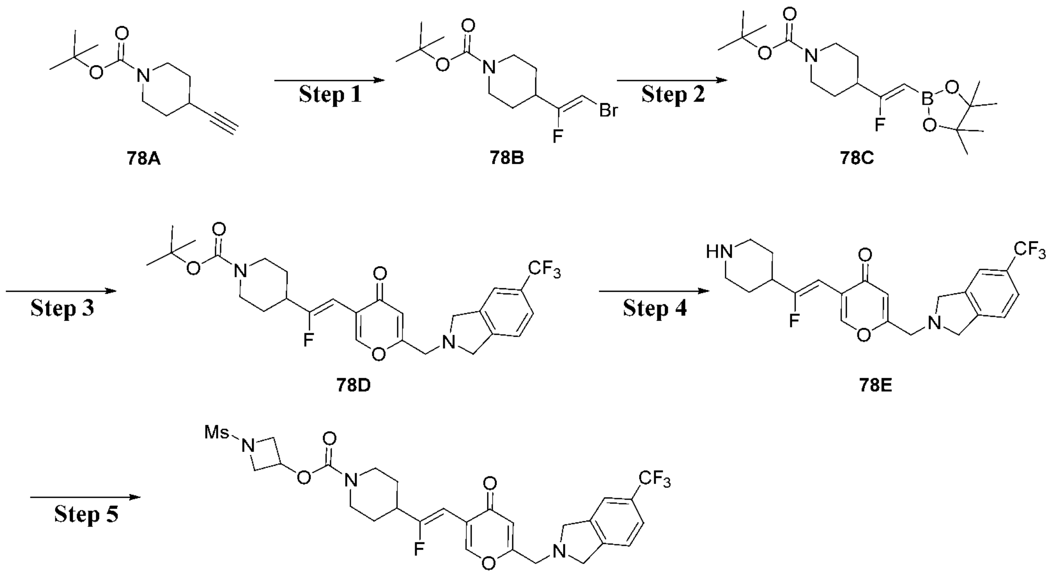


78A 78B 78C 78D 78E

Compound 78

Step 1:

N-bromosuccinimide (4.67 g, 26.26 mmol) and silver fluoride (7.57 g, 59.68 mmol) were added to a mixed solution of acetonitrile (90 mL) and water (10 mL), and at room temperature, 78A (5.00 g, 23.87 mmol) was added. After the addition, the mixture was reacted at 80°C overnight. After the reaction was completed, the reaction mixture was cooled to room temperature, and the reaction solution was subjected to suction filtration. The filtrate was extracted with ethyl acetate (100 mL×2), the organic phase was concentrated, and the residue was purified by column chromatography (petroleum ether/ethyl acetate =9/1, (v/v)) to obtain 78B (5.50 g, yield: 74%).

LC-MS (ESI): m/z= 252.2 $[M-56]^+$.

Step 2:

78B (3.00 g, 9.73 mmol) was dissolved in 1,4-dioxane (30 mL), and at room temperature, bis(pinacolato)diboron (3.71 g, 14.60 mmol), tricyclohexylphosphine (0.27 g, 0.96 mmol) and potassium acetate (3.36 g, 24.31 mmol) were added. After the addition, the system was subjected to nitrogen replacement three times, and tris(dibenzylidene-BASE acetone)dipalladium (0.89 g, 0.97 mmol) was then added. After the addition, the system was subjected to nitrogen replacement three times and reacted at 80°C overnight. After the reaction was completed, the reaction solution was subjected to suction filtration, the filtrate was concentrated, and the residue was purified by column chromatography (petroleum ether/ethyl acetate =9/1, (v/v)) to obtain 78C (3.40 g, yield: 98%).

LC-MS (ESI): m/z= 255.4 $[M-100]^+$.

Step 3:

41B (1.70 g, 3.83 mmol) was dissolved in 1,4-dioxane (30 mL) and water (6 mL), and at room temperature, 78C (1.70 g, 4.79 mmol), potassium carbonate (1.32 g, 9.55 mmol) and [1,1'-bis(diphenylphosphino)ferrocene]palladium dichloride (II) (0.35 g, 0.48 mmol) were added. After the addition, the mixture was subjected to nitrogen replacement three times and reacted at 90°C for 3 hrs. After the reaction was completed, the reaction mixture was concentrated, and the residue was purified by column chromatography (petroleum ether/ethyl acetate =1/1, (v/v)) to obtain 78D (0.60 g, yield: 23%).

LC-MS (ESI): m/z= 467.6 $[M-55]^+$.

Step 4:

78D (300 mg, 0.57 mmol) was dissolved in dichloromethane (8 mL), and at 0°C-5°C, trifluoroacetic acid (2 mL) was added. After the addition, the mixture was reacted at room temperature for 2.5 hrs. After the reaction was completed, the reaction mixture was concentrated to obtain the crude 78E, which was directly used in the next reaction.

Step 5:

1-Methylsulphonylazetidin-3-ol (232.67 mg, 1.54 mmol) was dissolved in tetrahydrofuran (10 mL), and at room temperature, N,N'-carbonyldiimidazole (249.55 mg, 1.54 mmol) was added. After the addition, the mixture was reacted at room temperature for 4.5 hrs, and triethylamine (288.39 mg, 2.85 mmol) was added dropwise to the reaction system at room temperature. After the addition, the mixture was reacted at room temperature for 2 hrs, and a solution of 78E (240 mg, 0.57 mmol) in tetrahydrofuran (1 mL) was added dropwise at 0°C-5°C. After the addition, the mixture was reacted at 60°C overnight. After the reaction was completed, the reaction solution was concentrated and purified by preparative HPLC to obtain compound 78 (195 mg, yield: 57%).
LC-MS (ESI): m/z= 600.2 $[M+H]^+$.
$^1H$ NMR (400 MHz, DMSO-$d_6$) δ 8.49 (s, 1H), 7.62 (s, 1H), 7.58-7.56 (d, 1H), 7.48-7.46 (d, 1H), 6.41 (s, 1H), 5.74-5.63 (d, 1H), 5.07 - 5.01 (m, 1H), 4.17-4.13 (m, 2H), 4.10-4.05 (m, 6H), 3.89-3.86 (m, 2H), 3.83 (s, 2H), 3.04 (s, 3H), 3.00-2.77 (m, 2H), 2.66 - 2.49 (m, 1H), 1.86-1.83 (d, 2H), 1.43-1.40 (d, 2H).

**Example 79:**

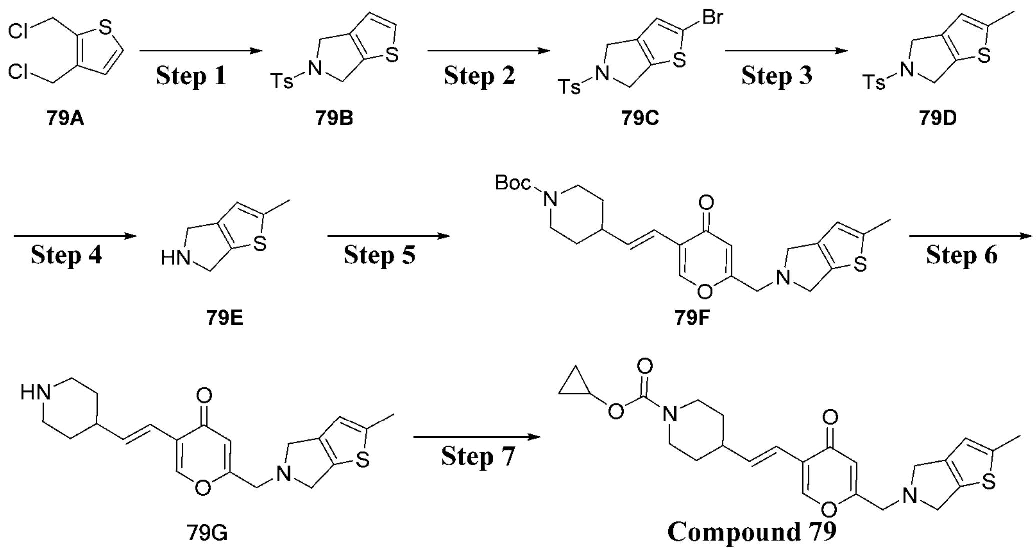


79A 79B 79C 79D 79E 79F 79G Compound 79

Step 1:

79A (6.50 g, 35.90 mmol, synthesized with reference to: Organic Letters, 2012, vol. 14, # 6, p. 1508 - 1511) was dissolved in N,N-dimethylformamide (65 mL), and sodium hydride (3.59 g, 89.75 mmol) was added at 0°C-5°C. After the addition, the mixture was reacted at room temperature for 30 min, and p-toluenesulphonamide (6.15 g, 35.90 mmol) was added at 0°C-5°C. After the addition, the mixture was reacted at room temperature for 3 hrs. After the reaction was completed, to the reaction solution was added water (200 mL) to quench the reaction, and the resulting mixture was extracted with ethyl acetate (200 mL×2). The organic phase was concentrated, and the residue was purified by column chromatography (petroleum ether/ethyl acetate =1/1, (v/v)) to obtain 79B (4.80 g, yield: 47%).
LC-MS (ESI): m/z= 280.2 $[M+H]^+$.

Step 2:

79B (0.55 g, 1.97 mmol) was dissolved in acetonitrile (11 mL), and at room temperature, N-bromosuccinimide (385.68 mg, 2.17 mmol) was added. After the addition, the mixture was reacted at room temperature for 1.5 hrs. After the reaction was completed, the reaction mixture was concentrated, and the residue was purified by column chromatography (petroleum ether/ethyl acetate =4/1, (v/v)) to obtain 79C (0.50 g, yield: 70%).

Step 3:

79C (0.50 g, 1.40 mmol) was dissolved in 1,4-dioxane (16 mL) and water (2 mL), and at room temperature, methyl

boronic acid (355.22 mg, 5.60 mmol) and potassium carbonate (967.47 mg,7.00 mmol) were added. After the addition, the system was subjected to nitrogen replacement three times, and then tetrakis(triphenylphosphine)palladium (161.78 mg, 0.14 mmol) was added. After the addition, the mixture was subjected to nitrogen replacement three times and reacted at 100°C overnight. After the reaction was completed, the reaction solution was subjected to suction filtration, the filtrate was concentrated, and the residue was purified by column chromatography (petroleum ether/ethyl acetate =4/1, (v/v)) to obtain 79D (0.25 g, yield: 60%).

LC-MS (ESI): m/z= 294.1 $[M+H]^+$.

Step 4:

79D (0.25 g, 0.85 mmol) was dissolved in aqueous hydrogen bromide solution (1 mL), and then at room temperature, phenol (385.159.99 mg, 1.70 mmol) was added. After the addition, the mixture was reacted at 100°C overnight. After the reaction was completed, the reaction solution was extracted with diethyl ether (15 mL×2), and the aqueous phase was directly concentrated to obtain crude 79E, which was directly used in the next reaction.

LC-MS (ESI): m/z= 140.1 $[M+H]^+$.

Step 5:

16C (0.18 g, 0.54 mmol) was dissolved in dichloromethane (10 mL), and triethylamine (136.61 mg, 1.35 mmol) and methanesulphonyl chloride (92.79 mg, 0.81 mmol) were added at 0°C-5°C. After the addition, the mixture was reacted at room temperature for 30 min, and 79E (90.21 mg, 0.65 mmol) was added at room temperature. After the addition, the mixture was reacted at room temperature overnight. After the reaction was completed, the reaction solution was concentrated and purified by column chromatography (petroleum ether/ethyl acetate =1/1, (v/v)) to obtain compound 79F (110 mg, yield: 44%).

LC-MS (ESI): m/z= 401.4 $[M-55]^+$.

Step 6:

79F (110 mg, 0.24 mmol) was dissolved in dichloromethane (4 mL), and at 0°C-5°C, trifluoroacetic acid (1 mL) was added. After the addition, the mixture was reacted at room temperature for 2 hrs. After the reaction was completed, the reaction mixture was concentrated to obtain crude 79G, which was directly used in the next reaction.

Step 7:

Cyclopropanol (37.64 mg, 0.65 mmol) was dissolved in tetrahydrofuran (10 mL), and at room temperature, N,N'-carbonyldiimidazole (105.07 mg, 0.65 mmol) was added. After the addition, the mixture was reacted at room temperature for 4.5 hrs, and triethylamine (121.43 mg, 1.20 mmol) was added dropwise to the reaction system at room temperature. After the addition, the mixture was reacted at room temperature for 2 hrs, and at 0°C-5°C, a solution of crude 79G in tetrahydrofuran (0.5 mL) was added dropwise. After the addition, the mixture was reacted at 60°C overnight. After the reaction was completed, the reaction solution was concentrated and purified by preparative HPLC to obtain compound 79 (5 mg, yield: 4%).

LC-MS (ESI): m/z= 411.8 $[M+H]^+$.

$^1H$ NMR (400 MHz, DMSO-$d_6$) δ 8.29 (s, 1H), 6.6\6-6.61 (dd, 1H), 6.58 (s, 1H), 6.31 (s, 1H), 6.13-6.09 (d, 1H), 3.99 - 3.94 (m, 3H), 3.82-3.81 (m, 4H), 2.83-2.77 (t, 2H), 2.40 (s, 3H), 2.25 - 2.21 (m, 1H), 1.68-1.65 (d, 2H), 1.28 - 1.14 (m, 4H), 0.65 - 0.56 (m, 4H).

**Example 80**

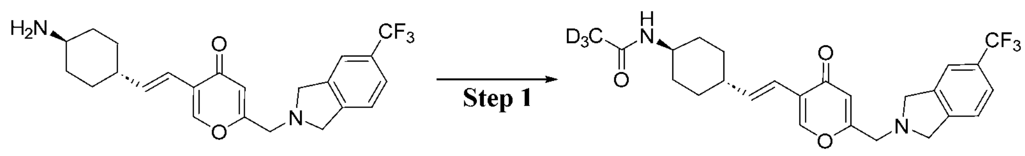


61D

Compound 80

Step 1:

61D (320 mg, 0.76 mmol) was dissolved in dichloromethane (10 mL), and at 0°C-5°C, triethylamine (153.81 mg, 1.52 mmol) and deuterated acetyl chloride (92.93 mg, 1.14 mmol) were successively added. After the dropwise addition was completed, the mixture was reacted at room temperature overnight. After the reaction was completed, the reaction solution was concentrated and purified by preparative HPLC to obtain compound 80 (160.00 mg, yield: 45%).
LC-MS (ESI): m/z= 464.5 $[M+H]^+$.
$^1$H NMR (400 MHz, DMSO-$d_6$) δ 8.30 (s, 1H), 7.69-7.67 (d, 1H), 7.62 (s, 1H), 7.58-7.56 (d, 1H), 7.48-7.46 (d, 1H), 6.66-6.60 (dd, 1H), 6.35 (s, 1H), 6.13-6.09 (d, 1H), 4.03 (s, 4H), 3.81 (s, 2H), 3.48-3.45 (m, 1H), 2.01-2.00 (m, 1H), 1.82-1.74 (m,4H), 1.24-1.13 (m, 4H).

**Example 81**

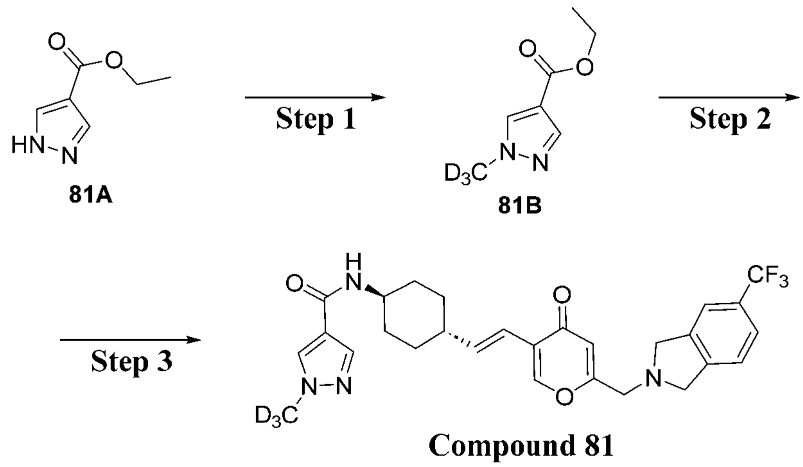


Compound 81

Step 1:

81A (2.6 g, 18.56 mmol) was dissolved in N,N-dimethylformamide (30 mL), and at 0°C-5°C, caesium carbonate (9.07 g, 27.84 mmol) and deuterated iodomethane (3.23 g, 22.27 mmol) were successively added. After the addition, the mixture was reacted at room temperature overnight. After the reaction was completed, to the reaction solution was added water (200 mL) to quench the reaction, and the resulting mixture was extracted with ethyl acetate (200 mL×2). The organic phases were combined, washed with water (200 mL×1) and saturated sodium chloride aqueous solution (200 mL×1), dried over anhydrous sodium sulphate and concentrated to obtain 81B (2.7 g, yield: 92%).
LC-MS (ESI): m/z= 158.2 $[M+H]^+$.

Step 2:

81B (2.7 g, 17.18 mmol) was dissolved in ethanol (30 mL), and at 0°C-5°C, a solution of sodium hydroxide (1.38 g, 34.61 mmol) in water (10 mL) was added. After the addition, the mixture was reacted at room temperature overnight. After the reaction was completed, the reaction mixture was concentrated. After concentration, the residue was dissolved in water, and adjusted to pH=2-3 with 1 N hydrochloric acid. A large amount of white solid was precipitated, and the mixture was subjected to suction filtration. The filter cake was concentrated to obtain 81C (1.30 g, yield: 56%).
LC-MS (ESI): m/z= 130.2 $[M+H]^+$.

Step 3:

61D (350 mg, 0.84 mmol) and 81C (119.32 mg, 0.92 mmol) were dissolved in N,N-dimethylformamide (10 mL), and at 0°C-5°C, N-methylimidazole (344.82 mg, 4.20 mmol) was added. After the addition, the mixture was reacted for 5 min, and then at 0°C-5°C, N,N,N',N'-tetramethylchloroformamidinium hexafluorophosphate (353.53 mg, 1.26 mmol) was added. After the addition, the mixture was reacted at room temperature overnight. After the reaction was completed, the reaction solution was concentrated and purified by preparative HPLC to obtain compound 81 (170.00 mg, yield: 38%).
LC-MS (ESI): m/z= 530.7 $[M+H]^+$.

$^1$H NMR (400 MHz, DMSO-$d_6$) δ 8.31 (s, 1H), 8.09 (s, 1H), 7.82 (s, 1H), 7.77-7.75 (d, 1H), 7.63 (s, 1H), 7.58-7.56 (d, 1H), 7.48-7.46 (d, 1H), 6.68-6.62 (dd, 1H), 6.36 (s, 1H), 6.15-6.11 (d,1H), 4.04 (s, 4H), 3.82 (s, 2H), 3.72 - 3.64 (m, 1H), 2.07 - 2.03 (m, 1H), 1.88-1.77 (m, 4H), 1.39-1.19 (m, 4H).

**Example 82**

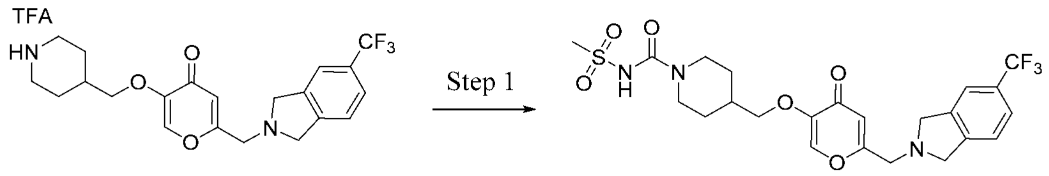


7D

**Compound 82**

Step 1:

Methanesulphonamide (190 mg, 2.0 mmol) was dissolved in DCM (10 mL), DIPEA (650 mg, 5 mmol) was added, and the mixture was reacted at room temperature for 2 h. Then 7D (408 mg, 1.0 mmol) was added and the mixture was stirred at room temperature for 12 h. After the reaction was completed, water (10 mL) was added to the reaction solution, the resulting mixture was extracted with DCM (15 mL×3), and the organic layers were combined, dried over anhydrous sodium sulphate, filtered and concentrated. The residue was separated and purified by silica gel column chromatography (dichloromethane: methanol (v/v) = 20:1) to obtain **compound 82** (150 mg, yield: 28%).
LC-MS (ESI): m/z = 530.5 $[M+H]^+$.
$^1$H NMR (400 MHz, DMSO-$d_6$) δ 8.13 (s, 1H), 7.62 (s, 1H), 7.57 (d, 1H), 7.47 (d, 1H), 6.38 (s, 1H), 4.22 (d, 2H), 4.03 (s, 4H), 3.80 (s, 2H), 3.67 (d, 2H), 3.31 (s, H), 2.70 (s, 3H), 1.92 (s, 2H), 1.92 - 1.75 (m, 1H), 1.69 - 1.55 (m, 2H), 1.07 - 0.97 (m, 2H).
$^{19}$F NMR (376 MHz, DMSO) δ -58.34 (s).

**Example 83**

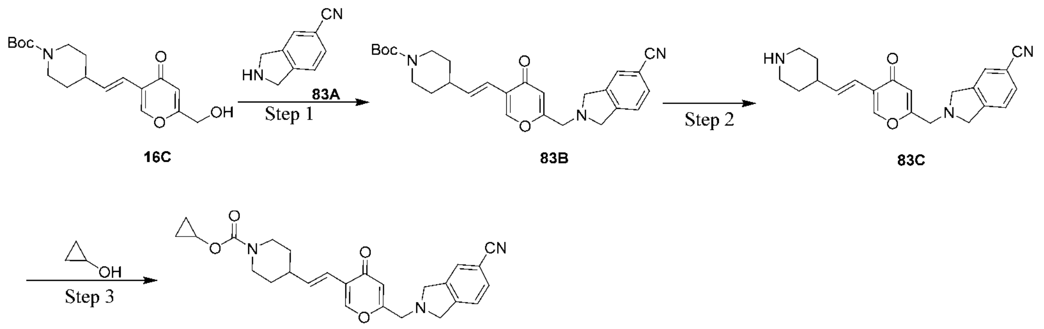


16C 83B 83C

**Compound 83**

Step 1:

Compound 16C (420 mg, 1.25 mmol) was dissolved in dry dichloromethane (20 mL), and in an ice bath, triethylamine (316 mg, 3.13 mmol) was added. After the addition, methanesulphonyl chloride (172 mg, 1.50 mmol) was slowly added in an ice bath and the resulting mixture was stirred at room temperature for 1 h. In an ice bath, a solution of compound 83A (218 mg, 1.51 mmol) and triethylamine (191 mg, 1.89 mmol) in dichloromethane (10 mL) was slowly added to the above-mentioned reaction solution, and the mixture was stirred and reacted at room temperature for 2 h. After the reaction was completed as monitored by TLC (petroleum ether: ethyl acetate =1:1 (v/v)), the reaction solution was diluted with dichloromethane (100 mL), water (50 mL) was added for liquid separation, and the organic phase was dried over anhydrous sodium sulphate, filtered, and concentrated under reduced pressure. The residue was purified by column chromatography (petroleum ether:ethyl acetate = 2:1 (v/v)) to obtain compound 83B (380 mg, yield: 65%).

LC-MS (ESI): $m/z$ = 406.1 $[M-56+H]^+$.

Step 2:

Compound 83B (380 mg, 0.82 mmol) was dissolved in dichloromethane (10 mL), trifluoroacetic acid (3 mL) was added, and the mixture was reacted at room temperature for 2 h. After the reaction was completed as monitored by TLC (petroleum ether: ethyl acetate = 1:1 (v/v)), the reaction solution was concentrated to obtain compound 83C (315 mg, crude), which was directly used in the next step without purification.

LC-MS (ESI): $m/z$ = 362.4 $[M+H]^+$.

Step 3:

Cyclopropanol (101 mg, 1.74 mmol) and N,N'-carbonyldiimidazole (282 mg, 1.74 mmol) were dissolved in tetrahydrofuran (15 mL), and the mixture was reacted at room temperature for 1 h. In an ice-water bath, triethylamine (308 mg, 3.04 mmol) was added. After the dropwise addition was completed, the mixture was reacted at room temperature for 2 h. Compound 83C (315 mg, 0.87 mmol) was added to the above-mentioned solution. After the addition, the mixture was reacted at 60°C overnight. After the reaction was completed as monitored by TLC (dichloromethane: methanol = 10:1 (v/v)), the reaction solution was concentrated, and the crude was purified by column chromatography (eluents: dichloromethane: methanol = 65:1 (v/v)) to obtain the target compound 83 (65 mg, yield: 17%).

LCMS $m/z$ = 446.4 $[M+1]^+$

$^1$H NMR (400 MHZ, DMSO-$d_6$) δ 8.31 (s, 1H), 7.72 (s, 1H), 7.68 (d, 1H), 7.46 (d, 1H), 6.65 (dd, 1H), 6.35 (s, 1H), 6.12 (d, 1H), 4.03 (s, 2H), 4.00 (s, 2H), 3.99 - 3.83 (m, 3H), 3.80 (s, 2H), 2.81 (t, 2H), 2.25 (td, 1H), 1.67 (d, 2H), 1.27 - 1.14 (m, 2H), 0.66 - 0.56 (m, 4H).

**Example 84**

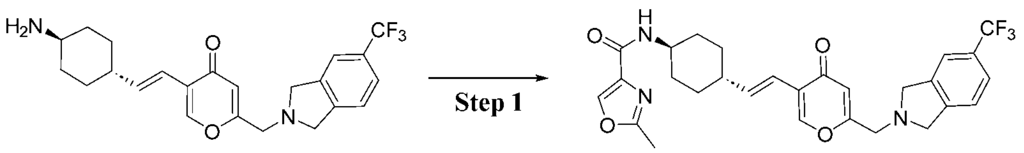


61D

Compound 84

Step 1:

61D (230 mg, 0.55 mmol) and 2-methyloxazole-4-carboxylic acid (76.90 mg, 0.61 mmol) were dissolved in N,N-dimethylformamide (10 mL), and at 0°C-5°C, N-methylimidazole (225.77 mg, 2.75 mmol) was added. After the addition, the mixture was reacted for 5 min, and then at 0°C-5°C, N,N,N',N'-tetramethylchloroformamidinium hexafluorophosphate (231.48 mg, 0.83 mmol) was added. After the dropwise addition was completed, the mixture was reacted at room temperature overnight. After the reaction was completed, the reaction solution was concentrated and purified by preparative HPLC to obtain compound 84 (65.00 mg, yield: 22%).

LC-MS (*ESI*): $m/z$ = 528.6 $[M+H]^+$.

$^1$H NMR (400 MHz, DMSO-$d_6$) δ 8.41 (s, 1H), 8.30 (s, 1H), 7.88-7.85 (d, 1H), 7.63 (s, 1H), 7.58-7.56 (d, 1H), 7.48-7.46 (d, 1H), 6.68-6.62 (dd, 1H), 6.36 (s, 1H), 6.14-6.10 (d, 1H), 4.04 (s, 4H), 3.82 (s, 2H), 3.72 - 3.67 (m, 1H), 2.44 (s, 3H), 2.03-2.01 (m, 1H), 1.83-1.75 (m, 4H), 1.50-1.42 (m, 2H), 1.27-1.19 (m, 2H).

**Example 85:**

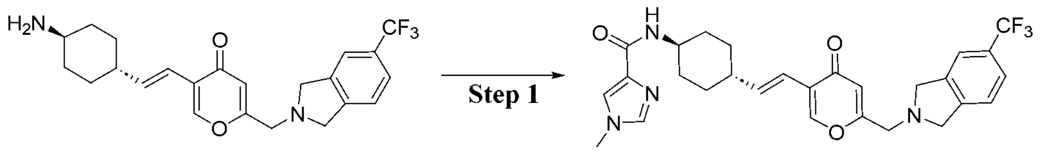


61D        Compound 85

Step 1:

61D (220 mg, 0.53 mmol) and 1-methyl-4-imidazole carboxylic acid (73.52 mg, 0.58 mmol) were dissolved in N,N-dimethylformamide (10 mL), and at 0°C-5°C, N-methylimidazole (217.56 mg, 2.65 mmol) was added. After the addition, the mixture was reacted for 5 min, and then at 0°C-5°C, N,N,N',N'-tetramethylchloroformamidinium hexafluorophosphate (233.06 mg, 0.80 mmol) was added. After the dropwise addition was completed, the mixture was reacted at room temperature overnight. After the reaction was completed, the reaction solution was concentrated and purified by preparative HPLC to obtain compound 85 (90.00 mg, yield: 32%).

LC-MS (ESI): m/z= 527.6 $[M+H]^+$.

$^1$H NMR (400 MHz, DMSO-$d_6$) δ 8.31 (s, 1H), 7.63 (s, 2H), 7.60-7.56 (m, 2H), 7.52-7.46 (m, 2H), 6.69-6.63 (dd, 1H), 6.36 (s, 1H), 6.14-6.10 (d, 1H), 4.04 (s, 4H), 3.82 (s, 2H), 3.72 - 3.65 (m, 4H), 2.04-1.99 (m, 1H), 1.85 - 1.76 (m, 4H), 1.47-1.38 (m, 2H), 1.28-1.19(m, 2H).

**Example 86**

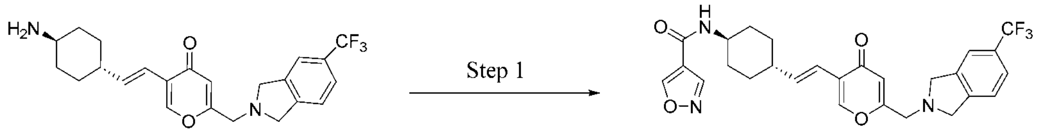


61D        Compound 86

Step 1:

**61D** (100.0 mg, 0.24 mmol) and 4-isoxazole carboxylic acid (35.0 mg, 0.31 mmol) were dissolved in N,N-dimethylformamide (8 mL), and the mixture was stirred at 0°C. N-methylimidazole (110.0 mg, 1.38 mmol) was then added, and N,N,N',N'-tetramethylchloroformamidinium hexafluorophosphate (100.0 mg, 0.36 mmol) was slowly added. After the addition, the resulting mixture was reacted at room temperature for 3 hours. After the reaction was completed as monitored by LC-MS, water (30 mL) was added, and the mixture was extracted with ethyl acetate (30 mL×2). The organic layer was dried over anhydrous sodium sulphate and filtered, and the filtrate was concentrated under reduced pressure. The residue was separated and purified by column chromatography (petroleum ether/ethyl acetate = 1/1) to obtain compound **86** (22.0 mg, yield: 17.8%).

$^1$H NMR (400 MHz, $CDCl_3$) δ 8.84 (s, 1H), 8.54 (s, 1H), 7.82 (s, 1H), 7.50 (d, 1H), 7.46 (s, 1H), 7.31 (d, 1H), 6.47-6.41 (m, 2H), 6.21 (d, 1H), 5.76 (d, 1H), 4.10 (s, 4H), 3.96-3.88 (m, 1H), 3.79 (s, 2H), 2.14-2.09 (m, 3H), 1.92-1.88 (m, 2H), 1.42-1.25 (m, 4H), LC-MS (ESI): m/z = 514.3 $[M+H]^+$.

**Example 87**

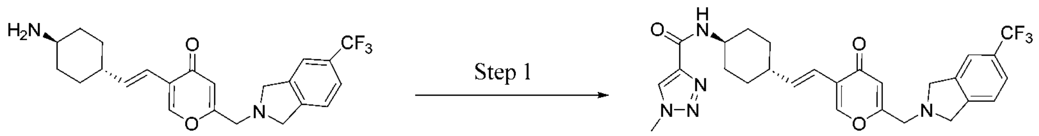


61D        Compound 87

Step 1:

61D (100.0 mg, 0.24 mmol) and 1-methyl-1H-1,2,3-triazole-4-carboxylic acid (40.0 mg, 0.31 mmol) were dissolved in N,N-dimethylformamide (8 mL), and the mixture was stirred at 0°C. N-methylimidazole (110.0 mg, 1.38 mmol) was then added, and N,N,N',N'-tetramethylchloroformamidinium hexafluorophosphate (100.0 mg, 0.36 mmol) was slowly added. After the addition, the resulting mixture was reacted at room temperature for 3 hours. After the reaction was completed as monitored by LC-MS, water (30 mL) was added, and the mixture was extracted with ethyl acetate (30 mL×2). The organic layer was dried over anhydrous sodium sulphate and filtered, and the filtrate was concentrated under reduced pressure. The residue was separated and purified by column chromatography (petroleum ether/ethyl acetate = 1/1) to obtain compound **87** (28.0 mg, yield: 22.1%).

$^1$H NMR (400 MHz, $CDCl_3$) δ 8.01 (s, 1H), 7.81 (s, 1H), 7.52 (d, 1H), 7.47 (s, 1H), 7.32 (d, 1H), 6.98 (d, 1H), 6.50 (dd, 1H), 6.43 (s, 1H), 6.18 (d, 1H), 4.17-4.14 (m, 7H), 3.96-3.90 (m, 1H), 3.84 (s, 2H), 2.14-2.09 (m, 3H), 1.91-1.85 (m, 2H), 1.42-1.29 (m, 4H), LC-MS (ESI): m/z = 528.4 $[M+H]^+$.

**Example 88**

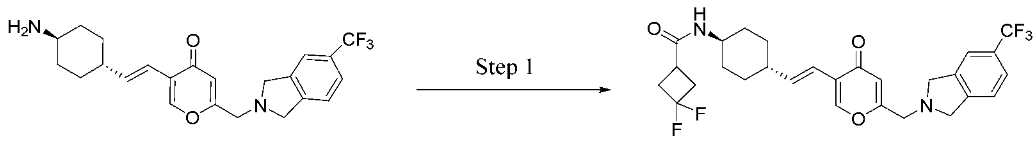


61D

Compound 88

Step 1:

**61D** (150.0 mg, 0.36 mmol) and 3,3-difluorocyclobutane carboxylic acid (63.0 mg, 0.46 mmol) were dissolved in N,N-dimethylformamide (8 mL), and the mixture was stirred at 0°C. N-methylimidazole (180.0 mg, 2.16 mmol) was then added, and N,N,N',N'-tetramethylchloroformamidinium hexafluorophosphate (150.0 mg, 0.54 mmol) was slowly added. After the addition, the resulting mixture was reacted at room temperature for 3 hours. After the reaction was completed as monitored by LC-MS, water (30 mL) was added, and the mixture was extracted with ethyl acetate (30 mL×2). The organic layer was dried over anhydrous sodium sulphate and filtered, and the filtrate was concentrated under reduced pressure. The residue was separated and purified by column chromatography (petroleum ether/ethyl acetate = 1/1) to obtain compound **88** (50.0 mg, yield: 25.8%).

$^1$H NMR (400 MHz, $CDCl_3$) δ 7.80 (s, 1H), 7.49 (d, 1H), 7.45 (s, 1H), 7.30 (d, 1H), 6.45 (dd, 1H), 6.40 (s, 1H), 6.17 (d, 1H), 5.27 (d, 1H), 4.09 (s, 4H), 3.78 (s, 2H), 3.76-3.70 (m, 1H), 2.91-2.82 (m, 2H), 2.74-2.66 (m, 3H), 2.06-2.00 (m, 3H), 1.89-1.82 (m, 2H), 1.37-1.27 (m, 2H), 1.22-1.12 (m, 2H),
LC-MS (ESI): m/z = 537.7 $[M+H]^+$.

**Example 89**

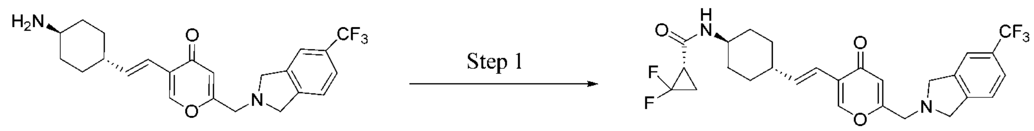


61D

Compound 89

Step 1:

**61D** (450.0 mg, 1.08 mmol) and (1S)-2,2-difluorocyclopropane-1-carboxylic acid (170.0 mg, 1.40 mmol) were dissolved in N,N-dimethylformamide (10 mL), and the mixture was stirred at 0°C. N-methylimidazole (530.0 mg, 6.48 mmol) was then added, and N,N,N',N'-tetramethylchloroformamidinium hexafluorophosphate (450.0 mg, 1.62 mmol) was slowly added. After the addition, the resulting mixture was reacted at room temperature for 3 hours. After the reaction was completed as monitored by LC-MS, water (30 mL) was added, and the mixture was extracted with ethyl acetate (30 mL×2).

The organic layer was dried over anhydrous sodium sulphate and filtered, and the filtrate was concentrated under reduced pressure. The residue was separated and purified by column chromatography (petroleum ether/ethyl acetate = 1/1) to obtain compound **89.**
LC-MS (ESI): m/z = 523.6 [M+H]+

**Example 90**

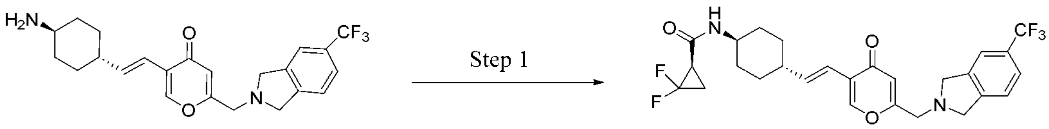


61D

Compound 90

Step 1:

**61D** (450.0 mg, 1.08 mmol) and (1R)-2,2-difluorocyclopropane-1-carboxylic acid (170.0 mg, 1.40 mmol) were dissolved in N,N-dimethylformamide (10 mL), and the mixture was stirred at 0°C. N-methylimidazole (530.0 mg, 6.48 mmol) was then added, and N,N,N',N'-tetramethylchloroformamidinium hexafluorophosphate (450.0 mg, 1.62 mmol) was slowly added. After the addition, the resulting mixture was reacted at room temperature for 3 hours. After the reaction was completed as monitored by LC-MS, water (30 mL) was added, and the mixture was extracted with ethyl acetate (30 mL×2). The organic layer was dried over anhydrous sodium sulphate and filtered, and the filtrate was concentrated under reduced pressure. The residue was separated and purified by column chromatography (petroleum ether/ethyl acetate = 1/1) to obtain compound **90.**
LC-MS (ESI): m/z = 523.6 [M+H]+.

**Example 91:**

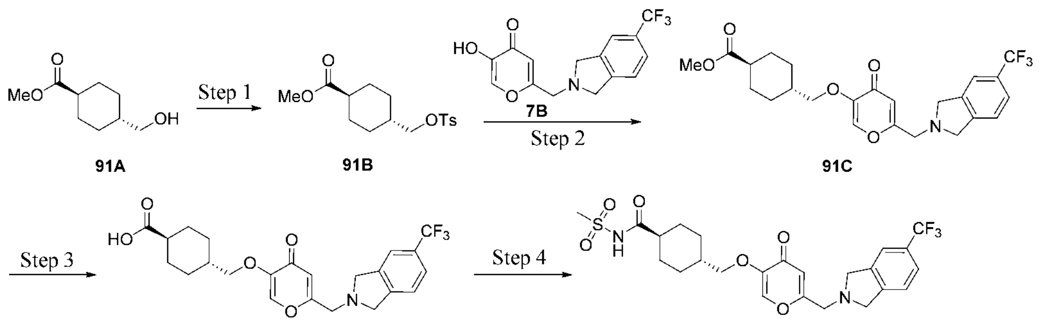


91A 91B 91C

91D

Compound 91

Step 1:

91A (2.0 g, 11.6 mmol) was dissolved in DCM (40 mL), and triethylamine (1.8 g, 17.4 mmol) and TsCl (2.7 g, 13.9 mmol) were successively added, and the mixture was stirred at room temperature for 4 h. After the reaction was completed, water (20 mL) was added to the reaction solution, the resulting mixture was extracted with DCM (30 mL×2), and the organic layers were combined, dried over anhydrous sodium sulphate, filtered and concentrated. The residue was separated and purified by silica gel column chromatography (petroleum ether: ethyl acetate (v/v)= 4/1) to obtain 91B (2.5 g, yield: 66%).
$^1$H NMR (400 MHz, $CDCl_3$) δ 7.78 (d, 2H), 7.34 (d, 2H), 3.83 (d, 2H), 3.66 (d, 3H), 2.45 (s, 3H), 2.24 - 2.16 (m, 1H), 2.05 - 1.90 (m, 2H), 1.88 - 1.73 (m, 2H), 1.72 - 1.58 (m, 1H), 1.44 - 1.34 (m, 2H), 1.02 - 0.92 (m, 2H).

Step 2:

91B (1.0 g, 3.1 mmol), 7B (0.8 g, 2.5 mmol), and caesium carbonate (2.0 g, 6.1 mmol) were dissolved in DMF (20

mL), and the mixture was reacted at 80°C for 2 h. After the reaction was completed, water (10 mL) was added to the reaction solution, the resulting mixture was extracted with EA (30 mL×3), and the organic layers were combined, dried over anhydrous sodium sulphate, filtered and concentrated. The residue was separated and purified by silica gel column chromatography (petroleum ether: ethyl acetate (v/v)= 3/1) to obtain 91C (0.8 g, yield: 56%).

LC-MS (ESI): *m/z* = 466.5 $[M+H]^+$.

Step 3:

91C (0.5 g, 1.1 mmol) was dissolved in THF (4 mL), water (1 mL) and lithium hydroxide (0.2 g, 8.8 mmol) were added, and the mixture was reacted at room temperature for 2 h. After the reaction was completed, THF was removed by distillation under reduced pressure. The solution was adjusted to pH 3 with diluted hydrochloric acid, a grey solid precipitated, and the mixture was filtered to obtain 91D (0.3 g, yield: 52%).

Step 4:

91D (0.2 g, 0.5 mmol) was dissolved in DMF (10 mL), CDI (0.2 g, 1.3 mmol) was then added, and the mixture was reacted at 65°C for 1 h. The reaction solution was cooled to 0°C. A solution of methanesulphonamide (250 mg, 2.6 mmol) and sodium hydride (88 mg, 2.2 mmol) in DMF (5 mL) was added, and the mixture was reacted at room temperature for 3 h. The solvent was concentrated and the residue was purified by silica gel column chromatography (dichloromethane/-methanol (v/v) = 10/1) to obtain compound 91 (50 mg, yield: 22%).

LC-MS (ESI): m/z = 529.5 $[M+H]^+$.

$^1H$ NMR (400 MHz, $CDCl_3$) δ 9.17 (s, 1H), 7.63 (s, 1H), 7.54 - 7.39 (m, 2H), 7.31 (d, 1H), 6.58 (s, 1H), 4.11 (s, 4H), 3.81 (s, 2H), 3.72 (d, 2H), 3.28 (s, 3H), 2.39 - 2.19 (m, 1H), 2.05 - 1.99 (m, 4H), 1.86 - 1.82 (m, 1H), 1.60 - 1.49 (m, 2H), 1.21 - 1.03 (m, 2H).

$^{19}F$ NMR (400 MHz, $CDCl_3$) δ -60.09 (s, 3F).

**Example 92:**

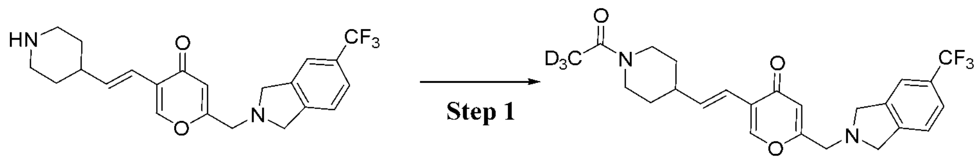


18E

Compound 92

Step 1:

18E (340 mg, 0.67 mmol) was dissolved in dichloromethane (10 mL), and at 0°C-5°C, triethylamine (338.99 mg, 3.35 mmol) and deuterated acetyl chloride (65.54 mg, 0.80 mmol) were successively added. After the dropwise addition was completed, the mixture was reacted at room temperature overnight. After the reaction was completed, the reaction solution was concentrated and purified by preparative HPLC to obtain compound 92 (150.00 mg, yield: 49%).

LC-MS (ESI): m/z= 450.2 $[M+H]^+$.

$^1H$ NMR (400 MHz, DMSO-$d_6$) δ 8.32 (s, 1H), 7.63 (s, 1H), 7.55-7.56 (d, 1H), 7.48-7.46 (d, 1H), 6.69-6.63 (m, 1H), 6.36 (s, 1H), 6.15-6.11 (d, 1H), 4.37-4.34 (d, 1H), 4.04 (s, 4H), 3.82 (s, 3H), 3.10 - 3.03 (m, 1H), 2.61- 2.54(m, 1H), 2.35 - 2.28 (m, 1H), 1.74-1.67 (t, 2H), 1.34 - 1.24 (m, 1H), 1.19-1.09 (m, 1H).

**Example 93:**

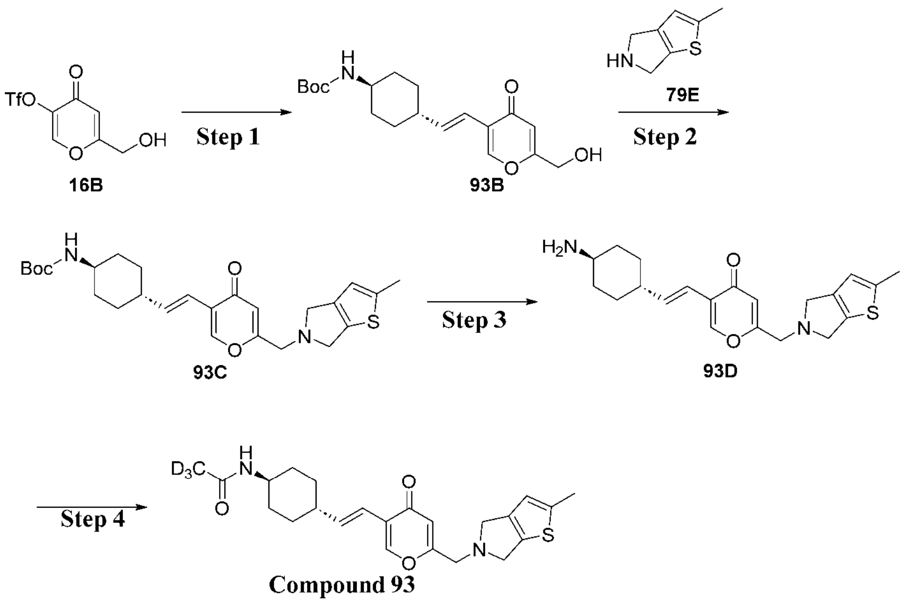


16B 93B 93C 93D Compound 93

Step 1:

16B (3.10 g, 11.31 mmol) was dissolved in 1,4-dioxane (30 mL) and water (6 mL), and at room temperature, 61B (5.96 g, 16.96 mmol), potassium carbonate (4.69 g, 33.93 mmol) and [1,1'-bis(diphenylphosphino)ferrocene]palladium dichloride (II) (0.83 g, 1.13 mmol) were added. After the addition, the mixture was subjected to nitrogen replacement three times and reacted at 85°C for 4 hrs. After the reaction was completed, the reaction mixture was concentrated, and the residue was purified by column chromatography (petroleum ether/ethyl acetate = 2/3, (v/v)) to obtain 93B (1.20 g, yield: 30%).
LC-MS (ESI): m/z= 294.4 $[M-55]^+$

Step 2:

93B (0.55 g, 1.57 mmol) was dissolved in dichloromethane (10 mL), and triethylamine (794.34 mg, 7.85 mmol) and methanesulphonyl chloride (215.81 mg, 1.88 mmol) were added at 0°C-5°C. After the addition, the mixture was reacted at room temperature for 30 min, and 79E (262.29 mg, 1.88 mmol) was added at room temperature. After the addition, the mixture was reacted at room temperature overnight. After the reaction was completed, the reaction solution was concentrated and purified by column chromatography (petroleum ether/ethyl acetate =1/1, (v/v)) to obtain compound 93C (192 mg, yield: 25%).

LC-MS (ESI): m/z= 471.8 $[M+H]^+$ .

Step 3:

93C (192 mg, 0.41 mmol) was dissolved in 1,4-dioxane (4 mL), and at 0°C-5°C, 4 N hydrogen chloride 1,4-dioxane solution (10 mL) was added dropwise. After the addition, the mixture was reacted at room temperature for 2 hrs. After the reaction was completed, the reaction mixture was concentrated to obtain crude 93D, which was directly used in the next reaction.

Step 4:

93D (151.90 mg, 0.41 mmol) was dissolved in dichloromethane (10 mL), and at 0°C-5°C, triethylamine (0.20 g, 2.02 mmol) and deuterated acetyl chloride (0.040 g, 0.49 mmol) were successively added. After the dropwise addition was completed, the mixture was reacted at room temperature overnight. After the reaction was completed, the reaction solution was concentrated and purified by preparative HPLC to obtain compound 93 (20.00 mg, yield: 11%).

LC-MS (ESI): m/z= 416.2 $[M+H]^+$.

$^1H$ NMR (400 MHz, DMSO-$d_6$) δ8.28 (s, 1H), 7.69-7.67 (d, 1H), 6.65 - 6.59 (m, 2H), 6.31 (s, 1H), 6.12-6.08 (d, 1H), 3.96 (s, 2H), 3.82-3.81 (m, 4H), 3.50-3.41 (m, 1H), 2.41 (s, 3H), 2.00-1.90 (m, 1H), 1.82-1.81 (m, 2H), 1.74 (s, 2H), 1.24-1.13 (m, 4H).

**Example 94:**

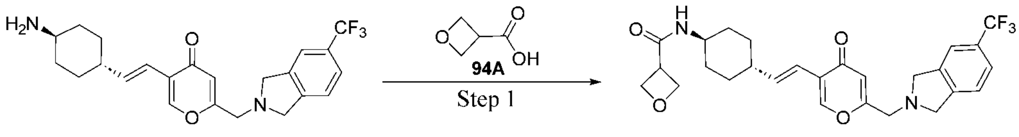


61D

Compound 94

Step 1:

Compound 61D (250 mg, 0.60 mmol) and 94A (92 mg, 0.90 mmol) were dissolved in N,N-dimethylformamide (10 mL), and N-methylimidazole (200 mg, 2.40 mmol) and TCFH (250 mg, 0.90 mmol) were successively added. After the addition, the mixture was reacted at room temperature for 3 h. Upon complete depletion of raw materials as monitored by LCMS, the reaction solution was concentrated, and purified by preparative HPLC to obtain compound 94 (105 mg, yield: 35%).

LC-MS (ESI): *m/z* = 503.20 $[M+H]^+$.

$^1H$ NMR (400 MHz, DMSO-$d_6$) δ 8.30 (s, 1H), 7.73 (d, 1H), 7.62 (s, 1H), 7.57 (d, 1H), 7.47 (d, 1H), 6.70-6.55 (m, 1H), 6.35 (s, 1H), 6.11 (d, 1H), 4.76-4.44 (m, 4H), 4.03 (s, 4H), 3.81 (s, 2H), 3.73-3.63 (m, 1H), 3.58-3.46 (m, 1H), 2.08-1.96 (m, 1H), 1.86-1.70 (m, 4H), 1.26-1.14 (m, 4H).

**Example 95:**

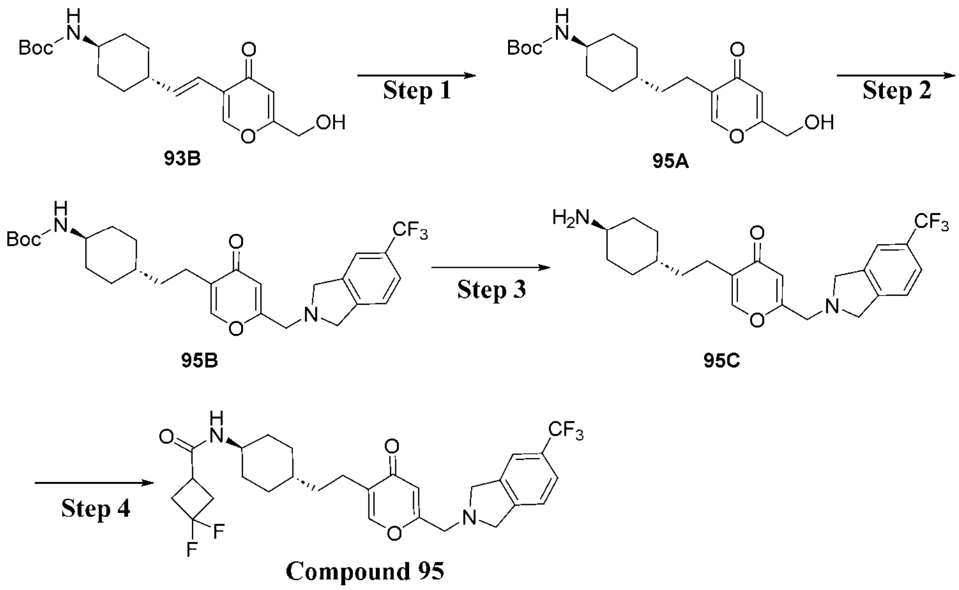


93B 95A 95B 95C

Compound 95

Step 1:

93B (0.90 g, 2.58 mmol) was dissolved in methanol (20 mL), and palladium on carbon (274.56 mg, 0.26 mmol) was added. After the addition, the reaction was subjected to hydrogen replacement three times, and reacted at room temperature overnight. After the reaction was completed, the reaction solution was concentrated and purified by column chromatography (petroleum ether/ethyl acetate = 1/2, (v/v)) to obtain compound 95A (520 mg, yield: 57%).

LC-MS (ESI): m/z= 295.3 $[M-56]^+$ .

Step 2:

95A (0.25 g, 0.71 mmol) was dissolved in dichloromethane (10 mL), and triethylamine (358.55 mg, 3.55 mmol)

and methanesulphonyl chloride (133.45 mg, 0.85 mmol) were added at 0°C-5°C. After the addition, the mixture was reacted at room temperature for 30 min, and 5-trifluoromethylisoindoline (157.21 mg, 0.84 mmol) was added at room temperature. After the addition, the mixture was reacted at room temperature overnight. After the reaction was completed, the reaction solution was concentrated and purified by column chromatography (petroleum ether/ethyl acetate = 3/2, (v/v)) to obtain compound 95B (300 mg, yield: 82%).

LC-MS (ESI): m/z= 521.3 $[M+H]^+$ .

Step 3:

95B (300 mg, 0.58 mmol) was dissolved in 1,4-dioxane (4 mL), and at 0°C-5°C, 4 N hydrogen chloride 1,4-dioxane solution (10 mL) was added dropwise. After the addition, the mixture was reacted at room temperature for 2 hrs. After the reaction was completed, the reaction mixture was concentrated to obtain crude 95C, which was directly used in the next reaction.

Step 4:

95C (242.70 mg, 0.58 mmol) was dissolved in dichloromethane (10 mL), and at 0°C-5°C, N-methylimidazole (238.09 mg, 2.90 mmol) and 3,3-difluorocyclobutane carboxylic acid (94.73 mg, 0.70 mmol) were successively added. The mixture was reacted at 0°C-5°C for 5 min, and then N,N,N',N'-tetramethylchloroformamidinium hexafluorophosphate (244.1 mg, 0.87 mmol) was then added at 0°C-5°C. After the addition, the mixture was reacted at room temperature overnight. After the reaction was completed, the reaction solution was concentrated and purified by preparative HPLC to obtain compound 95 (82.00 mg, yield: 26%).

LC-MS (ESI): m/z= 539.3 $[M+H]^+$.

$^1H$ NMR (400 MHz, DMSO-$d_6$) δ 8.08 (s, 1H), 7.85-7.83 (d, 1H), 7.63 (s, 1H), 7.58-7.56 (d, 1H), 7.48-7.46 (d, 1H), 6.31 (s, 1H), 4.03 (s, 4H), 3.80 (s, 2H), 3.51 - 3.43 (m, 1H), 2.83 - 2.75 (m, 1H), 2.69-2.60 (m, 4H), 2.26 - 2.22 (m, 2H), 1.79-1.74 (m, 4H), 1.36-1.30 (m, 2H), 1.16-1.07 (m, 3H), 1.00-0.84 (m, 2H).

**Example 96:**

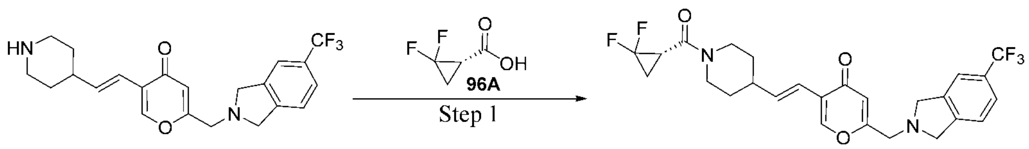


18E

Compound 96

Step 1:

Compound 18E (250 mg, 0.62 mmol) and 96A (110 mg, 0.90 mmol) were dissolved in N,N-dimethylformamide (10 mL), and N-methylimidazole (200 mg, 2.40 mmol) and TCFH (250 mg, 0.90 mmol) were successively added. After the addition, the mixture was reacted at room temperature for 3 h. Upon complete depletion of raw materials as monitored by LCMS, the reaction solution was concentrated, and purified by preparative HPLC to obtain compound 96 (65 mg, yield: 20%).

LC-MS (ESI): *m*/*z* = 509.50 $[M+H]^+$.

$^1H$ NMR (400 MHz, DMSO-$d_6$) δ 8.33 (s, 1H), 7.62 (s, 1H), 7.57 (d, 1H), 7.47 (d, 1H), 6.74-6.58 (m, 1H), 6.36 (s, 1H), 6.21-6.07 (m, 1H), 4.45-4.23 (m, 1H), 4.10-3.96 (m, 5H), 3.82 (s, 2H), 3.25-3.05 (m, 2H), 2.81-2.64 (m, 1H), 2.44-2.30 (m, 1H), 1.94-1.68 (m, 4H), 1.45-1.05 (m, 2H).

**Example 97:**

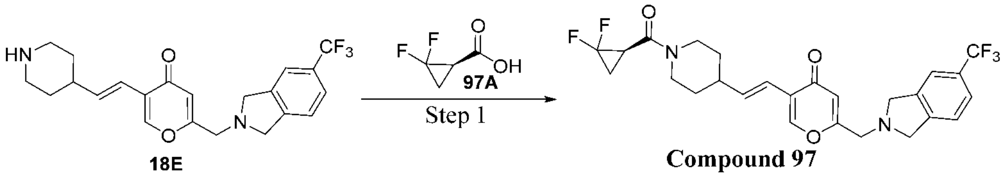


18E

Compound 97

Step 1:

Compound 18E (250 mg, 0.62 mmol) and 97A (110 mg, 0.90 mmol) were dissolved in N,N-dimethylformamide (10 mL), and N-methylimidazole (210 mg, 2.51 mmol) and TCFH (260 mg, 0.93 mmol) were successively added. After the addition, the mixture was reacted at room temperature for 3 h. Upon complete depletion of raw materials as monitored by LCMS, the reaction solution was concentrated, and purified by preparative HPLC to obtain compound 97 (60 mg, yield: 19%).

LC-MS (ESI): *m/z* = 509.60 $[M+H]^+$.

$^1H$ NMR (400 MHz, DMSO-$d_6$) δ 8.33 (s, 1H), 7.62 (s, 1H), 7.57 (d, 1H), 7.47 (d, 1H), 6.74-6.58 (m, 1H), 6.36 (s, 1H), 6.21-6.07 (m, 1H), 4.45-4.23 (m, 1H), 4.14-3.92 (m, 5H), 3.82 (s, 2H), 3.25-3.05 (m, 2H), 2.81-2.64 (m, 1H), 2.44-2.30 (m, 1H), 1.94-1.68 (m, 4H), 1.45-1.05 (m, 2H).

**Example 98:**

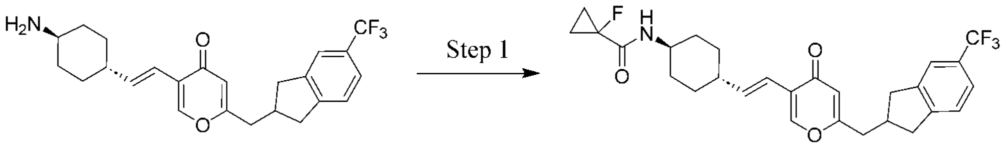


61D

98

Step 1:

**61D** (100.0 mg, 0.24 mmol) was dissolved in DMF (5 mL), and 1-fluorocyclopropane carboxylic acid (25.0 mg, 0.24 mmol) and N-methylimidazole (59.0 mg, 0.72 mmol) were added. At 0°C, N,N,N',N'-tetramethylchloroformamidinium hexafluorophosphate (84.0 mg, 0.3 mmol) was added and the resulting mixture was stirred at room temperature for 20 min. After the reaction was completed, water (10 mL) was added to the reaction solution, and the resulting mixture was extracted with ethyl acetate (15 mL×3). The organic layers were combined, washed with saturated sodium chloride aqueous solution (15 mL×3), dried over anhydrous sodium sulphate, filtered, and concentrated. The residue was separated and purified by silica gel column chromatography (dichloromethane: methanol (v/v) = 20:1) to obtain **compound 98** (40 mg, yield: 33%).

LC-MS (ESI): *m/z* = 504.2 $[M+H]^+$.

$^1H$ NMR (400 MHz, $CDCl_3$) δ 7.81 (s, 1H), 7.59 - 7.45 (m, 2H), 7.34 (d, 1H), 6.55 - 6.42 (m, 2H), 6.30 - 6.08 (m, 2H), 4.24 (s, 4H), 3.95 - 3.71 (m, 3H), 2.08 (d, 3H), 1.88 (d, 2H), 1.42 - 1.18 (m, 9H).

$^{19}F$ NMR (377 MHz, $CDCl_3$) δ -60.18 (s), -194.69 (s).

**Example 99:**

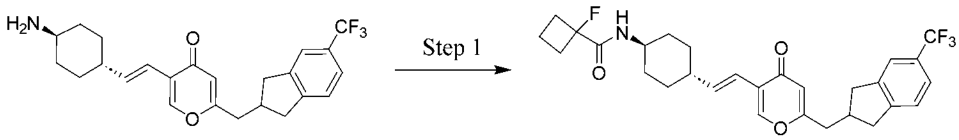


61D

99

Step 1:

**61D** (100.0 mg, 0.24 mmol) was dissolved in DMF (5 mL), and 1-fluorocyclobutane carboxylic acid (26.0 mg, 0.24 mmol) and N-methylimidazole (59.0 mg, 0.72 mmol) were added. At 0°C, N,N,N',N'-tetramethylchloroformamidinium hexafluorophosphate (84.0 mg, 0.3 mmol) was added and the resulting mixture was stirred at room temperature for 20 min. After the reaction was completed, water (10 mL) was added to the reaction solution, and the resulting mixture was extracted with ethyl acetate (15 mL×3). The organic layers were combined, washed with saturated sodium chloride aqueous solution (15 mL×3), dried over anhydrous sodium sulphate, filtered, and concentrated. The residue was separated and purified by silica gel column chromatography (dichloromethane: methanol (v/v) = 20:1) to obtain **compound 99** (45 mg, yield: 34%).
LC-MS (ESI): *m/z* = 518.2 [M+H]+.
$^1$H NMR (400 MHz, $CDCl_3$) δ 7.80 (s, 1H), 7.59 - 7.44 (m, 2H), 7.34 (d, 1H), 6.50 - 6.45 (m, 2H), 6.16 (d, 1H), 6.10 - 5.98 (m, 1H), 4.26 (s, 4H), 3.90 (s, 2H), 3.76 - 3.71 (m, 1H), 2.73 - 2.53 (m, 2H), 2.46 - 2.23 (m, 2H), 2.12 - 1.91 (m, 6H), 1.86 (d, 2H), 1.39 - 1.13 (m, 5H).
$^{19}$F NMR (377 MHz, $CDCl_3$) δ -60.20 (s), -142.50 (s).

**Example 100:**

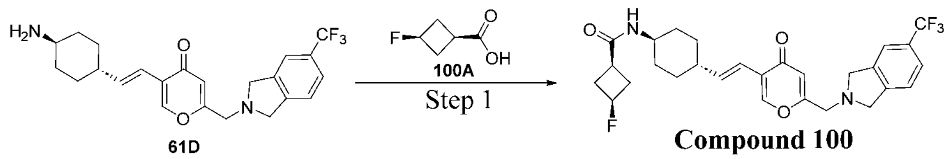

61D
Compound 100

Step 1:

Compound 61D (250 mg, 0.60 mmol) and 100A (92 mg, 0.78 mmol) were dissolved in N,N-dimethylformamide (10 mL), and N-methylimidazole (200 mg, 2.40 mmol) and TCFH (250 mg, 0.90 mmol) were successively added. After the addition, the mixture was reacted at room temperature for 3 h. Upon complete depletion of raw materials as monitored by LCMS, the reaction solution was concentrated, and purified by preparative HPLC to obtain compound 100 (105 mg, yield: 34%).
LC-MS (ESI): *m/z* = 519.60 [M+H]+.
$^1$H NMR (400 MHz, DMSO-$d_6$) δ 8.29 (s, 1H), 7.73 (d, 1H), 7.62 (s, 1H), 7.57 (d, 1H), 7.46 (d, 1H), 6.67-6.57 (m, 1H), 6.35 (s, 1H), 6.11 (d, 1H), 5.04-4.80 (m, 1H), 4.03 (s, 4H), 3.81 (s, 2H), 3.54-3.42 (m, 1H), 2.44-2.34 (m, 3H), 2.26-2.12 (m, 2H), 2.05-1.95 (m, 1H), 1.85-1.70 (m, 4H), 1.25-1.10 (m, 4H).

**Example 101:**

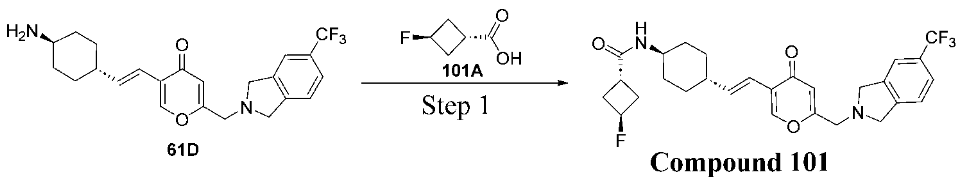

61D
Compound 101

Step 1:

Compound 61D (300 mg, 0.72 mmol) and 101A (100 mg, 0.85 mmol) were dissolved in N,N-dimethylformamide (10 mL), and N-methylimidazole (240 mg, 2.92 mmol) and TCFH (300 mg, 1.07 mmol) were successively added. After the addition, the mixture was reacted at room temperature for 3 h. Upon complete depletion of raw materials as monitored by LCMS, the reaction solution was concentrated, and purified by preparative HPLC to obtain compound 101 (130 mg, yield: 35%).
LC-MS (ESI): *m/z* = 519.60 [M+H]+.
$^1$H NMR (400 MHz, DMSO-$d_6$) δ 8.29 (s, 1H), 7.73 (d, 1H), 7.62 (s, 1H), 7.57 (d, 1H), 7.46 (d, 1H), 6.67-6.57 (m,

1H), 6.35 (s, 1H), 6.11 (d, 1H), 5.29-5.07 (m, 1H), 4.03 (s, 4H), 3.81 (s, 2H), 3.54-3.42 (m, 1H), 3.00-2.86 (m, 1H), 2.42-2.23 (m, 4H), 2.05-1.93 (m, 1H), 1.85-1.70 (m, 4H), 1.25-1.10 (m, 4H).

**Example 102:**

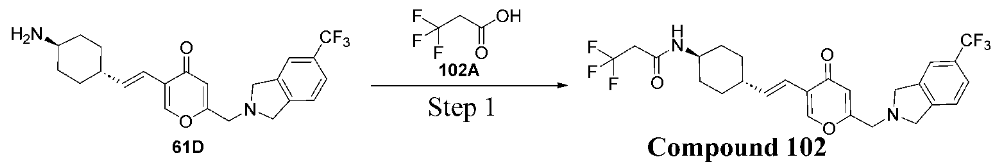


61D

Compound 102

Step 1:

Compound 61D (300 mg, 0.72 mmol) and 102A (140 mg, 1.08 mmol) were dissolved in N,N-dimethylformamide (10 mL), and N-methylimidazole (240 mg, 2.92 mmol) and TCFH (300 mg, 1.07 mmol) were successively added. After the addition, the mixture was reacted at room temperature for 3 h. Upon complete depletion of raw materials as monitored by LCMS, the reaction solution was concentrated, and purified by preparative HPLC to obtain compound 102 (110 mg, yield: 29%).

LC-MS (ESI): *m/z* = 529.50 $[M+H]^+$.

$^1H$ NMR (400 MHz, DMSO-$d_6$) δ 8.30 (s, 1H), 8.12 (d, 1H), 7.62 (s, 1H), 7.57 (d, 1H), 7.46 (d, 1H), 6.69-6.58 (m, 1H), 6.35 (s, 1H), 6.11 (d, 1H), 4.03 (s, 4H), 3.81 (s, 2H), 3.54-3.42 (m, 1H), 3.23-3.15 (m, 2H), 2.08-1.97 (m, 1H), 1.87-1.72 (m, 4H), 1.26-1.17 (m, 4H).

**Example 103**

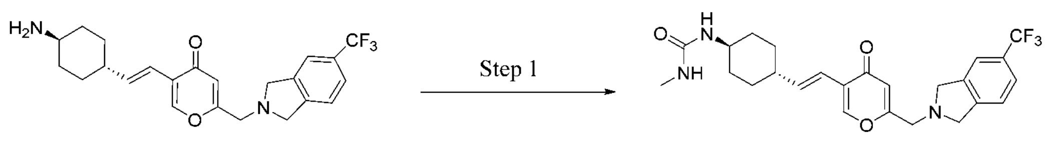


61D

Compound 103

Step 1:

61D (350.0 mg, 0.84 mmol) and N,N-diisopropylethylamine (330.0 mg, 2.52 mmol) were dissolved in dichloromethane (10 mL), the mixture was stirred at 0°C, and then methylaminoformyl chloride (120.0 mg, 1.26 mmol) was added. After the addition, the mixture was reacted at room temperature overnight. After the reaction was completed as monitored by LC-MS, water (20 mL) was added, and the mixture was extracted with dichloromethane (30 mL×2). The organic layer was dried over anhydrous sodium sulphate and filtered, and the filtrate was concentrated under reduced pressure. The residue was separated and purified by column chromatography (petroleum ether/ethyl acetate = 1/1) to obtain compound 103 (52.0 mg, yield: 13.0%).

$^1H$ NMR (400 MHz, $CDCl_3$) δ 7.80 (s, 1H), 7.49 (d, 1H), 7.45 (s, 1H), 7.30 (d, 1H), 6.47-6.40 (m, 2H), 6.17 (d, 1H), 4.19-4.09 (m, 6H), 3.78 (s, 2H), 3.52-3.47 (m, 1H), 2.76 (d, 3H), 2.09-2.05 (m, 3H), 1.86-1.83 (m, 2H), 1.35-1.26 (m, 2H), 1.19-1.11 (m, 2H), LC-MS (ESI): m/z = 476.5 $[M+H]^+$.

**Example 104:**

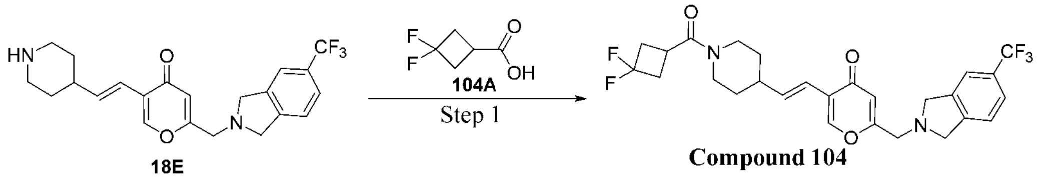


18E 104A Compound 104

Step 1:

Compound 18E (250 mg, 0.62 mmol) and 104A (130 mg, 0.92 mmol) were dissolved in N,N-dimethylformamide (10 mL), and N-methylimidazole (210 mg, 2.51 mmol) and TCFH (260 mg, 0.93 mmol) were successively added. After the addition, the mixture was reacted at room temperature for 3 h. Upon complete depletion of raw materials as monitored by LCMS, the reaction solution was concentrated, and purified by preparative HPLC to obtain compound 104 (60 mg, yield: 19%).

LC-MS (ESI): *m/z* = 523.20 [M+H]$^+$.

$^1$H NMR (400 MHz, DMSO-$d_6$) δ 8.31 (s, 1H), 7.62 (s, 1H), 7.57 (d, 1H), 7.47 (d, 1H), 6.74-6.58 (m, 1H), 6.36 (s, 1H), 6.14 (d, 1H), 4.44-4.27 (m, 1H), 4.03 (s, 4H), 3.85-3.70 (m, 3H), 3.27-3.21 (m, 1H), 3.10-2.94 (m, 1H), 2.82-2.63 (m, 5H), 2.38-2.26 (m, 1H), 1.76-1.65 (m, 2H), 1.30-1.14 (m, 2H).

**Example 105:**

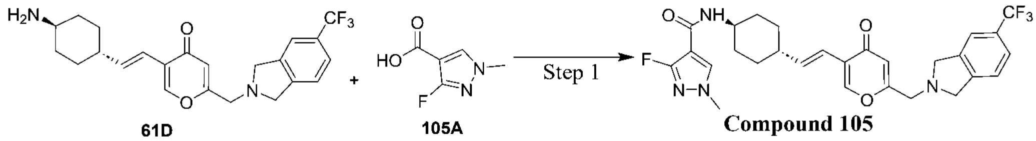


61D 105A Compound 105

Step 1:

Compound 105A (200 mg, 1.39 mmol) was dissolved in thionyl chloride (5 mL), and the mixture was heated to 60°C and reacted for 2 h. After the reaction was completed, the reaction solution was concentrated and diluted with dichloromethane (5 mL), and the mixture was added to a solution of compound 61D (400 mg, 0.96 mmol) and triethylamine (437 mg, 4.32 mmol) in dichloromethane (15 mL). At room temperature, the resulting mixture was reacted for 0.5 h. After the reaction was completed as monitored by TLC (dichloromethane: methanol = 20:1 (v/v)), water (40 mL) was added, and the mixture was extracted with dichloromethane (50 mL×3), dried over anhydrous sodium sulphate, filtered, and concentrated under reduced pressure. The residue was purified by column chromatography (dichloromethane: methanol = 65:1 (v/v)) to obtain a crude compound, which was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; and mobile phase B: water (containing 0.1% trifluoroacetic acid); gradient: isocratic elution using 30%-70% acetonitrile; cycle time: 15 minutes) to obtain compound 105 (12 mg, yield: 2.3%).

LCMS *m/z* = 545.5 [M+1]$^+$

$^1$H NMR (400 MHz, MeOD-$d_4$) δ 8.24 (s, 1H), 7.77 - 7.69 (m, 2H), 7.60 (d, 1H), 6.66 - 6.57 (m, 2H), 6.33 (d, 1H), 6.22 (d, 1H), 4.75 (s, 4H), 4.58 (s, 2H), 3.97 (s, 3H), 3.83 - 3.74 (m, 1H), 2.21 - 2.07 (m, 1H), 1.96 (dd, 4H), 1.52 - 1.24 (m, 4H).

**Example 106:**

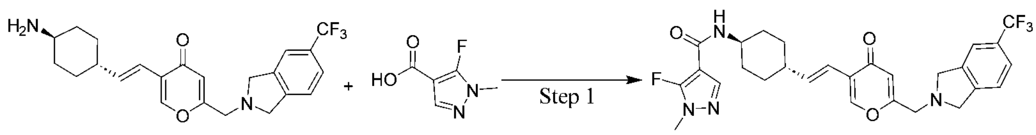


61D 106A Compound 106

Step 1:

Compound 106A (214 mg, 1.49 mmol) was dissolved in N,N-dimethylformamide (15 mL), and in an ice-water bath, N,N-diisopropylethylamine (692 mg, 5.35 mmol) and HATU (679 mg, 1.78 mmol) were successively added. After the addition, the mixture was reacted at room temperature for 1 h, and to the above-mentioned solution was added 61D (500 mg, 1.19 mmol). After the addition, the mixture was reacted at room temperature for 4 h. After the reaction was completed as monitored by TLC (dichloromethane:methanol = 20:1 (v/v)), water (45 mL) was added, and the resulting mixture was extracted with ethyl acetate (50 mL×3). The organic phases were combined, washed with saturated sodium chloride aqueous solution (40 mL×1), dried over anhydrous sodium sulphate, filtered, and concentrated under reduced pressure. The residue was purified by column chromatography (dichloromethane:methanol = 65:1 (v/v)) to obtain a crude compound, which was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; and mobile phase B: water (containing 0.1% ammonium acetate); gradient: isocratic elution using 30%-70% acetonitrile; cycle time: 15 minutes) to obtain compound 106 (145 mg, yield: 22%).

LCMS *m/z* = 545.3 $[M+1]^+$

$^1$H NMR (400 MHz, DMSO-$d_6$) δ 8.31 (s, 1H), 7.82 (d, 1H), 7.73 (d, 1H), 7.63 (s, 1H), 7.57 (d, 1H), 7.47 (d, 1H), 6.65 (dd, 1H), 6.36 (s, 1H), 6.13 (d, 1H), 4.04 (s, 4H), 3.82 (s, 2H), 3.69 (s, 3H), 3.68- 3.61 (m, 1H), 2.06 - 1.97 (m, 1H), 1.88 - 1.76 (m, 4H), 1.40 - 1.29 (m, 2H), 1.27 - 1.18(m, 2H)

**Example 107:**

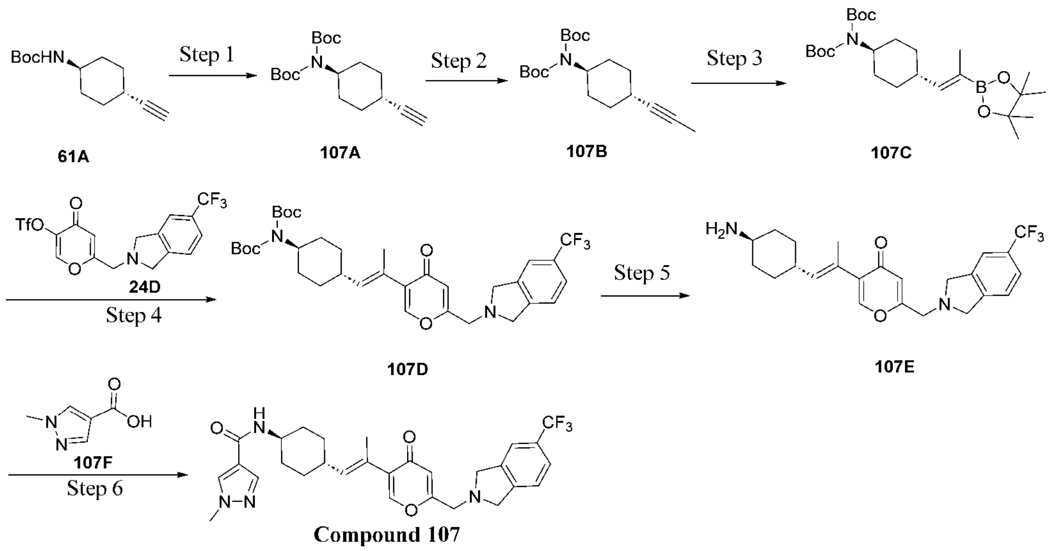


61A, 107A, 107B, 107C, 24D, 107D, 107E, 107F, Compound 107

Step 1:

Compound 61A (5.0 g, 22.39 mmol) was dissolved in tetrahydrofuran (50 mL), and then di-tert-butyl dicarbonate (12.0 g, 55.06 mmol) and DMAP (0.27 g, 2.24 mmol) were successively added. After the addition, the mixture was warmed to 65°C and reacted for 12 h. After the completion of the reaction, the mixture was diluted with water, and extracted with ethyl acetate, and the organic phases were combined, washed with saturated sodium chloride aqueous solution, dried over anhydrous sodium sulphate, filtered, and concentrated to obtain a crude, which was purified by column chromatography (eluents: petroleum ether:ethyl acetate =10:1) to obtain the target compound 107A (3.5 g, yield: 48%).

Step 2:

Compound 107A (3.0 g, 9.28 mmol) was dissolved in tetrahydrofuran (50 mL), and at -78°C, n-butyllithium (5.5 mL, 13.92 mmol) was slowly added dropwise. The mixture was stirred for 30 min, and then iodomethane (2.63g, 18.56 mmol) was added. After the addition, the mixture was gradually warmed to room temperature and reacted for 3 h. After the completion of the reaction, saturated ammonium chloride solution was added to quench the reaction. The resulting mixture

was extracted with ethyl acetate, and the organic phases were combined, washed with saturated sodium chloride aqueous solution, dried over anhydrous sodium sulphate, filtered and concentrated. The resulting crude was purified by column chromatography (eluents: petroleum ether:ethyl acetate=10:1) to obtain the target compound 107B (1.3 g, yield: 41%).

Step 3:

Compound 107B (0.8 g, 2.37 mmol) was dissolved in dry toluene (15 mL), pinacolborane (1.51 g, 11.83 mmol) was added dropwise, and bis(cyclopentadienyl)zirconium chloride hydride (0.061 g, 0.24 mmol) was added. After the addition, the system was subjected to nitrogen replacement, and stirred and reacted at 65°C for 18 h. After the reaction was completed as monitored by TLC (petroleum ether: ethyl acetate = 5:1 (v/v)), the reaction solution was concentrated to obtain compound 107C (crude), which was directly used in the next step.

Step 4:

Compound 107C (1.10 g, 2.36 mmol) and 24D (0.40 g, 0.90 mmol) were dissolved in 1,4-dioxane (20 mL), and [1,1'-bis(diphenylphosphino)ferrocene]palladium dichloride (II) (0.066 g, 0.09 mmol), potassium carbonate (0.37 g, 2.70 mmol) and water (4 mL) were successively added. After the addition, the mixture was subjected to nitrogen replacement three times, and stirred and reacted at 100°C for 6 h. After the completion of the reaction, water (30 mL) was added, the resulting mixture was extracted with ethyl acetate (50 mL×3), and the organic phases were combined, washed with saturated sodium chloride aqueous solution (40 mL×1), dried over anhydrous sodium sulphate and filtered. The filtrate was concentrated under reduced pressure and purified by column chromatography (petroleum ether:ethyl acetate = 2:1 (v/v)) to obtain compound 107D (0.25 g, yield: 44%).

LC-MS (ESI): m/z = 477.30 $[M-156+H]^+$.

Step 5:

Compound 107D (150 mg, 0.24 mmol) was dissolved in dichloromethane (5 mL), hydrochloric acid/1,4-dioxane solution (5 mL) was added, and the mixture was reacted at room temperature for 2 h. After the reaction was completed as monitored by TLC (petroleum ether: ethyl acetate = 1:1 (v/v)), the reaction solution was concentrated to obtain compound 107E (110 mg, crude), which was directly used in the next step without purification.

LC-MS (ESI): m/z = 217.10 $[M/2+H]^+$.

Step 6:

Compound 107F (44 mg, 0.35 mmol) was dissolved in DMF (5 mL), N-methylimidazole (76 mg, 0.92 mmol) and TCFH (97 mg, 0.35 mmol) were added, and the resulting mixture was stirred at room temperature for 10 min. 107E (100 mg, 0.23 mmol) was subsequently added and the mixture was reacted at room temperature for 2 h. After the reaction was completed, water (25 mL) was added to the reaction solution, and the resulting mixture was extracted with ethyl acetate (15 mL×3). The organic layers were combined, washed with saturated sodium chloride aqueous solution (25 mL), dried over anhydrous sodium sulphate, filtered, and concentrated. The residue was separated and purified by silica gel column chromatography (dichloromethane: methanol (v/v) = 15:1) to obtain compound 107 (30 mg, yield: 24%).

LC-MS (ESI): m/z = 541.30 $[M+H]^+$.

$^1H$ NMR (400 MHz, DMSO-$d_6$) δ 8.09 (s, 1H), 8.03 (s, 1H), 7.82 (s, 1H), 7.76 (d, 1H), 7.63 (s, 1H), 7.57 (d, 1H), 7.47 (d, 1H), 6.32 (s, 1H), 5.54 (d, 1H), 4.04 (s, 4H), 3.83 (s, 3H), 3.81 (s, 2H), 3.72-3.62 (m, 1H), 2.30-2.19 (m, 1H), 1.89-1.80 (m, 5H), 1.73-1.65 (m, 2H), 1.42-1.28 (m, 2H), 1.25-1.15 (m, 2H).

**Example 108:**

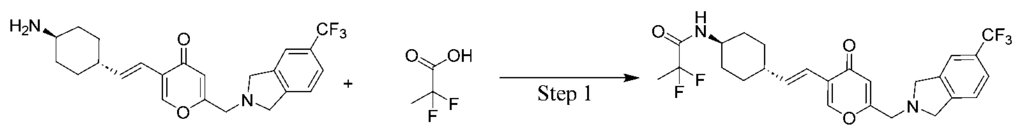


61D 108A Compound 108

Step 1:

Compound 108A (220 mg, 2.00 mmol) was dissolved in N,N-dimethylformamide (15 mL), and in an ice-water bath, N,N-diisopropylethylamine (582 mg, 4.50 mmol) and HATU (837 mg, 2.20 mmol) were successively added. After the addition, the mixture was reacted at room temperature for 30 min, and to the above-mentioned solution was added 61D (418 mg, 1.00 mmol). After the addition, the mixture was reacted at room temperature for 4 h. After the reaction was completed as monitored by TLC (dichloromethane: methanol = 20:1 (v/v)), water (45 mL) was added, and the resulting mixture was extracted with ethyl acetate (50 mL×3). The organic phases were combined, washed with saturated sodium chloride aqueous solution (40 mL×1), dried over anhydrous sodium sulphate, filtered, and concentrated under reduced pressure. The residue was purified by column chromatography (dichloromethane: methanol = 65:1 (v/v)) to obtain a crude compound, which was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; and mobile phase B: water (containing 0.1% ammonium acetate); gradient: isocratic elution using 30%-70% acetonitrile; cycle time: 15 minutes) to obtain compound 108 (248 mg, yield: 49%).

LCMS *m/z* = 511.5 $[M+1]^+$
$^1$H NMR (400 MHz, DMSO-$d_6$) δ 8.46 (d, 1H), 8.30 (s, 1H), 7.62 (s, 1H), 7.57 (d, 1H), 7.47 (d, 1H), 6.64 (dd, 1H), 6.35 (s, 1H), 6.11 (d, 1H), 4.04 (s, 4H), 3.81 (s, 2H), 3.62 - 3.53 (m, 1H), 2.06 - 1.95 (m, 1H), 1.81 - 1.65 (m, 7H), 1.39 (dd, 2H), 1.29 - 1.14 (m, 2H).
$^{19}$F NMR (376 MHz, DMSO-$d_6$) δ -58.35 (s), -95.36 (s), -95.42 (s).

Example 109:

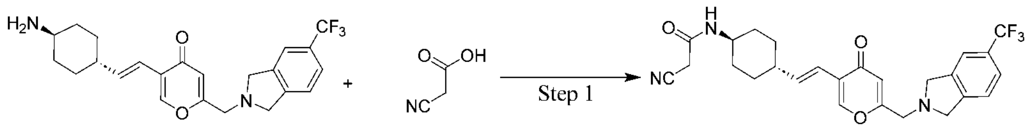

61D
109A
Compound 109

Step 1:

Compound 109A (170 mg, 2.00 mmol) was dissolved in N,N-dimethylformamide (15 mL), and in an ice-water bath, N,N-diisopropylethylamine (582 mg, 4.50 mmol) and HATU (837 mg, 2.20 mmol) were successively added. After the addition, the mixture was reacted at room temperature for 30 min, and to the above-mentioned solution was added 61D (418 mg, 1.00 mmol). After the addition, the mixture was reacted at room temperature for 4 h. After the reaction was completed as monitored by TLC (dichloromethane: methanol = 20:1 (v/v)), water (45 mL) was added, and the resulting mixture was extracted with ethyl acetate (50 mL×3). The organic phases were combined, washed with saturated sodium chloride aqueous solution (40 mL×1), dried over anhydrous sodium sulphate, filtered, and concentrated under reduced pressure. The residue was separated and purified by column chromatography (dichloromethane: methanol = 70:1 (v/v)), and concentrated to obtain a solid, which was slurried with ethyl acetate: dichloromethane = 20:1 (v/v), and filtered. The solid was dried to obtain compound 109 (68 mg, yield: 14%).

LCMS *m/z* = 486.7 $[M+1]^+$
$^1$H NMR (400 MHz, DMSO-$d_6$) δ 8.30 (s, 1H), 8.12 (d, 1H), 7.62 (s, 1H), 7.57 (d, 1H), 7.47 (d, 1H), 6.63 (dd, 1H), 6.35 (s, 1H), 6.11 (d, 1H), 4.03 (s, 4H), 3.81 (s, 2H), 3.56 (s, 2H), 3.54 - 3.42 (m, 1H), 2.08 - 1.98 (m, 1H), 1.89 - 1.80 (m, 2H), 1.78 - 1.72 (m, 2H), 1.26 - 1.18 (m, 4H).
$^{19}$F NMR (376 MHz, DMSO-$d_6$) δ -58.35 (s).

**Example 110:**

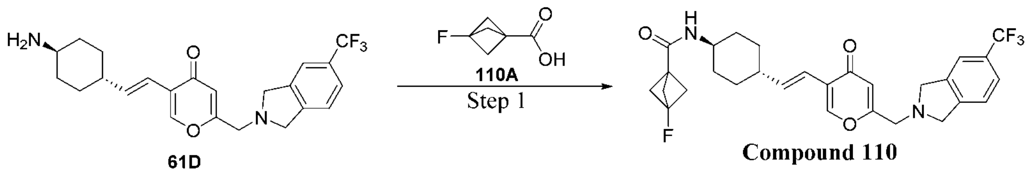


61D

Compound 110

Step 1:

Compound 110A (120 mg, 0.92 mmol) was dissolved in DMF (10 mL), N-methylimidazole (200 mg, 2.44 mmol) and TCFH (340 mg, 1.21 mmol) were added, and the resulting mixture was stirred at room temperature for 10 min. 61D (250 mg, 0.60 mmol) was subsequently added and the mixture was reacted at room temperature for 2 h. After the reaction was completed, water (30 mL) was added to the reaction solution, and the resulting mixture was extracted with ethyl acetate (20 mL×3). The organic layers were combined, washed with saturated sodium chloride aqueous solution (30 mL), dried over anhydrous sodium sulphate, filtered, and concentrated. The residue was separated and purified by silica gel column chromatography (dichloromethane:methanol (v/v) = 15:1) to obtain compound 110 (100 mg, yield: 31%).

LC-MS (ESI): m/z = 531.20 $[M+H]^+$.

$^1H$ NMR (400 MHz, DMSO-$d_6$) δ 8.29 (s, 1H), 7.68 (d, 1H), 7.62 (s, 1H), 7.57 (d, 1H), 7.47 (d, 1H), 6.68-6.55 (m, 1H), 6.35 (s, 1H), 6.10 (d, 1H), 4.03 (s, 4H), 3.81 (s, 2H), 3.57-3.46 (m, 1H), 2.23 (d, 6H), 2.04-1.94 (m, 1H), 1.80-1.70 (m, 4H), 1.34-1.12 (m, 4H).

**Example 111:**

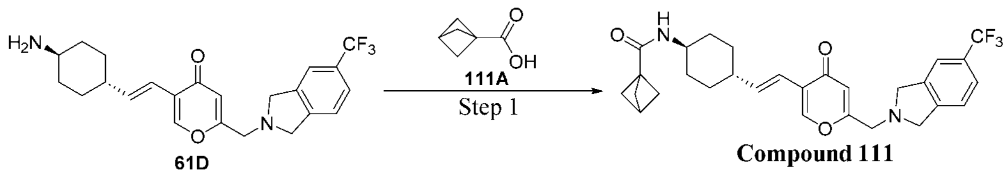


61D

Compound 111

Step 1:

Compound 111A (100 mg, 0.89 mmol) was dissolved in DMF (10 mL), N-methylimidazole (200 mg, 2.44 mmol) and TCFH (340 mg, 1.21 mmol) were added, and the resulting mixture was stirred at room temperature for 10 min. 61D (250 mg, 0.60 mmol) was subsequently added and the mixture was reacted at room temperature for 2 h. After the reaction was completed, water (30 mL) was added to the reaction solution, and the resulting mixture was extracted with ethyl acetate (20 mL×3). The organic layers were combined, washed with saturated sodium chloride aqueous solution (30 mL), dried over anhydrous sodium sulphate, filtered, and concentrated. The residue was separated and purified by silica gel column chromatography (dichloromethane:methanol (v/v) = 15:1) to obtain compound 111 (70 mg, yield: 23%).

LC-MS (ESI): m/z = 513.30 $[M+H]^+$.

$^1H$ NMR (400 MHz, DMSO-$d_6$) δ 8.30 (s, 1H), 7.63 (s, 1H), 7.57 (d, 1H), 7.47 (d, 1H), 7.43 (d, 1H), 6.67-6.57 (m, 1H), 6.35 (s, 1H), 6.11 (d, 1H), 4.03 (s, 4H), 3.81 (s, 2H), 3.52-3.42 (m, 1H), 2.38 (s, 1H), 2.04-1.96 (m, 1H), 1.92 (s, 6H), 1.79-1.70 (m, 4H), 1.34-1.11 (m, 4H).

Example 112

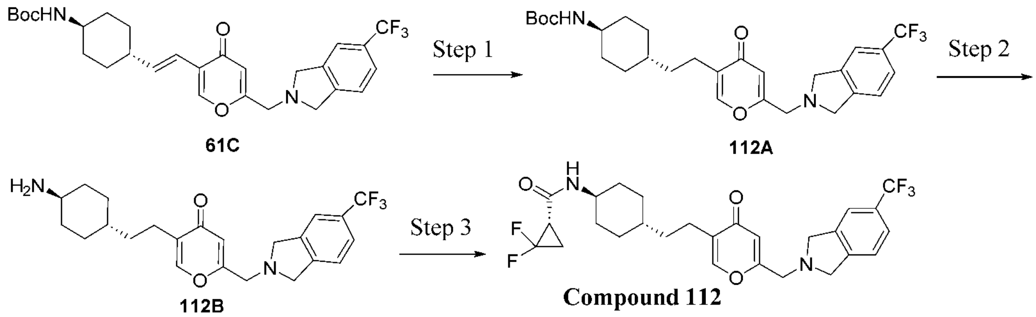


61C 112A

112B Compound 112

Step 1:

**61C** (1.0 g, 1.93 mmol), methanol (9 mL), ethyl acetate (9 mL) and dichloromethane (3 mL) were successively added to a single-neck flask, and then 10% palladium on carbon (100.0 mg) was added. After the addition, the mixture was subjected to hydrogen replacement three times, and reacted at room temperature for 0.5 hours. After the reaction was completed as monitored by LC-MS, the mixture was subjected to suction filtration, and the filter cake was washed with dichloromethane (20 mL×2) and concentrated to obtain compound **112A** (1.0 g, yield: 99.5%).

LC-MS (ESI): m/z = 465.5 $[M-56+H]^+$.

Step 2:

Compound **112A** (1.0 g, 1.92 mmol) was dissolved in dichloromethane (20 mL) and 4 M hydrogen chloride dioxane solution (20 mL) was added. After the addition, the mixture was reacted at room temperature for 2 hours. After the reaction was completed as monitored by LC-MS, the reaction solution was concentrated to obtain compound **112B** (940 mg, crude), which was directly used in the next step without purification.

LC-MS (ESI): m/z = 421.4 $[M+H]^+$.

Step 3:

**112B** (470.0 mg, 1.12 mmol) and (1S)-2,2-difluorocyclopropane-1-carboxylic acid (180.0 mg, 1.46 mmol) were dissolved in N,N-dimethylformamide (15 mL), and the mixture was stirred at 0°C. N-methylimidazole (550.0 mg, 6.72 mmol) was then added, and N,N,N',N'-tetramethylchloroformamidinium hexafluorophosphate (470.0 mg, 1.68 mmol) was slowly added. After the addition, the resulting mixture was reacted at room temperature for 3 hours. After the reaction was completed as monitored by LC-MS, water (30 mL) was added, and the mixture was extracted with ethyl acetate (30 mL×2). The organic layer was dried over anhydrous sodium sulphate and filtered, and the filtrate was concentrated under reduced pressure. The residue was separated and purified by column chromatography (petroleum ether/ethyl acetate = 1/1) to obtain compound **112** (205.0 mg, yield: 34.9%).

$^1H$ NMR (400 MHz, $CDCl_3$) δ 7.62 (s, 1H), 7.50 (d, 1H), 7.46 (s, 1H), 7.31 (d, 1H), 6.39 (s, 1H), 5.49 (d, 1H), 4.13 (s, 4H), 3.79-3.72 (m, 3H), 2.38-2.34 (m, 2H), 2.24-2.16 (m, 1H), 2.12-2.00 (m, 3H), 1.85-1.83 (m, 2H), 1.68-1.59 (m, 1H), 1.45-1.39 (m, 2H), 1.28-1.24 (m, 1H), 1.19-1.01 (m, 4H), LC-MS (ESI): m/z = 525.1 $[M+H]^+$.

**Example 113**

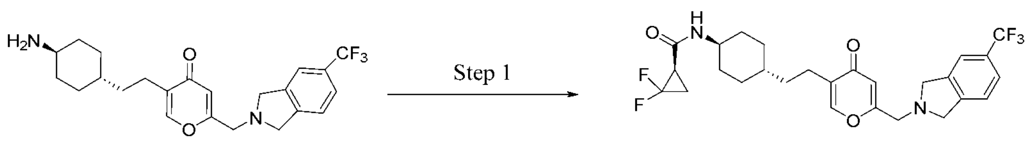


112B Compound 113

Step 1:

**112B** (470.0 mg, 1.12 mmol) and (1R)-2,2-difluorocyclopropane-1-carboxylic acid (180.0 mg, 1.46 mmol) were dissolved in N,N-dimethylformamide (15 mL), and the mixture was stirred at 0°C. N-methylimidazole (550.0 mg, 6.72 mmol) was then added, and N,N,N',N'-tetramethylchloroformamidinium hexafluorophosphate (470.0 mg, 1.68 mmol) was

slowly added. After the addition, the resulting mixture was reacted at room temperature for 3 hours. After the reaction was completed as monitored by LC-MS, water (30 mL) was added, and the mixture was extracted with ethyl acetate (30 mL×2). The organic layer was dried over anhydrous sodium sulphate and filtered, and the filtrate was concentrated under reduced pressure. The residue was separated and purified by column chromatography (petroleum ether/ethyl acetate = 1/1) to obtain compound **113** (210.0 mg, yield: 35.8%).

$^1$H NMR (400 MHz, $CDCl_3$) δ 7.62 (s, 1H), 7.50 (d, 1H), 7.46 (s, 1H), 7.31 (d, 1H), 6.38 (s, 1H), 5.51 (d, 1H), 4.12 (s, 4H), 3.79-3.72 (m, 3H), 2.38-2.34 (m, 2H), 2.24-2.16 (m, 1H), 2.12-2.00 (m, 3H), 1.85-1.83 (m, 2H), 1.68-1.59 (m, 1H), 1.45-1.39 (m, 2H), 1.28-1.24 (m, 1H), 1.19-1.01 (m, 4H), LC-MS (ESI): m/z = 525.1 $[M+H]^+$.

**Example 114:**

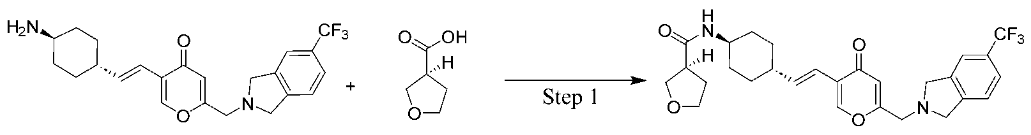


61D 114A Compound 114

Step 1:

Compound 114A (232 mg, 2.00 mmol) was dissolved in N,N-dimethylformamide (15 mL), and in an ice-water bath, N,N-diisopropylethylamine (582 mg, 4.50 mmol) and HATU (837 mg, 2.20 mmol) were successively added. After the addition, the mixture was reacted at room temperature for 30 min, and to the above-mentioned solution was added 61D (418 mg, 1.00 mmol). After the addition, the mixture was reacted at room temperature for 4 h. After the reaction was completed as monitored by TLC (dichloromethane:methanol = 20:1 (v/v)), water (45 mL) was added, and the resulting mixture was extracted with ethyl acetate (50 mL×3). The organic phases were combined, washed with saturated sodium chloride aqueous solution (40 mL×1), dried over anhydrous sodium sulphate, filtered, and concentrated under reduced pressure. The residue was purified by column chromatography (dichloromethane:methanol = 65:1 (v/v)) to obtain a crude compound, which was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; and mobile phase B: water (containing 0.1% trifluoroacetic acid); gradient: isocratic elution using 20%-80% acetonitrile; cycle time: 15 minutes) to obtain compound 114 (156 mg, yield: 30%).

LCMS *m/z* = 517.5 $[M+1]^+$
$^1$H NMR (400 MHz, DMSO-$d_6$) δ 8.35 (s, 1H), 7.79 (d, 1H), 7.75 (s, 1H), 7.69 (d, 1H), 7.57 (d, 1H), 6.67 (dd, 1H), 6.51 (s, 1H), 6.12 (d, 1H), 4.52 (s, 4H), 4.32 (s, 2H), 3.82 (t, 1H), 3.71 (dd, 1H), 3.64 (dd, 1H), 3.60 - 3.54 (m, 1H), 3.54 - 3.43 (m, 1H), 2.91 - 2.83 (m, 1H), 2.09 - 1.98 (m, 1H), 1.95 (dd, 2H), 1.87 - 1.70 (m, 4H), 1.28 - 1.13 (m, 4H).
$^{19}$F NMR (376 MHz, DMSO-$d_6$) δ -58.62 (s).

**Example 115:**

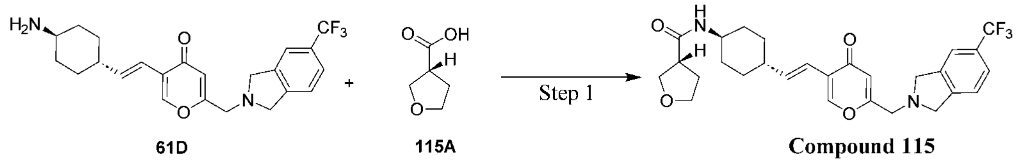


61D 115A Compound 115

Step 1:

Compound 115A (232 mg, 2.00 mmol) was dissolved in N,N-dimethylformamide (15 mL), and in an ice-water bath, N,N-diisopropylethylamine (582 mg, 4.50 mmol) and HATU (837 mg, 2.20 mmol) were successively added. After the addition, the mixture was reacted at room temperature for 30 min, and to the above-mentioned solution was added 61D (418 mg, 1.00 mmol). After the addition, the mixture was reacted at room temperature for 4 h. After the reaction was completed as monitored by TLC (dichloromethane:methanol= 20:1 (v/v)), water (45 mL) was added, and the resulting

mixture was extracted with ethyl acetate (50 mL×3). The organic phases were combined, washed with saturated sodium chloride aqueous solution (40 mL×1), dried over anhydrous sodium sulphate, filtered, and concentrated under reduced pressure. The residue was separated and purified by column chromatography (dichloromethane:methanol = 65:1 (v/v)), and concentrated to obtain a solid, which was slurried with ethyl acetate:dichloromethane = 20:1 (v/v), and filtered. The solid was dried to obtain compound 115 (182 mg, yield: 35%).

LCMS *m/z* = 517.5 [M+1]$^+$
$^1$H NMR (400 MHz, DMSO-$d_6$) δ 8.30 (s, 1H), 7.78 (d, 1H), 7.63 (s, 1H), 7.57 (d, 1H), 7.47 (d, 1H), 6.63 (dd, 1H), 6.35 (s, 1H), 6.11 (d, 1H), 4.03 (s, 4H), 3.86 - 3.78 (m, 3H), 3.71 (dd, 1H), 3.64 (dd, 1H), 3.60 - 3.54 (m, 1H), 3.53 - 3.43 (m, 1H), 2.91 - 2.83 (m, 1H), 2.04 - 1.98 (m, 1H), 1.94 (dd, 2H), 1.86 - 1.71 (d, 4H), 1.27 - 1.14 (m, 4H).
$^{19}$F NMR (376 MHz, DMSO) δ -58.34 (s).

**Example 116:**

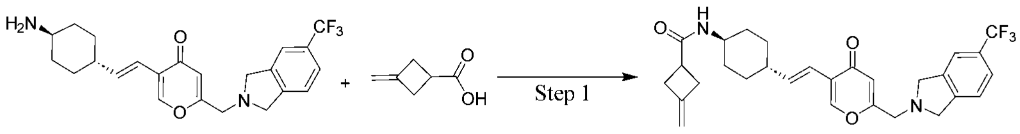


61D 116A Compound 116

Step 1:

Compound 116A (224 mg, 2.00 mmol) was dissolved in N,N-dimethylformamide (15 mL), and in an ice-water bath, N,N-diisopropylethylamine (582 mg, 4.50 mmol) and HATU (837 mg, 2.20 mmol) were successively added. After the addition, the mixture was reacted at room temperature for 30 min, and to the above-mentioned solution was added 61D (418 mg, 1.00 mmol). After the addition, the mixture was reacted at room temperature for 4 h. After the reaction was completed as monitored by TLC (dichloromethane:methanol = 20:1 (v/v)), water (45 mL) was added, and the resulting mixture was extracted with ethyl acetate (50 mL×3). The organic phases were combined, washed with saturated sodium chloride aqueous solution (40 mL×1), dried over anhydrous sodium sulphate, filtered, and concentrated under reduced pressure. The residue was separated and purified by column chromatography (dichloromethane:methanol = 70:1 (v/v)), and concentrated to obtain a solid, which was slurried with ethyl acetate:dichloromethane = 20:1 (v/v), and filtered. The solid was dried to obtain compound 116 (158 mg, yield: 31%).

LCMS *m/z* = 513.5 [M+1]$^+$
$^1$H NMR (400 MHz, DMSO-$d_6$) δ 8.30 (s, 1H), 7.66 (d, 1H), 7.63 (s, 1H), 7.57 (d, 1H), 7.47 (d, 1H), 6.63 (dd, 1H), 6.35 (s, 1H), 6.11 (d, 1H), 4.78 - 4.68 (m, 2H), 4.03 (s, 4H), 3.81 (s, 2H), 3.55 - 3.42 (m, 1H), 2.98 - 2.90 (m, 1H), 2.83 - 2.72 (m, 2H), 2.72 - 2.62 (m, 2H), 2.06 - 1.92 (m, 1H), 1.86 - 1.69 (m, 4H), 1.25 - 1.17 (m, 4H).
$^{19}$F NMR (376 MHz, DMSO) δ -58.34 (s).

**Example 117**

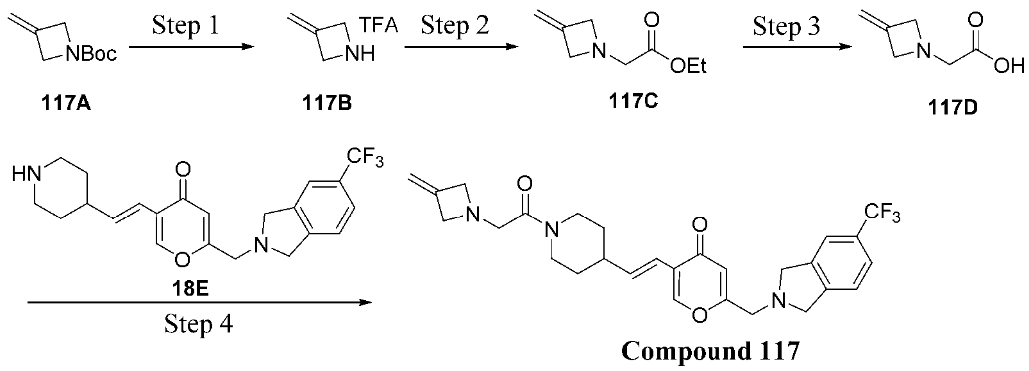


117A 117B 117C 117D 18E Compound 117

Step 1

117A (1.7 g, 10.0 mmol) was dissolved in DCM (20 mL), trifluoroacetic acid (5 mL) was added, and the mixture was stirred at room temperature for 1 h. After the reaction was completed, the reaction solution was concentrated, and the residue was separated and purified by silica gel column chromatography (dichloromethane/methanol (v/v) = 10/1) to obtain 117B (1.5 g, 88%).

LC-MS (ESI): m/z = 70.1 $[M+H]^+$.

Step 2

117B (1.5 g, 8.8 mmol) was dissolved in acetonitrile (30 mL), potassium carbonate (1.4 g, 10 mmol) and ethyl chloroacetate (1.2 g, 10.0 mmol) were successively added, and the mixture was reacted at 70°C for 4 h. After the reaction was completed, the reaction solution was concentrated, water (20 mL) was added, the resulting mixture was extracted with EA (30 mL×2), and the organic layers were combined, dried over anhydrous sodium sulphate, filtered and concentrated. The residue was separated and purified by silica gel column chromatography (petroleum ether: ethyl acetate (v/v) = 4/1) to obtain 117C (0.8 g, 58%).

LC-MS (ESI): m/z = 156.2 $[M+H]^+$.

Step 3

117C (0.8 g, 5.2 mmol) was dissolved in a mixed solution of THF/MeOH/$H_2O$ = (1/1/1) (20 mL), lithium hydroxide (1.0 g, 25.0 mmol) was added, and the mixture was reacted at room temperature for 2 h. After the reaction was completed, the organic solvent was removed by distillation under reduced pressure. The solution was adjusted to pH 3 with diluted hydrochloric acid, and freeze-dried to obtain 117D (2.3 g, crude).

Step 4

117D (1.0 g, crude) was dissolved in DMF (10 mL), 18E (0.4 g, 1.0 mmol) and N-methylimidazole (328 mg, 4.0 mmol) were added, and at 0°C, TCFH (336 mg, 1.2 mmol) was added. The resulting mixture was stirred at room temperature for 20 min. After the reaction was completed, water (20 mL) was added to the reaction solution, and the resulting mixture was extracted with ethyl acetate (15 mL×3). The organic layers were combined, washed with saturated sodium chloride aqueous solution (25 mL×3), dried over anhydrous sodium sulphate, filtered, and concentrated. The residue was separated and purified by silica gel column chromatography (dichloromethane:methanol (v/v) = 20:1) to obtain compound 117 (150 mg).

LC-MS (ESI): m/z = 514.5 $[M+H]^+$.

$^1H$ NMR (400 MHz, $CDCl_3$) δ 7.80 (s, 1H), 7.49 (d, 1H), 7.46 (s, 1H), 7.31 (d, 1H), 6.57 - 6.51 (m, 1H), 6.41 (s, 1H), 6.18 (d, 1H), 4.96 - 4.89 (m, 2H), 4.56 (d, 1H), 4.17 (s, 4H), 4.09 (s, 4H), 3.87 (d, 1H), 3.78 (s, 2H), 3.54 (t, 2H), 3.06 (t, 1H), 2.65 (t, 1H), 2.43 - 2.28 (m, 1H), 1.82 (t, 2H), 1.47 -1.31 (m, 2H).

$^{19}F$ NMR (377 MHz, $CDCl_3$) δ -60.06 (s).

Example 118:

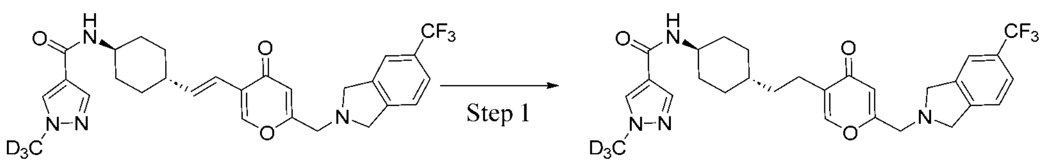


Compound 81 Compound 118

Step 1:

Compound 81 (350 mg, 0.66 mmol) was dissolved in methanol (10 mL), and then 10% palladium on carbon (7.00 mg, 0.066 mmol) was added. After the addition, the system was subjected to hydrogen replacement three times, and the mixture was reacted at room temperature for 1 hr. After the reaction was completed, the mixture was filtered, and the filtrate was concentrated and purified by preparative HPLC to obtain compound 118 (14 mg, yield: 4%).

LC-MS (ESI): m/z= 532.3 [M+H]$^+$.

$^1$H NMR (400 MHz, DMSO-$d_6$) δ 8.10-8.08 (d, 2H), 7.83-7.81 (m, 1H), 7.74-7.72 (d, 1H), 7.63 (s, 1H), 7.58-7.56 (d, 1H), 7.48-7.46 (d, 1H), 6.32 (s, 1H), 4.04 (s, 4H), 3.82-3.80 (m, 2H), 3.69 - 3.61 (m, 1H), 2.28 - 2.25 (m, 2H), 1.84-1.75 (m, 4H), 1.38 - 1.18 (m, 5H), 1.03-0.94 (m, 2H).

**Example 119:**

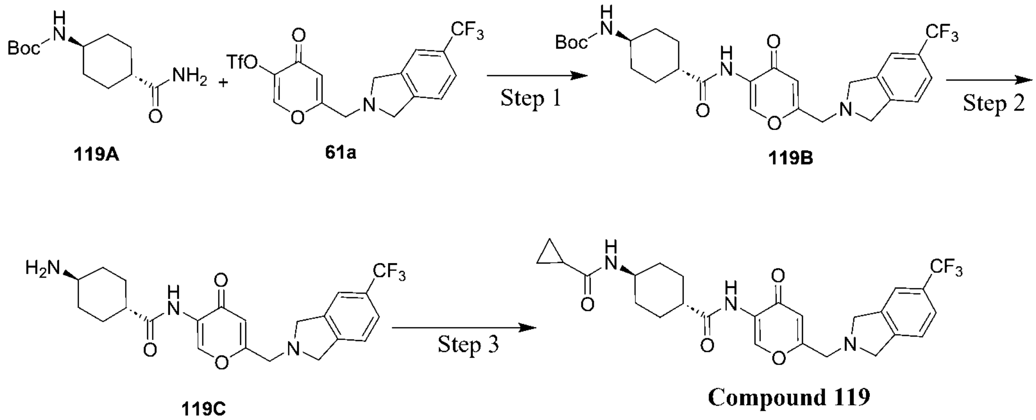


119A 61a 119B

119C Compound 119

Step 1:

Compound 119A (550 mg, 2.27 mmol) and 61a (1.11 g, 2.50 mmol) were dissolved in 1,4-dioxane (55 mL), and 4,5-bis(diphenylphosphino)-9,9-dimethylxanthene (263 mg, 0.454 mmol), caesium carbonate (2.59 g, 7.94 mmol) and tris(dibenzylideneacetone)dipalladium (208 mg, 0.227 mmol) were successively added. After the addition, the mixture was subjected to nitrogen replacement three times, and stirred and reacted at 100°C for 18 h. After the completion of the reaction, water (50 mL) was added, the resulting mixture was extracted with ethyl acetate (100 mL×3), and the organic phases were combined, washed with saturated sodium chloride aqueous solution (40 mL×1), dried over anhydrous sodium sulphate and filtered. The filtrate was concentrated under reduced pressure and purified by column chromatography (petroleum ether:ethyl acetate = 2:1 (v/v)) to obtain compound 119B (320 mg, yield: 27%).

LC-MS (ESI): *m*/*z* = 480.2 [M-56+H]$^+$.

Step 2:

Compound 119B (160 mg, 0.30 mmol) was dissolved in dichloromethane (20 mL), trifluoroacetic acid (5 mL) was added, and the mixture was reacted at room temperature for 2 h. After the reaction was completed as monitored by TLC (petroleum ether: ethyl acetate = 1:1 (v/v)), the reaction solution was concentrated to obtain the trifluoroacetate of compound 119C (164 mg, crude), which was directly used in the next step without purification.

LC-MS (ESI): *m*/*z* = 436.2 [M+H]$^+$.

Step 4:

Trifluoroacetate of compound 119C (164 mg, 0.30 mmol) was dissolved in dichloromethane (10 mL), and in an ice-water bath, triethylamine (137 mg, 1.35 mmol) and cyclopropanecarbonyl chloride (63 mg, 0.6 mmol) were successively added. After the dropwise addition was completed, the mixture was reacted for another 2 h in an ice-water bath. After the reaction was completed as monitored by TLC (dichloromethane: methanol = 20:1 (v/v)), the reaction solution was concentrated, and the crude was purified by column chromatography (eluents: dichloromethane: methanol = 65:1 (v/v)) to obtain the target compound 119 (84 mg, yield: 56%).

LCMS *m*/*z* = 504.4 [M+1]$^+$

$^1$H NMR (400 MHZ, DMSO-$d_6$) δ 9.25 (s, 1H), 9.20 (s, 1H), 7.95 (d, 1H), 7.75 (s, 1H), 7.69 (d, 1H), 7.58 (d, 1H), 6.67 (s, 1H), 4.53 (s, 4H), 4.36 (s, 2H), 3.75 - 3.25 (m, 1H),2.66 (t, 1H), 1.88 - 1.76 (m, 4H), 1.55 - 1.45 (m, 1H), 1.46 - 1.33 (m, 2H), 1.25 - 1.16 (m, 2H), 0.68 - 0.55 (m, 4H).

**Example 120:**

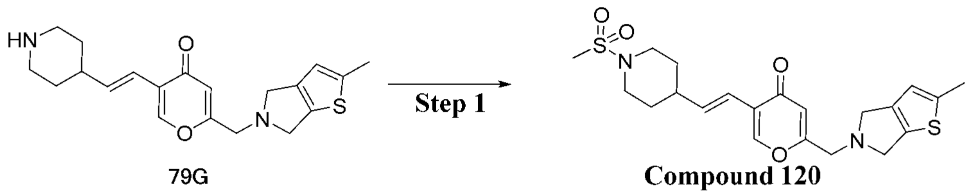


79G Compound 120

Step 1:

79G (400 mg, 1.12 mmol) was dissolved in dichloromethane (10 mL), and at 0°C-5°C, triethylamine (570 mg, 5.6 mmol) and methanesulphonyl chloride (150 mg, 1.34 mmol) were successively added. After the dropwise addition was completed, the mixture was reacted at room temperature for 2 hrs. After the reaction was completed, the reaction solution was concentrated and purified by preparative HPLC to obtain compound 92 (80.00 mg, yield: 16%).

LC-MS (ESI): m/z= 435.2 $[M+H]^+$.

$^1H$ NMR (400 MHz, DMSO-$d_6$) δ8.31 (s, 1H), 6.71-6.65 (dd, 1H), 6.59 (s, 1H), 6.33 (s, 1H), 6.17-6.13 (d, 1H), 4.00-3.93 (m, 2H), 3.83-3.81 (m,4H), 3.58-3.56 (m, 2H), 2.85 (s, 3H), 2.79-2.72 (m, 2H), 2.41 (s, 3H), 2.23 - 2.16 (m, 1H), 1.81-1.78 (m, 2H), 1.43-1.33 (m, 2H).

**Example 121 and example 122:**

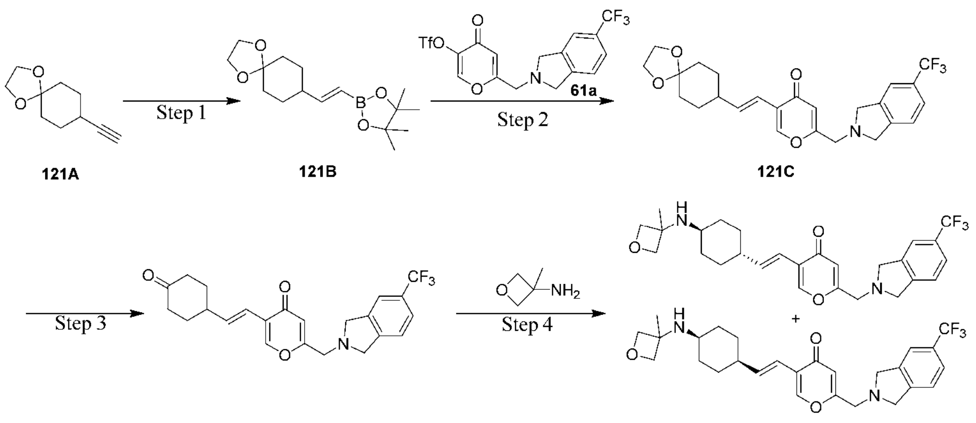


121A 121B 121C 121D Compound 121 and compound 122

Step 1:

121A (2.50 g, 15.04 mmol) was dissolved in dry toluene (60 mL), pinacolborane (3.85 g, 30.08 mmol) was added dropwise, and bis(cyclopentadienyl)zirconium chloride hydride (0.78 g, 3.01 mmol) was added. After the addition, the system was subjected to nitrogen replacement, and stirred and reacted at 65°C for 18 h. After the reaction was completed as monitored by TLC (petroleum ether:ethyl acetate = 2:1 (v/v)), the reaction solution was concentrated, and the crude was directly used in the next step.

Step 2:

Compound 121B (4.24 g, 15.04 mmol) and 61a (4.45 g, 10.03 mmol) were dissolved in 1,4-dioxane (60 mL), and [1,1'-bis(diphenylphosphino)ferrocene]palladium dichloride (II) (0.73 g, 1.00 mmol), potassium carbonate (4.85 g, 35.09 mmol) and water (12 mL) were successively added. After the addition, the mixture was subjected to nitrogen replacement three times, and stirred and reacted at 85°C for 18 h. After the completion of the reaction, water (100 mL) was added, the resulting mixture was extracted with ethyl acetate (100 mL×3), and the organic phases were combined, washed with saturated sodium chloride aqueous solution (40 mL×1), dried over anhydrous sodium sulphate and filtered. The filtrate

was concentrated under reduced pressure and purified by column chromatography (petroleum ether:ethyl acetate = 2:1 (v/v)) to obtain compound 121C (3.5 g, yield: 76%).

LC-MS (ESI): *m*/*z* = 462.10 [M+H]$^+$.

Step 3:

Compound 121C (3.5 g, 7.58 mmol) was dissolved in tetrahydrofuran (35 mL), 6 N aqueous hydrochloric acid solution (35 mL) was added, and the mixture was reacted at room temperature for 2 h. After the reaction was completed as monitored by TLC (petroleum ether:ethyl acetate = 1:1 (v/v)), water (100 mL) was added, the resulting mixture was extracted with ethyl acetate (100 mL×3), and the organic phases were combined, washed with saturated sodium chloride aqueous solution (40 mL×1), dried over anhydrous sodium sulphate and filtered. The filtrate was concentrated under reduced pressure and purified by column chromatography (petroleum ether:ethyl acetate = 1:1 (v/v)) to obtain compound 121D (2.5 g, yield: 79%).

LC-MS (ESI): *m*/*z* = 418.20 [M+H]$^+$.

Step 4:

Compound 121D (550 mg, 1.32 mmol) and 3-methyl 3-aminooxetane (172 mg, 1.98 mmol) were dissolved in 1,2-dichloroethane (15 mL), and then glacial acetic acid (79 mg, 1.32 mmol) was added. After the dropwise addition was completed, the mixture was reacted at room temperature for 1 h, and then sodium triacetoxyborohydride (977 mg, 4.61 mmol) was slowly added. After the addition, the mixture was reacted overnight. After the reaction was completed as monitored by TLC (dichloromethane:methanol = 20:1 (v/v)), water (50 mL) was added, and the mixture was extracted with dichloromethane (100 mL×2), dried over anhydrous sodium sulphate, filtered, and concentrated under reduced pressure. The residue was purified by column chromatography (eluents: dichloromethane:methanol =60:1 (v/v)) to obtain a crude target compound. The crude was subjected to chiral preparative SFC to obtain P1 (121 mg, yield: 19%, retention time: 6.00 minutes, assigned as compound 121), and P2 (135 mg, yield: 21%, retention time: 7.18 minutes, assigned as compound 122).

Preparative SFC method: 1. Instrument: SFC Prep 150 AP; chromatographic column: TORUS 2-PIC (19 mm×250 mm). 2. The sample was dissolved in methanol and filtered with a 0.45 μm filter to prepare a sample solution. 3. Preparative chromatography conditions: a. composition of mobile phases A and B: mobile phase A: $CO_2$; mobile phase B: methanol. b. isocratic elution, mobile phase B: 13%; c. flow rate: 50 ml/min. After separation, the fraction was dried in a water bath at 50°C by a rotary evaporator to obtain a product. Then the solvent was dried at -80°C with a lyophilizer to obtain P1 and P2.

Compound 121, LCMS m/z = 489.7 [M+H]$^+$.

$^1$H NMR (400 MHZ, DMSO-*d*$_6$) δ 8.34 (s, 1H), 7.63 (s, 1H), 7.58 (d, 1H), 7.48 (d, 1H), 6.85 (dd, 1H), 6.38 (s, 1H), 6.17 (d, 1H), 4.67 (s, 2H), 4.34 (s, 2H), 4.04 (s, 4H), 3.83 (s, 2H), 2.42 - 2.34 (m, 1H), 1.80 - 1.39 (m, 12H).

Compound 122, LCMS m/z = 489.7 [M+H]$^+$.

$^1$H NMR (400 MHZ, DMSO-*d*$_6$) δ 8.30 (s, 1H), 7.63 (s, 1H), 7.57 (d, 1H), 7.47 (d, 1H), 6.62 (dd, 1H), 6.35 (s, 1H), 6.11 (d, 1H), 4.67 (s, 2H), 4.35 (s, 2H), 4.03 (s, 4H), 3.82 (s, 2H), 2.10 - 1.99 (m, 1H), 1.84 - 1.70 (m, 4H), 1.69 - 1.50 (m, 3H), 1.44 - 1.30 (m, 2H), 1.29 - 1.11 (m, 3H).

**Biological test:**

1. Inhibitory effects of compounds on testosterone level in NCI-H295R cells

The purpose of the experimental study was intended to detect the testosterone level in the NCI-H295R cells by adopting the Enzyme linked immunosorbent assay (ELISA), so as to evaluate the inhibitory effects of the compounds on the testosterone level in the NCI-H295R cells.

The H295R cells were purchased from ATCC, the cell complete medium contained DMEM: F12+ 10% FBS +0.00625 mg/ml insulin +0.00625 mg/ml transferrin+6.25 ng/ml selenium +1.25 mg/ml BSA+0.00535 mg/ml linoleic acid, and the cells were cultured in a 37°C, 5% $CO_2$ incubator. The cells in the exponential growth phase were collected, and cultured in a cytokine-free medium (phenol red 1640-free +1% PS+10% css FBS+1.25 mg/ml BSA +0.00625 mg/ml insulin + 0.00625 mg/ml transferrin+6.25 ng/ml selenium+0.00535 mg/ml linoleic acid) for three days. The cells in the exponential growth phase were collected on the third day. The cell suspension was adjusted with a cytokine-free medium to a corresponding concentration and plated at 60000 cells/well, with the volume being 90 μL/well. Subsequently, 10 μL of the compounds at different concentrations was added and the plate was incubated in a $CO_2$ incubator for three days. After the incubation was completed, according to the operation instructions for testosterone Elisa kit (Beyotime, PT872), 50 μL of the cell culture supernatant was collected from each well and centrifuged at 500 g for 5 min at room temperature and then

the resulting supernatant was collected. The samples were added to the corresponding wells at 25 μl/well, and then formulated horseradish peroxidase labelled testosterone was added at 75 μl/well. The mixture was fully mixed for 10 seconds. The reaction wells were sealed with a plate-sealing film (white) and the mixture was incubated at room temperature in the dark for 120 min. The plate was washed three times with a volume of 300 μl per well, and then placed onto thick absorbent paper and dried. Chromogenic reagent TMB solution was added at 100 μl/well. The reaction wells were sealed with a plate-sealing film (white) and the mixture was incubated at room temperature in the dark for 15-20 min. Subsequently, a termination solution was added at 50 μl/well, and after the mixture was fully mixed, the absorbance value $OD_{450}$ was immediately measured. The absorbance readings were processed using Graphpad Prim 8.0 software and S-shaped concentration curves were plotted and the $IC_{50}$ values were calculated using a four-parameter non-linear regression model. The results were processed according to formula (1), the inhibition rate of the compound at different concentrations was calculated, and the $IC_{50}$ value of the compound with an inhibition rate of 50% was calculated using Graphpad Prim 8.0 software, wherein $RLU_{compound}$ was the readout of the treated group, and $RLU_{control}$ was the average value of the solvent control group.

$$\text{Inhibition rate\%} = 100 - RLU_{compound} / RLU_{control} \times 100 \quad \text{Formula (1)}$$

Table 2 Inhibitory effects of compounds on testosterone level in NCI-H295R cells

| Compound | Testosterone level in H295R (Elisa) | | |
|---|---|---|---|
| | inh.% 1000 nM; | inh.% 100 nM | $IC_{50}$ nM |
| Compound 6 | 90.8% | 79.5% | NA |
| Compound 10 | 79.7% | 63% | NA |
| Compound 12 | 83.4% | 85.6% | NA |
| Compound 15 | 84.2% | 51% | 13 |
| Compound 16 | 79.6% | 55% | 98.3 |
| Compound 17 | 93.6% | 91.6% | NA |
| Compound 18 | 92.5% | 80.8% | 100 |
| Compound 19 | 78.3% | 64.8% | NA |
| Compound 20 | 95.2% | 87.3% | NA |
| Compound 25 | 77% | 56% | NA |
| Compound 26 | 78% | 61% | NA |
| Compound 27 | 68% | 78% | 50.1 |
| Compound 32 | 64.3% | 76.1% | 117 |
| Compound 33 | NA | NA | 68 |
| Compound 34 | NA | NA | 122 |
| Compound 35 | 75.5% | 72.8% | NA |
| Compound 37 | 74.1% | 73.8% | NA |
| Compound 47 | 69.3% | 72.2% | NA |
| Compound 48 | 78.5% | 72.4% | NA |
| Compound 49 | 75.2% | 67.3% | NA |
| Compound 52 | 74.9% | 55.8% | 98.2 |
| Compound 53 | 67.3% | 61% | NA |
| Compound 54 | 72.2% | 53.2% | NA |
| Compound 55 | 72.7% | 63% | NA |
| Compound 56 | 79.3% | 53.6% | NA |
| Compound 60 | 78.5% | 57.7% | NA |

(continued)

| Compound | Testosterone level in H295R (Elisa) | | |
|---|---|---|---|
| | inh.% 1000 nM; | inh.% 100 nM | $IC_{50}$ nM |
| Compound 61 | 79% | 71% | 162 |
| Compound 62 | 79% | 67% | NA |
| Compound 63 | 75% | 64% | NA |
| Compound 64 | 75% | 73% | NA |
| Compound 65 | 78% | 80% | NA |
| Compound 66 | 82% | 78% | NA |
| Compound 68 | 86% | 81% | NA |
| Compound 69 | 76% | 62% | NA |
| Compound 70 | 83% | 60% | NA |
| Compound 72 | 68% | 58% | NA |
| Compound 73 | 87% | 83% | NA |
| Compound 75 | 78% | 69% | NA |
| Compound 77 | 84% | 71% | NA |
| Compound 79 | 80% | 61% | NA |
| Compound 80 | 84% | 71% | NA |
| Compound 81 | 83% | 63% | 102 |
| Compound 88 | 86% | 76% | 59 |
| Compound 89 | 87% | 75% | NA |
| Compound 90 | 85% | 80% | 73 |
| Compound 95 | 81% | 57% | NA |
| Compound 98 | 78% | 61% | 41 |
| Compound 100 | 85% | 70% | NA |
| Compound 101 | 71% | 73% | NA |
| Compound 102 | 82% | 79% | NA |
| Compound 106 | 93% | 69% | NA |
| Compound 107 | 78% | 60% | NA |
| Compound 108 | 84% | 75% | NA |
| Compound 112 | 78% | 74% | NA |
| Compound 113 | 77% | 76% | NA |
| Compound 116 | 66% | 60% | NA |
| Compound 118 | 83% | 70% | 36 |
| Compound 119 | 81% | 60% | NA |
| Compound 122 | 82% | 63% | NA |
| NA: not detected. | | | |

Conclusion: The compounds of the present invention, such as example compounds, have significant inhibitory effects on the testosterone level in NCI-H295R cells.

2. The purpose of the experimental study was intended to detect the pregnenolone level in the NCI-H295R cells by adopting the Enzyme linked immunosorbent assay (ELISA), so as to evaluate the inhibitory effects of the compounds on the pregnenolone level in the NCI-H295R cells.

The H295R cells were purchased from ATCC (CRL-2128), the cell complete medium contained DMEM: F12+ 10% FBS +0.00625 mg/ml insulin+0.00625 mg/mL transferrin+6.25 ng/mL selenium+1.25 mg/mL BSA+0.00535 mg/mL linoleic acid, and the cells were cultured in a 37°C, 5% $CO_2$ incubator. The cells in the exponential growth phase were collected, and cultured in a cytokine-free medium (phenol red 1640-free +1% PS+10% css FBS+1.25 mg/mL BSA +0.00625 mg/mL insulin+0.00625 mg/mL transferrin+6.25 ng/mL selenium+0.00535 mg/mL linoleic acid) for three days. The cells in the exponential growth phase were collected on the third day. The cell suspension was adjusted with a cytokine-free medium to a corresponding concentration and plated at 60000 cells/well, with the volume being 90 μL/well. Subsequently, 10 μL of the compounds at different concentrations was added and the plate was incubated in a $CO_2$ incubator for three days. After the incubation was completed, the cell culture supernatant was collected and centrifuged at 2°C-8°C at 800×g for 10 min. The resulting supernatant was collected. Determination was carried out according to the operation instructions for pregnenolone Elisa kit (Antibodies-A73791), and the $OD_{450}$ signal was read.

Data processing:

2.1 Standard curve plotting. The standard concentration was taken as the abscissa and the $OD_{450}$ value as the ordinate, the coordinate points of various standards were connected with a smooth line and a curve was fitted according to the four parameter logistic (4-PL) method.

2.2 Sample quantification: The corresponding concentration (CONC) of testosterone/pregnenolone in the sample was calculated using the absorbance value of the sample and the standard curve. According to formula (1), the inhibition rate of the compound at various concentrations was calculated, and S-shaped concentration curves were plotted and the $IC_{50}$ values (i.e., concentration of the compound at which the inhibition rate was 50%) were calculated using a three parameter non-linear regression model in Graphpad Prim 8.0 software. CONC $_{cpd}$ was the concentration of the treated group, and CONC $_{ctrl}$ was the average concentration value of the solvent control group.

$$\text{Inhibition rate\%} = 100\%\text{-CONC}_{cpd} / \text{CONC}_{ctrl} \times 100\% \quad \text{Formula (1)}$$

Table 3 Inhibitory effects of compounds on pregnenolone level in NCI-H295R cells

| Compound | Pregnenolone level in H295R (Elisa) | | |
|---|---|---|---|
| | inh.% 1000 nM | inh.% 100 nM | $IC_{50}$ nM |
| Compound 15 | NA | NA | 382 |
| Compound 17 | 75.2% | 64.9% | 773 |
| Compound 18 | 50.5% | 40.7% | NA |
| Compound 27 | 67.5% | 65.5% | NA |
| Compound 61 | NA | NA | 370 |
| Compound 80 | 59.1% | 38.1% | 190 |
| Compound 81 | NA | NA | 146 |
| Compound 84 | NA | NA | 121 |
| Compound 88 | 64.5% | 41.6% | 94 |
| Compound 89 | 64.9% | 41.6% | NA |
| Compound 90 | 67.0% | 43.4% | 119 |
| Compound 95 | 66% | 50% | NA |
| Compound 98 | 70% | 62% | NA |
| Compound 116 | 75% | 48% | NA |
| Compound 118 | NA | NA | 71 |
| NA: not detected. | | | |

Conclusion: The compounds of the present invention, such as example compounds, have significant inhibitory effects on the pregnenolone level in NCI-H295R cells.

3. Pharmacokinetic test in rats

3.1 Experimental animals: Male SD rats (about 220 g, 6-8 weeks old, 6 rats/compound) were purchased from Chengdu Dossy Experimental Animals Co., Ltd.

3.2 Experimental design: On the day of the experiment, the SD rats were randomly grouped by body weight. The animals were fasted with water available for 12-14 h one day before the administration, and were fed 4 h after the administration.

Table 4 Administration information

| Group | Number | Administration information | | | | | |
|---|---|---|---|---|---|---|---|
| | Male | Test substance | Administration dosage (mg/kg) | Administration concentration (mg/mL) | Administration volume (mL/kg) | Collected sample | Administration mode |
| G1 | 3 | Compound 17 | 2.5 | 0.5 | 5 | Plasma | Intravenous administration |
| G2 | 3 | Compound 17 | 10 | 1 | 10 | Plasma | Intragastric administration |
| G3 | 3 | Compound 31 | 2.5 | 0.5 | 5 | Plasma | Intravenous administration |
| G4 | 3 | Compound 31 | 10 | 1 | 10 | Plasma | Intragastric administration |
| G5 | 3 | Compound 33 | 10 | 1 | 10 | Plasma | Intragastric administration |
| G6 | 3 | Compound 35 | 2.5 | 0.5 | 5 | Plasma | Intravenous administration |
| G7 | 3 | Compound 35 | 10 | 1 | 10 | Plasma | Intragastric administration |
| G8 | 3 | Compound 52 | 2.5 | 0.5 | 5 | Plasma | Intravenous administration |
| G9 | 3 | Compound 52 | 10 | 1 | 10 | Plasma | Intragastric administration |
| G10 | 3 | Compound 61 | 2.5 | 0.5 | 5 | Plasma | Intravenous administration |
| G11 | 3 | Compound 61 | 10 | 1 | 10 | Plasma | Intragastric administration |
| G12 | 3 | Compound 63 | 10 | 1 | 10 | Plasma | Intragastric administration |
| G13 | 3 | Compound 64 | 2.5 | 0.5 | 5 | Plasma | Intravenous administration |
| G14 | 3 | Compound 64 | 10 | 1 | 10 | Plasma | Intragastric administration |
| G15 | 3 | Compound 81 | 2.5 | 0.5 | 5 | Plasma | Intravenous administration |
| G16 | 3 | Compound 81 | 10 | 1 | 10 | Plasma | Intragastric administration |
| G17 | 3 | Compound 98 | 2.5 | 0.5 | 5 | Plasma | Intravenous administration |
| G18 | 3 | Compound 98 | 10 | 1 | 10 | Plasma | Intragastric administration |

(continued)

| Group | Number | Administration information | | | | | |
|---|---|---|---|---|---|---|---|
| | Male | Test substance | Administration dosage (mg/kg) | Administration concentration (mg/mL) | Administration volume (mL/kg) | Collected sample | Administration mode |
| G19 | 3 | Compound 100 | 2.5 | 0.5 | 5 | Plasma | Intravenous administration |
| G20 | 3 | Compound 100 | 10 | 1 | 10 | Plasma | Intragastric administration |
| G21 | 3 | Compound 101 | 2.5 | 0.5 | 5 | Plasma | Intravenous administration |
| G22 | 3 | Compound 101 | 10 | 1 | 10 | Plasma | Intragastric administration |
| G23 | 3 | Compound 107 | 2.5 | 0.5 | 5 | Plasma | Intravenous administration |
| G24 | 3 | Compound 107 | 10 | 1 | 10 | Plasma | Intragastric administration |
| G25 | 3 | Compound 118 | 2.5 | 0.5 | 5 | Plasma | Intravenous administration |
| G26 | 3 | Compound 118 | 10 | 1 | 10 | Plasma | Intragastric administration |
| vehicle for intravenous administration: 5%DMA+5%HS-15+90%NS; vehicle for intragastric administration: 0.5% MC | | | | | | | |

Before and after the administration, 0.1 ml of blood was taken from the orbit of the animals under isoflurane anaesthesia and placed in an EDTAK2 centrifuge tube. The blood was centrifuged at 5000 rpm and 4°C for 10 min to collect plasma. Blood sampling time points for the intravenous administration group: 0, 5 min, 15 min, 30 min, 1 h, 2 h, 4 h, 6 h, 8 h and 24 h; blood sampling time points for the intragastric administration group: 0, 5 min, 15 min, 30 min, 1 h, 2 h, 4 h, 6 h, 8 h and 24 h. Before analysis and detection, all samples were stored at -80°C.

Table 5 Pharmacokinetic parameters of test compounds in plasma of rats

| Test compound | Administration mode | CL (mL/min/kg) | $Vd_{ss}$ (L/kg) | $AUC_{0-t}$ (hr*ng/mL) | $T_{1/2}$ (h) |
|---|---|---|---|---|---|
| Compound 17 | Intravenous administration (2.5 mg/kg) | 24.4 | 2.53 | 1676 | 1.59 |
| Compound 17 | Intragastric administration (10 mg/kg) | - | - | 5235 | 2.72 |
| Compound 31 | Intravenous administration (2.5 mg/kg) | 9.48 | 3.41 | 4266 | 5.16 |
| Compound 31 | Intragastric administration (10 mg/kg) | - | - | 13357 | 5.65 |
| Compound 33 | Intragastric administration (10 mg/kg) | - | - | 19600 | 2.04 |
| Compound 35 | Intravenous administration (2.5 mg/kg) | 4.66 | 0.788 | 9101 | 2.71 |
| Compound 35 | Intragastric administration (10 mg/kg) | - | - | 18251 | 2.84 |
| Compound 52 | Intravenous administration (2.5 mg/kg) | 12.3 | 1.39 | 3297 | 1.37 |
| Compound 52 | Intragastric administration (10 mg/kg) | - | - | 10485 | 2.21 |
| Compound 61 | Intravenous administration (2.5 mg/kg) | 7.49 | 1.42 | 5594 | 2.58 |
| Compound 61 | Intragastric administration (10 mg/kg) | - | - | 17389 | 3.03 |
| Compound 63 | Intragastric administration (10 mg/kg) | - | - | 19079 | 2.18 |
| Compound 64 | Intravenous administration (2.5 mg/kg) | 12.1 | 2.09 | 3437 | 2.65 |
| Compound 64 | Intragastric administration (10 mg/kg) | - | - | 10094 | 2.92 |

(continued)

| Test compound | Administration mode | CL (mL/min/kg) | $Vd_{ss}$ (L/kg) | $AUC_{0-t}$ (hr*ng/mL) | $T_{1/2}$ (h) |
|---|---|---|---|---|---|
| Compound 81 | Intravenous administration (2.5 mg/kg) | 3.22 | 0.704 | 12894 | 3.18 |
| Compound 81 | Intragastric administration (10 mg/kg) | - | - | 40460 | 4.29 |
| Compound 98 | Intravenous administration (2.5 mg/kg) | 7.06 | 3.66 | 5311 | 7.11 |
| Compound 98 | Intragastric administration (10 mg/kg) | - | - | 11689 | 8.04 |
| Compound 100 | Intravenous administration (2.5 mg/kg) | 8.86 | 1.29 | 4657 | 1.8 |
| Compound 100 | Intragastric administration (10 mg/kg) | - | - | 17501 | 2.29 |
| Compound 101 | Intravenous administration (2.5 mg/kg) | 7.45 | 1.47 | 5640 | 2.96 |
| Compound 101 | Intragastric administration (10 mg/kg) | - | - | 11557 | 3.59 |
| Compound 107 | Intravenous administration (2.5 mg/kg) | 6.96 | 1.55 | 6028 | 3.39 |
| Compound 107 | Intragastric administration (10 mg/kg) | - | - | 22451 | 4.15 |
| Compound 118 | Intravenous administration (2.5 mg/kg) | 8.18 | 0.755 | 5199 | 1.58 |
| Compound 118 | Intragastric administration (10 mg/kg) | - | - | 11366 | 4.41 |

Conclusion: The compounds of the present invention, such as the compounds in the examples, have good bioavailability and pharmacokinetic characteristics.

4. Pharmacokinetic test in mice

4.1. Experimental animals: Male ICR mice, 20-25 g, 6 mice/compound, were purchased from Chengdu Dossy Experimental Animals Co., Ltd.

4.2 Experimental design: on the day of the experiment, the ICR mice were randomly grouped according to their body weights. The animals were fasted with water available for 12-14 h one day before the administration, and were fed 4 h after the administration.

Table 6 Administration information

| Group | Number | Administration information | | | | | |
|---|---|---|---|---|---|---|---|
| | Male | Test substance | Administra tion dosage (mg/kg) | Administra tion concentrati on (mg/mL) | Administra tion volume (mL/kg) | Collected sample | Administrati on mode |
| G1 | 3 | Compound 15 | 2.5 | 0.5 | 5 | Plasma | Intravenous administrati on |
| G2 | 3 | Compound 15 | 10 | 1 | 10 | Plasma | Intragastric administrati on |
| G3 | 3 | Compound 17 | 2.5 | 0.5 | 5 | Plasma | Intravenous administrati on |
| G4 | 3 | Compound 17 | 10 | 1 | 10 | Plasma | Intragastric administrati on |
| G5 | 3 | Compound 33 | 2.5 | 0.5 | 5 | Plasma | Intravenous administrati on |

(continued)

| Group | Number | Administration information | | | | | |
|---|---|---|---|---|---|---|---|
| | Male | Test substance | Administra tion dosage (mg/kg) | Administra tion concentrati on (mg/mL) | Administra tion volume (mL/kg) | Collected sample | Administrati on mode |
| G6 | 3 | Compound 33 | 10 | 1 | 10 | Plasma | Intragastric administrati on |
| G7 | 3 | Compound 35 | 2.5 | 0.5 | 5 | Plasma | Intravenous administrati on |
| G8 | 3 | Compound 35 | 10 | 1 | 10 | Plasma | Intragastric administrati on |
| G9 | 3 | Compound 54 | 2.5 | 0.5 | 5 | Plasma | Intravenous administrati on |
| G10 | 3 | Compound 54 | 10 | 1 | 10 | Plasma | Intragastric administrati on |
| G11 | 3 | Compound 61 | 2.5 | 0.5 | 5 | Plasma | Intravenous administrati on |
| G12 | 3 | Compound 61 | 10 | 1 | 10 | Plasma | Intragastric administrati on |
| G13 | 3 | Compound 81 | 2.5 | 0.5 | 5 | Plasma | Intravenous administrati on |
| G14 | 3 | Compound 81 | 10 | 1 | 10 | Plasma | Intragastric administrati on |
| G15 | 3 | Compound 88 | 2.5 | 0.5 | 5 | Plasma | Intravenous administrati on |
| G16 | 3 | Compound 88 | 10 | 1 | 10 | Plasma | Intragastric administrati on |
| G17 | 3 | Compound 89 | 2.5 | 0.5 | 5 | Plasma | Intravenous administrati on |
| G18 | 3 | Compound 89 | 10 | 1 | 10 | Plasma | Intragastric administrati on |
| G19 | 3 | Compound 90 | 2.5 | 0.5 | 5 | Plasma | Intravenous administrati on |
| G20 | 3 | Compound 90 | 10 | 1 | 10 | Plasma | Intragastric administrati on |

(continued)

| Group | Number | Administration information | | | | | |
|---|---|---|---|---|---|---|---|
| | Male | Test substance | Administra tion dosage (mg/kg) | Administra tion concentrati on (mg/mL) | Administra tion volume (mL/kg) | Collected sample | Administrati on mode |
| G21 | 3 | Compound 98 | 2.5 | 0.5 | 5 | Plasma | Intravenous administrati on |
| G22 | 3 | Compound 98 | 10 | 1 | 10 | Plasma | Intragastric administrati on |
| G21 | 3 | Compound 118 | 2.5 | 0.5 | 5 | Plasma | Intravenous administrati on |
| G22 | 3 | Compound 118 | 10 | 1 | 10 | Plasma | Intragastric administrati on |
| vehicle for intravenous administration: 5%DMA+5%HS-15+90%NS; vehicle for intragastric administration: 0.5% MC | | | | | | | |

Before and after administration, 0.06 mL of blood was taken from the orbit under isoflurane anaesthesia and placed in an EDTAK2 centrifuge tube. The tube was centrifuged at 5000 rpm at 4°C for 10 min, and plasma was collected. The blood collection time points for the intravenous administration group and intragastric administration group were 0, 5 min, 15 min, 30 min, 1 h, 2 h, 4 h, 7 h and 24 h. Before analysis and detection, all samples were stored at -80°C, and the samples were quantitatively analysed by LC-MS/MS.

Table 7 Pharmacokinetic parameters of test compounds in mouse plasma

| Test compound | Administration mode | CL (mL/min/kg) | $Vd_{ss}$ (L/kg) | $AUC_{0-t}$ (hr*ng/mL) | $T_{1/2}$ (h) |
|---|---|---|---|---|---|
| Compound 15 | Intravenous administration (2.5 mg/kg) | 6.21 | 1.61 | 6705 | 3.54 |
| Compound 15 | Intragastric administration (10 mg/kg) | - | - | 25396 | 3.19 |
| Compound 17 | Intravenous administration (2.5 mg/kg) | 8.73 | 1.81 | 4792 | 2.96 |
| Compound 17 | Intragastric administration (10 mg/kg) | - | - | 17827 | 2.42 |
| Compound 33 | Intravenous administration (2.5 mg/kg) | 19.9 | 1.19 | 2093 | 0.908 |
| Compound 33 | Intragastric administration (10 mg/kg) | - | - | 8432 | 2.45 |
| Compound 35 | Intravenous administration (2.5 mg/kg) | 3.34 | 0.557 | 12463 | 2.68 |
| Compound 35 | Intragastric administration (10 mg/kg) | - | - | 24202 | 2.85 |
| Compound 54 | Intravenous administration (2.5 mg/kg) | 7.19 | 1.17 | 5867 | 2.98 |
| Compound 54 | Intragastric administration (10 mg/kg) | - | - | 28097 | 2.97 |
| Compound 61 | Intravenous administration (2.5 mg/kg) | 6.8 | 1.75 | 6085 | 3.73 |
| Compound 61 | Intragastric administration (10 mg/kg) | - | - | 32115 | 3.47 |
| Compound 81 | Intravenous administration (2.5 mg/kg) | 1.69 | 0.86 | 22635 | 6.99 |
| Compound 81 | Intragastric administration (10 mg/kg) | - | - | 68455 | 5.38 |
| Compound 88 | Intravenous administration (2.5 mg/kg) | 4.82 | 1.54 | 8546 | 4.49 |
| Compound 88 | Intragastric administration (10 mg/kg) | - | - | 23563 | 4.48 |
| Compound 89 | Intravenous administration (2.5 mg/kg) | 4.9 | 1.63 | 8458 | 5.10 |
| Compound 89 | Intragastric administration (10 mg/kg) | - | - | 52530 | 8.11 |

(continued)

| Test compound | Administration mode | CL (mL/min/kg) | $Vd_{ss}$ (L/kg) | $AUC_{0-t}$ (hr*ng/mL) | $T_{1/2}$ (h) |
|---|---|---|---|---|---|
| Compound 90 | Intravenous administration (2.5 mg/kg) | 3.67 | 1.73 | 10554 | 6.73 |
| Compound 90 | Intragastric administration (10 mg/kg) | - | - | 40624 | 6.36 |
| Compound 98 | Intravenous administration (2.5 mg/kg) | 7.38 | 2.38 | 5573 | 4.62 |
| Compound 98 | Intragastric administration (10 mg/kg) | - | - | 10176 | 5.2 |
| Compound 118 | Intravenous administration (2.5 mg/kg) | 5.22 | 0.966 | 7975 | 2.78 |
| Compound 118 | Intragastric administration (10 mg/kg) | - | - | 27954 | 3.45 |
| Vehicle for administration: 0.5% MC<br>Conclusion: The compounds of the present invention, such as the compounds in the examples, have good pharmacokinetic characteristics in mice. | | | | | |

## Claims

1. A compound as represented by formula (I), or a stereoisomer, tautomer, deuterated compound, solvate, co-crystal or pharmaceutically acceptable salt, characterise in that

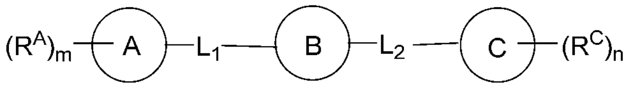

(I)

ring A is selected from 4- to 8-membered cycloalkyl, 4- to 7-membered monocyclic heterocycloalkyl, 6- to 12-membered bicyclic heterocycloalkyl, or 8- to 14-membered tricyclic heterocycloalkyl;
ring B is

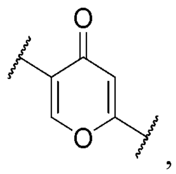,

and ring B is connected to $L_1$ on the left side;
ring C is selected from phenyl, 5- to 6-membered heteroaryl, 6- to 12-membered bicyclic carbocyclyl, 6- to 12-membered bicyclic heterocycloalkyl, or 8-to 14-membered tricyclic heterocycloalkyl, and ring C is not

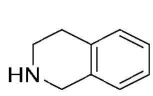, 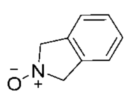,

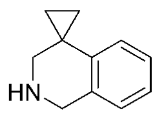, 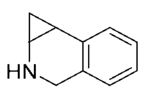 and 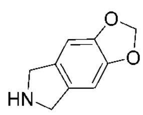;

$L_1$ is $W_1$-$R^{La}$-$W_2$, and $L_1$ is connected to A on the left side;
$L_2$ is $W_3$-$R^{Lb}$-$W_4$, and $L_2$ is connected to B on the left side, and $L_1$ and $L_2$ are not both a bond;
$R^{La}$ and $R^{Lb}$ are each independently selected from a bond, $C_{1-4}$ alkylene, $C_{2-4}$ alkenylene, or $C_{2-4}$ alkynylene, wherein the alkylene and alkenylene are optionally further substituted with 1-4 $R^{L1}$;
each $R^{L1}$ is independently selected from halogen, $C_{1-4}$ alkyl, $C_{2-4}$ alkenyl, $C_{1-4}$ alkoxy, or 3- to 6-membered

cycloalkyl, wherein the alkyl, alkenyl, alkoxy, and cycloalkyl are optionally further substituted with 1-4 substituents selected from halogen, CN, OH and $NH_2$, or two $R^{L1}$ on the same carbon atom or two adjacent carbon atoms, together with the atom to which they are connected, form 3- to 8-membered cycloalkyl, 5- to 10-membered heterocycloalkyl, or 5- to 6-membered heteroaryl;

$W_1$, $W_2$, $W_3$, and $W_4$ are each independently selected from a bond, -O-, -S-, - $NR^{W1}$-, -Se-, or -C(O)-;

$R^{W1}$ is selected from H, halogen, or $C_{1-4}$ alkyl;

each $R^A$ is independently selected from halogen, =O, CN, COOH, $C_{1-6}$ alkyl, $C_{2-6}$ alkenyl, $C_{2-6}$ alkynyl, -O-$C_{1-4}$ alkyl, -$S(O)_2$-$C_{1-4}$ alkyl, -$S(O)_2$-$(CH_2)_{1-3}$-$R^a$, - P(O)-($C_{1-4}$ alkyl)2, -C(O)-O-$(CH_2)_p$-$R^a$, -$(CH_2)_{1-2}$-C(O)-$R^a$, -C(O)-$(CH_2)_{1-2}$-$R^a$, - C(O)-O-halo $C_{1-4}$ alkyl, -C(O)-(6- to 9-membered bicyclic heterocycloalkyl), -NH-C(O)-$(CH_2)_p$-$R^a$, -NH-$R^a$, -N($CH_3$)-C(O)-$(CH_2)_p$-$R^a$, -NH-$SO_2$-$(CH_2)_{1-2}$-$R^a$, -NH-C(O)-O-$R^a$, -C(O)-$(CH_2)_p$-(4- to 6-membered monocyclic heterocycloalkyl), -C(O)-$(CH_2)_p$-(4- to 6-membered monocyclic cycloalkyl), -NH-C(O)-NH-$R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), -C(O)-NH-$S(O)_2$-$C_{1-4}$ alkyl, -NH-C(O)deuterated $C_{1-2}$ alkyl, -NH-C(O)-NH-$C_{1-2}$ alkyl or -NH-C(O)-$C_{1-2}$ alkyl, wherein the alkyl, alkenyl, alkynyl, cycloalkyl, and heterocycloalkyl are optionally further substituted with 1-4 groups selected from halogen, OH, $NH_2$, CN, -$S(O)_2$-$CH_3$, -O-halo $C_{1-4}$ alkyl, or -NH-$S(O)_2$-$CH_3$;

$R^a$ is selected from deuterated $C_{1-4}$ alkyl, $C_{2-4}$ alkynyl, 3- to 5-membered cycloalkyl, 3- to 6-membered heterocycloalkyl, or 5- to 6-membered heteroaryl, wherein the alkynyl, cycloalkyl, heterocycloalkyl, and heteroaryl are optionally further substituted with 1-4 groups selected from halogen, OH, =O, $C_{1-4}$ alkyl, deuterated $C_{1-4}$ alkyl, $C_{1-4}$ alkoxy, $C_{2-4}$ alkynyl, -O-halo $C_{1-4}$ alkyl, -$S(O)_2$-$CH_3$ or =$CH_2$;

each $R^C$ is independently selected from H, CN, halogen, OH, $C_{1-4}$ alkyl, halo $C_{1-4}$ alkyl, -O-halo $C_{1-4}$ alkyl, -Se-halo $C_{1-4}$ alkyl, $C_{2-4}$ alkenyl, $C_{2-4}$ alkynyl, =O, $C_{3-6}$ cycloalkyl, -$SCF_3$, -$SF_5$, -$(NH)_q$-P(O)($C_{1-4}$ alkyl)$_2$, -$(CH_2)_p$-O-$C_{1-4}$ alkyl, -O-$(CH_2)_p$-$C_{3-6}$ cycloalkyl or -$(CH_2)_p$-O-$C_{3-6}$ cycloalkyl, or any two $R^C$, together with the atom to which they are connected, form 3- to 8-membered cycloalkyl;

q is selected from 0 or 1;

each p is independently selected from 0, 1, 2, 3, or 4;

m is selected from 1, 2, 3, or 4;

n is selected from 1, 2, 3, or 4;

provided that

(1) when ring A is

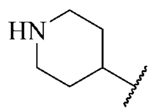

,

$R^A$ is not -$S(O)_2$-$(CH_2)_{0-1}CH_3$, and

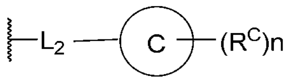

is not

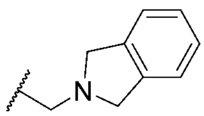

and

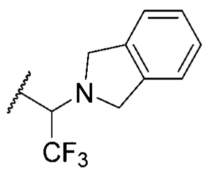

;

(2) when $R^A$ is -$S(O)_2$-$(CH_2)_{0-1}CH_3$, ring A is not

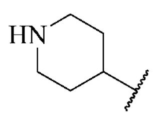

and

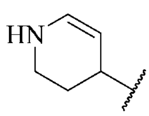

,

and

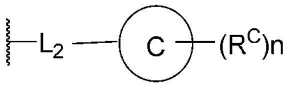

is not

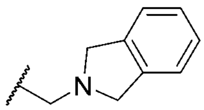 and 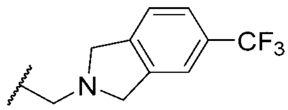 ;

(3) when ring C is

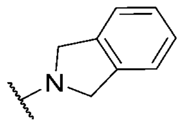 ,

$R^C$ is not H, and

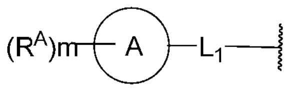

is not

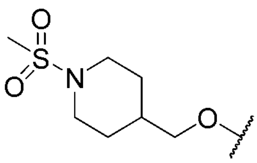 , 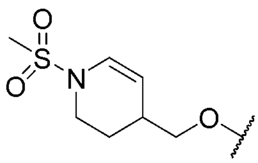 , 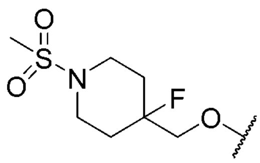

and

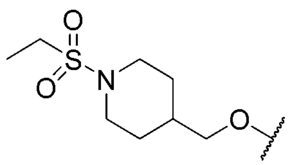 ;

(4) when $R^C$ is H, ring C is not

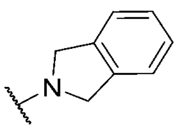 , and 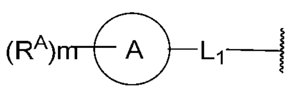

is not

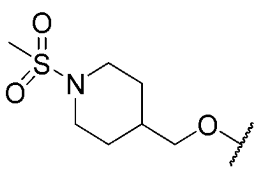 and 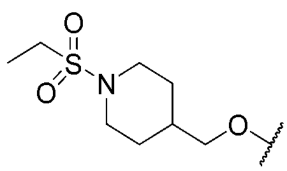 .

**2.** The compound as represented by formula (I), or the stereoisomer, tautomer, deuterated compound, solvate, co-crystal or pharmaceutically acceptable salt according to claim 1, **characterized in that**

ring A is selected from 4- to 8-membered cycloalkyl, 4- to 7-membered monocyclic heterocycloalkyl, 5- to 6-membered heterocycloalkyl fused 5- to 6-membered heterocycloalkyl, 4- to 6-membered heterocycloalkyl spiro 5- to 6-membered heterocycloalkyl, 5- to 6-membered heterocycloalkyl spiro 4- to 6-memberedcycloalkyl, 5- to 6-membered heterocycloalkyl spiro 5- to 6-membered cycloalkyl, 5- to 6-membered heterocycloalkyl spiro 3- to 6-membered cycloalkyl, 10- to 14-membered partially unsaturated tricyclic heterocycloalkyl, or 7- to 8-membered bicyclic bridged heterocyclyl;

ring C is selected from phenyl, 5-membered heteroaryl, 6-membered heteroaryl, 5- to 7-membered heterocycloalkyl fused phenyl, 5- to 6-membered carbocyclyl fused phenyl, 5- to 6-membered heterocycloalkyl fused 5- to 6-membered heteroaryl, 5- to 6-membered heterocycloalkyl fused 3- to 6-membered cycloalkyl, or 9- to 12-membered tricyclic heterocycloalkyl;

$R^{La}$ and $R^{Lb}$ are each independently selected from a bond, $C_{1-2}$ alkylene, $C_{2-4}$ alkenylene, or $C_{2-4}$ alkynylene, wherein the alkylene and alkenylene are optionally further substituted with 1-4 $R^{L1}$;

each $R^{L1}$ is independently selected from halogen, $C_{1-2}$ alkyl, $C_{2-4}$ alkenyl, $C_{1-2}$ alkoxy, or 3- to 6-membered cycloalkyl, wherein the alkyl, alkoxy, and cycloalkyl are optionally further substituted with 1-4 substituents selected from halogen, CN, OH and $NH_2$, or two $R^{L1}$ on the same carbon atom or two adjacent carbon atoms, together with the atom to which they are connected, form 3- to 6-membered cycloalkyl;

$R^{W1}$ is selected from H, halogen, or $C_{1-2}$ alkyl;

each $R^A$ is independently selected from halogen, =O, CN, COOH, $C_{1-4}$ alkyl, $C_{2-4}$ alkenyl, $C_{2-4}$ alkynyl, -O$C_{1-4}$ alkyl, -$S(O)_2$-$C_{1-4}$ alkyl, -$S(O)_2$-$(CH_2)_{1-3}$-$R^a$, -P(O)-($C_{1-4}$ alkyl)$_2$, -C(O)-O-$(CH_2)_p$-$R^a$, -$(CH_2)_{1-2}$-C(O)-$R^a$, -C(O)-$(CH_2)_{1-2}$-$R^a$, -NH-$R^a$, - C(O)-O-halo $C_{1-4}$ alkyl, -C(O)-(6- to 9-membered bicyclic heterocycloalkyl), -NH-C(O)-$(CH_2)_p$-$R^a$, -N($CH_3$)-C(O)-$(CH_2)_p$-$R^a$, -NH-$SO_2$-$(CH_2)_{1-2}$-$R^a$, -NH-C(O)-O-$R^a$, -C(O)-$(CH_2)_p$-(4- to 6-membered monocyclic heterocycloalkyl), -C(O)-$(CH_2)_p$-(4-to 6-membered monocyclic cycloalkyl), -NH-C(O)-NH-$R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), -C(O)-NH-$S(O)_2$-$C_{1-4}$ alkyl, -NH-C(O)deuterated $C_{1-2}$ alkyl, -NH-C(O)-NH-$C_{1-2}$ alkyl or -NH-C(O)-$C_{1-2}$ alkyl, wherein the alkyl, alkenyl, alkynyl, cycloalkyl, and heterocycloalkyl are optionally further substituted with 1-4 groups selected from halogen, OH, $NH_2$, CN, -$S(O)_2$-$CH_3$, or -NH-$S(O)_2$-$CH_3$;

$R^a$ is selected from deuterated $C_{1-2}$ alkyl, $C_{2-4}$ alkynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, $C_{1-2}$ alkyl, OH, =O, -$S(O)_2$-$CH_3$, $C_{1-2}$ alkoxy, deuterated $C_{1-2}$ alkyl or =$CH_2$;

each $R^C$ is independently selected from H, CN, halogen, OH, $C_{1-4}$ alkyl, halo $C_{1-4}$ alkyl, -O-halo $C_{1-4}$ alkyl, -Se-halo $C_{1-2}$ alkyl, $C_{2-4}$ alkenyl, $C_{2-4}$ alkynyl, =O, $C_{3-6}$ cycloalkyl, -$SCF_3$, -$SF_5$, -$(NH)_q$-P(O)($C_{1-4}$ alkyl)$_2$, -$(CH_2)_p$-O-$C_{1-4}$ alkyl, -O-$(CH_2)_p$-$C_{3-6}$ cycloalkyl or -$(CH_2)_p$-O-$C_{3-6}$ cycloalkyl, or any two $R^C$, together with the atom to which they are connected, form 3- to 6-membered cycloalkyl;

each p is independently selected from 0, 1, 2, or 3;

m is selected from 1, 2, or 3;

n is selected from 1, 2, or 3.

**3.** The compound as represented by formula (I), or the stereoisomer, tautomer, deuterated compound, solvate, co-crystal or pharmaceutically acceptable salt according to claim 2, **characterised in that**

ring A is selected from 4- to 6-membered cycloalkyl, 4- to 7-membered monocyclic heterocycloalkyl, 5-membered heterocycloalkyl fused 5-membered heterocycloalkyl, 5-membered heterocycloalkyl fused 6-membered heterocycloalkyl, 5-membered heterocycloalkyl spiro 5-membered heterocycloalkyl, 5-membered heterocycloalkyl spiro 6-membered heterocycloalkyl, 4-membered heterocycloalkyl spiro 6-membered heterocycloalkyl, 4-membered cycloalkyl spiro 6-membered heterocycloalkyl, 5-membered cycloalkyl spiro 6-membered heterocycloalkyl, 5-membered heterocycloalkyl fused 5-membered cycloalkyl, 6-membered heterocycloalkyl fused 5-membered cycloalkyl, or 10- to 14-membered partially unsaturated tricyclic heterocycloalkyl;

ring C is selected from 5- to 7-membered heterocycloalkyl fused phenyl, 5- to 6-membered heterocycloalkyl fused 5- to 6-membered heteroaryl, 5- to 6-membered heterocycloalkyl fused 3- to 6-membered cycloalkyl, or 9- to 12-membered tricyclic heterocycloalkyl;

each $R^A$ is independently selected from halogen, =O, CN, COOH, $C_{1-4}$ alkyl, $C_{2-4}$ alkenyl, $C_{2-4}$ alkynyl, -O$C_{1-4}$ alkyl, -$S(O)_2$-$C_{1-4}$ alkyl, -$S(O)_2$-$(CH_2)_{1-3}$-$R^a$, -C(O)-O-$(CH_2)_p$-$R^a$, -$(CH_2)_{1-2}$-C(O)-$R^a$, -C(O)-$(CH_2)_{1-2}$-$R^a$, -C(O)-O-halo $C_{1-4}$ alkyl, -NH-$R^a$, -C(O)-(6- to 9-membered bicyclic heterocycloalkyl), -N($CH_3$)-C(O)-$(CH_2)_p$-$R^a$,

-NH-C(O)-O-$R^a$, -C(O)-$(CH_2)_p$-(4- to 6-membered monocyclic heterocycloalkyl), - C(O)-$(CH_2)_p$-(4- to 6-membered monocyclic cycloalkyl), -NH-C(O)-NH-$R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), -C(O)-NH-$S(O)_2$-$C_{1-4}$ alkyl, -NH-C(O)deuterated $C_{1-2}$ alkyl, -NH-C(O)-NH-$C_{1-2}$ alkyl or -NH-C(O)-$C_{1-2}$ alkyl, wherein the alkyl, alkenyl, alkynyl, cycloalkyl, and heterocycloalkyl are optionally further substituted with 1-4 groups selected from halogen, OH, $NH_2$, CN, -$S(O)_2$-$CH_3$, or -NH-$S(O)_2$-$CH_3$;

$R^a$ is selected from deuterated $C_{1-2}$ alkyl, ethynyl, 3- to 5-membered cycloalkyl, 4- to 6-membered heterocycloalkyl, or 5-membered heteroaryl, wherein the cycloalkyl, heterocycloalkyl, and heteroaryl are optionally further substituted with groups selected from halogen, OH, =O, $C_{1-2}$ alkyl, deuterated $C_{1-2}$ alkyl, $C_{1-2}$ alkoxy, -$S(O)_2$-$CH_3$ or =$CH_2$;

each p is independently selected from 0, 1, or 2;

m is selected from 1, 2, or 3;

n is selected from 1, 2, or 3.

**4.** The compound as represented by formula (I), or the stereoisomer, tautomer, deuterated compound, solvate, cocrystal or pharmaceutically acceptable salt according to claim 1, **characterised in that**

ring A has one of the following structures:

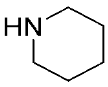, 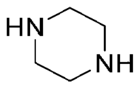, 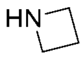,

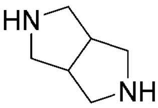, 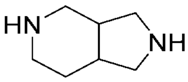, 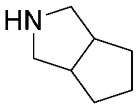, 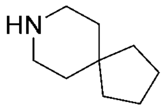, 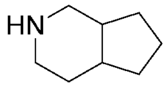,

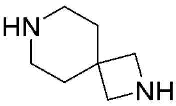, 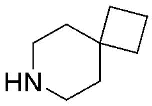, 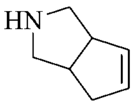, 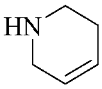, 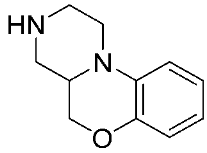,

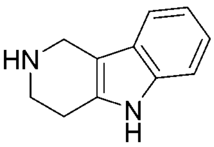, 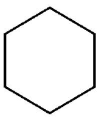, 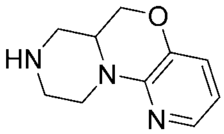, 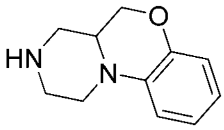, 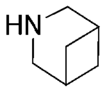, 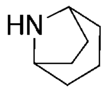,

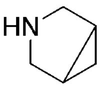, 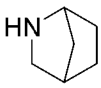, 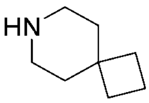, 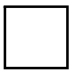, 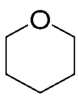, 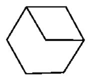, 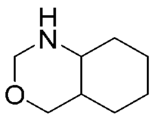, 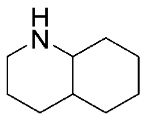,

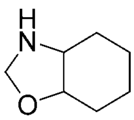, 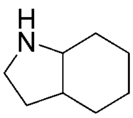, or 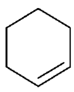;

ring C has one of the following structures:

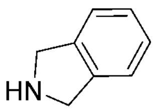, 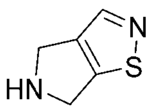,

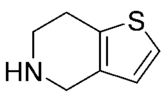, 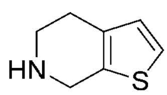, 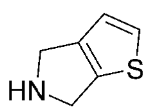, 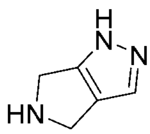, 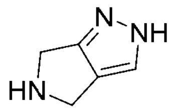, 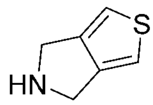,

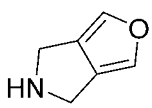, 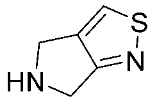, 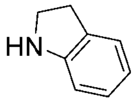, 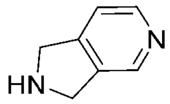, 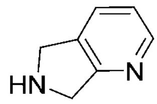, 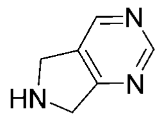,

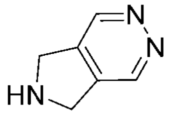, 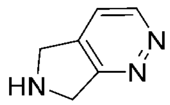, 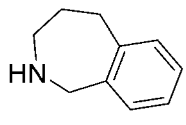, 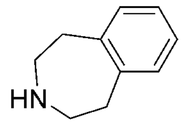, 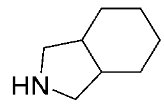,

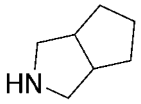, 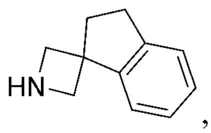, 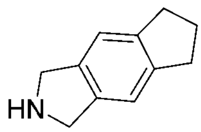, 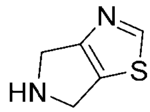, or 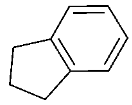;

$L_1$ is selected from a bond, -($C_{1-2}$ alkylene)-O-, -($C_{1-2}$ alkylene)-Se-, $C_{1-2}$ alkylene, -O-($C_{1-2}$ alkylene)-, $C_{2-4}$ alkenylene, -O-, or $C_{2-4}$ alkynylene, wherein the alkylene and alkenylene are optionally further substituted with 1-3 $R^{L1}$;

$L_2$ is selected from a bond, $C_{1-2}$ alkylene, -NH-, or -O-, wherein the alkylene, alkenylene and cycloalkyl are optionally further substituted with 1-3 $R^{L1}$;

each $R^{L1}$ is independently selected from halogen, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl, or two $R^{L1}$ on the same carbon atom or two adjacent carbon atoms, together with the atom to which they are connected, form 3- to 6-membered cycloalkyl;

each $R^A$ is independently selected from =O, $C_{1-4}$ alkyl, $-S(O)_2-C_{1-4}$ alkyl, - $S(O)_2-(CH_2)_{1-3}-R^a$, $-C(O)-O-(CH_2)_p-R^a$, $-(CH_2)_{1-2}-C(O)-R^a$, $-C(O)-(CH_2)_{1-2}-R^a$, - C(O)-O-halo $C_{1-3}$ alkyl, -C(O)-(6- to 9-membered bicyclic heterocycloalkyl), $-NH-C(O)-(CH_2)_p-R^a$, $-NH-R^a$, $-N(CH_3)-C(O)-(CH_2)_p-R^a$, $-NH-SO_2-(CH_2)_{1-2}-R^a$, -NH-C(O)-O-$R^a$, $-C(O)-(CH_2)_p$-(4- to 6-membered monocyclic heterocycloalkyl), $-C(O)-(CH_2)_p$-(4- to 6-membered monocyclic cycloalkyl), -NH-C(O)-NH-$R^a$, -C(O)-deuterated $C_{1-2}$ alkyl, -C(O)-NH-(4- to 6-membered monocyclic cycloalkyl), -C(O)-NH-(4- to 6-membered monocyclic heterocycloalkyl), $-C(O)-NH-S(O)_2-C_{1-4}$ alkyl, -NH-C(O) deuterated $C_{1-2}$ alkyl, -NH-C(O)-NH-$C_{1-2}$ alkyl or -NH-C(O)-$C_{1-2}$ alkyl, wherein the alkyl, cycloalkyl, and heterocycloalkyl are optionally further substituted with 1-4 groups selected from halogen, OH, $NH_2$, CN, $-S(O)_2-CH_3$, or $-NH-S(O)_2-CH_3$;

$R^a$ is selected from deuterated $C_{1-2}$ alkyl, ethynyl, 3- to 5-membered cycloalkyl, 4- to 6-membered heterocycloalkyl, or 5- to 6-membered heteroaryl, wherein the cycloalkyl, heterocycloalkyl, and heteroaryl are optionally further substituted with groups selected from halogen, methyl, deuterated methyl, methoxy, ethoxy, OH, =O, $-S(O)_2-CH_3$ or $=CH_2$;

each $R^C$ is independently selected from H, CN, F, Cl, Br, OH, $-SF_5$, methyl, difluoromethyl, trifluoromethyl, ethyl, ethynyl, cyclopropyl, -O-cyclopropyl, $-O-CH_2$-cyclopropyl, cyclobutyl, -O-cyclobutyl, $-O-CH_2$-cyclobutyl, or -Se-trifluoromethyl, or any two $R^C$, together with the atom to which they are connected, form 3- to 6-membered cycloalkyl;

each p is independently selected from 0, 1, or 2;

m is selected from 1 or 2;

n is selected from 1 or 2.

**5.** The compound as represented by formula (I), or the stereoisomer, tautomer, deuterated compound, solvate, co-crystal or pharmaceutically acceptable salt according to claim 1, **characterised in that**

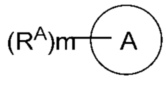

has one of the following structures:

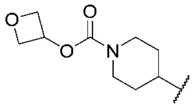 ,

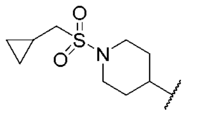 , 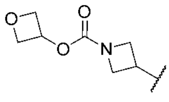 , 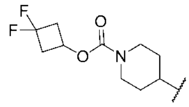 , 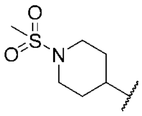 , 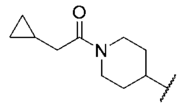 ,

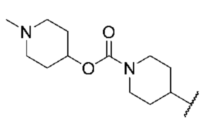 , 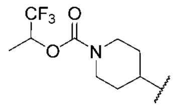 , 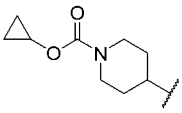 , 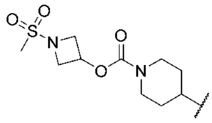 , 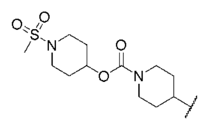 ,

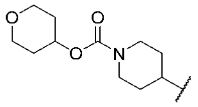 , 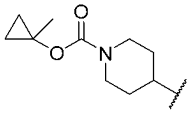 , 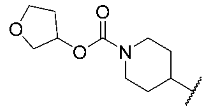 , 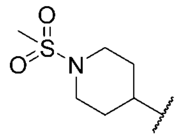 , 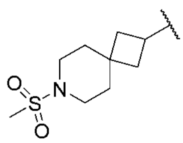 ,

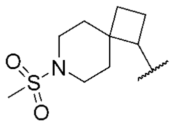 , 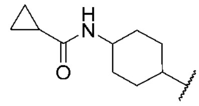 , 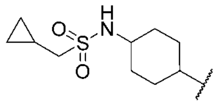 , 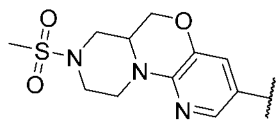 ,

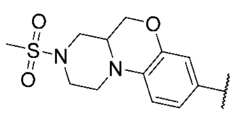 , 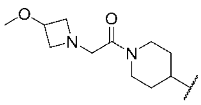 , 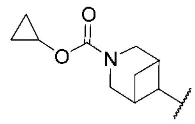 , 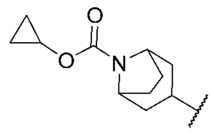 ,

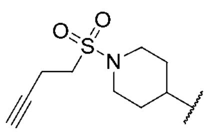 , 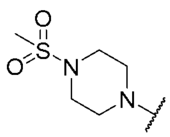 , 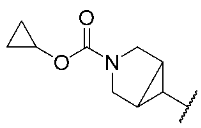 , 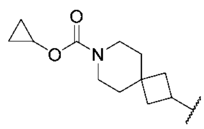 , 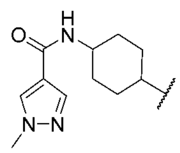 ,

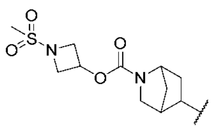 , 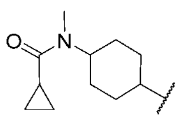 , 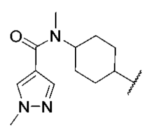 , 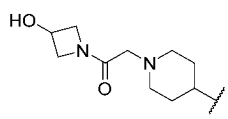 , 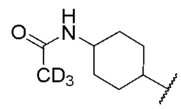 ,

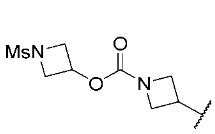 , 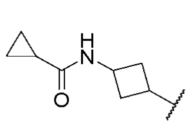 , 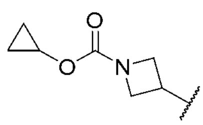 , 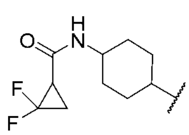 , 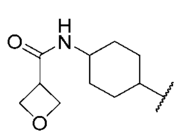 ,

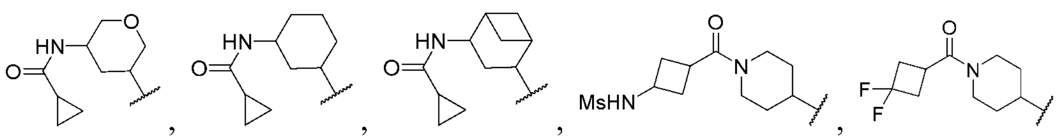 , , , , ,

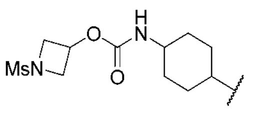 , 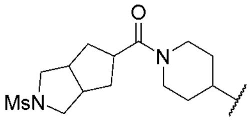 , 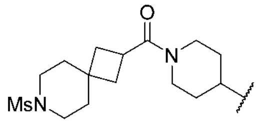 , 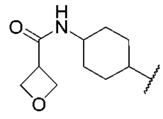 ,

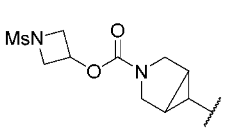 , 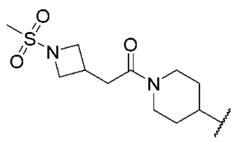 , 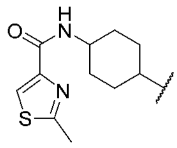 , 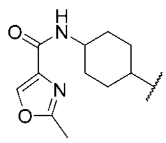 , 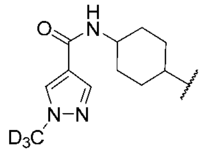 ,

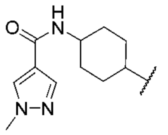 , 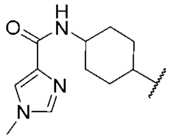 , 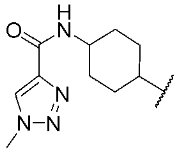 , 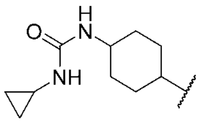 , 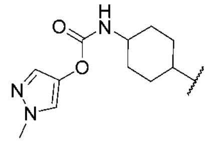 ,

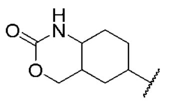 , 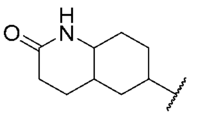 , 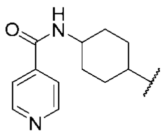 , 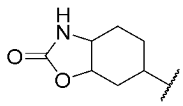 , 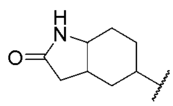 ,

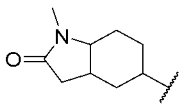 , 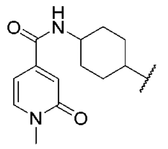 , 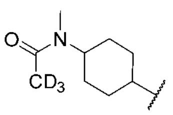 , 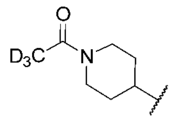 , 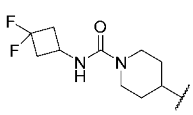 ,

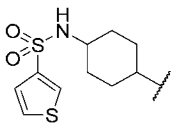 , 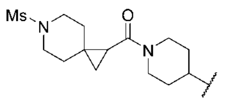 , 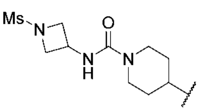 , 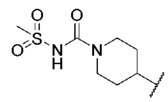 , 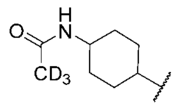 ,

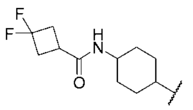 , 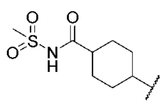 , 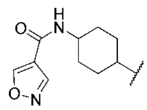 , 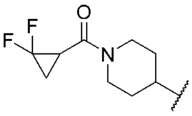 , 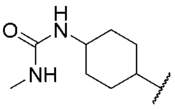 ,

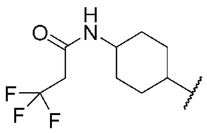 , 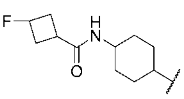 , 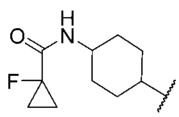 , 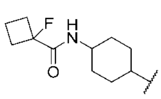 , 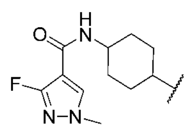 ,

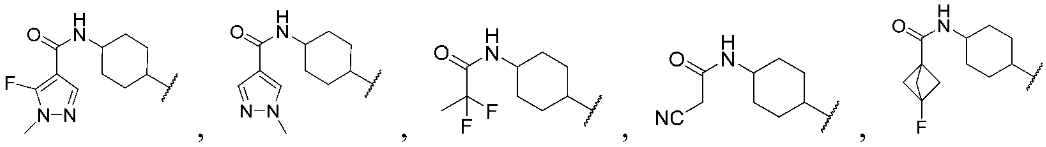 , , , , ,

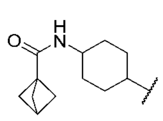, 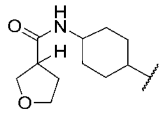, 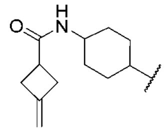, 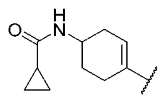, 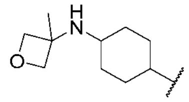,

or

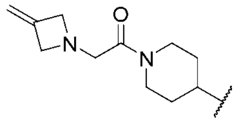;

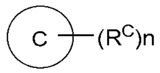

has one of the following structures:

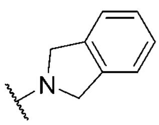, 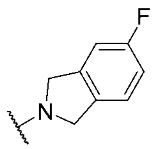,

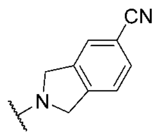, 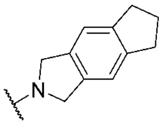, 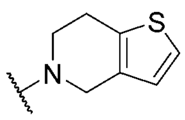, 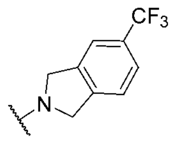, 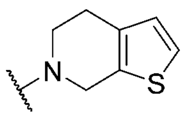,

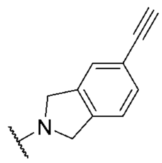, 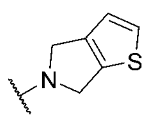, 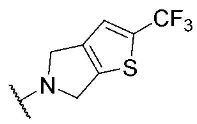, 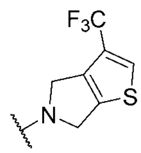, 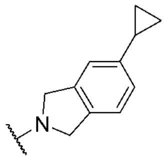, 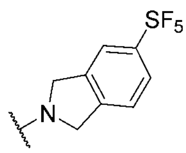,

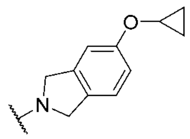, 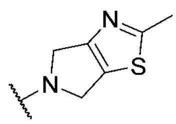, 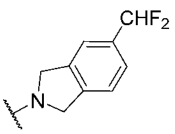, 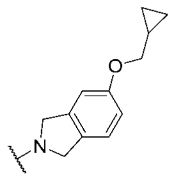, 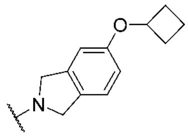,

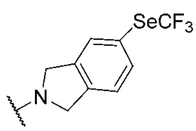, 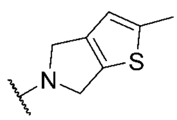, or 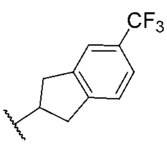;

$L_1$ is selected from a bond, $-CH_2-O-$, $-CH_2-Se-$, $-CH=CH-$, $-CF=CH-$, $-CH_2-$, $-CH_2CH_2-$,

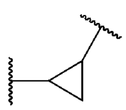,

-O-, $-C(CH_3)=CH-$, $-CH=C(CH_3)-$, or

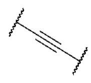;

$L_2$ is $-CH_2-$.

**6.** The compound, or the stereoisomer, tautomer, deuterated compound, solvate, co-crystal or pharmaceutically acceptable salt according to claim 1, **characterised in that** the compound has a structure of formula (I-a) or (I-c):

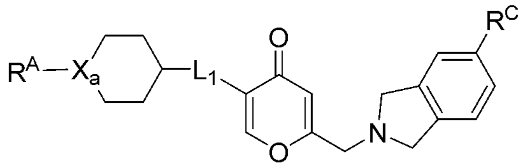

(I-a)

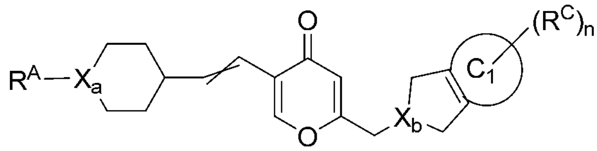

(I-c)

wherein

Xa is selected from CH or N;
$X_b$ is selected from CH or N;
ring $C_1$ is selected from 5- to 6-membered heteroaryl, phenyl or benzo 5- to 6-membered cycloalkyl.

**7.** The compound, or the stereoisomer, tautomer, deuterated compound, solvate, co-crystal or pharmaceutically acceptable salt according to claim 6, **characterised in that**

$R^A$ is selected from $-S(O)_2-(CH_2)_{1-3}-R^a$, $-C(O)-O-(CH_2)_p-R^a$, $-(CH_2)_{1-2}-C(O)-R^a$, $-C(O)-(CH_2)_{1-2}-R^a$, -C(O)-O-halo $C_{1-4}$ alkyl, -C(O)-(6- to 9-membered bicyclic heterocycloalkyl), $-NH-C(O)-(CH_2)_p-R^a$, $-N(CH_3)-C(O)-(CH_2)_p-R^a$, $-NH-SO_2-(CH_2)_{1-2}-R^a$, $-NH-C(O)-O-R^a$, $-NH-R^a$, $-C(O)-(CH_2)_p$-(4- to 6-membered monocyclic heterocycloalkyl), $-C(O)-(CH_2)_p$-(4- to 6-membered monocyclic cycloalkyl), $-NH-C(O)-NH-R^a$, -C(O)-deuterated $C_{1-4}$ alkyl, -C(O)-NH-(4- to 6-membered monocyclic cycloalkyl), -C(O)-NH-(4- to 6-membered monocyclic heterocycloalkyl), $-C(O)-NH-S(O)_2-C_{1-4}$ alkyl, -NH-C(O)deuterated $C_{1-2}$ alkyl, - $NH-C(O)-NH-C_{1-2}$ alkyl or $-NH-C(O)-C_{1-2}$ alkyl, wherein the alkyl, cycloalkyl, and heterocycloalkyl are optionally further substituted with 1-3 groups selected from halogen, OH, $NH_2$, CN, $-S(O)_2-CH_3$, -O-halo $C_{1-4}$ alkyl, or $-NH-S(O)_2-CH_3$;
$R^a$ is selected from deuterated $C_{1-4}$ alkyl, $C_{2-4}$ alkynyl, 3- to 5-membered cycloalkyl, 3- to 6-membered heterocycloalkyl, or 5- to 6-membered heteroaryl, wherein the alkynyl, cycloalkyl, heterocycloalkyl, and heteroaryl are optionally further substituted with 1-4 groups selected from halogen, OH, =O, $C_{1-4}$ alkyl, deuterated $C_{1-4}$ alkyl, $C_{1-4}$ alkoxy, $C_{2-4}$ alkynyl, -O-halo $C_{1-4}$ alkyl, $-S(O)_2-CH_3$ or $=CH_2$;
$R^C$ is selected from F, Cl, halo $C_{1-2}$ alkyl, $C_{2-4}$ alkynyl, 3-4-membered cycloalkyl, or $-O-C_{3-4}$ cycloalkyl;
$L_1$ is selected from $-(C_{1-2}$ alkylene)-O-, $C_{1-2}$ alkylene, $C_{2-4}$ alkenylene, or -C(O)-NH-;
n is selected from 1 or 2;
p is selected from 0, 1 or 2.

**8.** The compound, or the stereoisomer, tautomer, deuterated compound, solvate, co-crystal or pharmaceutically acceptable salt according to claim 6, **characterised in that**

$R^A$ is selected from $-C(O)-O-(CH_2)_p-R^a$, $-C(O)-(CH_2)_{1-2}-R^a$, $-NH-C(O)-(CH_2)_p-R^a$, $-N(CH_3)-C(O)-(CH_2)_p-R^a$, or $-NH-C(O)-O-R^a$;
$R^a$ is selected from 3- to 5-membered cycloalkyl, 3- to 6-membered heterocycloalkyl, or 5- to 6-membered heteroaryl, wherein the cycloalkyl, heterocycloalkyl, and heteroaryl are optionally further substituted with 1-4

groups selected from halogen, $C_{1-4}$ alkyl, deuterated $C_{1-4}$ alkyl, or $-S(O)_2-CH_3$;
$R^C$ is selected from F, Cl, $CHF_2$, $CF_3$, ethynyl, cyclopropyl, cyclobutyl, -O-cyclopropyl, or -O-cyclobutyl.

**9.** The compound, or the stereoisomer, tautomer, deuterated compound, solvate, co-crystal or pharmaceutically acceptable salt according to claim 1 or 6, **characterised in that** $L_1$ is selected from -CH=CH-.

**10.** The compound, or the stereoisomer, tautomer, deuterated compound, solvate, co-crystal or pharmaceutically acceptable salt according to claim 1, **characterised in that** the compound is selected from one of the structures in Table 1.

**11.** A pharmaceutical composition or pharmaceutical preparation, **characterised in that** the pharmaceutical composition or pharmaceutical preparation comprises the compound, or the stereoisomer, tautomer, deuterated compound, solvate, co-crystal or pharmaceutically acceptable salt thereof according to any one of claims 1-10, and a pharmaceutically acceptable carrier and/or excipient.

**12.** The pharmaceutical composition or pharmaceutical preparation according to claim 11, **characterised in that** the pharmaceutical composition or pharmaceutical composition comprises 1-1500 mg of the compound, or the stereoisomer, tautomer, deuterated compound, solvate, co-crystal or pharmaceutically acceptable salt thereof according to any one of claims 1-10, and a carrier and/or excipient.

**13.** Use of the compound, or the stereoisomer, tautomer, deuterated compound, solvate, co-crystal or pharmaceutically acceptable salt thereof according to any one of claims 1-10, or the composition according to claim 11 or 12 in the preparation of a drug for treating/preventing a CYP11A1-mediated disease.

**14.** The use according to claim 13, **characterised in that** the CYP11A1-mediated disease is treating steroid hormone-dependent cancer.

**INTERNATIONAL SEARCH REPORT**

International application No.
**PCT/CN2024/083998**

**A. CLASSIFICATION OF SUBJECT MATTER**

C07D405/14(2006.01)i; C07D209/44(2006.01)i; C07D405/06(2006.01)i; C07D417/14(2006.01)i; A61P35/00(2006.01)i; A61K31/404(2006.01)i

According to International Patent Classification (IPC) or to both national classification and IPC

**B. FIELDS SEARCHED**

Minimum documentation searched (classification system followed by classification symbols)

IPC:C07D,A61P,A61K

Documentation searched other than minimum documentation to the extent that such documents are included in the fields searched

Electronic data base consulted during the international search (name of data base and, where practicable, search terms used)

CNTXT, WPABS, ENTXTC, CAPLUS(STN), REGISTRY(STN), CNKI: 海思科制药, CYP11A1, 胆固醇侧链裂解酶, 细胞色素p450单加氧酶, 吡喃-4-酮, 吡喃, 癌症, 肿瘤, pyran+, pyran-4-one, cytochrome P450 monooxygenase, cancer, tumor, tumour, 结构式检索 structural formula search.

**C. DOCUMENTS CONSIDERED TO BE RELEVANT**

| Category* | Citation of document, with indication, where appropriate, of the relevant passages | Relevant to claim No. |
|---|---|---|
| X | CN 110139861 A (ORION CORP.) 16 August 2019 (2019-08-16) claims 1-2 and 4-27, description, paragraphs [0008], [0094], and [0745], and description pages 84, 101-102, 120, 122, 125-126, 132, 134, 137-139, 144, 151, and 155 | 1-14 |
| X | REG. "RN 1331127-48-0 etc." *STN*, 11 September 2011 (2011-09-11), pages 1-4 | 1-3 |
| X | CN 115551831 A (ORION CORP.) 30 December 2022 (2022-12-30) claims 1, 5-6, 9-24, 26-29, 38-39, and 52, description paragraphs [0008], [0355], and [0941] | 1-14 |
| A | WO 2022184975 A1 (ORION CORP.) 09 September 2022 (2022-09-09) description, pages 1-21 | 1-14 |
| A | WO 2022269134 A1 (ORION CORP.) 29 December 2022 (2022-12-29) description, pages 1-30 | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 May 2024** | **03 June 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/083998**

**C. DOCUMENTS CONSIDERED TO BE RELEVANT**

| Category* | Citation of document, with indication, where appropriate, of the relevant passages | Relevant to claim No. |
| --- | --- | --- |
| A | WO 2022117920 A1 (ORION CORP.) 09 June 2022 (2022-06-09)<br>claims 1-32 | 1-14 |

Form PCT/ISA/210 (second sheet) (July 2022)

**INTERNATIONAL SEARCH REPORT**
**Information on patent family members**

International application No.
**PCT/CN2024/083998**

| Patent document cited in search report | Publication date (day/month/year) | Patent family member(s) | Publication date (day/month/year) |
|---|---|---|---|
| CN 110139861 A | 16 August 2019 | CA 3047370 A1 | 28 June 2018 |
| | | EP 3868756 A1 | 25 August 2021 |
| | | MX 2019007373 A | 18 September 2019 |
| | | WO 2018115591 A1 | 28 June 2018 |
| | | JP 2020502229 A | 23 January 2020 |
| | | JP 7025432 B2 | 24 February 2022 |
| | | TW 202229264 A | 01 August 2022 |
| | | TWI 796205 B | 11 March 2023 |
| | | EA 201991513 A1 | 29 November 2019 |
| | | EA 039309 B1 | 12 January 2022 |
| | | AU 2021203497 A1 | 01 July 2021 |
| | | AU 2021203497 B2 | 10 November 2022 |
| | | RS 62198 B1 | 31 August 2021 |
| | | LT 3558981 T | 25 August 2021 |
| | | US 2020299280 A1 | 24 September 2020 |
| | | US 11098032 B2 | 24 August 2021 |
| | | AU 2017380282 A1 | 01 August 2019 |
| | | AU 2017380282 B2 | 24 June 2021 |
| | | EP 3558981 A1 | 30 October 2019 |
| | | EP 3558981 B1 | 26 May 2021 |
| | | PH 12019550111 A1 | 09 March 2020 |
| | | HRP 20211255 T1 | 12 November 2021 |
| | | KR 20190095950 A | 16 August 2019 |
| | | KR 102491308 B1 | 27 January 2023 |
| | | UA 124640 C2 | 20 October 2021 |
| | | KR 20230019496 A | 08 February 2023 |
| | | BR 112019012906 A2 | 03 December 2019 |
| | | TN 2019000189 A1 | 05 October 2020 |
| | | SI 3558981 T1 | 30 September 2021 |
| | | MA 47102 B1 | 30 September 2021 |
| | | ES 2880151 T3 | 23 November 2021 |
| | | HUE 056540 T2 | 28 February 2022 |
| | | MA 55983 A | 23 March 2022 |
| | | IL 285729 A | 30 September 2021 |
| | | IL 285729 B | 01 February 2022 |
| | | AR 110412 A1 | 27 March 2019 |
| | | CO 2019007321 A2 | 31 July 2019 |
| | | TW 201835072 A | 01 October 2018 |
| | | TWI 762544 B | 01 May 2022 |
| | | JP 2022078068 A | 24 May 2022 |
| | | JP 7286825 B2 | 05 June 2023 |
| | | PT 3558981 T | 06 July 2021 |
| | | IL 267484 A | 29 August 2019 |
| | | IL 267484 B | 30 September 2021 |
| | | PL 3558981 T3 | 06 December 2021 |
| | | DK 3558981 T3 | 26 July 2021 |
| | | CL 2019001728 A1 | 27 September 2019 |
| | | US 2021347765 A1 | 11 November 2021 |
| | | US 2019359601 A1 | 28 November 2019 |
| | | US 10717726 B2 | 21 July 2020 |

Form PCT/ISA/210 (patent family annex) (July 2022)

**INTERNATIONAL SEARCH REPORT**
**Information on patent family members**

International application No.
**PCT/CN2024/083998**

| Patent document cited in search report | Publication date (day/month/year) | Patent family member(s) | Publication date (day/month/year) |
|---|---|---|---|
| | | CY 1124461 T1 | 22 July 2022 |
| | | PE 20191137 A1 | 02 September 2019 |
| CN 115551831 A | 30 December 2022 | WO 2021229152 A1 | 18 November 2021 |
| | | JP 2023525137 A | 14 June 2023 |
| | | AU 2021269872 A1 | 24 November 2022 |
| | | US 2023192677 A1 | 22 June 2023 |
| | | EP 4149924 A1 | 22 March 2023 |
| | | KR 20230010723 A | 19 January 2023 |
| | | CA 3178067 A1 | 18 November 2021 |
| WO 2022184975 A1 | 09 September 2022 | JP 2024511295 A | 13 March 2024 |
| | | BR 112023017472 A2 | 07 November 2023 |
| | | PE 20231939 A1 | 05 December 2023 |
| | | CL 2023002585 A1 | 02 February 2024 |
| | | CO 2023011531 A2 | 30 November 2023 |
| | | IL 305508 A | 01 October 2023 |
| | | EP 4301732 A1 | 10 January 2024 |
| | | CA 3210591 A1 | 09 September 2022 |
| | | AU 2022228710 A1 | 07 September 2023 |
| | | KR 20230165773 A | 05 December 2023 |
| WO 2022269134 A1 | 29 December 2022 | AU 2022297768 A1 | 18 January 2024 |
| | | KR 20240024937 A | 26 February 2024 |
| | | EP 4359384 A1 | 01 May 2024 |
| | | BR 112023027282 A2 | 12 March 2024 |
| | | IL 309484 A | 01 February 2024 |
| | | CA 3223714 A1 | 29 December 2022 |
| WO 2022117920 A1 | 09 June 2022 | EP 4255902 A1 | 11 October 2023 |
| | | KR 20230117187 A | 07 August 2023 |
| | | CA 3200556 A1 | 30 November 2021 |
| | | US 2024124431 A1 | 18 April 2024 |
| | | MX 2023006290 A | 21 August 2023 |
| | | JP 2023551340 A | 07 December 2023 |
| | | AU 2021391977 A1 | 13 July 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202310320916 A
- WO 202310428094 A
- WO 202310667272 A
- WO 202310821299 A
- WO 202310979027 A
- WO 202311059482 A
- WO 202311216619 A
- WO 202311669033 A
- WO 2018115591 A1
- WO 2007087231 A2


### Non-patent literature cited in the description


- *Organic Letters*, 2012, vol. 14 (6), 1508-1511